# EUROPÄISCHE PATENTSCHRIFT

(11) **EP 2 177 605 B1**
(45) Veröffentlichungstag und Bekanntmachung des Hinweises auf die Patenterteilung: **10.12.2014**
(21) Anmeldenummer: 09175508.2
(22) Anmeldetag: 04.10.2007
(51) Int. Cl.: C12N 9/02, C12N 15/82

(54) **Delta-5 Desaturasen und Verfahren zur Herstellung mehrfach ungesättigter Fettsäuren in transgenen nicht-humanen Organismen**
Delta-5 Desaturases and method for producing polyunsaturated fatty acids in transgenic non-human organisms
Delta-5 deasturase et procédé de production d'acides gras polyinstaurés dans des organimes transgéniques non-humains

(30) Priorität: 06.10.2006 EP 06121888
(43) Veröffentlichungstag der Anmeldung: 21.04.2010
(62) Teilanmeldung aus: 07820931.9
(73) Patentinhaber: BASF Plant Science GmbH, 67056 Ludwigshafen (DE)
(72) Erfinder: Bauer, Jörg, Dr., 67117 Limburgerhof (DE); Wetjen, Tom, Dr.-Ing., 68163 Mannheim (DE)
(74) Vertreter: Krieger, Stephan Gerhard

(56) Entgegenhaltungen:
- WO-A-2006/069710
- DERELLE EVELYNE ET AL: "Genome analysis of the smallest free-living eukaryote Ostreococcus tauri unveils many unique features." PROCEEDINGS OF THE NATIONAL ACADEMY OF SCIENCES OF THE UNITED STATES OF AMERICA 1 AUG 2006, Bd. 103, Nr. 31, 1. August 2006 (2006-08-01), Seiten 11647-11652, XP002466956 ISSN: 0027-8424 -& DATABASE EMBL [Online] 30. April 2005 (2005-04-30), "Ostreococcus tauri chromosome 01 contig 1, DNA sequence." XP002565451 gefunden im EBI accession no. EMBL:CR954201 Database accession no. CR954201 -& DATABASE EMBL [Online] 30. April 2005 (2005-04-30), "Ostreococcus tauri chromosome 12 contig 1, DNA sequence." XP002565452 gefunden im EBI accession no. EMBL:CR954212 Database accession no. CR954212
- PALENIK BRIAN ET AL: "The tiny eukaryote Ostreococcus provides genomic insights into the paradox of plankton speciation." PROCEEDINGS OF THE NATIONAL ACADEMY OF SCIENCES OF THE UNITED STATES OF AMERICA 1 MAY 2007, Bd. 104, Nr. 18, 1. Mai 2007 (2007-05-01), Seiten 7705-7710, XP002466957 ISSN: 0027-8424 -& DATABASE EMBL [Online] 20. April 2007 (2007-04-20), "Ostreococcus lucimarinus CCE9901 chromosome 1, complete sequence." XP002565453 gefunden im EBI accession no. EMBL:CP000581 Database accession no. CP000581 -& DATABASE EMBL [Online] 20. April 2007 (2007-04-20), "Ostreococcus lucimarinus CCE9901 chromosome 12, complete sequence." XP002565454 gefunden im EBI accession no. EMBL:CP000592 Database accession no. CP000592
- WORDEN ALEXANDRA Z ET AL: "Assessing the dynamics and ecology of marine picophytoplankton: The importance of the eukaryotic component.", LIMNOLOGY AND OCEANOGRAPHY, vol. 49, no. 1, January 2004 (2004-01), pages 168-179, ISSN: 0024-3590

### SEQUENCE LISTING

<110> BASF Plant Science GmbH
<120> verfahren zur Herstellung von mehrfach ungsättigten Fettsäuren in transgenen organismen
<130> BPS64509EPDIV1
<140>
   <141>
<160> 44
<170> PatentIn version 3.1
<210> 1
   <211> 1696
   <212> DNA
   <213> Ostreococcus lucimarinus
<220>
   <223> Delta-12 desaturase
<220>
   <221> CDS
   <222> (374)..(1462)
<400> 1
   cctttgtaac gccccgattc gttcgcgcgt ttcgctcgcc tcgcgtccgc cttcgcgcct      60
   cgcgggataa ggctctcccg tcgcgtcgtc gcgttcgcac tcaccggcgc tcgcgcgtcg     120
   accgctttcc cgcggcaccg cgatccacga acgacagtcg cgcgtcatgc tccgcgctct     180
   gacgctcccg cgcgctcgcg cgctcccgcg cgccgcgttc tctcgctcga cgacgtcacg     240
   tcgcgatcgc gcgcgcgtcg cccgcgccgc gcgcgccgac gccgcgacga cctacgacac     300
   gtcgcgacac gaacaactgc gctcgggctt gaacgccaag gcgctgaacg ccgaggcgaa     360
   gacgttcccg acg atg caa gag gtg ctg cgc gcg atc ccg agg gag tgc        409
                  Met Gln Glu Val Leu Arg Ala Ile Pro Arg Glu Cys
                  1               5                   10
   ttc gag cgg gac acg gga aag tcg ctc gcg tac gcg gcg tgc tcg acg       457
   Phe Glu Arg Asp Thr Gly Lys Ser Leu Ala Tyr Ala Ala Cys Ser Thr
           15                  20                  25
   gcg atc acg ctg gcg tgc ggc gcg ctg gcg tgg gcg tgc gtg ccg gtg       505
   Ala Ile Thr Leu Ala Cys Gly Ala Leu Ala Trp Ala Cys Val Pro Val
       30                  35                  40
   acg gcg gcg tat tgg ccg gtg tgg gtg gcg tac gcg ttc gtg acg gga     553
   Thr Ala Ala Tyr Trp Pro Val Trp Val Ala Tyr Ala Phe Val Thr Gly
    45                 50                  55                  60
   acg gcg gcg acc ggg tgc tgg gtc gcg gcg cac gag tgc gga cac ggc     601
   Thr Ala Ala Thr Gly Cys Trp Val Ala Ala His Glu Cys Gly His Gly
                   65                  70                  75
   gcg ttt agc gac gat aaa acg att caa gac gcg gtg gga tac gcg ctg     649
   Ala Phe Ser Asp Asp Lys Thr Ile Gln Asp Ala Val Gly Tyr Ala Leu
               80                  85                  90
   cac tcg ttg ttg ttg gtg ccg tat ttt tcg tgg cag cgg tcg cac gcg     697
   His Ser Leu Leu Leu Val Pro Tyr Phe Ser Trp Gln Arg Ser His Ala
           95                  100                 105
   gtg cat cac tcg agg act aat cac gtg ctc gag gga gag acg cac gtg     745
   Val His His Ser Arg Thr Asn His Val Leu Glu Gly Glu Thr His Val
       110                 115                 120
   ccg gcg cgg ttg ggg acg gag gac gcg aac gtg atg ttc aag ttg cgc     793
   Pro Ala Arg Leu Gly Thr Glu Asp Ala Asn Val Met Phe Lys Leu Arg
   125                 130                 135                 140
   ggg ttg ctc ggc gaa ggg ccg ttt acg ttt ttg aac ctc gtc gga gtg     841
   Gly Leu Leu Gly Glu Gly Pro Phe Thr Phe Leu Asn Leu Val Gly Val
                   145                 150                 155
   ttc gcc ttg ggg tgg ccg att tat ttg ctc acc ggc gcc agc ggt ggg     889
   Phe Ala Leu Gly Trp Pro Ile Tyr Leu Leu Thr Gly Ala Ser Gly Gly
               160                 165                 170
   ccg gtt cgc ggg aac acg aac cac ttc ttg ccg ttc atg ggc gag aag     937
   Pro Val Arg Gly Asn Thr Asn His Phe Leu Pro Phe Met Gly Glu Lys
           175                 180                 185
   ggt aag cac gcg ttg ttc ccg ggg aag tgg gcg aag aag gtt tgg cag     985
   Gly Lys His Ala Leu Phe Pro Gly Lys Trp Ala Lys Lys Val Trp Gln
       190                 195                 200
   agc gac gtc ggc gtc gtg gcg gtt ttg gga gcg ctc gcg gcg tgg gcg     1033
   Ser Asp Val Gly Val Val Ala Val Leu Gly Ala Leu Ala Ala Trp Ala
   205                 210                 215                 220
   gcg cac agc ggt gtc gcc acc gtc atg gcg ctc tac gtc gga ccg tac     1081
   Ala His Ser Gly Val Ala Thr Val Met Ala Leu Tyr Val Gly Pro Tyr
                   225                 230                 235
   atg gtg acc aac ttt tgg ctc gtc ctg tac acg tgg ttg cag cac act     1129
   Met Val Thr Asn Phe Trp Leu Val Leu Tyr Thr Trp Leu Gln His Thr
               240                 245                 250
   gac gtg gac gtg ccg cac ttt gag ggc gac gac tgg aac ttg gtc aag     1177
   Asp Val Asp Val Pro His Phe Glu Gly Asp Asp Trp Asn Leu Val Lys
           255                 260                 265
   ggc gcg ttt atg acg att gat cgc ccg tac ggg cca gtg ttt gac ttt     1225
   Gly Ala Phe Met Thr Ile Asp Arg Pro Tyr Gly Pro Val Phe Asp Phe
       270                 275                 280
   ttg cac cac cgc atc ggc agc acg cac gtc gcg cat cac atc aac cac     1273
   Leu His His Arg Ile Gly Ser Thr His Val Ala His His Ile Asn His
   285                 290                 295                 300
   acc atc ccg cac tac cac gcc aag aag gcc acc gag gcg ttg caa aag     1321
   Thr Ile Pro His Tyr His Ala Lys Lys Ala Thr Glu Ala Leu Gln Lys
                   305                 310                 315
   gcg ttc ccg gat ctg tac ctg tat gac ccg acg ccc atc gcc acg gcg     1369
   Ala Phe Pro Asp Leu Tyr Leu Tyr Asp Pro Thr Pro Ile Ala Thr Ala
               320                 325                 330
   act tgg cgc gtc ggc agc aag tgc atc gcc gtc gtc aag aag ggc gac     1417
   Thr Trp Arg Val Gly Ser Lys Cys Ile Ala Val Val Lys Lys Gly Asp
           335                 340                 345
   gag tgg gtg ttc acg gac aag cag ctc gac ccg gtc gcg gcg tga         1462
   Glu Trp Val Phe Thr Asp Lys Gln Leu Asp Pro Val Ala Ala
       350                 355                 360
   gcgcgcgtgt gacgaggata ttaagtttta gttagagaga cgagcattgt gattatgatt   1522
   atatacgtgc gtgtttattt cacatgctgg aattcgccga agagcgaaac ggcgtattgt   1582
   atcaccgctc gccccgcgag cgctctgctt cgtgtcgacg cgagaaaaat gtgcgctgct   1642
   cggttttcga gcggcacaga gatcgaatga tctacagata ttaatcgcgt ttgt 1696
<210> 2
   <211> 362
   <212> PRT
   <213> Ostreococcus lucimarinus
<400> 2
   Met Gln Glu Val Leu Arg Ala Ile Pro Arg Glu Cys Phe Glu Arg Asp
   1               5                   10                  15
   Thr Gly Lys Ser Leu Ala Tyr Ala Ala Cys Ser Thr Ala Ile Thr Leu
               20                  25                  30
   Ala Cys Gly Ala Leu Ala Trp Ala Cys Val Pro Val Thr Ala Ala Tyr
           35                  40                  45
   Trp Pro Val Trp Val Ala Tyr Ala Phe Val Thr Gly Thr Ala Ala Thr
       50                  55                  60
   Gly Cys Trp Val Ala Ala His Glu Cys Gly His Gly Ala Phe Ser Asp
   65                  70                  75                  80
   Asp Lys Thr Ile Gln Asp Ala Val Gly Tyr Ala Leu His Ser Leu Leu
                   85                  90                  95
   Leu Val Pro Tyr Phe Ser Trp Gln Arg Ser His Ala Val His His Ser
               100                 105                 110
   Arg Thr Asn His Val Leu Glu Gly Glu Thr His Val Pro Ala Arg Leu
           115                 120                 125
   Gly Thr Glu Asp Ala Asn Val Met Phe Lys Leu Arg Gly Leu Leu Gly
       130                 135                 140
   Glu Gly Pro Phe Thr Phe Leu Asn Leu Val Gly Val Phe Ala Leu Gly
   145                 150                 155                 160
   Trp Pro Ile Tyr Leu Leu Thr Gly Ala Ser Gly Gly Pro Val Arg Gly
                   165                 170                 175
   Asn Thr Asn His Phe Leu Pro Phe Met Gly Glu Lys Gly Lys His Ala
               180                 185                 190
   Leu Phe Pro Gly Lys Trp Ala Lys Lys Val Trp Gln Ser Asp Val Gly
           195                 200                 205
   Val Val Ala Val Leu Gly Ala Leu Ala Ala Trp Ala Ala His Ser Gly
       210                 215                 220
   Val Ala Thr Val Met Ala Leu Tyr Val Gly Pro Tyr Met Val Thr Asn
   225                 230                 235                 240
   Phe Trp Leu Val Leu Tyr Thr Trp Leu Gln His Thr Asp Val Asp Val
                   245                 250                 255
   Pro His Phe Glu Gly Asp Asp Trp Asn Leu Val Lys Gly Ala Phe Met
               260                 265                 270
   Thr Ile Asp Arg Pro Tyr Gly Pro Val Phe Asp Phe Leu His His Arg
           275                 280                 285
   Ile Gly Ser Thr His Val Ala His His Ile Asn His Thr Ile Pro His
       290                 295                 300
   Tyr His Ala Lys Lys Ala Thr Glu Ala Leu Gln Lys Ala Phe Pro Asp
   305                 310                 315                 320
   Leu Tyr Leu Tyr Asp Pro Thr Pro Ile Ala Thr Ala Thr Trp Arg Val
                   325                 330                 335
   Gly Ser Lys Cys Ile Ala Val Val Lys Lys Gly Asp Glu Trp Val Phe
               340                 345                 350
   Thr Asp Lys Gln Leu Asp Pro Val Ala Ala
           355                 360
<210> 3
   <211> 2039
   <212> DNA
   <213> Ostreococcus lucimarinus
<220>
   <223> Delta-12 desaturase
<220>
   <221> CDS
   <222> (572)..(1900)
<400> 3
   cgttctcgcc ctcgcgcgcg tcgcgaccga cgtcttctac gcttaccgac gcgcccgcgt      60
   ccgcgaccgc gcgtctggcg cgtgctcgag cgtgccgtcg attcgccgcg ccgccgtcgc     120
   gcgcgcgcgt cgcgccgatc ccgcggcgcg ctcgccgacg cgcgttcgcc gcgcgcgcgc     180
   gctcgatcga ccactcgcgc gcgccgaggc gcgacgcagt gcgggcgcgc gcgccgaggc     240
   gcggtcggtc ggtcggtcgc cgcgtgatcg cgggcgctcc gatgatggcc atccaaagcg     300
   ctcgaatgcg tggagcgacg cgatatcagc ctgcgaatgt tcacataaca taactctgac     360
   tgcgaatgtc cgaacacgcg gcgttcgcgg tcgcggtcct ccgcgttcgt cgcgcacacg     420
   cgcgcgcgcg aacgacgccc gacgtcgacg cgagcgacga gcgacgcaaa gtttttacgg     480
   cgagaatcgc gcgcgcccag gacgcacgcg cgcccaggac gtctcgacgc gacgcgcacg     540
   acgcaaacgc ggatagcgaa cgacgaggac g atg atg acg acg gca aac gtc        592
                                     Met Met Thr Thr Ala Asn Val
                                     1               5
   tcg cgg gtc gcc gcg acg cgc gcg atg acg acg tcg acg acg tcg acg       640
   Ser Arg Val Ala Ala Thr Arg Ala Met Thr Thr Ser Thr Thr Ser Thr
           10                  15                  20
   cga cgc gtg atg gcg agt aaa tac atc gcg cga acg gcg acg acg acg       688
   Arg Arg Val Met Ala Ser Lys Tyr Ile Ala Arg Thr Ala Thr Thr Thr
       25                  30                  35
   acg acg gat gcg cgc gga cgg gcg cac cga cgc gcg ggc gcg atc gcg       736
   Thr Thr Asp Ala Arg Gly Arg Ala His Arg Arg Ala Gly Ala Ile Ala
   40                  45                  50                  55
   ctc ggt ggg ttt gaa gat ctt ccc agg agc ggg ctg gag gga cag gcg       784
   Leu Gly Gly Phe Glu Asp Leu Pro Arg Ser Gly Leu Glu Gly Gln Ala
                   60                  65                  70
   ctc acg ttc ccg cac aag aag gag ttc cct tcg cgc gcg gcg gtg ttg       832
   Leu Thr Phe Pro His Lys Lys Glu Phe Pro Ser Arg Ala Ala Val Leu
               75                  80                  85
   tca aac att ccc gat gaa tgc ttc aag aag gat acg gcc aag tcg ttg       880
   Ser Asn Ile Pro Asp Glu Cys Phe Lys Lys Asp Thr Ala Lys Ser Leu
           90                  95                  100
   atg tac gcc gcg gtg tcg acg gca atg acg gtg ggg tgc ggc ttg atc       928
   Met Tyr Ala Ala Val Ser Thr Ala Met Thr Val Gly Cys Gly Leu Ile
       105                 110                 115
   gcc gcg gcg aca ttg ccg ttg cag gcg gcg tgg tgg ccg gcg tgg ctt       976
   Ala Ala Ala Thr Leu Pro Leu Gln Ala Ala Trp Trp Pro Ala Trp Leu
   120                 125                 130                 135
   gcg tac gcc gcg gtg aac ggt acg atc gcc act gga tgc tgg gtg atc      1024
   Ala Tyr Ala Ala Val Asn Gly Thr Ile Ala Thr Gly Cys Trp Val Ile
                   140                 145                 150
   gcg cac gag tgc ggc cat aac gcg ttc agc gat aac cga ttc att caa      1072
   Ala His Glu Cys Gly His Asn Ala Phe Ser Asp Asn Arg Phe Ile Gln
               155                 160                 165
   gac gcg gtc ggg tac gcg ttg cac tcg gcg ctc ctc gtg ccg tac ttt      1120
   Asp Ala Val Gly Tyr Ala Leu His Ser Ala Leu Leu Val Pro Tyr Phe
           170                 175                 180
   tcc tgg cag cgg tcg cac gct gtc cac cac tca cga acg aac cac ttg      1168
   Ser Trp Gln Arg Ser His Ala Val His His Ser Arg Thr Asn His Leu
       185                 190                 195
   acg gaa gga gaa acg cac gtg ccg tac gtc aag ggc gaa ttg aag ggt      1216
   Thr Glu Gly Glu Thr His Val Pro Tyr Val Lys Gly Glu Leu Lys Gly
   200                 205                 210                 215
   gac ttg aac ctc aag gcg aag aag aac ttg ggt gag ggc ccg ttc gcg       1264
   Asp Leu Asn Leu Lys Ala Lys Lys Asn Leu Gly Glu Gly Pro Phe Ala
                   220                 225                 230
   att ctt cag ctc gtc acc cac ttg gtt ttc ggc tgg ccg gcg tac ttg       1312
   Ile Leu Gln Leu Val Thr His Leu Val Phe Gly Trp Pro Ala Tyr Leu
               235                 240                 245
   ctc acc ggc gcg acg ggc ggg agc gcg aga ggc gtc acc aac cat ttc       1360
   Leu Thr Gly Ala Thr Gly Gly Ser Ala Arg Gly Val Thr Asn His Phe
           250                 255                 260
   ttg cct aac atc aat acc ggc gct ttg gag ctg ttc ccg gga agc tgg       1408
   Leu Pro Asn Ile Asn Thr Gly Ala Leu Glu Leu Phe Pro Gly Ser Trp
       265                 270                 275
   aag aag aaa gtg tac tac tcg gac att ggt gtt ttc gcg ttc gtc ggc       1456
   Lys Lys Lys Val Tyr Tyr Ser Asp Ile Gly Val Phe Ala Phe Val Gly
   280                 285                 290                 295
   gtt ctc gcc gcc tgg gtt gcc cag tgc ggt tgg gct ccg ttt gtc gcc       1504
   Val Leu Ala Ala Trp Val Ala Gln Cys Gly Trp Ala Pro Phe Val Ala
                   300                 305                 310
   ctg tac ctg gga ccg tat ttg ttt gtc aac ttt tgg ctc gtc ttg tac       1552
   Leu Tyr Leu Gly Pro Tyr Leu Phe Val Asn Phe Trp Leu Val Leu Tyr
               315                 320                 325
   acg tgg ttg cag cac acc gat gtg gac gtc caa cac ttg gcc gcg gac       1600
   Thr Trp Leu Gln His Thr Asp Val Asp Val Gln His Leu Ala Ala Asp
           330                 335                 340
   gag tgg tcg tac atc aag ggc gca ttc ttg acg atc gac cgc ccg tac       1648
   Glu Trp Ser Tyr Ile Lys Gly Ala Phe Leu Thr Ile Asp Arg Pro Tyr
       345                 350                 355
   gga ccc gtg ttt gac ttc tta cac cac cgc atc gga tcc act cac gtg       1696
   Gly Pro Val Phe Asp Phe Leu His His Arg Ile Gly Ser Thr His Val
   360                 365                 370                 375
   gcg cac cac gtt gag tgc gcg att ccg cac tac aag gcg gtc gaa gcg       1744
   Ala His His Val Glu Cys Ala Ile Pro His Tyr Lys Ala Val Glu Ala
                   380                 385                 390
   acc gag gcg ttg aag acg aag tat ccg gag tac tat ttg tat gac ccg       1792
   Thr Glu Ala Leu Lys Thr Lys Tyr Pro Glu Tyr Tyr Leu Tyr Asp Pro
               395                 400                 405
   acg ccg att tgg gcg gca atg atg cgc gtc gcg tcc aag tgc gtc gct       1840
   Thr Pro Ile Trp Ala Ala Met Met Arg Val Ala Ser Lys Cys Val Ala
           410                 415                 420
   gtc gag aag cgc ggc gaa ggc aag ggt gcg atg tgg act ttc act gac       1888
   Val Glu Lys Arg Gly Glu Gly Lys Gly Ala Met Trp Thr Phe Thr Asp
       425                 430                 435
   ggc acg gcg taa ttatgaatga ttattagtat ttagcagctc ttcgcgcaca           1940
   Gly Thr Ala
   440
   cgcagacaca aacattttat tgatacacac taacgaaact actacgtgcg attccagttg     2000
   cgaaagacga gtacacactt gtacacacta acgaaacta                            2039
<210> 4
   <211> 442
   <212> PRT
   <213> Ostreococcus lucimarinus
<400> 4
   Met Met Thr Thr Ala Asn Val Ser Arg Val Ala Ala Thr Arg Ala Met
   1               5                   10                  15
   Thr Thr Ser Thr Thr Ser Thr Arg Arg Val Met Ala Ser Lys Tyr Ile
               20                  25                  30
   Ala Arg Thr Ala Thr Thr Thr Thr Thr Asp Ala Arg Gly Arg Ala His
           35                  40                  45
   Arg Arg Ala Gly Ala Ile Ala Leu Gly Gly Phe Glu Asp Leu Pro Arg
       50                  55                  60
   Ser Gly Leu Glu Gly Gln Ala Leu Thr Phe Pro His Lys Lys Glu Phe
   65                  70                  75                  80
   Pro Ser Arg Ala Ala Val Leu Ser Asn Ile Pro Asp Glu Cys Phe Lys
                   85                  90                  95
   Lys Asp Thr Ala Lys Ser Leu Met Tyr Ala Ala Val Ser Thr Ala Met
               100                 105                 110
   Thr Val Gly Cys Gly Leu Ile Ala Ala Ala Thr Leu Pro Leu Gln Ala
           115                 120                 125
   Ala Trp Trp Pro Ala Trp Leu Ala Tyr Ala Ala Val Asn Gly Thr Ile
       130                 135                 140
   Ala Thr Gly Cys Trp Val Ile Ala His Glu Cys Gly His Asn Ala Phe
   145                 150                 155                 160
   Ser Asp Asn Arg Phe Ile Gln Asp Ala Val Gly Tyr Ala Leu His Ser
                   165                 170                 175
   Ala Leu Leu Val Pro Tyr Phe Ser Trp Gln Arg Ser His Ala Val His
               180                 185                 190
   His Ser Arg Thr Asn His Leu Thr Glu Gly Glu Thr His Val Pro Tyr
           195                 200                 205
   Val Lys Gly Glu Leu Lys Gly Asp Leu Asn Leu Lys Ala Lys Lys Asn
       210                 215                 220
   Leu Gly Glu Gly Pro Phe Ala Ile Leu Gln Leu Val Thr His Leu Val
   225                 230                 235                 240
   Phe Gly Trp Pro Ala Tyr Leu Leu Thr Gly Ala Thr Gly Gly Ser Ala
                   245                 250                 255
   Arg Gly Val Thr Asn His Phe Leu Pro Asn Ile Asn Thr Gly Ala Leu
               260                 265                 270
   Glu Leu Phe Pro Gly Ser Trp Lys Lys Lys Val Tyr Tyr Ser Asp Ile
           275                 280                 285
   Gly Val Phe Ala Phe Val Gly Val Leu Ala Ala Trp Val Ala Gln Cys
       290                 295                 300
   Gly Trp Ala Pro Phe Val Ala Leu Tyr Leu Gly Pro Tyr Leu Phe Val
   305                 310                 315                 320
   Asn Phe Trp Leu Val Leu Tyr Thr Trp Leu Gln His Thr Asp Val Asp
                   325                 330                 335
   Val Gln His Leu Ala Ala Asp Glu Trp Ser Tyr Ile Lys Gly Ala Phe
               340                 345                 350
   Leu Thr Ile Asp Arg Pro Tyr Gly Pro Val Phe Asp Phe Leu His His
           355                 360                 365
   Arg Ile Gly Ser Thr His Val Ala His His Val Glu Cys Ala Ile Pro
       370                 375                 380
   His Tyr Lys Ala Val Glu Ala Thr Glu Ala Leu Lys Thr Lys Tyr Pro
   385                 390                 395                 400
   Glu Tyr Tyr Leu Tyr Asp Pro Thr Pro Ile Trp Ala Ala Met Met Arg
                   405                 410                 415
   Val Ala Ser Lys Cys Val Ala Val Glu Lys Arg Gly Glu Gly Lys Gly
               420                 425                 430
   Ala Met Trp Thr Phe Thr Asp Gly Thr Ala
           435                 440
<210> 5
   <211> 1737
   <212> DNA
   <213> Ostreococcus lucimarinus
<220>
   <223> Delta-4 desaturase
<220>
   <221> CDS
   <222> (218)..(1618)
<400> 5
   gcgcaccgtc gcgcgcgcgt tcgcgacgac gacgacgtcg agcgcgaggt gtgattatcg 60
   ccgtcgagcg acgacgacgc gcgcgacgac gagcgcggtg cgcgcggacg cgcggcgcgc 120
   gcgatcgggg aggtatgcga tcgttccgcg atcgatcgag ggcgaaggga acgccgcggt 180
   cgtcgaaaga agcgtcgagg gcgaggaggg ggagaag atg atg tcc ctg gag gcg     235
                                            Met Met Ser Leu Glu Ala
                                             1               5
   aga cgc gag gcg ctg agg ttg ccg cgc ccg gac gtc gcg gcg ccg ggg     283
   Arg Arg Glu Ala Leu Arg Leu Pro Arg Pro Asp Val Ala Ala Pro Gly
               10                  15                  20
   atg gag gat ccg tgg aac gat gag aag tgg caa aag gtg aag tgg acg     331
   Met Glu Asp Pro Trp Asn Asp Glu Lys Trp Gln Lys Val Lys Trp Thr
           25                  30                  35
   gtg ttt cga gac gtg gcg tac gac ctc gga ccg ttc ttt gag aag cat     379
   Val Phe Arg Asp Val Ala Tyr Asp Leu Gly Pro Phe Phe Glu Lys His
       40                  45                  50
   ccg ggc ggg aac tgg ttg ttg aac ctc gcc atc ggt cgc gac tgc acg     427
   Pro Gly Gly Asn Trp Leu Leu Asn Leu Ala Ile Gly Arg Asp Cys Thr
   55                  60                  65                  70
   gcg ctc atg gaa tcc tat cac ttg cga cca gag gtg gcg acg gcg cgc     475
   Ala Leu Met Glu Ser Tyr His Leu Arg Pro Glu Val Ala Thr Ala Arg
                   75                  80                  85
   ttc agg atg tta ccc aag ctc gag ggg ttc ccg gtc gac gcc gtg gcg     523
   Phe Arg Met Leu Pro Lys Leu Glu Gly Phe Pro Val Asp Ala Val Ala
               90                  95                  100
   aag tca ccg aga ccg aac gat tcg ccg ctg tac aac aac atc cgc aat     571
   Lys Ser Pro Arg Pro Asn Asp Ser Pro Leu Tyr Asn Asn Ile Arg Asn
           105                 110                 115
   cgc gtt cgc gag gaa ctt ttc ccg gaa gaa ggg aag aac aaa cac aga     619
   Arg Val Arg Glu Glu Leu Phe Pro Glu Glu Gly Lys Asn Lys His Arg
       120                 125                 130
   atg ggt ggc gac cac gcg acg ata acg att ttg tct ttc gcc gcg ttt     667
   Met Gly Gly Asp His Ala Thr Ile Thr Ile Leu Ser Phe Ala Ala Phe
   135                 140                 145                 150
   gcg tac ggt gtg tac gcc acc atc ccg ggc ttt ctc tcg ggt tgc ctc     715
   Ala Tyr Gly Val Tyr Ala Thr Ile Pro Gly Phe Leu Ser Gly Cys Leu
                   155                 160                 165
   ctt ggc tta gct ggt gcg tgg atc ggg ctc acg att cag cat tgc gcc     763
   Leu Gly Leu Ala Gly Ala Trp Ile Gly Leu Thr Ile Gln His Cys Ala
               170                 175                 180
   aat cac ggg gcg atg tca ccg tcg ccc gcc gtc aac ggc gtc ctc ggt     811
   Asn His Gly Ala Met Ser Pro Ser Pro Ala Val Asn Gly Val Leu Gly
           185                 190                 195
   ttg acg aac gat ctc atc ggc ggc tcg tct ttg atg tgg cga tac cac     859
   Leu Thr Asn Asp Leu Ile Gly Gly Ser Ser Leu Met Trp Arg Tyr His
       200                 205                 210
   cac cag gtg agc cac cac att cac tgc aac gac aat gct atg gat caa     907
   His Gln Val Ser His His Ile His Cys Asn Asp Asn Ala Met Asp Gln
   215                 220                 225                 230
   gac gtg tac acc gcc atg cct ctc ttg cgt ttc gac gct cgt cgt ccc     955
   Asp Val Tyr Thr Ala Met Pro Leu Leu Arg Phe Asp Ala Arg Arg Pro
                   235                 240                 245
   aag gcg tgg tac cac cgc ttc cag cac tgg tac atg ttc ttg gcc ttt     1003
   Lys Ala Trp Tyr His Arg Phe Gln His Trp Tyr Met Phe Leu Ala Phe
               250                 255                 260
   ccg ttg ttg cag gtg gcg ttc caa gtc ggc gac atc gtc ggt ttg ttc     1051
   Pro Leu Leu Gln Val Ala Phe Gln Val Gly Asp Ile Val Gly Leu Phe
           265                 270                 275
   act cgc gat acc gaa ggc gca aag ctc cac ggg gcg acc act tgg gaa     1099
   Thr Arg Asp Thr Glu Gly Ala Lys Leu His Gly Ala Thr Thr Trp Glu
       280                 285                 290
   ctt gcc acc gta gtt atc ggc aag ttt gtg cac ttc ggt ttg ttg gct     1147
   Leu Ala Thr Val Val Ile Gly Lys Phe Val His Phe Gly Leu Leu Ala
   295                 300                 305                 310
   gca cct ttg atg agc cac gcg gct tcc gcc gtc gtc gcc ggc atc gtc     1195
   Ala Pro Leu Met Ser His Ala Ala Ser Ala Val Val Ala Gly Ile Val
                   315                 320                 325
   ggt ttc atg gcg tgc caa gga gtc gtc ctc gcg tgt acg ttc gcg gtg     1243
   Gly Phe Met Ala Cys Gln Gly Val Val Leu Ala Cys Thr Phe Ala Val
               330                 335                 340
   agt cac aac gtg ccc gag gcc aaa ctc cct gaa gat acc ggt gga gag     1291
   Ser His Asn Val Pro Glu Ala Lys Leu Pro Glu Asp Thr Gly Gly Glu
           345                 350                 355
   gcg tgg gaa aga gat tgg ggc gtg cag cag ttg gtg act agc gct gac        1339
   Ala Trp Glu Arg Asp Trp Gly Val Gln Gln Leu Val Thr Ser Ala Asp
       360                 365                 370
   tgg ggt gga aag att gga aac ttt ttc acc ggc ggt ctg aac ttg caa        1387
   Trp Gly Gly Lys Ile Gly Asn Phe Phe Thr Gly Gly Leu Asn Leu Gln
   375                 380                 385                 390
   gtc gag cat cac ctg ttc ccg gcg att tgc ttt gtg cac tac ccc gcg        1435
   Val Glu His His Leu Phe Pro Ala Ile Cys Phe Val His Tyr Pro Ala
                   395                 400                 405
   atc gcg aaa att gtc gcg gaa gaa gcg gcg aag atg ggc att cct tac        1483
   Ile Ala Lys Ile Val Ala Glu Glu Ala Ala Lys Met Gly Ile Pro Tyr
               410                 415                 420
   tcg tca tac aga aca ctt ccc ggc att ttc gtc gcg ttc tgg aaa ttc        1531
   Ser Ser Tyr Arg Thr Leu Pro Gly Ile Phe Val Ala Phe Trp Lys Phe
           425                 430                 435
   gtc aga gac atg ggc acg gcg gag caa atc gac gac gtt tta ctt ccg        1579
   Val Arg Asp Met Gly Thr Ala Glu Gln Ile Asp Asp Val Leu Leu Pro
       440                 445                 450
   aag ttc gcg aac ccg caa ctt tcg cca gcc atc aat tag aattataact         1628
   Lys Phe Ala Asn Pro Gln Leu Ser Pro Ala Ile Asn
   455                 460                 465
   taccttacgc gcttgttgac ttcaaacgag caaacgacag aaatacgatt attgtgatat      1688
   acttacacct ccgcctcatc attgttttga tctaattcta acttacaaa                  1737
<210> 6
   <211> 466
   <212> PRT
   <213> Ostreococcus lucimarinus
<400> 6
   Met Met Ser Leu Glu Ala Arg Arg Glu Ala Leu Arg Leu Pro Arg Pro
   1               5                   10                  15
   Asp Val Ala Ala Pro Gly Met Glu Asp Pro Trp Asn Asp Glu Lys Trp
               20                  25                  30
   Gln Lys Val Lys Trp Thr Val Phe Arg Asp Val Ala Tyr Asp Leu Gly
           35                  40                  45
   Pro Phe Phe Glu Lys His Pro Gly Gly Asn Trp Leu Leu Asn Leu Ala
       50                  55                  60
   Ile Gly Arg Asp Cys Thr Ala Leu Met Glu Ser Tyr His Leu Arg Pro
   65                  70                  75                  80
   Glu Val Ala Thr Ala Arg Phe Arg Met Leu Pro Lys Leu Glu Gly Phe
                   85                  90                  95
   Pro Val Asp Ala Val Ala Lys Ser Pro Arg Pro Asn Asp Ser Pro Leu
               100                 105                 110
   Tyr Asn Asn Ile Arg Asn Arg Val Arg Glu Glu Leu Phe Pro Glu Glu
           115                 120                 125
   Gly Lys Asn Lys His Arg Met Gly Gly Asp His Ala Thr Ile Thr Ile
       130                 135                 140
   Leu Ser Phe Ala Ala Phe Ala Tyr Gly Val Tyr Ala Thr Ile Pro Gly
   145                 150                 155                 160
   Phe Leu Ser Gly Cys Leu Leu Gly Leu Ala Gly Ala Trp Ile Gly Leu
                   165                 170                 175
   Thr Ile Gln His Cys Ala Asn His Gly Ala Met Ser Pro Ser Pro Ala
               180                 185                 190
   Val Asn Gly Val Leu Gly Leu Thr Asn Asp Leu Ile Gly Gly Ser Ser
           195                 200                 205
   Leu Met Trp Arg Tyr His His Gln Val Ser His His Ile His Cys Asn
       210                 215                 220
   Asp Asn Ala Met Asp Gln Asp Val Tyr Thr Ala Met Pro Leu Leu Arg
   225                 230                 235                 240
   Phe Asp Ala Arg Arg Pro Lys Ala Trp Tyr His Arg Phe Gln His Trp
                   245                 250                 255
   Tyr Met Phe Leu Ala Phe Pro Leu Leu Gln Val Ala Phe Gln Val Gly
               260                 265                 270
   Asp Ile Val Gly Leu Phe Thr Arg Asp Thr Glu Gly Ala Lys Leu His
           275                 280                 285
   Gly Ala Thr Thr Trp Glu Leu Ala Thr Val Val Ile Gly Lys Phe Val
       290                 295                 300
   His Phe Gly Leu Leu Ala Ala Pro Leu Met Ser His Ala Ala Ser Ala
   305                 310                 315                 320
   Val Val Ala Gly Ile Val Gly Phe Met Ala Cys Gln Gly Val Val Leu
                   325                 330                 335
   Ala Cys Thr Phe Ala Val Ser His Asn Val Pro Glu Ala Lys Leu Pro
               340                 345                 350
   Glu Asp Thr Gly Gly Glu Ala Trp Glu Arg Asp Trp Gly Val Gln Gln
           355                 360                 365
   Leu Val Thr Ser Ala Asp Trp Gly Gly Lys Ile Gly Asn Phe Phe Thr
       370                 375                 380
   Gly Gly Leu Asn Leu Gln Val Glu His His Leu Phe Pro Ala Ile Cys
   385                 390                 395                 400
   Phe Val His Tyr Pro Ala Ile Ala Lys Ile Val Ala Glu Glu Ala Ala
                   405                 410                 415
   Lys Met Gly Ile Pro Tyr Ser Ser Tyr Arg Thr Leu Pro Gly Ile Phe
               420                 425                 430
   Val Ala Phe Trp Lys Phe Val Arg Asp Met Gly Thr Ala Glu Gln Ile
           435                 440                 445
   Asp Asp Val Leu Leu Pro Lys Phe Ala Asn Pro Gln Leu Ser Pro Ala
       450                 455                 460
   Ile Asn
   465
<210> 7
   <211> 1604
   <212> DNA
   <213> Ostreococcus lucimarinus
<220>
   <223> Delta-5 desaturase
<220>
   <221> CDS
   <222> (156)..(1535)
<400> 7
   tcactcagcc gtccgcgatc cgcgcgcgcg tcgcgttcga ccgcgtcgcg cccagaacga      60
   acgtagaacc gaatcgcccg tcccgcgtcg cgcggatgaa tgcaaaaaac aagatgactg     120
   gatgacgtcc cgcgccacac gatcgcgcgc gcgtc atg ccc tcc gcc gcg cgc        173
                                          Met Pro Ser Ala Ala Arg
                                          1               5
   tcc cga gcg tcg aag cgc gcg aac gcg acg acg gac gtc gcg acg acc       221
   Ser Arg Ala Ser Lys Arg Ala Asn Ala Thr Thr Asp Val Ala Thr Thr
               10                  15                  20
   gct ccc gag gcg acg ctc gac ccg acg cgc gcg tac acg cgc tat cgc       269
   Ala Pro Glu Ala Thr Leu Asp Pro Thr Arg Ala Tyr Thr Arg Tyr Arg
           25                  30                  35
   ggc gtc gtc tac gac gtc acc gag ttt cag cat cgc cat ccc ggc ggc       317
   Gly Val Val Tyr Asp Val Thr Glu Phe Gln His Arg His Pro Gly Gly
       40                  45                  50
   gcg caa ttg ctc tcg ctc tgc gtc gga cgc gac gcg acg atc ctg atc       365
   Ala Gln Leu Leu Ser Leu Cys Val Gly Arg Asp Ala Thr Ile Leu Ile
   55                  60                  65                  70
   gag agc cat cac ttg cga cct gag gtg gtg cgc aag tac atg aag acg       413
   Glu Ser His His Leu Arg Pro Glu Val Val Arg Lys Tyr Met Lys Thr
                   75                  80                  85
   ctg ccc gtg gtg gag ggc gcg gcg ggc gcg ttc ggg aag gag gag acg       461
   Leu Pro Val Val Glu Gly Ala Ala Gly Ala Phe Gly Lys Glu Glu Thr
               90                  95                  100
   ttt ccg aaa ccg ctc gat tcg gcg gtg tat cga gcg ata caa cgc cga       509
   Phe Pro Lys Pro Leu Asp Ser Ala Val Tyr Arg Ala Ile Gln Arg Arg
   105 110 115
   gtg cga gat gaa gtc gtg gaa ccg atg aaa gca aag tct gga agg gag 557
   Val Arg Asp Glu Val Val Glu Pro Met Lys Ala Lys Ser Gly Arg Glu
   120 125 130
   gca cac gga cgc ggt ggg tgc gtc gtg gac gcg ggg gtg gtc gtg ttg 605
   Ala His Gly Arg Gly Gly Cys Val Val Asp Ala Gly Val Val Val Leu
   135 140 145 150
   acg ttt gtc gcg gcg atg gtg gcg tat tgg cgc gcg ccc tcg gcg ctc 653
   Thr Phe Val Ala Ala Met Val Ala Tyr Trp Arg Ala Pro Ser Ala Leu
   155 160 165
   acg ggg tgc gcg ttg gga ctg gcg ggg tac tgg agc ggc act ggg ttg 701
   Thr Gly Cys Ala Leu Gly Leu Ala Gly Tyr Trp Ser Gly Thr Gly Leu
   170 175 180
   caa cac acg gcg aat cac gga gga ttg gcg aag agt gga ttt tgg aat 749
   Gln His Thr Ala Asn His Gly Gly Leu Ala Lys Ser Gly Phe Trp Asn
   185 190 195
   cag ttt tgg gga tgg ctc ggg aac gac gtg gcg atc ggg aag agt tcg 797
   Gln Phe Trp Gly Trp Leu Gly Asn Asp Val Ala Ile Gly Lys Ser Ser
   200 205 210
   gtg gag tgg cgg tat cat cac atg gtg agc cat cac tcg tac tgc aac 845
   Val Glu Trp Arg Tyr His His Met Val Ser His His Ser Tyr Cys Asn
   215 220 225 230
   gac gcc gat ctc gat caa gac gtg tac acc gcg tta ccg ttg ttg cga 893
   Asp Ala Asp Leu Asp Gln Asp Val Tyr Thr Ala Leu Pro Leu Leu Arg
   235 240 245
   ctt gac cca tcg cag gag ttg aag tgg ttc cat cgg tac caa gcg ttt 941
   Leu Asp Pro Ser Gln Glu Leu Lys Trp Phe His Arg Tyr Gln Ala Phe
   250 255 260
   tac gcg ccg ctc atg tgg ccg ttt tta tgg ctc gcc gcg caa gtc ggt 989
   Tyr Ala Pro Leu Met Trp Pro Phe Leu Trp Leu Ala Ala Gln Val Gly
   265 270 275
   gac gcg caa aac att tta atc gat cga gcg tct ccc ggc gtc gag tac 1037
   Asp Ala Gln Asn Ile Leu Ile Asp Arg Ala Ser Pro Gly Val Glu Tyr
   280 285 290
   aag ggc ttg atg aag aac gaa atc gcg ctg tac ctg ctc ggt aaa gtt 1085
   Lys Gly Leu Met Lys Asn Glu Ile Ala Leu Tyr Leu Leu Gly Lys Val
   295 300 305 310
   ctg cac ttc ggc tta ctt ctc ggc gtt ccg gcg tac atc cac ggg ttg 1133
   Leu His Phe Gly Leu Leu Leu Gly Val Pro Ala Tyr Ile His Gly Leu
   315 320 325
   tcc aac gtc atc gtg ccg ttc ctc gcg tac ggc gcg ttt ggc tcg ttc 1181
   Ser Asn Val Ile Val Pro Phe Leu Ala Tyr Gly Ala Phe Gly Ser Phe
   330 335 340
   gtc ctt tgc tgg ttc ttc atc gtc agc cac aac ttg gag gcg ttg acc 1229
   Val Leu Cys Trp Phe Phe Ile Val Ser His Asn Leu Glu Ala Leu Thr
   345 350 355
   ccg atg aac ttg agc aag agc act aag aac gat tgg ggt gcg tgg caa 1277
   Pro Met Asn Leu Ser Lys Ser Thr Lys Asn Asp Trp Gly Ala Trp Gln
   360 365 370
   atc gaa acg tcc gcg tcg tgg ggt aac agc ttc tgg agc ttc ttt tcc 1325
   Ile Glu Thr Ser Ala Ser Trp Gly Asn Ser Phe Trp Ser Phe Phe Ser
   375 380 385 390
   ggt ggt ttg aac ttg caa atc gag cac cac ttg ttt ccg gga tgc gcg 1373
   Gly Gly Leu Asn Leu Gln Ile Glu His His Leu Phe Pro Gly Cys Ala
   395 400 405
   cac aat ctg tac ccg aag atg gtt ccc atc atc aag gat gag tgc aaa 1421
   His Asn Leu Tyr Pro Lys Met Val Pro Ile Ile Lys Asp Glu Cys Lys
   410 415 420
   aag gct ggc ctc gcg tac acc ggc tat ggt ggc tac ttt gga ttg ttg 1469
   Lys Ala Gly Leu Ala Tyr Thr Gly Tyr Gly Gly Tyr Phe Gly Leu Leu
   425 430 435
   ccc atc acg cgc gac atg ttt tcc tat tta cac aag atg ggc cat caa 1517
   Pro Ile Thr Arg Asp Met Phe Ser Tyr Leu His Lys Met Gly His Gln
   440 445 450
   cga ccg aag gcc atg tag aaatccgaac aaaccgttgt acctcttgtt 1565
   Arg Pro Lys Ala Met
   455
   tgatgaatac agtatttatt ttacaacgca actacgcat 1604
<210> 8
   <211> 459
   <212> PRT
   <213> Ostreococcus lucimarinus
<400> 8
   Met Pro Ser Ala Ala Arg Ser Arg Ala Ser Lys Arg Ala Asn Ala Thr
   1               5                   10                  15
   Thr Asp Val Ala Thr Thr Ala Pro Glu Ala Thr Leu Asp Pro Thr Arg
               20                  25                  30
   Ala Tyr Thr Arg Tyr Arg Gly Val Val Tyr Asp Val Thr Glu Phe Gln
           35                  40                  45
   His Arg His Pro Gly Gly Ala Gln Leu Leu Ser Leu Cys Val Gly Arg
       50                  55                  60
   Asp Ala Thr Ile Leu Ile Glu Ser His His Leu Arg Pro Glu Val Val
   65                  70                  75                  80
   Arg Lys Tyr Met Lys Thr Leu Pro Val Val Glu Gly Ala Ala Gly Ala
                   85                  90                  95
   Phe Gly Lys Glu Glu Thr Phe Pro Lys Pro Leu Asp Ser Ala Val Tyr
               100                 105                 110
   Arg Ala Ile Gln Arg Arg Val Arg Asp Glu Val Val Glu Pro Met Lys
           115                 120                 125
   Ala Lys Ser Gly Arg Glu Ala His Gly Arg Gly Gly Cys Val Val Asp
       130                 135                 140
   Ala Gly Val Val Val Leu Thr Phe Val Ala Ala Met Val Ala Tyr Trp
   145                 150                 155                 160
   Arg Ala Pro Ser Ala Leu Thr Gly Cys Ala Leu Gly Leu Ala Gly Tyr
                   165                 170                 175
   Trp Ser Gly Thr Gly Leu Gln His Thr Ala Asn His Gly Gly Leu Ala
               180                 185                 190
   Lys Ser Gly Phe Trp Asn Gln Phe Trp Gly Trp Leu Gly Asn Asp Val
           195                 200                 205
   Ala Ile Gly Lys Ser Ser Val Glu Trp Arg Tyr His His Met Val Ser
       210                 215                 220
   His His Ser Tyr Cys Asn Asp Ala Asp Leu Asp Gln Asp Val Tyr Thr
   225                 230                 235                 240
   Ala Leu Pro Leu Leu Arg Leu Asp Pro Ser Gln Glu Leu Lys Trp Phe
                   245                 250                 255
   His Arg Tyr Gln Ala Phe Tyr Ala Pro Leu Met Trp Pro Phe Leu Trp
               260                 265                 270
   Leu Ala Ala Gln Val Gly Asp Ala Gln Asn Ile Leu Ile Asp Arg Ala
           275                 280                 285
   Ser Pro Gly Val Glu Tyr Lys Gly Leu Met Lys Asn Glu Ile Ala Leu
       290                 295                 300
   Tyr Leu Leu Gly Lys Val Leu His Phe Gly Leu Leu Leu Gly Val Pro
   305                 310                 315                 320
   Ala Tyr Ile His Gly Leu Ser Asn Val Ile Val Pro Phe Leu Ala Tyr
                   325                 330                 335
   Gly Ala Phe Gly Ser Phe Val Leu Cys Trp Phe Phe Ile Val Ser His
               340                 345                 350
   Asn Leu Glu Ala Leu Thr Pro Met Asn Leu Ser Lys Ser Thr Lys Asn
           355                 360                 365
   Asp Trp Gly Ala Trp Gln Ile Glu Thr Ser Ala Ser Trp Gly Asn Ser
       370                 375                 380
   Phe Trp Ser Phe Phe Ser Gly Gly Leu Asn Leu Gln Ile Glu His His
   385                 390                 395                 400
   Leu Phe Pro Gly Cys Ala His Asn Leu Tyr Pro Lys Met Val Pro Ile
                   405                 410                 415
   Ile Lys Asp Glu Cys Lys Lys Ala Gly Leu Ala Tyr Thr Gly Tyr Gly
               420                 425                 430
   Gly Tyr Phe Gly Leu Leu Pro Ile Thr Arg Asp Met Phe Ser Tyr Leu
           435                 440                 445
   His Lys Met Gly His Gln Arg Pro Lys Ala Met
       450                 455
<210> 9
   <211> 2297
   <212> DNA
   <213> Ostreococcus lucimarinus
<220>
   <223> Delta-5 desaturase
<220>
   <221> CDS
   <222> (629)..(2104)
<400> 9
   cgcgcgcgcg tcgccgcgac ccccctcgaa gtcgcgtacc gttcgcccgc gtcgctcgcg      60
   tcgcgctctc gccgtctcgc gcgcgcgtcg cgtcgcgatg cgtcttcgcg cgtcgtcgcg     120
   atcggcgtct tccggcgtcc acgattcgcg cgcgcgcgtc ctcctccgcg gcgctcgcgc     180
   gcgcgcgtcg atcgcgtcgc gtcgatcgcg gtctctctcg cggttcctcg cggttcgcgc     240
   ctccctcgcg cgcgcgtcga gcgcgcgccg tcgtctgcgg cgccgtcgcc gtttcccgcg     300
   cgattcgtcc gacacgcgtc cgacacgcgt gcgacacgcg cgtttacata ccacaaatac     360
   cacacagtgt acgaccaaca ttaccgtact gtagaagaaa tgatggtcac tcgatgtata     420
   cagaccccgt cactgatccc catacgcgac cgaccgcccc gcccggtgac gtcggcgcgc     480
   gcgcgcgtcg ccacacgacg ccgcgcgctc gccgcgcgcg ctcgactcgc gcgccgcgtc     540
   gcgcgccgtc gcgctcaccg cgcgcccgcg accgccggcg tcgcgcgtcg acgacgccgc     600
   gcggcgaacg agggcgcgac gcgacgcg atg acg acg gtc gcg gag atc gtc        652
                                  Met Thr Thr Val Ala Glu Ile Val
                                  1               5
   gac gac gac gcg cgg cgc gcg gga aag ggc gcg acg acg cga cga cga       700
   Asp Asp Asp Ala Arg Arg Ala Gly Lys Gly Ala Thr Thr Arg Arg Arg
       10                  15                  20
   cgc gcg acg acg cgc gtc gcg cgc gat gac gcg cgc gag gcg acg tac       748
   Arg Ala Thr Thr Arg Val Ala Arg Asp Asp Ala Arg Glu Ala Thr Tyr
   25                  30                  35                  40
   acg gcg gcg gag gtg gcg agg cac gcg cgc gcg gac gac tgc tgg gtg       796
   Thr Ala Ala Glu Val Ala Arg His Ala Arg Ala Asp Asp Cys Trp Val
                   45                  50                  55
   atc gtg cga ggc ggc gtg tac gac gtg acg agg ttc gtg ccg aga cac       844
   Ile Val Arg Gly Gly Val Tyr Asp Val Thr Arg Phe Val Pro Arg His
               60                  65                  70
   ccg gga ggg aac atg ata tac gtc aag gcg ggg ggc gag tgc acg gcg       892
   Pro Gly Gly Asn Met Ile Tyr Val Lys Ala Gly Gly Glu Cys Thr Ala
           75                  80                  85
   ctg ttc gac tcg tat cat ccc gag cgc gcg agg gcg acg ctg gag aag       940
   Leu Phe Asp Ser Tyr His Pro Glu Arg Ala Arg Ala Thr Leu Glu Lys
       90                  95                  100
   tat agg atc ggc gcg ctg cgg agg gac gcg ggg gaa cgc gag gac gag       988
   Tyr Arg Ile Gly Ala Leu Arg Arg Asp Ala Gly Glu Arg Glu Asp Glu
   105                 110                 115                 120
   gac gtg gtg gag tac ttg aag gac gat ctg agg gag gga gag ttt tac      1036
   Asp Val Val Glu Tyr Leu Lys Asp Asp Leu Arg Glu Gly Glu Phe Tyr
                   125                 130                 135
   gcg gat tgt aag gcg ggg gcg gcg aag tat ttt aag gat aat aag ctc      1084
   Ala Asp Cys Lys Ala Gly Ala Ala Lys Tyr Phe Lys Asp Asn Lys Leu
               140                 145                 150
   gat ccg cgc gtg cac tgg gag atg tac gcg aag acg ttg gtg att ttg      1132
   Asp Pro Arg Val His Trp Glu Met Tyr Ala Lys Thr Leu Val Ile Leu
           155                 160                 165
   acg ggc gtc gtc gtc gga cac tac ggg tcg ttt ttc gcg ccg tcg gcg      1180
   Thr Gly Val Val Val Gly His Tyr Gly Ser Phe Phe Ala Pro Ser Ala
       170                 175                 180
   tcc ttc gcc gcg gcg ttg gcg ctc gcg gtg ctg cac ggc acg tgc aag      1228
   Ser Phe Ala Ala Ala Leu Ala Leu Ala Val Leu His Gly Thr Cys Lys
   185                 190                 195                 200
   gcg gaa gta gga gtg tcg atc cag cac gac gcc aat cac ggg gcg tac      1276
   Ala Glu Val Gly Val Ser Ile Gln His Asp Ala Asn His Gly Ala Tyr
                   205                 210                 215
   gga aac aat cgc act tgg ttg cac gcg atg cag ttg acc ttg gac gcc      1324
   Gly Asn Asn Arg Thr Trp Leu His Ala Met Gln Leu Thr Leu Asp Ala
               220                 225                 230
   gtc ggc gcg tcg agc ttc atg tgg aag cag cag cac gtc gcc gga cat      1372
   Val Gly Ala Ser Ser Phe Met Trp Lys Gln Gln His Val Ala Gly His
           235                 240                 245
   cac gcg tac acc aac gtc gaa ggc atc gat ccc gac att cga tgc tcg      1420
   His Ala Tyr Thr Asn Val Glu Gly Ile Asp Pro Asp Ile Arg Cys Ser
       250                 255                 260
   gaa aag gac gtg cga cgg gtg aac gag cat cag cct cac gag ccg tac      1468
   Glu Lys Asp Val Arg Arg Val Asn Glu His Gln Pro His Glu Pro Tyr
   265                 270                 275                 280
   cac aga gtt caa cac gtc tac ttg gcg ttg atg tac ggc ttg ttg tcg      1516
   His Arg Val Gln His Val Tyr Leu Ala Leu Met Tyr Gly Leu Leu Ser
                   285                 290                 295
   ttc aaa tcg tgc ttc gtg gac gac ttc aac gcc ttc ttc tcc ggc cga      1564
   Phe Lys Ser Cys Phe Val Asp Asp Phe Asn Ala Phe Phe Ser Gly Arg
               300                 305                 310
   atc ggt tgg gtg aag gtg atg aag ttt act cgc ggt gaa gcc gtg gcg      1612
   Ile Gly Trp Val Lys Val Met Lys Phe Thr Arg Gly Glu Ala Val Ala
           315                 320                 325
   ttt tgg gga agc aag ctc gcg tgg gcg ttt tac tac ctc tac ttg ccc      1660
   Phe Trp Gly Ser Lys Leu Ala Trp Ala Phe Tyr Tyr Leu Tyr Leu Pro
       330                 335                 340
   gcc aag tac tcc cat cgc tcg atc ggt cag ctc ttg gcg ctc tgg acc      1708
   Ala Lys Tyr Ser His Arg Ser Ile Gly Gln Leu Leu Ala Leu Trp Thr
   345                 350                 355                 360
   gtc acc gag ttc gtc acc ggt tgg ttg ctg gcg ttc atg ttc caa gtc      1756
   Val Thr Glu Phe Val Thr Gly Trp Leu Leu Ala Phe Met Phe Gln Val
                   365                 370                 375
   gcc cac gtc gtc ggc gac gtc cac ttc ttc cgc ctc aac gaa aag aac      1804
   Ala His Val Val Gly Asp Val His Phe Phe Arg Leu Asn Glu Lys Asn
               380                 385                 390
   cag ctc aac aag ggc tgg ggc gag gcg caa ctc atg act tcg gct gat      1852
   Gln Leu Asn Lys Gly Trp Gly Glu Ala Gln Leu Met Thr Ser Ala Asp
           395                 400                 405
   ttc gcg cac ggc agc aag ttt tgg acc cac ttc tcc ggc ggc tta aac      1900
   Phe Ala His Gly Ser Lys Phe Trp Thr His Phe Ser Gly Gly Leu Asn
       410                 415                 420
   tat caa gtc gtc cac cac ctc ttc ccg ggc gtg tgt cac gtg cac tac      1948
   Tyr Gln Val Val His His Leu Phe Pro Gly Val Cys His Val His Tyr
   425                 430                 435                 440
   ccc gcg ctc gcg ccg atc atc aag gct gcc gcg gat aag cac gga tta      1996
   Pro Ala Leu Ala Pro Ile Ile Lys Ala Ala Ala Asp Lys His Gly Leu
                   445                 450                 455
   cac tat caa atc tac ccc acg ttt tgg tcc gcg ctg cgc gcg cac ttc      2044
   His Tyr Gln Ile Tyr Pro Thr Phe Trp Ser Ala Leu Arg Ala His Phe
               460                 465                 470
   tcc cac ctc gcc cgc gtc ggc cac gag gcg tac gtg cct tcc ctc cga      2092
   Ser His Leu Ala Arg Val Gly His Glu Ala Tyr Val Pro Ser Leu Arg
           475                 480                 485
   acc gtc ggg tga gcgcgcctcg cgcgcggcca tttattcata cctttacctc          2144
   Thr Val Gly
       490
   accccgacgc gtcgatcgct cgccggcgct cccatcgcat ttattttaat ctccacgtct    2204
   tccgtccatc cctcgcgcct tcgcctcgcg ctcgcgctcg cctcgcctcg cccgggcttc 2264
   ctccccactc cgccgcgcgc cgtcgcgcgc gcg 2297
<210> 10
   <211> 491
   <212> PRT
   <213> Ostreococcus lucimarinus
<400> 10
   Met Thr Thr Val Ala Glu Ile Val Asp Asp Asp Ala Arg Arg Ala Gly
   1               5                   10                  15
   Lys Gly Ala Thr Thr Arg Arg Arg Arg Ala Thr Thr Arg Val Ala Arg
               20                  25                  30
   Asp Asp Ala Arg Glu Ala Thr Tyr Thr Ala Ala Glu Val Ala Arg His
           35                  40                  45
   Ala Arg Ala Asp Asp Cys Trp Val Ile Val Arg Gly Gly Val Tyr Asp
       50                  55                  60
   Val Thr Arg Phe Val Pro Arg His Pro Gly Gly Asn Met Ile Tyr Val
   65                  70                  75                  80
   Lys Ala Gly Gly Glu Cys Thr Ala Leu Phe Asp Ser Tyr His Pro Glu
                   85                  90                  95
   Arg Ala Arg Ala Thr Leu Glu Lys Tyr Arg Ile Gly Ala Leu Arg Arg
               100                 105                 110
   Asp Ala Gly Glu Arg Glu Asp Glu Asp Val Val Glu Tyr Leu Lys Asp
           115                 120                 125
   Asp Leu Arg Glu Gly Glu Phe Tyr Ala Asp Cys Lys Ala Gly Ala Ala
       130                 135                 140
   Lys Tyr Phe Lys Asp Asn Lys Leu Asp Pro Arg Val His Trp Glu Met
   145                 150                 155                 160
   Tyr Ala Lys Thr Leu Val Ile Leu Thr Gly Val Val Val Gly His Tyr
                   165                 170                 175
   Gly Ser Phe Phe Ala Pro Ser Ala Ser Phe Ala Ala Ala Leu Ala Leu
               180                 185                 190
   Ala Val Leu His Gly Thr Cys Lys Ala Glu Val Gly Val Ser Ile Gln
           195                 200                 205
   His Asp Ala Asn His Gly Ala Tyr Gly Asn Asn Arg Thr Trp Leu His
       210                 215                 220
   Ala Met Gln Leu Thr Leu Asp Ala Val Gly Ala Ser Ser Phe Met Trp
   225                 230                 235                 240
   Lys Gln Gln His Val Ala Gly His His Ala Tyr Thr Asn Val Glu Gly
                   245                 250                 255
   Ile Asp Pro Asp Ile Arg Cys Ser Glu Lys Asp Val Arg Arg Val Asn
               260                 265                 270
   Glu His Gln Pro His Glu Pro Tyr His Arg Val Gln His Val Tyr Leu
           275                 280                 285
   Ala Leu Met Tyr Gly Leu Leu Ser Phe Lys Ser Cys Phe Val Asp Asp
       290                 295                 300
   Phe Asn Ala Phe Phe Ser Gly Arg Ile Gly Trp Val Lys Val Met Lys
   305                 310                 315                 320
   Phe Thr Arg Gly Glu Ala Val Ala Phe Trp Gly Ser Lys Leu Ala Trp
                   325                 330                 335
   Ala Phe Tyr Tyr Leu Tyr Leu Pro Ala Lys Tyr Ser His Arg Ser Ile
               340                 345                 350
   Gly Gln Leu Leu Ala Leu Trp Thr Val Thr Glu Phe Val Thr Gly Trp
           355                 360                 365
   Leu Leu Ala Phe Met Phe Gln Val Ala His Val Val Gly Asp Val His
       370                 375                 380
   Phe Phe Arg Leu Asn Glu Lys Asn Gln Leu Asn Lys Gly Trp Gly Glu
   385                 390                 395                 400
   Ala Gln Leu Met Thr Ser Ala Asp Phe Ala His Gly Ser Lys Phe Trp
                   405                 410                 415
   Thr His Phe Ser Gly Gly Leu Asn Tyr Gln Val Val His His Leu Phe
               420                 425                 430
   Pro Gly Val Cys His Val His Tyr Pro Ala Leu Ala Pro Ile Ile Lys
           435                 440                 445
   Ala Ala Ala Asp Lys His Gly Leu His Tyr Gln Ile Tyr Pro Thr Phe
       450                 455                 460
   Trp Ser Ala Leu Arg Ala His Phe Ser His Leu Ala Arg Val Gly His
   465                 470                 475                 480
   Glu Ala Tyr Val Pro Ser Leu Arg Thr Val Gly
                   485                 490
<210> 11
   <211> 1412
   <212> DNA
   <213> Ostreococcus lucimarinus
<220>
   <223> Delta-5 elongase
<220>
   <221> CDS
   <222> (254)..(1150)
<400> 11
   cgcgcgcgca ctttttcgcc caccgcgcgc cgtcgcgcac tgcgccgtcg tcgcgcgtcg      60
   cgcgaacgcc gacatgtcaa acgttacgtt cgccgccgtc tcgcgcgcgt cgctccgtct     120
   ctctcgcgcg cgtcgtgcgt cgagcgccgc gaaatgtcgc cgagcgtgcg ctatccgttc     180
   gccaccgcga catcgtcgcc actgacgcgc gcgtcgcccg tcgctcgtcg ctcgcgcgcg     240
   cgcacgcgga gac atg gcg cag ttt ccg ctc gtt tcg ctg tgt gct ttc        289
                  Met Ala Gln Phe Pro Leu Val Ser Leu Cys Ala Phe
                  1               5                   10
   gcg gtg tac ggc tac gcg acg tac gcg tac gcg ttc gaa tgg tcg cac       337
   Ala Val Tyr Gly Tyr Ala Thr Tyr Ala Tyr Ala Phe Glu Trp Ser His
           15                  20                  25
   gcg cgc acg ccc gga ggt ttg gcg aac gtc gac gcg cag cga tgg atc       385
   Ala Arg Thr Pro Gly Gly Leu Ala Asn Val Asp Ala Gln Arg Trp Ile
       30                  35                  40
   ggt gat ctc tcg ttc gcg ctc ccg gcg tgc gcg acg acg gcg tac ttg       433
   Gly Asp Leu Ser Phe Ala Leu Pro Ala Cys Ala Thr Thr Ala Tyr Leu
   45                  50                  55                  60
   atg ttt tgc ctc gtc ggg ccg cgc gtg atg gcg aag cga gag gcg ttc       481
   Met Phe Cys Leu Val Gly Pro Arg Val Met Ala Lys Arg Glu Ala Phe
                   65                  70                  75
   gat ccg aaa ggg ctc atg ctg gcg tac aac gcg tat cag acg gcg ttc       529
   Asp Pro Lys Gly Leu Met Leu Ala Tyr Asn Ala Tyr Gln Thr Ala Phe
               80                  85                  90
   aac gtg tgc gtg ctc ggg atg ttt att cgg gag atc gtg acg ctg aaa       577
   Asn Val Cys Val Leu Gly Met Phe Ile Arg Glu Ile Val Thr Leu Lys
           95                  100                 105
   caa ccg acg tgg ggg tcg aag atg ccg tgg agc gat aaa cgg tcg ttt       625
   Gln Pro Thr Trp Gly Ser Lys Met Pro Trp Ser Asp Lys Arg Ser Phe
       110                 115                 120
   aac atc ttg ctc ggg gtg tgg ttt cat tac aac aac aag tat ttg gaa       673
   Asn Ile Leu Leu Gly Val Trp Phe His Tyr Asn Asn Lys Tyr Leu Glu
   125                 130                 135                 140
   ttg ctg gac acg gcg ttt atg att gcc cgc aag aag acg aat caa ctg       721
   Leu Leu Asp Thr Ala Phe Met Ile Ala Arg Lys Lys Thr Asn Gln Leu
                   145                 150                 155
   agc ttt ttg cac gtg tat cat cac gca ctg ttg att tgg gca tgg tgg       769
   Ser Phe Leu His Val Tyr His His Ala Leu Leu Ile Trp Ala Trp Trp
               160                 165                 170
   ttc gtg tgt cat ctg atg gcg acg aac gat tgc gtc gac gcg tac ttt       817
   Phe Val Cys His Leu Met Ala Thr Asn Asp Cys Val Asp Ala Tyr Phe
                   175                     180                     185
   ggt gcg gcg tgt aac tcg ttc att cac atc gtg atg tac tcg tac tat       865
   Gly Ala Ala Cys Asn Ser Phe Ile His Ile Val Met Tyr Ser Tyr Tyr
       190                     195                     200
   tta atg gcg gcg ctc ggc gtg cga tgc ccc tgg aag cgt tac atc acg       913
   Leu Met Ala Ala Leu Gly Val Arg Cys Pro Trp Lys Arg Tyr Ile Thr
   205                     210                     215                 220
   cag gcg caa atg tta caa ttt gtc atc gtt ttc gtc cac gcc gtg ttc       961
   Gln Ala Gln Met Leu Gln Phe Val Ile Val Phe Val His Ala Val Phe
                   225                     230                     235
   gtc tta cga gaa aag cac tgc cca gtc tca tta cca tgg gcg caa atg      1009
   Val Leu Arg Glu Lys His Cys Pro Val Ser Leu Pro Trp Ala Gln Met
               240                     245                     250
   ttc gtc atg gcg aac atg ctc gtg tta ttc ggt aac ttc tac ctc aag      1057
   Phe Val Met Ala Asn Met Leu Val Leu Phe Gly Asn Phe Tyr Leu Lys
           255                     260                     265
   gcg tac gcc gcc aaa ccg tcg ggc aaa tcc tcg acg cgc gtc tcc gcc      1105
   Ala Tyr Ala Ala Lys Pro Ser Gly Lys Ser Ser Thr Arg Val Ser Ala
       270                     275                     280
   gcc aaa ccg gca acc agg cgc act cga agc cgt aag atc gat tga          1150
   Ala Lys Pro Ala Thr Arg Arg Thr Arg Ser Arg Lys Ile Asp
   285                     290                     295
   tagtcgcgta gtgtggtcgt ttcactagtc ttcgtattgt ttcttcgcaa taatattgga    1210
   atattggact tttcctgtac agatcgagcg ctcggccgcg ctgtttcgca acgaagcgcg    1270
   cgcgcgccca gcccgaagct tcagcgcgca tcgcgacatc gtcctcacat tcgtacgcgc    1330
   gacgttcacg cctcgtcgta aacgcgctcg atcgatcgaa agactttaaa cacgccgaac    1390
   gaaggcgcga acgagtccaa cg                                             1412
<210> 12
   <211> 298
   <212> PRT
   <213> Ostreococcus lucimarinus
<400> 12
   Met Ala Gln Phe Pro Leu Val Ser Leu Cys Ala Phe Ala Val Tyr Gly
   1               5                   10                  15
   Tyr Ala Thr Tyr Ala Tyr Ala Phe Glu Trp Ser His Ala Arg Thr Pro
               20                  25                  30
   Gly Gly Leu Ala Asn Val Asp Ala Gln Arg Trp Ile Gly Asp Leu Ser
           35                  40                  45
   Phe Ala Leu Pro Ala Cys Ala Thr Thr Ala Tyr Leu Met Phe Cys Leu
       50                  55                  60
   Val Gly Pro Arg Val Met Ala Lys Arg Glu Ala Phe Asp Pro Lys Gly
   65                  70                  75                  80
   Leu Met Leu Ala Tyr Asn Ala Tyr Gln Thr Ala Phe Asn Val Cys Val
                   85                  90                  95
   Leu Gly Met Phe Ile Arg Glu Ile Val Thr Leu Lys Gln Pro Thr Trp
               100                 105                 110
   Gly Ser Lys Met Pro Trp Ser Asp Lys Arg Ser Phe Asn Ile Leu Leu
           115                 120                 125
   Gly Val Trp Phe His Tyr Asn Asn Lys Tyr Leu Glu Leu Leu Asp Thr
       130                 135                 140
   Ala Phe Met Ile Ala Arg Lys Lys Thr Asn Gln Leu Ser Phe Leu His
   145                 150                 155                 160
   Val Tyr His His Ala Leu Leu Ile Trp Ala Trp Trp Phe Val Cys His
                   165                 170                 175
   Leu Met Ala Thr Asn Asp Cys Val Asp Ala Tyr Phe Gly Ala Ala Cys
               180                 185                 190
   Asn Ser Phe Ile His Ile Val Met Tyr Ser Tyr Tyr Leu Met Ala Ala
           195                 200                 205
   Leu Gly Val Arg Cys Pro Trp Lys Arg Tyr Ile Thr Gln Ala Gln Met
       210                 215                 220
   Leu Gln Phe Val Ile Val Phe Val His Ala Val Phe Val Leu Arg Glu
   225                 230                 235                 240
   Lys His Cys Pro Val Ser Leu Pro Trp Ala Gln Met Phe Val Met Ala
                   245                 250                 255
   Asn Met Leu Val Leu Phe Gly Asn Phe Tyr Leu Lys Ala Tyr Ala Ala
               260                 265                 270
   Lys Pro Ser Gly Lys Ser Ser Thr Arg Val Ser Ala Ala Lys Pro Ala
           275                 280                 285
   Thr Arg Arg Thr Arg Ser Arg Lys Ile Asp
       290                 295
<210> 13
   <211> 2077
   <212> DNA
   <213> Ostreococcus lucimarinus
<220>
   <223> Delta-6 desaturase
<220>
   <221> CDS
   <222> (388)..(1836)
<400> 13
   cgccgcgcgg cgacgttcaa cgtagcgaat tgaaacgaat tagcatgtgc gaataacgcg      60
   agagacgatg attcgatcgc gacgaccacc gcgtcgaccg cgcgcgcggt cgcgcgaggc     120
   gacattaaaa cgcgcgcgct cggcgctcgc gcgcgacgca gggagcacaa gatgatgaca     180
   ctggcggctc gctttatcgt cacatcgcgt cgtcgcgcgc gcgcggcggc gtccgtgacc     240
   gcgcgcaccg cgaaacaaac acgcgcgatc aaagtttcgc gcgcgccgca cgcctcgaag     300
   acgcgcggcg acgacgtcag acgcgttcaa agcgacgaat tccacaaaac tgatcgttcg     360
   ctttaaatcc tgattaaaac gcgagag atg tgc gtc gaa acg acc gaa ggc aca     414
                                 Met Cys Val Glu Thr Thr Glu Gly Thr
                                  1               5
   tcg cga acg atg gcg aac gaa cgc acg agc tcg tcg tcg tcg ctg agc       462
   Ser Arg Thr Met Ala Asn Glu Arg Thr Ser Ser Ser Ser Ser Leu Ser
    10                  15                  20                  25
   gaa ggc gga acg ccg acg gtg acg gtc ggg atg gga agc gaa gac gcg       510
   Glu Gly Gly Thr Pro Thr Val Thr Val Gly Met Gly Ser Glu Asp Ala
                    30                  35                  40
   ggg aag aag act cga aac gcg agc gtc acg gcg tgg acg aaa gag ttg       558
   Gly Lys Lys Thr Arg Asn Ala Ser Val Thr Ala Trp Thr Lys Glu Leu
                45                  50                  55
   gag ccg cac gcg atc gcg aag acg ttc gaa cgg cgg tac gtg acg atc       606
   Glu Pro His Ala Ile Ala Lys Thr Phe Glu Arg Arg Tyr Val Thr Ile
            60                  65                  70
   gaa ggc gtg gaa tac gat gtg acg gat ttt aag cat ccc gga gga tcg       654
   Glu Gly Val Glu Tyr Asp Val Thr Asp Phe Lys His Pro Gly Gly Ser
        75                  80                  85
   gtt att tat tac atg ctg tcg aac acg gga gcg gac gcg acg gag gct       702
   Val Ile Tyr Tyr Met Leu Ser Asn Thr Gly Ala Asp Ala Thr Glu Ala
    90                  95                 100                 105
   ttt aaa gag ttt cat tat cgg tcg aaa aag gcg cgc aag gcg ttg gcg       750
   Phe Lys Glu Phe His Tyr Arg Ser Lys Lys Ala Arg Lys Ala Leu Ala
                   110                 115                 120
   gcg ttg ccg cat aag cca gtg gac gcg gcg acg cgg gaa ccg atc gaa 798
   Ala Leu Pro His Lys Pro Val Asp Ala Ala Thr Arg Glu Pro Ile Glu
   125 130 135
   gat gag gcg atg ctg aag gat ttc gcg cag tgg cgc aag gaa ttg gag 846
   Asp Glu Ala Met Leu Lys Asp Phe Ala Gln Trp Arg Lys Glu Leu Glu
   140 145 150
   cgt gag gga ttt ttt aag ccc tcg ccg gcg cac gtg gcg tat cga ttc 894
   Arg Glu Gly Phe Phe Lys Pro Ser Pro Ala His Val Ala Tyr Arg Phe
   155 160 165
   gcc gag ctc gcg gcg atg ttc gcg ctc ggc acg gcg ttg atg cac gcg 942
   Ala Glu Leu Ala Ala Met Phe Ala Leu Gly Thr Ala Leu Met His Ala
   170 175 180 185
   cgt tgg cac gtc gct tcc gtg atc gtg tac tcg tgt ttc ttc ggc gcg 990
   Arg Trp His Val Ala Ser Val Ile Val Tyr Ser Cys Phe Phe Gly Ala
   190 195 200
   cga tgc ggt tgg gtg cag cac gag ggt ggg cac aat tcg ttg act gga 1038
   Arg Cys Gly Trp Val Gln His Glu Gly Gly His Asn Ser Leu Thr Gly
   205 210 215
   aac att tgg tgg gac aag cga atc caa gcc ttc gcc gcg ggg ttc ggc 1086
   Asn Ile Trp Trp Asp Lys Arg Ile Gln Ala Phe Ala Ala Gly Phe Gly
   220 225 230
   ttg gcg tcg agt ggc gac atg tgg aac aac atg cac aac aag cat cac 1134
   Leu Ala Ser Ser Gly Asp Met Trp Asn Asn Met His Asn Lys His His
   235 240 245
   gcg acg ccc caa aag gtg cga cac gat atg gat ctc gac acc act ccc 1182
   Ala Thr Pro Gln Lys Val Arg His Asp Met Asp Leu Asp Thr Thr Pro
   250 255 260 265
   acg gtg gcg ttc ttc aac tcc gcg gtt gaa gaa aat cgc ccg cgg gga 1230
   Thr Val Ala Phe Phe Asn Ser Ala Val Glu Glu Asn Arg Pro Arg Gly
   270 275 280
   ttc agt aag ttg tgg ttg cgc ctt caa gcg tgg acc ttc gtg ccc gtg 1278
   Phe Ser Lys Leu Trp Leu Arg Leu Gln Ala Trp Thr Phe Val Pro Val
   285 290 295
   acg tcc ggt atg gtt ttg ttc ttc tgg atg ttc gtc ttg cac ccg cgt 1326
   Thr Ser Gly Met Val Leu Phe Phe Trp Met Phe Val Leu His Pro Arg
   300 305 310
   aac gcg ctg cga cgc aaa agc ttc gaa gaa gcg gct tgg atg ttt tcc 1374
   Asn Ala Leu Arg Arg Lys Ser Phe Glu Glu Ala Ala Trp Met Phe Ser
   315 320 325
   gcg cac gtc att cgc acg gcg gtt atc aaa gcc gtc acc ggc tac tcc 1422
   Ala His Val Ile Arg Thr Ala Val Ile Lys Ala Val Thr Gly Tyr Ser
   330 335 340 345
   tgg atc gcc tcg tac ggc ttg ttc gcg gcg acg atg tgg gcg agc gga 1470
   Trp Ile Ala Ser Tyr Gly Leu Phe Ala Ala Thr Met Trp Ala Ser Gly
   350 355 360
   tgt tac ttg ttc gcg cac ttt tcc acg tct cac acg cac ttg gat gtc 1518
   Cys Tyr Leu Phe Ala His Phe Ser Thr Ser His Thr His Leu Asp Val
   365 370 375
   gtg ccg agc gat aaa cac ctc tcg tgg gtg cga tac gcc gtc gat cac 1566
   Val Pro Ser Asp Lys His Leu Ser Trp Val Arg Tyr Ala Val Asp His
   380 385 390
   acg atc gac atc aat ccg aac aac agc gtc gtc aac tgg ttg atg ggc 1614
   Thr Ile Asp Ile Asn Pro Asn Asn Ser Val Val Asn Trp Leu Met Gly
   395 400 405
   tac ttg aac tgc caa gtc atc cat cac ctg ttc ccg gat atg cct cag 1662
   Tyr Leu Asn Cys Gln Val Ile His His Leu Phe Pro Asp Met Pro Gln
   410 415 420 425
   ttc cgc caa ccc gaa gtc tcc cgc cga ttc gtc ccg ttt gcg aag aag 1710
   Phe Arg Gln Pro Glu Val Ser Arg Arg Phe Val Pro Phe Ala Lys Lys
   430 435 440
   tgg aac tta aac tac aag gtc ttg acg tat tat ggg gcc tgg aag gcg 1758
   Trp Asn Leu Asn Tyr Lys Val Leu Thr Tyr Tyr Gly Ala Trp Lys Ala
   445 450 455
   acg ttc ggc aac ttg aac gac gtc ggg aag cac tat tac gtg cac gga 1806
   Thr Phe Gly Asn Leu Asn Asp Val Gly Lys His Tyr Tyr Val His Gly
   460 465 470
   tct cag cgc gtc aaa tca aag tcg gcg tga gtttcgatga gctcttcgcc 1856
   Ser Gln Arg Val Lys Ser Lys Ser Ala
   475 480
   ctcgcgatgt cgcctcgcgc gacgttctct catcttatcc catcttgcat catctcgcgc 1916
   ggcacttcac tagctcgccg cgcgttatct ccaagattta ccgctttgtg aacgtttatt 1976
   ctagcaacct ttagggtttt tttcgcgaac gagacgtcaa cttgtctgcc gcccatcgca 2036
   acgttccgcc acaccacggc tcgccgacgc ccgctcgcgc g 2077
<210> 14
   <211> 482
   <212> PRT
   <213> Ostreococcus lucimarinus
<400> 14
   Met Cys Val Glu Thr Thr Glu Gly Thr Ser Arg Thr Met Ala Asn Glu
   1               5                   10                  15
   Arg Thr Ser Ser Ser Ser Ser Leu Ser Glu Gly Gly Thr Pro Thr Val
               20                  25                  30
   Thr Val Gly Met Gly Ser Glu Asp Ala Gly Lys Lys Thr Arg Asn Ala
           35                  40                  45
   Ser Val Thr Ala Trp Thr Lys Glu Leu Glu Pro His Ala Ile Ala Lys
       50                  55                  60
   Thr Phe Glu Arg Arg Tyr Val Thr Ile Glu Gly Val Glu Tyr Asp Val
   65                  70                  75                  80
   Thr Asp Phe Lys His Pro Gly Gly Ser Val Ile Tyr Tyr Met Leu Ser
                   85                  90                  95
   Asn Thr Gly Ala Asp Ala Thr Glu Ala Phe Lys Glu Phe His Tyr Arg
               100                 105                 110
   Ser Lys Lys Ala Arg Lys Ala Leu Ala Ala Leu Pro His Lys Pro Val
           115                 120                 125
   Asp Ala Ala Thr Arg Glu Pro Ile Glu Asp Glu Ala Met Leu Lys Asp
       130                 135                 140
   Phe Ala Gln Trp Arg Lys Glu Leu Glu Arg Glu Gly Phe Phe Lys Pro
   145                 150                 155                 160
   Ser Pro Ala His Val Ala Tyr Arg Phe Ala Glu Leu Ala Ala Met Phe
                   165                 170                 175
   Ala Leu Gly Thr Ala Leu Met His Ala Arg Trp His Val Ala Ser Val
               180                 185                 190
   Ile Val Tyr Ser Cys Phe Phe Gly Ala Arg Cys Gly Trp Val Gln His
           195                 200                 205
   Glu Gly Gly His Asn Ser Leu Thr Gly Asn Ile Trp Trp Asp Lys Arg
       210                 215                 220
   Ile Gln Ala Phe Ala Ala Gly Phe Gly Leu Ala Ser Ser Gly Asp Met
   225                 230                 235                 240
   Trp Asn Asn Met His Asn Lys His His Ala Thr Pro Gln Lys Val Arg
                   245                 250                 255
   His Asp Met Asp Leu Asp Thr Thr Pro Thr Val Ala Phe Phe Asn Ser
               260                 265                 270
   Ala Val Glu Glu Asn Arg Pro Arg Gly Phe Ser Lys Leu Trp Leu Arg
           275                 280                 285
   Leu Gln Ala Trp Thr Phe Val Pro Val Thr Ser Gly Met Val Leu Phe
       290                 295                 300
   Phe Trp Met Phe Val Leu His Pro Arg Asn Ala Leu Arg Arg Lys Ser
   305                 310                 315                 320
   Phe Glu Glu Ala Ala Trp Met Phe Ser Ala His Val Ile Arg Thr Ala
                   325                 330                 335
   Val Ile Lys Ala Val Thr Gly Tyr Ser Trp Ile Ala Ser Tyr Gly Leu
               340                 345                 350
   Phe Ala Ala Thr Met Trp Ala Ser Gly Cys Tyr Leu Phe Ala His Phe
           355                 360                 365
   Ser Thr Ser His Thr His Leu Asp Val Val Pro Ser Asp Lys His Leu
       370                 375                 380
   Ser Trp Val Arg Tyr Ala Val Asp His Thr Ile Asp Ile Asn Pro Asn
   385                 390                 395                 400
   Asn Ser Val Val Asn Trp Leu Met Gly Tyr Leu Asn Cys Gln Val Ile
                   405                 410                 415
   His His Leu Phe Pro Asp Met Pro Gln Phe Arg Gln Pro Glu Val Ser
               420                 425                 430
   Arg Arg Phe Val Pro Phe Ala Lys Lys Trp Asn Leu Asn Tyr Lys Val
           435                 440                 445
   Leu Thr Tyr Tyr Gly Ala Trp Lys Ala Thr Phe Gly Asn Leu Asn Asp
       450                 455                 460
   Val Gly Lys His Tyr Tyr Val His Gly Ser Gln Arg Val Lys Ser Lys
   465                 470                 475                 480
   Ser Ala
<210> 15
   <211> 864
   <212> DNA
   <213> Ostreococcus lucimarinus
<220>
   <223> Delta-6 elongase
<220>
   <221> CDS
   <222> (1)..(864)
<400> 15
   atg ttg cga cac tac tgg aag gcg tgg gac tca atc att tcg aaa gta      48
   Met Leu Arg His Tyr Trp Lys Ala Trp Asp Ser Ile Ile Ser Lys Val
   1               5                   10                  15
   gtt ttc tcc tgc gct gac tgg tta ggc tgg gag ctc gac ccg ctg agc      96
   Val Phe Ser Cys Ala Asp Trp Leu Gly Trp Glu Leu Asp Pro Leu Ser
               20                  25                  30
   ccc acc acc tca cat tta ccc gcc ata acc tca cca act cct ctg atc      144
   Pro Thr Thr Ser His Leu Pro Ala Ile Thr Ser Pro Thr Pro Leu Ile
           35                  40                  45
   acc agc ctt ctc gtg tac ttg gtc acg gtc gtt gta tcg tac cgc gtc      192
   Thr Ser Leu Leu Val Tyr Leu Val Thr Val Val Val Ser Tyr Arg Val
       50                  55                  60
   tta tcc gca aca aca aac aca aag att tgg gat ccc aca tgg tta aaa      240
   Leu Ser Ala Thr Thr Asn Thr Lys Ile Trp Asp Pro Thr Trp Leu Lys
   65                  70                  75                  80
   gca tcg gtg att tgc cac aat gca ttc ctc ata tta cta agt ttg tat      288
   Ala Ser Val Ile Cys His Asn Ala Phe Leu Ile Leu Leu Ser Leu Tyr
                   85                  90                  95
   atg tgc atc ggt tgt atc gtc gaa gca tac aaa agt gga tat aag cta      336
   Met Cys Ile Gly Cys Ile Val Glu Ala Tyr Lys Ser Gly Tyr Lys Leu
               100                 105                 110
   tgg gga aat aag ttc aac gtg aat gaa aag cag ctt gcg ttt tac atc      384
   Trp Gly Asn Lys Phe Asn Val Asn Glu Lys Gln Leu Ala Phe Tyr Ile
           115                 120                 125
   tac ctc ttt tac gtg agc aag ata tat gag ttc gtt gat aca ttc ata      432
   Tyr Leu Phe Tyr Val Ser Lys Ile Tyr Glu Phe Val Asp Thr Phe Ile
       130                 135                 140
   atg cta ctg aag aga aac tta cgc caa gta agc ttt ttg cat gtc tac      480
   Met Leu Leu Lys Arg Asn Leu Arg Gln Val Ser Phe Leu His Val Tyr
   145                 150                 155                 160
   cat cac agc act att tcg ttt ata tgg tgg atg atc gca cgt cga gct      528
   His His Ser Thr Ile Ser Phe Ile Trp Trp Met Ile Ala Arg Arg Ala
                   165                 170                 175
   ccg ggt ggg gat gct tac ttc agt gca gca ctg aac tct tgg gta cac      576
   Pro Gly Gly Asp Ala Tyr Phe Ser Ala Ala Leu Asn Ser Trp Val His
               180                 185                 190
   gtg tgc atg tac acg tac tat ctg cta tcg gct ctg atc ggg aaa aac      624
   Val Cys Met Tyr Thr Tyr Tyr Leu Leu Ser Ala Leu Ile Gly Lys Asn
           195                 200                 205
   aac gac aaa cgt gtt aag tac ctt tgg tgg ggt cga cat ttg acg cag      672
   Asn Asp Lys Arg Val Lys Tyr Leu Trp Trp Gly Arg His Leu Thr Gln
       210                 215                 220
   atg cag atg ctt caa ttt cta tgc aat ctg tta cag gcc gtg tac tgt      720
   Met Gln Met Leu Gln Phe Leu Cys Asn Leu Leu Gln Ala Val Tyr Cys
   225                 230                 235                 240
   gca tat ttt tca gaa tac ccg aag ttc ttg tca aag ata ctc ctt ttt       768
   Ala Tyr Phe Ser Glu Tyr Pro Lys Phe Leu Ser Lys Ile Leu Leu Phe
                   245                 250                 255
   tac atg att agc ctg ctg gcg ctg ttt gga cat ttc tat tac tcc aag       816
   Tyr Met Ile Ser Leu Leu Ala Leu Phe Gly His Phe Tyr Tyr Ser Lys
               260                 265                 270
   cac atc gcc acc gcg aag ctc agg aaa aaa cac acc aag aag gcg tga       864
   His Ile Ala Thr Ala Lys Leu Arg Lys Lys His Thr Lys Lys Ala
           275                 280                 285
<210> 16
   <211> 287
   <212> PRT
   <213> Ostreococcus lucimarinus
<400> 16
   Met Leu Arg His Tyr Trp Lys Ala Trp Asp Ser Ile Ile Ser Lys Val
   1               5                   10                  15
   Val Phe Ser Cys Ala Asp Trp Leu Gly Trp Glu Leu Asp Pro Leu Ser
               20                  25                  30
   Pro Thr Thr Ser His Leu Pro Ala Ile Thr Ser Pro Thr Pro Leu Ile
           35                  40                  45
   Thr Ser Leu Leu Val Tyr Leu Val Thr Val Val Val Ser Tyr Arg Val
       50                  55                  60
   Leu Ser Ala Thr Thr Asn Thr Lys Ile Trp Asp Pro Thr Trp Leu Lys
   65                  70                  75                  80
   Ala Ser Val Ile Cys His Asn Ala Phe Leu Ile Leu Leu Ser Leu Tyr
                   85                  90                  95
   Met Cys Ile Gly Cys Ile Val Glu Ala Tyr Lys Ser Gly Tyr Lys Leu
               100                 105                 110
   Trp Gly Asn Lys Phe Asn Val Asn Glu Lys Gln Leu Ala Phe Tyr Ile
           115                 120                 125
   Tyr Leu Phe Tyr Val Ser Lys Ile Tyr Glu Phe Val Asp Thr Phe Ile
       130                 135                 140
   Met Leu Leu Lys Arg Asn Leu Arg Gln Val Ser Phe Leu His Val Tyr
   145                 150                 155                 160
   His His Ser Thr Ile Ser Phe Ile Trp Trp Met Ile Ala Arg Arg Ala
                   165                 170                 175
   Pro Gly Gly Asp Ala Tyr Phe Ser Ala Ala Leu Asn Ser Trp Val His
               180                 185                 190
   Val Cys Met Tyr Thr Tyr Tyr Leu Leu Ser Ala Leu Ile Gly Lys Asn
           195                 200                 205
   Asn Asp Lys Arg Val Lys Tyr Leu Trp Trp Gly Arg His Leu Thr Gln
       210                 215                 220
   Met Gln Met Leu Gln Phe Leu Cys Asn Leu Leu Gln Ala Val Tyr Cys
   225                 230                 235                 240
   Ala Tyr Phe Ser Glu Tyr Pro Lys Phe Leu Ser Lys Ile Leu Leu Phe
                   245                 250                 255
   Tyr Met Ile Ser Leu Leu Ala Leu Phe Gly His Phe Tyr Tyr Ser Lys
               260                 265                 270
   His Ile Ala Thr Ala Lys Leu Arg Lys Lys His Thr Lys Lys Ala
           275                 280                 285
<210> 17
   <211> 903
   <212> DNA
   <213> Ostreococcus tauri
<220>
   <221> CDS
   <222> (1)..(903)
   <223> Delta-5-Elongase
<400> 17
   atg agc gcc tcc ggt gcg ctg ctg ccc gcg atc gcg ttc gcc gcg tac 48
   Met Ser Ala Ser Gly Ala Leu Leu Pro Ala Ile Ala Phe Ala Ala Tyr
   1 5 10 15
   gcg tac gcg acg tac gcc tac gcc ttt gag tgg tcg cac gcg aat ggc 96
   Ala Tyr Ala Thr Tyr Ala Tyr Ala Phe Glu Trp Ser His Ala Asn Gly
   20 25 30
   atc gac aac gtc gac gcg cgc gag tgg atc ggt gcg ctg tcg ttg agg 144
   Ile Asp Asn Val Asp Ala Arg Glu Trp Ile Gly Ala Leu Ser Leu Arg
   35 40 45
   ctc ccg gcg atc gcg acg acg atg tac ctg ttg ttc tgc ctg gtc gga 192
   Leu Pro Ala Ile Ala Thr Thr Met Tyr Leu Leu Phe Cys Leu Val Gly
   50 55 60
   ccg agg ttg atg gcg aag cgc gag gcg ttc gac ccg aag ggg ttc atg 240
   Pro Arg Leu Met Ala Lys Arg Glu Ala Phe Asp Pro Lys Gly Phe Met
   65 70 75 80
   ctg gcg tac aat gcg tat cag acg gcg ttc aac gtc gtc gtg ctc ggg 288
   Leu Ala Tyr Asn Ala Tyr Gln Thr Ala Phe Asn Val Val Val Leu Gly
   85 90 95
   atg ttc gcg cga gag atc tcg ggg ctg ggg cag ccc gtg tgg ggg tca 336
   Met Phe Ala Arg Glu Ile Ser Gly Leu Gly Gln Pro Val Trp Gly Ser
   100 105 110
   acc atg ccg tgg agc gat aga aaa tcg ttt aag atc ctc ctc ggg gtg 384
   Thr Met Pro Trp Ser Asp Arg Lys Ser Phe Lys Ile Leu Leu Gly Val
   115 120 125
   tgg ttg cac tac aac aac caa tat ttg gag cta ttg gac act gtg ttc 432
   Trp Leu His Tyr Asn Asn Gln Tyr Leu Glu Leu Leu Asp Thr Val Phe
   130 135 140
   atg gtt gcg cgc aag aag acg aag cag ttg agc ttc ttg cac gtt tat 480
   Met Val Ala Arg Lys Lys Thr Lys Gln Leu Ser Phe Leu His Val Tyr
   145 150 155 160
   cat cac gcc ctg ttg atc tgg gcg tgg tgg ttg gtg tgt cac ttg atg 528
   His His Ala Leu Leu Ile Trp Ala Trp Trp Leu Val Cys His Leu Met
   165 170 175
   gcc acg aac gat tgt atc gat gcc tac ttc ggc gcg gcg tgc aac tcg 576
   Ala Thr Asn Asp Cys Ile Asp Ala Tyr Phe Gly Ala Ala Cys Asn Ser
   180 185 190
   ttc att cac atc gtg atg tac tcg tat tat ctc atg tcg gcg ctc ggc 624
   Phe Ile His Ile Val Met Tyr Ser Tyr Tyr Leu Met Ser Ala Leu Gly
   195 200 205
   att cga tgc ccg tgg aag cga tac atc acc cag gct caa atg ctc caa 672
   Ile Arg Cys Pro Trp Lys Arg Tyr Ile Thr Gln Ala Gln Met Leu Gln
   210 215 220
   ttc gtc att gtc ttc gcg cac gcc gtg ttc gtg ctg cgt cag aag cac 720
   Phe Val Ile Val Phe Ala His Ala Val Phe Val Leu Arg Gln Lys His
   225 230 235 240
   tgc ccg gtc acc ctt cct tgg gcg caa atg ttc gtc atg acg aac atg      768
   Cys Pro Val Thr Leu Pro Trp Ala Gln Met Phe Val Met Thr Asn Met
                   245                 250                 255
   ctc gtg ctc ttc ggg aac ttc tac ctc aag gcg tac tcg aac aag tcg      816
   Leu Val Leu Phe Gly Asn Phe Tyr Leu Lys Ala Tyr Ser Asn Lys Ser
               260                 265                 270
   cgc ggc gac ggc gcg agt tcc gtg aaa cca gcc gag acc acg cgc gcg      864
   Arg Gly Asp Gly Ala Ser Ser Val Lys Pro Ala Glu Thr Thr Arg Ala
           275                 280                 285
   ccc agc gtg cga cgc acg cga tct cga aaa att gac taa                  903
   Pro Ser Val Arg Arg Thr Arg Ser Arg Lys Ile Asp
       290                 295                 300
<210> 18
   <211> 300
   <212> PRT
   <213> Ostreococcus tauri
<400> 18
   Met Ser Ala Ser Gly Ala Leu Leu Pro Ala Ile Ala Phe Ala Ala Tyr
   1               5                   10                  15
   Ala Tyr Ala Thr Tyr Ala Tyr Ala Phe Glu Trp Ser His Ala Asn Gly
               20                  25                  30
   Ile Asp Asn Val Asp Ala Arg Glu Trp Ile Gly Ala Leu Ser Leu Arg
           35                  40                  45
   Leu Pro Ala Ile Ala Thr Thr Met Tyr Leu Leu Phe Cys Leu Val Gly
       50                  55                  60
   Pro Arg Leu Met Ala Lys Arg Glu Ala Phe Asp Pro Lys Gly Phe Met
   65                  70                  75                  80
   Leu Ala Tyr Asn Ala Tyr Gln Thr Ala Phe Asn Val Val Val Leu Gly
                   85                  90                  95
   Met Phe Ala Arg Glu Ile Ser Gly Leu Gly Gln Pro Val Trp Gly Ser
               100                 105                 110
   Thr Met Pro Trp Ser Asp Arg Lys Ser Phe Lys Ile Leu Leu Gly Val
           115                 120                 125
   Trp Leu His Tyr Asn Asn Gln Tyr Leu Glu Leu Leu Asp Thr Val Phe
       130                 135                 140
   Met Val Ala Arg Lys Lys Thr Lys Gln Leu Ser Phe Leu His Val Tyr
   145                 150                 155                 160
   His His Ala Leu Leu Ile Trp Ala Trp Trp Leu Val Cys His Leu Met
                   165                 170                 175
   Ala Thr Asn Asp Cys Ile Asp Ala Tyr Phe Gly Ala Ala Cys Asn Ser
               180                 185                 190
   Phe Ile His Ile Val Met Tyr Ser Tyr Tyr Leu Met Ser Ala Leu Gly
           195                 200                 205
   Ile Arg Cys Pro Trp Lys Arg Tyr Ile Thr Gln Ala Gln Met Leu Gln
       210                 215                 220
   Phe Val Ile Val Phe Ala His Ala Val Phe Val Leu Arg Gln Lys His
   225                 230                 235                 240
   Cys Pro Val Thr Leu Pro Trp Ala Gln Met Phe Val Met Thr Asn Met
                   245                 250                 255
   Leu Val Leu Phe Gly Asn Phe Tyr Leu Lys Ala Tyr Ser Asn Lys Ser
               260                 265                 270
   Arg Gly Asp Gly Ala Ser Ser Val Lys Pro Ala Glu Thr Thr Arg Ala
           275                 280                 285
   Pro Ser Val Arg Arg Thr Arg Ser Arg Lys Ile Asp
       290                 295                 300
<210> 19
   <211> 879
   <212> DNA
   <213> Ostreococcus tauri
<220>
   <221> CDS
   <222> (1)..(879)
   <223> Delta-6-Elongase
<400> 19
   atg agt ggc tta cgt gca ccc aac ttt tta cac aga ttc tgg aca aag      48
   Met Ser Gly Leu Arg Ala Pro Asn Phe Leu His Arg Phe Trp Thr Lys
   1               5                   10                  15
   tgg gac tac gcg att tcc aaa gtc gtc ttc acg tgt gcc gac agt ttt      96
   Trp Asp Tyr Ala Ile Ser Lys Val Val Phe Thr Cys Ala Asp Ser Phe
               20                  25                  30
   cag tgg gac atc ggg cca gtg agt tcg agt acg gcg cat tta ccc gcc     144
   Gln Trp Asp Ile Gly Pro Val Ser Ser Ser Thr Ala His Leu Pro Ala
           35                  40                  45
   att gaa tcc cct acc cca ctg gtg act agc ctc ttg ttc tac tta gtc     192
   Ile Glu Ser Pro Thr Pro Leu Val Thr Ser Leu Leu Phe Tyr Leu Val
       50                  55                  60
   aca gtt ttc ttg tgg tat ggt cgt tta acc agg agt tca gac aag aaa     240
   Thr Val Phe Leu Trp Tyr Gly Arg Leu Thr Arg Ser Ser Asp Lys Lys
   65                  70                  75                  80
   att aga gag cct acg tgg tta aga aga ttc ata ata tgt cat aat gcg     288
   Ile Arg Glu Pro Thr Trp Leu Arg Arg Phe Ile Ile Cys His Asn Ala
                   85                  90                  95
   ttc ttg ata gtc ctc agt ctt tac atg tgc ctt ggt tgt gtg gcc caa     336
   Phe Leu Ile Val Leu Ser Leu Tyr Met Cys Leu Gly Cys Val Ala Gln
               100                 105                 110
   gcg tat cag aat gga tat act tta tgg ggt aat gaa ttc aag gcc acg      384
   Ala Tyr Gln Asn Gly Tyr Thr Leu Trp Gly Asn Glu Phe Lys Ala Thr
           115                 120                 125
   gaa act cag ctt gct ctc tac att tac att ttt tac gta agt aaa ata      432
   Glu Thr Gln Leu Ala Leu Tyr Ile Tyr Ile Phe Tyr Val Ser Lys Ile
       130                 135                 140
   tac gag ttt gta gat act tac att atg ctt ctc aag aat aac ttg cgg      480
   Tyr Glu Phe Val Asp Thr Tyr Ile Met Leu Leu Lys Asn Asn Leu Arg
   145                 150                 155                 160
   caa gta agt ttc cta cac att tat cac cac agc acg att tcc ttt att      528
   Gln Val Ser Phe Leu His Ile Tyr His His Ser Thr Ile Ser Phe Ile
                   165                 170                 175
   tgg tgg atc att gct cgg agg gct ccg ggt ggt gat gct tac ttc agc      576
   Trp Trp Ile Ile Ala Arg Arg Ala Pro Gly Gly Asp Ala Tyr Phe Ser
               180                 185                 190
   gcg gcc ttg aac tca tgg gta cac gtg tgc atg tac acc tat tat cta      624
   Ala Ala Leu Asn Ser Trp Val His Val Cys Met Tyr Thr Tyr Tyr Leu
           195                 200                 205
   tta tca acc ctt att gga aaa gaa gat cct aag cgt tcc aac tac ctt      672
   Leu Ser Thr Leu Ile Gly Lys Glu Asp Pro Lys Arg Ser Asn Tyr Leu
       210                 215                 220
   tgg tgg ggt cgc cac cta acg caa atg cag atg ctt cag ttt ttc ttc      720
   Trp Trp Gly Arg His Leu Thr Gln Met Gln Met Leu Gln Phe Phe Phe
   225                 230                 235                 240
   aac gta ctt caa gcg ttg tac tgc gct tcg ttc tct acg tat ccc aag      768
   Asn Val Leu Gln Ala Leu Tyr Cys Ala Ser Phe Ser Thr Tyr Pro Lys
                   245                 250                 255
   ttt ttg tcc aaa att ctg ctc gtc tat atg atg agc ctt ctc ggc ttg      816
   Phe Leu Ser Lys Ile Leu Leu Val Tyr Met Met Ser Leu Leu Gly Leu
               260                 265                 270
   ttt ggg cat ttc tac tat tcc aag cac ata gca gca gct aag ctc cag      864
   Phe Gly His Phe Tyr Tyr Ser Lys His Ile Ala Ala Ala Lys Leu Gln
           275                 280                 285
   aaa aaa cag cag tga                                                  879
   Lys Lys Gln Gln
       290
<210> 20
   <211> 292
   <212> PRT
   <213> Ostreococcus tauri
<400> 20
   Met Ser Gly Leu Arg Ala Pro Asn Phe Leu His Arg Phe Trp Thr Lys
   1               5                   10                  15
   Trp Asp Tyr Ala Ile Ser Lys Val Val Phe Thr Cys Ala Asp Ser Phe
               20                  25                  30
   Gln Trp Asp Ile Gly Pro Val Ser Ser Ser Thr Ala His Leu Pro Ala
           35                  40                  45
   Ile Glu Ser Pro Thr Pro Leu Val Thr Ser Leu Leu Phe Tyr Leu Val
       50                  55                  60
   Thr Val Phe Leu Trp Tyr Gly Arg Leu Thr Arg Ser Ser Asp Lys Lys
   65                  70                  75                  80
   Ile Arg Glu Pro Thr Trp Leu Arg Arg Phe Ile Ile Cys His Asn Ala
                   85                  90                  95
   Phe Leu Ile Val Leu Ser Leu Tyr Met Cys Leu Gly Cys Val Ala Gln
               100                 105                 110
   Ala Tyr Gln Asn Gly Tyr Thr Leu Trp Gly Asn Glu Phe Lys Ala Thr
           115                 120                 125
   Glu Thr Gln Leu Ala Leu Tyr Ile Tyr Ile Phe Tyr Val Ser Lys Ile
       130                 135                 140
   Tyr Glu Phe Val Asp Thr Tyr Ile Met Leu Leu Lys Asn Asn Leu Arg
   145                 150                 155                 160
   Gln Val Ser Phe Leu His Ile Tyr His His Ser Thr Ile Ser Phe Ile
                   165                 170                 175
   Trp Trp Ile Ile Ala Arg Arg Ala Pro Gly Gly Asp Ala Tyr Phe Ser
               180                 185                 190
   Ala Ala Leu Asn Ser Trp Val His Val Cys Met Tyr Thr Tyr Tyr Leu
           195                 200                 205
   Leu Ser Thr Leu Ile Gly Lys Glu Asp Pro Lys Arg Ser Asn Tyr Leu
       210                 215                 220
   Trp Trp Gly Arg His Leu Thr Gln Met Gln Met Leu Gln Phe Phe Phe
   225                 230                 235                 240
   Asn Val Leu Gln Ala Leu Tyr Cys Ala Ser Phe Ser Thr Tyr Pro Lys
                   245                 250                 255
   Phe Leu Ser Lys Ile Leu Leu Val Tyr Met Met Ser Leu Leu Gly Leu
               260                 265                 270
   Phe Gly His Phe Tyr Tyr Ser Lys His Ile Ala Ala Ala Lys Leu Gln
           275                 280                 285
   Lys Lys Gln Gln
       290
<210> 21
   <211> 879
   <212> DNA
   <213> Ostreococcus tauri
<220>
   <221> CDS
   <222> (1)..(879)
   <223> Delta-6-Elongase
<400> 21
   atg agt ggc tta cgt gca ccc aac ttt tta cac aga ttc tgg aca aag     48
   Met Ser Gly Leu Arg Ala Pro Asn Phe Leu His Arg Phe Trp Thr Lys
   1               5                   10                  15
   tgg gac tac gcg att tcc aaa gtc gtc ttc acg tgt gcc gac agt ttt     96
   Trp Asp Tyr Ala Ile Ser Lys Val Val Phe Thr Cys Ala Asp Ser Phe
               20                  25                  30
   cag tgg gac atc ggg cca gtg agt tcg agt acg gcg cat tta ccc gcc     144
   Gln Trp Asp Ile Gly Pro Val Ser Ser Ser Thr Ala His Leu Pro Ala
           35                  40                  45
   att gaa tcc cct acc cca ctg gtg act agc ctc ttg ttc tac tta gtc     192
   Ile Glu Ser Pro Thr Pro Leu Val Thr Ser Leu Leu Phe Tyr Leu Val
       50                  55                  60
   aca gtt ttc ttg tgg tat ggt cgt tta acc agg agt tca gac aag aaa     240
   Thr Val Phe Leu Trp Tyr Gly Arg Leu Thr Arg Ser Ser Asp Lys Lys
   65                  70                  75                  80
   att aga gag cct acg tgg tta aga aga ttc ata ata tgt cat aat gcg     288
   Ile Arg Glu Pro Thr Trp Leu Arg Arg Phe Ile Ile Cys His Asn Ala
                   85                  90                  95
   ttc ttg ata gtc ctc agt ctt tac atg tgc ctt ggt tgt gtg gcc caa     336
   Phe Leu Ile Val Leu Ser Leu Tyr Met Cys Leu Gly Cys Val Ala Gln
               100                 105                 110
   gcg tat cag aat gga tat act tta tgg ggt aat gaa ttc aag gcc acg     384
   Ala Tyr Gln Asn Gly Tyr Thr Leu Trp Gly Asn Glu Phe Lys Ala Thr
           115                 120                 125
   gaa act cag ctt gct ctc tac att tac att ttt tac gta agt aaa ata     432
   Glu Thr Gln Leu Ala Leu Tyr Ile Tyr Ile Phe Tyr Val Ser Lys Ile
       130                 135                 140
   tac gag ttt gta gat act tac att atg ctt ctc aag aat aac ttg cgg     480
   Tyr Glu Phe Val Asp Thr Tyr Ile Met Leu Leu Lys Asn Asn Leu Arg
   145                 150                 155                 160
   caa gta aga ttc cta cac act tat cac cac agc acg att tcc ttt att     528
   Gln Val Arg Phe Leu His Thr Tyr His His Ser Thr Ile Ser Phe Ile
                   165                 170                 175
   tgg tgg atc att gct cgg agg gct ccg ggt ggt gat gct tac ttc agc     576
   Trp Trp Ile Ile Ala Arg Arg Ala Pro Gly Gly Asp Ala Tyr Phe Ser
               180                 185                 190
   gcg gcc ttg aac tca tgg gta cac gtg tgc atg tac acc tat tat cta     624
   Ala Ala Leu Asn Ser Trp Val His Val Cys Met Tyr Thr Tyr Tyr Leu
           195                 200                 205
   tta tca acc ctt att gga aaa gaa gat cct aag cgt tcc aac tac ctt     672
   Leu Ser Thr Leu Ile Gly Lys Glu Asp Pro Lys Arg Ser Asn Tyr Leu
       210                 215                 220
   tgg tgg ggt cgc cac cta acg caa atg cag atg ctt cag ttt ttc ttc     720
   Trp Trp Gly Arg His Leu Thr Gln Met Gln Met Leu Gln Phe Phe Phe
   225                 230                 235                 240
   aac gta ctt caa gcg ttg tac tgc gct tcg ttc tct acg tat ccc aag     768
   Asn Val Leu Gln Ala Leu Tyr Cys Ala Ser Phe Ser Thr Tyr Pro Lys
                   245                 250                 255
   ttt ttg tcc aaa att ctg ctc gtc tat atg atg agc ctt ctc ggc ttg     816
   Phe Leu Ser Lys Ile Leu Leu Val Tyr Met Met Ser Leu Leu Gly Leu
                   260                      265                      270
   ttt ggg cat ttc tac tat tcc aag cac ata gca gca gct aag ctc cag       864
   Phe Gly His Phe Tyr Tyr Ser Lys His Ile Ala Ala Ala Lys Leu Gln
           275                     280                     285
   aaa aaa cag cag tga                                                   879
   Lys Lys Gln Gln
       290
<210> 22
   <211> 292
   <212> PRT
   <213> Ostreococcus tauri
<400> 22
   Met Ser Gly Leu Arg Ala Pro Asn Phe Leu His Arg Phe Trp Thr Lys
   1               5                   10                  15
   Trp Asp Tyr Ala Ile Ser Lys Val Val Phe Thr Cys Ala Asp Ser Phe
               20                  25                  30
   Gln Trp Asp Ile Gly Pro Val Ser Ser Ser Thr Ala His Leu Pro Ala
           35                  40                  45
   Ile Glu Ser Pro Thr Pro Leu Val Thr Ser Leu Leu Phe Tyr Leu Val
       50                  55                  60
   Thr Val Phe Leu Trp Tyr Gly Arg Leu Thr Arg Ser Ser Asp Lys Lys
   65                  70                  75                  80
   Ile Arg Glu Pro Thr Trp Leu Arg Arg Phe Ile Ile Cys His Asn Ala
                   85                  90                  95
   Phe Leu Ile Val Leu Ser Leu Tyr Met Cys Leu Gly Cys Val Ala Gln
               100                 105                 110
   Ala Tyr Gln Asn Gly Tyr Thr Leu Trp Gly Asn Glu Phe Lys Ala Thr
           115                 120                 125
   Glu Thr Gln Leu Ala Leu Tyr Ile Tyr Ile Phe Tyr Val Ser Lys Ile
       130                 135                 140
   Tyr Glu Phe Val Asp Thr Tyr Ile Met Leu Leu Lys Asn Asn Leu Arg
   145                 150                 155                 160
   Gln Val Arg Phe Leu His Thr Tyr His His Ser Thr Ile Ser Phe Ile
                   165                 170                 175
   Trp Trp Ile Ile Ala Arg Arg Ala Pro Gly Gly Asp Ala Tyr Phe Ser
               180                 185                 190
   Ala Ala Leu Asn Ser Trp Val His Val Cys Met Tyr Thr Tyr Tyr Leu
           195                 200                 205
   Leu Ser Thr Leu Ile Gly Lys Glu Asp Pro Lys Arg Ser Asn Tyr Leu
       210                 215                 220
   Trp Trp Gly Arg His Leu Thr Gln Met Gln Met Leu Gln Phe Phe Phe
   225                 230                 235                 240
   Asn Val Leu Gln Ala Leu Tyr Cys Ala Ser Phe Ser Thr Tyr Pro Lys
                   245                 250                 255
   Phe Leu Ser Lys Ile Leu Leu Val Tyr Met Met Ser Leu Leu Gly Leu
               260                 265                 270
   Phe Gly His Phe Tyr Tyr Ser Lys His Ile Ala Ala Ala Lys Leu Gln
           275                 280                 285
   Lys Lys Gln Gln
       290
<210> 23
   <211> 1611
   <212> DNA
   <213> Ostreococcus tauri
<220>
   <221> CDS
   <222> (1)..(1611)
   <223> Delta-4-Desaturase
<400> 23
   atg tac ctc gga cgc ggc cgt ctc gag agc ggg acg acg cga ggg atg      48
   Met Tyr Leu Gly Arg Gly Arg Leu Glu Ser Gly Thr Thr Arg Gly Met
   1               5                   10                  15
   atg cgg acg cac gcg cgg cga ccg tcg acg acg tcg aat ccg tgc gcg      96
   Met Arg Thr His Ala Arg Arg Pro Ser Thr Thr Ser Asn Pro Cys Ala
               20                  25                  30
   cgg tca cgc gtg cgt aag acg acg gag cga tcg ctc gcg cga gtg cga     144
   Arg Ser Arg Val Arg Lys Thr Thr Glu Arg Ser Leu Ala Arg Val Arg
           35                  40                  45
   cga tcg acg agt gag aag gga agc gcg ctc gtg ctc gag cga gag agc     192
   Arg Ser Thr Ser Glu Lys Gly Ser Ala Leu Val Leu Glu Arg Glu Ser
       50                  55                  60
   gaa cgg gag aag gag gag gga ggg aaa gcg cga gcg gag gga ttg cga     240
   Glu Arg Glu Lys Glu Glu Gly Gly Lys Ala Arg Ala Glu Gly Leu Arg
   65                  70                  75                  80
   ttc caa cgc ccg gac gtc gcc gcg ccg ggg gga gcg gat cct tgg aac     288
   Phe Gln Arg Pro Asp Val Ala Ala Pro Gly Gly Ala Asp Pro Trp Asn
                   85                  90                  95
   gac gag aag tgg aca aag acc aag tgg acg gta ttc aga gac gtc gcg     336
   Asp Glu Lys Trp Thr Lys Thr Lys Trp Thr Val Phe Arg Asp Val Ala
               100                 105                 110
   tac gat ctc gat cct ttc ttc gct cga cac ccc gga gga gac tgg ctc     384
   Tyr Asp Leu Asp Pro Phe Phe Ala Arg His Pro Gly Gly Asp Trp Leu
           115                 120                 125
   ctg aac ttg gcc gtg gga cga gac tgc acc gcg ctc atc gaa tcc tat     432
   Leu Asn Leu Ala Val Gly Arg Asp Cys Thr Ala Leu Ile Glu Ser Tyr
       130                 135                 140
   cac ttg cga cca gag gtg gcg acg gct cgt ttc aga atg ctg ccc aaa 480
   His Leu Arg Pro Glu Val Ala Thr Ala Arg Phe Arg Met Leu Pro Lys
   145 150 155 160
   ctc gag gat ttt ccc gtc gag gcc gtg ccc aag tcc ccg aga ccg aac 528
   Leu Glu Asp Phe Pro Val Glu Ala Val Pro Lys Ser Pro Arg Pro Asn
   165 170 175
   gat tcg ccg tta tac aac aac att cgc aac cga gtc cgc gaa gag ctc 576
   Asp Ser Pro Leu Tyr Asn Asn Ile Arg Asn Arg Val Arg Glu Glu Leu
   180 185 190
   ttc cca gag gag gga aag aat atg cac aga cag ggc ggc gac cac ggc 624
   Phe Pro Glu Glu Gly Lys Asn Met His Arg Gln Gly Gly Asp His Gly
   195 200 205
   gac ggt gac gat tct ggg ttt cgc cgc ctt ttg ctt atg ccg tgt acc 672
   Asp Gly Asp Asp Ser Gly Phe Arg Arg Leu Leu Leu Met Pro Cys Thr
   210 215 220
   tat tcc ctt ccg ggg gtt cct ttc cgg ctg cct cct cgg gtc tcg cgg 720
   Tyr Ser Leu Pro Gly Val Pro Phe Arg Leu Pro Pro Arg Val Ser Arg
   225 230 235 240
   ggg cgt gga ttg gtc tca cga ttc agg cac tgc gcc aac cac ggc gcg 768
   Gly Arg Gly Leu Val Ser Arg Phe Arg His Cys Ala Asn His Gly Ala
   245 250 255
   atg tct cct tcg ccg gcc gtt aac ggc gtc ctc ggt ttg acg aac gat 816
   Met Ser Pro Ser Pro Ala Val Asn Gly Val Leu Gly Leu Thr Asn Asp
   260 265 270
   ctc atc ggc ggc tcg tcc ttg atg tgg aga tat cac cac caa gtc agc 864
   Leu Ile Gly Gly Ser Ser Leu Met Trp Arg Tyr His His Gln Val Ser
   275 280 285
   cac cac att cat tgc aac gac aac gcc atg gat caa gac gtg tac acg 912
   His His Ile His Cys Asn Asp Asn Ala Met Asp Gln Asp Val Tyr Thr
   290 295 300
   gcg atg cca tta ttg cgt ttc gac gct cgc cgg ccc aag tcc tgg tac 960
   Ala Met Pro Leu Leu Arg Phe Asp Ala Arg Arg Pro Lys Ser Trp Tyr
   305 310 315 320
   cat cgc ttc cag cag tgg tac atg ttt tta gcg ttc ccg ttg ttg cag 1008
   His Arg Phe Gln Gln Trp Tyr Met Phe Leu Ala Phe Pro Leu Leu Gln
   325 330 335
   gtt gcc ttc caa gtc gga gac att gcc gca ctg ttc acg cgt gat acc 1056
   Val Ala Phe Gln Val Gly Asp Ile Ala Ala Leu Phe Thr Arg Asp Thr
   340 345 350
   gaa ggc gct aag ctt cac ggg gcg acg acg tgg gag ctt acc acg gtt 1104
   Glu Gly Ala Lys Leu His Gly Ala Thr Thr Trp Glu Leu Thr Thr Val
   355 360 365
   gtc ctc ggt aag att gtg cac ttc ggt ctt ttg ttg ggg ccg ttg atg 1152
   Val Leu Gly Lys Ile Val His Phe Gly Leu Leu Leu Gly Pro Leu Met
   370 375 380
   aac cac gcg gtg agt tct gtt ttg ctg ggg atc gtc ggt ttc atg gcg 1200
   Asn His Ala Val Ser Ser Val Leu Leu Gly Ile Val Gly Phe Met Ala
   385 390 395 400
   tgc caa ggt ata gtt ctg gcg tgc acg ttt gct gtg agt cac aat gtc 1248
   Cys Gln Gly Ile Val Leu Ala Cys Thr Phe Ala Val Ser His Asn Val
   405 410 415
   gcg gag gcg aag ata cct gag gac acc gga gga gaa gcc tgg gag aga      1296
   Ala Glu Ala Lys Ile Pro Glu Asp Thr Gly Gly Glu Ala Trp Glu Arg
               420                 425                 430
   gat tgg ggt gtc cag cag ttg gtg act agc gcc gac tgg ggt gga aag      1344
   Asp Trp Gly Val Gln Gln Leu Val Thr Ser Ala Asp Trp Gly Gly Lys
           435                 440                 445
   ata ggt aac ttc ttc acg ggt ggc ctc aac ttg caa gtt gag cac cac      1392
   Ile Gly Asn Phe Phe Thr Gly Gly Leu Asn Leu Gln Val Glu His His
       450                 455                 460
   ttg ttt ccg gcg att tgc ttc gtc cac tac ccg gac atc gcg aag atc      1440
   Leu Phe Pro Ala Ile Cys Phe Val His Tyr Pro Asp Ile Ala Lys Ile
   465                 470                 475                 480
   gtg aag gaa gaa gcg gcc aag ctc aac atc cct tac gcg tct tac agg      1488
   Val Lys Glu Glu Ala Ala Lys Leu Asn Ile Pro Tyr Ala Ser Tyr Arg
                   485                 490                 495
   act ctt cct ggt att ttc gtc caa ttc tgg aga ttt atg aag gac atg      1536
   Thr Leu Pro Gly Ile Phe Val Gln Phe Trp Arg Phe Met Lys Asp Met
               500                 505                 510
   ggc acg gct gag caa att ggt gaa gtt cca ttg ccg aag att ccc aac      1584
   Gly Thr Ala Glu Gln Ile Gly Glu Val Pro Leu Pro Lys Ile Pro Asn
           515                 520                 525
   ccg cag ctc gcg ccg aag ctc gct tag                                  1611
   Pro Gln Leu Ala Pro Lys Leu Ala
       530                 535
<210> 24
   <211> 536
   <212> PRT
   <213> Ostreococcus tauri
<400> 24
   Met Tyr Leu Gly Arg Gly Arg Leu Glu Ser Gly Thr Thr Arg Gly Met
   1               5                   10                  15
   Met Arg Thr His Ala Arg Arg Pro Ser Thr Thr Ser Asn Pro Cys Ala
               20                  25                  30
   Arg Ser Arg Val Arg Lys Thr Thr Glu Arg Ser Leu Ala Arg Val Arg
           35                  40                  45
   Arg Ser Thr Ser Glu Lys Gly Ser Ala Leu Val Leu Glu Arg Glu Ser
       50                  55                  60
   Glu Arg Glu Lys Glu Glu Gly Gly Lys Ala Arg Ala Glu Gly Leu Arg
   65                  70                  75                  80
   Phe Gln Arg Pro Asp Val Ala Ala Pro Gly Gly Ala Asp Pro Trp Asn
                   85                  90                  95
   Asp Glu Lys Trp Thr Lys Thr Lys Trp Thr Val Phe Arg Asp Val Ala
               100                 105                 110
   Tyr Asp Leu Asp Pro Phe Phe Ala Arg His Pro Gly Gly Asp Trp Leu
           115                 120                 125
   Leu Asn Leu Ala Val Gly Arg Asp Cys Thr Ala Leu Ile Glu Ser Tyr
   130 135 140
   His Leu Arg Pro Glu Val Ala Thr Ala Arg Phe Arg Met Leu Pro Lys
   145 150 155 160
   Leu Glu Asp Phe Pro Val Glu Ala Val Pro Lys Ser Pro Arg Pro Asn
   165 170 175
   Asp Ser Pro Leu Tyr Asn Asn Ile Arg Asn Arg Val Arg Glu Glu Leu
   180 185 190
   Phe Pro Glu Glu Gly Lys Asn Met His Arg Gln Gly Gly Asp His Gly
   195 200 205
   Asp Gly Asp Asp Ser Gly Phe Arg Arg Leu Leu Leu Met Pro Cys Thr
   210 215 220
   Tyr Ser Leu Pro Gly Val Pro Phe Arg Leu Pro Pro Arg Val Ser Arg
   225 230 235 240
   Gly Arg Gly Leu Val Ser Arg Phe Arg His Cys Ala Asn His Gly Ala
   245 250 255
   Met Ser Pro Ser Pro Ala Val Asn Gly Val Leu Gly Leu Thr Asn Asp
   260 265 270
   Leu Ile Gly Gly Ser Ser Leu Met Trp Arg Tyr His His Gln Val Ser
   275 280 285
   His His Ile His Cys Asn Asp Asn Ala Met Asp Gln Asp Val Tyr Thr
   290 295 300
   Ala Met Pro Leu Leu Arg Phe Asp Ala Arg Arg Pro Lys Ser Trp Tyr
   305 310 315 320
   His Arg Phe Gln Gln Trp Tyr Met Phe Leu Ala Phe Pro Leu Leu Gln
   325 330 335
   Val Ala Phe Gln Val Gly Asp Ile Ala Ala Leu Phe Thr Arg Asp Thr
   340 345 350
   Glu Gly Ala Lys Leu His Gly Ala Thr Thr Trp Glu Leu Thr Thr Val
   355 360 365
   Val Leu Gly Lys Ile Val His Phe Gly Leu Leu Leu Gly Pro Leu Met
   370 375 380
   Asn His Ala Val Ser Ser Val Leu Leu Gly Ile Val Gly Phe Met Ala
   385 390 395 400
   Cys Gln Gly Ile Val Leu Ala Cys Thr Phe Ala Val Ser His Asn Val
   405 410 415
   Ala Glu Ala Lys Ile Pro Glu Asp Thr Gly Gly Glu Ala Trp Glu Arg
   420 425 430
   Asp Trp Gly Val Gln Gln Leu Val Thr Ser Ala Asp Trp Gly Gly Lys
   435 440 445
   Ile Gly Asn Phe Phe Thr Gly Gly Leu Asn Leu Gln Val Glu His His
   450 455 460
   Leu Phe Pro Ala Ile Cys Phe Val His Tyr Pro Asp Ile Ala Lys Ile
   465 470 475 480
   Val Lys Glu Glu Ala Ala Lys Leu Asn Ile Pro Tyr Ala Ser Tyr Arg
   485 490 495
   Thr Leu Pro Gly Ile Phe Val Gln Phe Trp Arg Phe Met Lys Asp Met
               500                 505                 510
   Gly Thr Ala Glu Gln Ile Gly Glu Val Pro Leu Pro Lys Ile Pro Asn
           515                 520                 525
   Pro Gln Leu Ala Pro Lys Leu Ala
       530                 535
<210> 25
   <211> 606
   <212> DNA
   <213> Ostreococcus tauri
<220>
   <221> CDS
   <222> (1)..(606)
   <223> Delta-5-Desaturase
<400> 25
   atg tac ggt ttg cta tcg ctc aag tcg tgc ttc gtc gac gat ttc aac      48
   Met Tyr Gly Leu Leu Ser Leu Lys Ser Cys Phe Val Asp Asp Phe Asn
   1               5                   10                  15
   gcc tac ttc tcc gga cgc atc ggc tgg gtc aag gtg atg aag ttc acc      96
   Ala Tyr Phe Ser Gly Arg Ile Gly Trp Val Lys Val Met Lys Phe Thr
               20                  25                  30
   cgc ggc gag gcg atc gca ttt tgg ggc acc aag ctc ttg tgg gcc gcg      144
   Arg Gly Glu Ala Ile Ala Phe Trp Gly Thr Lys Leu Leu Trp Ala Ala
           35                  40                  45
   tat tac ctc gcg ttg ccg cta aag atg tcg cat cgg ccg ctc gga gaa      192
   Tyr Tyr Leu Ala Leu Pro Leu Lys Met Ser His Arg Pro Leu Gly Glu
       50                  55                  60
   ctc ctc gca ctc tgg gcc gtc acc gag ttc gtc acc gga tgg ctg ttg      240
   Leu Leu Ala Leu Trp Ala Val Thr Glu Phe Val Thr Gly Trp Leu Leu
   65                  70                  75                  80
   gcg ttc atg ttc caa gtc gcc cac gtc gtc ggc gag gtt cac ttc ttc      288
   Ala Phe Met Phe Gln Val Ala His Val Val Gly Glu Val His Phe Phe
                   85                  90                  95
   acc ctc gac gcg aag aac cgc gtg aac ttg gga tgg gga gag gca cag      336
   Thr Leu Asp Ala Lys Asn Arg Val Asn Leu Gly Trp Gly Glu Ala Gln
               100                 105                 110
   ctc atg tcg agc gcg gat ttc gcc cac gga tcc aag ttt tgg acg cac      384
   Leu Met Ser Ser Ala Asp Phe Ala His Gly Ser Lys Phe Trp Thr His
           115                 120                 125
   ttc tcc gga ggc tta aac tac caa gtc gtc cac cat ctc ttc ccg ggc      432
   Phe Ser Gly Gly Leu Asn Tyr Gln Val Val His His Leu Phe Pro Gly
       130                 135                 140
   gtc tgc cac gtg cac tat ccc gcg ctc gcg cca att att aag gcg gca      480
   Val Cys His Val His Tyr Pro Ala Leu Ala Pro Ile Ile Lys Ala Ala
   145                 150                 155                 160
   gct gag aag cac ggc ctc cac tac cag att tac ccc acg ttt tgg tcc      528
   Ala Glu Lys His Gly Leu His Tyr Gln Ile Tyr Pro Thr Phe Trp Ser
                   165                 170                 175
   gcc ctg cgc gcg cac ttc cgg cac ctc gcc aac gtc ggc cgc gcc gcg      576
   Ala Leu Arg Ala His Phe Arg His Leu Ala Asn Val Gly Arg Ala Ala
               180                 185                 190
   tac gta ccg tcc ctc caa acc gtc gga tga                              606
   Tyr Val Pro Ser Leu Gln Thr Val Gly
           195                 200
<210> 26
   <211> 201
   <212> PRT
   <213> Ostreococcus tauri
<400> 26
   Met Tyr Gly Leu Leu Ser Leu Lys Ser Cys Phe Val Asp Asp Phe Asn
   1               5                   10                  15
   Ala Tyr Phe Ser Gly Arg Ile Gly Trp Val Lys Val Met Lys Phe Thr
               20                  25                  30
   Arg Gly Glu Ala Ile Ala Phe Trp Gly Thr Lys Leu Leu Trp Ala Ala
           35                  40                  45
   Tyr Tyr Leu Ala Leu Pro Leu Lys Met Ser His Arg Pro Leu Gly Glu
       50                  55                  60
   Leu Leu Ala Leu Trp Ala Val Thr Glu Phe Val Thr Gly Trp Leu Leu
   65                  70                  75                  80
   Ala Phe Met Phe Gln Val Ala His Val Val Gly Glu Val His Phe Phe
                   85                  90                  95
   Thr Leu Asp Ala Lys Asn Arg Val Asn Leu Gly Trp Gly Glu Ala Gln
               100                 105                 110
   Leu Met Ser Ser Ala Asp Phe Ala His Gly Ser Lys Phe Trp Thr His
           115                 120                 125
   Phe Ser Gly Gly Leu Asn Tyr Gln Val Val His His Leu Phe Pro Gly
       130                 135                 140
   Val Cys His Val His Tyr Pro Ala Leu Ala Pro Ile Ile Lys Ala Ala
   145                 150                 155                 160
   Ala Glu Lys His Gly Leu His Tyr Gln Ile Tyr Pro Thr Phe Trp Ser
                   165                 170                 175
   Ala Leu Arg Ala His Phe Arg His Leu Ala Asn Val Gly Arg Ala Ala
               180                 185                 190
   Tyr Val Pro Ser Leu Gln Thr Val Gly
           195                 200
<210> 27
   <211> 714
   <212> DNA
   <213> Ostreococcus tauri
<220>
   <221> CDS
   <222> (1)..(714)
   <223> Delta-5-Desaturase
<400> 27
   atg gtg agc cat cac tcg tac tgt aac gac gcg gat ttg gat cag gat       48
   Met Val Ser His His Ser Tyr Cys Asn Asp Ala Asp Leu Asp Gln Asp
   1               5                   10                  15
   gtg tac acc gca ctg ccg ctc ctg cgc ctg gac ccg tct cag gag ttg       96
   Val Tyr Thr Ala Leu Pro Leu Leu Arg Leu Asp Pro Ser Gln Glu Leu
               20                  25                  30
   aag tgg ttt cat cga tac cag gcg ttt tac gcc ccg ctc atg tgg ccg      144
   Lys Trp Phe His Arg Tyr Gln Ala Phe Tyr Ala Pro Leu Met Trp Pro
           35                  40                  45
   ttt ttg tgg ctc gcg gcg cag ttt ggc gac gcg cag aac atc ctg atc      192
   Phe Leu Trp Leu Ala Ala Gln Phe Gly Asp Ala Gln Asn Ile Leu Ile
       50                  55                  60
   gac cga gcg tcg ccg ggc gtc gcg tac aag gga ttg atg gcg aac gag      240
   Asp Arg Ala Ser Pro Gly Val Ala Tyr Lys Gly Leu Met Ala Asn Glu
   65                  70                  75                  80
   gtc gcg ctg tac gtt ctc ggt aag gtt tta cac ttt ggt ctt ctc ctc      288
   Val Ala Leu Tyr Val Leu Gly Lys Val Leu His Phe Gly Leu Leu Leu
                   85                  90                  95
   ggc gtt cct gcg tac ttg cac gga ttg tcc aac gcg atc gtt cca ttc      336
   Gly Val Pro Ala Tyr Leu His Gly Leu Ser Asn Ala Ile Val Pro Phe
               100                 105                 110
   ttg gcg tac ggc gca ttc ggc tcc ttc gtc ctg tgc tgg ttc ttc atc      384
   Leu Ala Tyr Gly Ala Phe Gly Ser Phe Val Leu Cys Trp Phe Phe Ile
           115                 120                 125
   gtc agc cat aac ctc gaa gcg ctg aca ccc gtt aac ctt aac aag tcc      432
   Val Ser His Asn Leu Glu Ala Leu Thr Pro Val Asn Leu Asn Lys Ser
       130                 135                 140
   acg aag aac gac tgg ggg gcg tgg cag atc gag aca tcg gcg tct tgg      480
   Thr Lys Asn Asp Trp Gly Ala Trp Gln Ile Glu Thr Ser Ala Ser Trp
   145                 150                 155                 160
   ggc aac gcg ttc tgg agc ttc ttc tct gga ggt ctg aac ctg caa atc      528
   Gly Asn Ala Phe Trp Ser Phe Phe Ser Gly Gly Leu Asn Leu Gln Ile
                   165                 170                 175
   gag cac cac ctc ttc ccg ggc atg gcg cac aac ctg tac ccg aag atg      576
   Glu His His Leu Phe Pro Gly Met Ala His Asn Leu Tyr Pro Lys Met
               180                 185                 190
   gtg ccg atc atc aag gac gag tgt gcg aaa gcg ggc gtt cgc tac acc       624
   Val Pro Ile Ile Lys Asp Glu Cys Ala Lys Ala Gly Val Arg Tyr Thr
           195                 200                 205
   ggt tac ggt ggc tac acc ggc ctg ctc ccg atc acc cgc gac atg ttc       672
   Gly Tyr Gly Gly Tyr Thr Gly Leu Leu Pro Ile Thr Arg Asp Met Phe
       210                 215                 220
   tcc tac ctc cat aag tgt ggc cga acg gcg aaa cta gcc taa               714
   Ser Tyr Leu His Lys Cys Gly Arg Thr Ala Lys Leu Ala
   225                 230                 235
<210> 28
   <211> 237
   <212> PRT
   <213> Ostreococcus tauri
<400> 28
   Met Val Ser His His Ser Tyr Cys Asn Asp Ala Asp Leu Asp Gln Asp
   1               5                   10                  15
   Val Tyr Thr Ala Leu Pro Leu Leu Arg Leu Asp Pro Ser Gln Glu Leu
               20                  25                  30
   Lys Trp Phe His Arg Tyr Gln Ala Phe Tyr Ala Pro Leu Met Trp Pro
           35                  40                  45
   Phe Leu Trp Leu Ala Ala Gln Phe Gly Asp Ala Gln Asn Ile Leu Ile
       50                  55                  60
   Asp Arg Ala Ser Pro Gly Val Ala Tyr Lys Gly Leu Met Ala Asn Glu
   65                  70                  75                  80
   Val Ala Leu Tyr Val Leu Gly Lys Val Leu His Phe Gly Leu Leu Leu
                   85                  90                  95
   Gly Val Pro Ala Tyr Leu His Gly Leu Ser Asn Ala Ile Val Pro Phe
               100                 105                 110
   Leu Ala Tyr Gly Ala Phe Gly Ser Phe Val Leu Cys Trp Phe Phe Ile
           115                 120                 125
   Val Ser His Asn Leu Glu Ala Leu Thr Pro Val Asn Leu Asn Lys Ser
       130                 135                 140
   Thr Lys Asn Asp Trp Gly Ala Trp Gln Ile Glu Thr Ser Ala Ser Trp
   145                 150                 155                 160
   Gly Asn Ala Phe Trp Ser Phe Phe Ser Gly Gly Leu Asn Leu Gln Ile
                   165                 170                 175
   Glu His His Leu Phe Pro Gly Met Ala His Asn Leu Tyr Pro Lys Met
               180                 185                 190
   Val Pro Ile Ile Lys Asp Glu Cys Ala Lys Ala Gly Val Arg Tyr Thr
           195                 200                 205
   Gly Tyr Gly Gly Tyr Thr Gly Leu Leu Pro Ile Thr Arg Asp Met Phe
       210                 215                 220
   Ser Tyr Leu His Lys Cys Gly Arg Thr Ala Lys Leu Ala
   225                 230                 235
<210> 29
   <211> 1371
   <212> DNA
   <213> Ostreococcus tauri
<220>
   <221> CDS
   <222> (1)..(1371)
   <223> Delta-6-Desaturase
<400> 29
   atg tgc gtg gag acg gaa aat aac gat ggg atc ccc acg gtg gag atc      48
   Met Cys Val Glu Thr Glu Asn Asn Asp Gly Ile Pro Thr Val Glu Ile
   1               5                   10                  15
   gcg ttc gac ggt gag cgc gag cgg gcg gag gca aac gtg aag ctg tcc      96
   Ala Phe Asp Gly Glu Arg Glu Arg Ala Glu Ala Asn Val Lys Leu Ser
               20                  25                  30
   gcg gag aag atg gag ccg gcg gcg ctg gcg aag acg ttc gcg agg cgg     144
   Ala Glu Lys Met Glu Pro Ala Ala Leu Ala Lys Thr Phe Ala Arg Arg
           35                  40                  45
   tac gtc gtg atc gag ggg gtg gag tac gat gtg acg gat ttt aag cac     192
   Tyr Val Val Ile Glu Gly Val Glu Tyr Asp Val Thr Asp Phe Lys His
       50                  55                  60
   ccg gga gga acg gtt att ttc tat gcg ttg tca aac acc ggg gcg gac     240
   Pro Gly Gly Thr Val Ile Phe Tyr Ala Leu Ser Asn Thr Gly Ala Asp
   65                  70                  75                  80
   gcg acg gaa gcg ttc aag gag ttt cat cat cgg tcg aga aag gcg agg     288
   Ala Thr Glu Ala Phe Lys Glu Phe His His Arg Ser Arg Lys Ala Arg
                   85                  90                  95
   aaa gcc ttg gcg gcg ctc ccg tct cga ccg gcc aag acg gcc aag gtg     336
   Lys Ala Leu Ala Ala Leu Pro Ser Arg Pro Ala Lys Thr Ala Lys Val
               100                 105                 110
   gac gac gcg gag atg ctc caa gat ttc gcc aag tgg cgg aaa gaa ttg     384
   Asp Asp Ala Glu Met Leu Gln Asp Phe Ala Lys Trp Arg Lys Glu Leu
           115                 120                 125
   gag aga gat gga ttc ttc aag ccc tct ccg gcg cac gtg gcg tat cgc     432
   Glu Arg Asp Gly Phe Phe Lys Pro Ser Pro Ala His Val Ala Tyr Arg
       130                 135                 140
   ttc gcc gag ctc gcg gcg atg tac gct ctc ggg acg tac ctg atg tac     480
   Phe Ala Glu Leu Ala Ala Met Tyr Ala Leu Gly Thr Tyr Leu Met Tyr
   145                 150                 155                 160
   gct cga tac gtc gtc tcc tcg gtg ctc gtg tac gct tgc ttt ttc ggc     528
   Ala Arg Tyr Val Val Ser Ser Val Leu Val Tyr Ala Cys Phe Phe Gly
                   165                 170                 175
   gcc cga tgc ggt tgg gtg cag cac gag ggc gga cac agc tcg ctg acg       576
   Ala Arg Cys Gly Trp Val Gln His Glu Gly Gly His Ser Ser Leu Thr
               180                 185                 190
   ggc aac att tgg tgg gac aag cgc atc cag gcc ttc aca gcc ggg ttc       624
   Gly Asn Ile Trp Trp Asp Lys Arg Ile Gln Ala Phe Thr Ala Gly Phe
           195                 200                 205
   ggt ctc gcc ggt agc ggc gac atg tgg aac tcg atg cac aac aag cat       672
   Gly Leu Ala Gly Ser Gly Asp Met Trp Asn Ser Met His Asn Lys His
       210                 215                 220
   cac gcg acg cct caa aag gtt cgt cac gac atg gat ctg gac acc acc       720
   His Ala Thr Pro Gln Lys Val Arg His Asp Met Asp Leu Asp Thr Thr
   225                 230                 235                 240
   ccc gcg gtg gcg ttc ttc aac acc gcg gtg gaa gac aat cgt ccc cgt       768
   Pro Ala Val Ala Phe Phe Asn Thr Ala Val Glu Asp Asn Arg Pro Arg
                   245                 250                 255
   ggc ttt agc aag tac tgg ttg cgc ctt cag gcg tgg acc ttc atc ccc       816
   Gly Phe Ser Lys Tyr Trp Leu Arg Leu Gln Ala Trp Thr Phe Ile Pro
               260                 265                 270
   gtg acg tcc ggc ttg gtg ctc ctt ttc tgg atg ttt ttc ctc cac ccc       864
   Val Thr Ser Gly Leu Val Leu Leu Phe Trp Met Phe Phe Leu His Pro
           275                 280                 285
   tcc aag gct ttg aag ggt ggc aag tac gaa gag ttg gtg tgg atg ctc       912
   Ser Lys Ala Leu Lys Gly Gly Lys Tyr Glu Glu Leu Val Trp Met Leu
       290                 295                 300
   gcc gcg cac gtc atc cgc acg tgg acg atc aag gcg gtg acc gga ttc       960
   Ala Ala His Val Ile Arg Thr Trp Thr Ile Lys Ala Val Thr Gly Phe
   305                 310                 315                 320
   acc gcg atg cag tcc tac ggc tta ttt ttg gcg acg agc tgg gtg agc      1008
   Thr Ala Met Gln Ser Tyr Gly Leu Phe Leu Ala Thr Ser Trp Val Ser
                   325                 330                 335
   ggc tgc tat ctg ttt gca cac ttc tcc acg tcg cac acg cac ctg gat      1056
   Gly Cys Tyr Leu Phe Ala His Phe Ser Thr Ser His Thr His Leu Asp
               340                 345                 350
   gtg gtg ccc gcg gac gag cat ctc tcc tgg gtt cga tac gcc gtc gat      1104
   Val Val Pro Ala Asp Glu His Leu Ser Trp Val Arg Tyr Ala Val Asp
           355                 360                 365
   cac acg atc gac atc gat ccg agt caa ggt tgg gtg aac tgg ttg atg      1152
   His Thr Ile Asp Ile Asp Pro Ser Gln Gly Trp Val Asn Trp Leu Met
       370                 375                 380
   ggc tac ctc aac tgc caa gtc atc cac cac ctc ttt ccg agc atg ccg      1200
   Gly Tyr Leu Asn Cys Gln Val Ile His His Leu Phe Pro Ser Met Pro
   385                 390                 395                 400
   cag ttc cgc cag ccc gag gta tct cgc cgc ttc gtc gcc ttt gcg aaa      1248
   Gln Phe Arg Gln Pro Glu Val Ser Arg Arg Phe Val Ala Phe Ala Lys
                   405                 410                 415
   aag tgg aac ctc aac tac aag gtc atg acc tac gcc ggt gcg tgg aag      1296
   Lys Trp Asn Leu Asn Tyr Lys Val Met Thr Tyr Ala Gly Ala Trp Lys
               420                 425                 430
   gca acg ctc gga aac ctc gac aac gtg ggt aag cac tac tac gtg cac      1344
   Ala Thr Leu Gly Asn Leu Asp Asn Val Gly Lys His Tyr Tyr Val His
           435                 440                     445
   ggc caa cac tcc gga aag acg gcg taa                                  1371
   Gly Gln His Ser Gly Lys Thr Ala
       450                 455
<210> 30
   <211> 456
   <212> PRT
   <213> Ostreococcus tauri
<400> 30
   Met Cys Val Glu Thr Glu Asn Asn Asp Gly Ile Pro Thr Val Glu Ile
   1               5                   10                  15
   Ala Phe Asp Gly Glu Arg Glu Arg Ala Glu Ala Asn Val Lys Leu Ser
               20                  25                  30
   Ala Glu Lys Met Glu Pro Ala Ala Leu Ala Lys Thr Phe Ala Arg Arg
           35                  40                  45
   Tyr Val Val Ile Glu Gly Val Glu Tyr Asp Val Thr Asp Phe Lys His
       50                  55                  60
   Pro Gly Gly Thr Val Ile Phe Tyr Ala Leu Ser Asn Thr Gly Ala Asp
   65                  70                  75                  80
   Ala Thr Glu Ala Phe Lys Glu Phe His His Arg Ser Arg Lys Ala Arg
                   85                  90                  95
   Lys Ala Leu Ala Ala Leu Pro Ser Arg Pro Ala Lys Thr Ala Lys Val
               100                 105                 110
   Asp Asp Ala Glu Met Leu Gln Asp Phe Ala Lys Trp Arg Lys Glu Leu
           115                 120                 125
   Glu Arg Asp Gly Phe Phe Lys Pro Ser Pro Ala His Val Ala Tyr Arg
       130                 135                 140
   Phe Ala Glu Leu Ala Ala Met Tyr Ala Leu Gly Thr Tyr Leu Met Tyr
   145                 150                 155                 160
   Ala Arg Tyr Val Val Ser Ser Val Leu Val Tyr Ala Cys Phe Phe Gly
                   165                 170                 175
   Ala Arg Cys Gly Trp Val Gln His Glu Gly Gly His Ser Ser Leu Thr
               180                 185                 190
   Gly Asn Ile Trp Trp Asp Lys Arg Ile Gln Ala Phe Thr Ala Gly Phe
           195                 200                 205
   Gly Leu Ala Gly Ser Gly Asp Met Trp Asn Ser Met His Asn Lys His
       210                 215                 220
   His Ala Thr Pro Gln Lys Val Arg His Asp Met Asp Leu Asp Thr Thr
   225                 230                 235                 240
   Pro Ala Val Ala Phe Phe Asn Thr Ala Val Glu Asp Asn Arg Pro Arg
                           245                 250                 255
   Gly Phe Ser Lys Tyr Trp Leu Arg Leu Gln Ala Trp Thr Phe Ile Pro
               260                 265                 270
   Val Thr Ser Gly Leu Val Leu Leu Phe Trp Met Phe Phe Leu His Pro
           275                 280                 285
   Ser Lys Ala Leu Lys Gly Gly Lys Tyr Glu Glu Leu Val Trp Met Leu
       290                 295                 300
   Ala Ala His Val Ile Arg Thr Trp Thr Ile Lys Ala Val Thr Gly Phe
   305                 310                 315                 320
   Thr Ala Met Gln Ser Tyr Gly Leu Phe Leu Ala Thr Ser Trp Val Ser
                   325                 330                 335
   Gly Cys Tyr Leu Phe Ala His Phe Ser Thr Ser His Thr His Leu Asp
               340                 345                 350
   Val Val Pro Ala Asp Glu His Leu Ser Trp Val Arg Tyr Ala Val Asp
           355                 360                 365
   His Thr Ile Asp Ile Asp Pro Ser Gln Gly Trp Val Asn Trp Leu Met
       370                 375                 380
   Gly Tyr Leu Asn Cys Gln Val Ile His His Leu Phe Pro Ser Met Pro
   385                 390                 395                 400
   Gln Phe Arg Gln Pro Glu Val Ser Arg Arg Phe Val Ala Phe Ala Lys
                   405                 410                 415
   Lys Trp Asn Leu Asn Tyr Lys Val Met Thr Tyr Ala Gly Ala Trp Lys
               420                 425                 430
   Ala Thr Leu Gly Asn Leu Asp Asn Val Gly Lys His Tyr Tyr Val His
           435                 440                 445
   Gly Gln His Ser Gly Lys Thr Ala
       450                 455
<210> 31
   <211> 1086
   <212> DNA
   <213> Ostreococcus tauri
<220>
   <221> CDS
   <222> (1)..(1086)
   <223> Delta-12-Desaturase
<400> 31
   atg cag gag ggg gtg cga aac att ccg aac gag tgc ttt gag acg gga       48
   Met Gln Glu Gly Val Arg Asn Ile Pro Asn Glu Cys Phe Glu Thr Gly
   1               5                   10                  15
   cat ctt gaa aga ccc tgg cgt tcc ggc cgg tgt ggg cgc gat ccc ggt       96
   His Leu Glu Arg Pro Trp Arg Ser Gly Arg Cys Gly Arg Asp Pro Gly
               20                  25                  30
   tcg aat tgg ggc gct ggc ttc cgc ttt ttt tcg ctc aag ggg ttt tgg      144
   Ser Asn Trp Gly Ala Gly Phe Arg Phe Phe Ser Leu Lys Gly Phe Trp
           35                  40                  45
   tgg ccg gcg tgg tgg gcg tac gcg ttc gtg acg ggg acg gcg gcc act      192
   Trp Pro Ala Trp Trp Ala Tyr Ala Phe Val Thr Gly Thr Ala Ala Thr
       50                  55                  60
   ggg tgt tgg gtc gcc gcg cac gag tgc ggg cac ggc gcg ttc agc gat      240
   Gly Cys Trp Val Ala Ala His Glu Cys Gly His Gly Ala Phe Ser Asp
   65                  70                  75                  80
   aac aag acg ttg caa gat gcg gtt gga tac gtg ttg cac tcg ttg ctc      288
   Asn Lys Thr Leu Gln Asp Ala Val Gly Tyr Val Leu His Ser Leu Leu
                   85                  90                  95
   ttg gtg ccg tac ttt tct tgg cag cga tca cac gcg gtg cat cac tcg      336
   Leu Val Pro Tyr Phe Ser Trp Gln Arg Ser His Ala Val His His Ser
               100                 105                 110
   agg acg aat cac gtt ctt gag ggc gag acg cac gtg ccg gcg cgc ttg      384
   Arg Thr Asn His Val Leu Glu Gly Glu Thr His Val Pro Ala Arg Leu
           115                 120                 125
   ggg acg gaa gac gcc aac gtc gtg ttc aag ctt cgc gaa ttg atc ggt      432
   Gly Thr Glu Asp Ala Asn Val Val Phe Lys Leu Arg Glu Leu Ile Gly
       130                 135                 140
   gaa ggg ccg ttc acg ttt ttc aac ctc gtc ggc gtc ttc gcg ctc gga      480
   Glu Gly Pro Phe Thr Phe Phe Asn Leu Val Gly Val Phe Ala Leu Gly
   145                 150                 155                 160
   tgg ccg att tac ttg ctc acc ggc gcg agc ggc gga ccg gtg cgc ggt      528
   Trp Pro Ile Tyr Leu Leu Thr Gly Ala Ser Gly Gly Pro Val Arg Gly
                   165                 170                 175
   aac acg aac cac ttc tta ccc ttc atg ggc gag aaa ggt aag cac gcg      576
   Asn Thr Asn His Phe Leu Pro Phe Met Gly Glu Lys Gly Lys His Ala
               180                 185                 190
   ctg ttc ccg ggt aag tgg gcg aag aag gtg tgg cag tct gac atc ggc      624
   Leu Phe Pro Gly Lys Trp Ala Lys Lys Val Trp Gln Ser Asp Ile Gly
           195                 200                 205
   gtt gtt gcc gtc ctg ggc gcg ctc gcg gct tgg gcg gcg cac agc ggg      672
   Val Val Ala Val Leu Gly Ala Leu Ala Ala Trp Ala Ala His Ser Gly
       210                 215                 220
   att gcc aca gtg atg gca ctc tac gtc ggc ccg tac atg gtg acc aac      720
   Ile Ala Thr Val Met Ala Leu Tyr Val Gly Pro Tyr Met Val Thr Asn
   225                 230                 235                 240
   ttt tgg ctc gtc ttg tac acg tgg tta cag cac acc gac gtt gac gtg      768
   Phe Trp Leu Val Leu Tyr Thr Trp Leu Gln His Thr Asp Val Asp Val
                   245                 250                 255
   ccg cac ttc gag ggc gac gat tgg aac ttg gtc aag ggg gca ttc atg      816
   Pro His Phe Glu Gly Asp Asp Trp Asn Leu Val Lys Gly Ala Phe Met
               260                 265                 270
   acg atc gat cgc ccg tac ggc cca gtt ttt gat ttc ttg cac cac cgc      864
   Thr Ile Asp Arg Pro Tyr Gly Pro Val Phe Asp Phe Leu His His Arg
           275                 280                 285
   atc ggc agc acg cac gtc gcg cac cac atc aac aca cca ttc ccg cat      912
   Ile Gly Ser Thr His Val Ala His His Ile Asn Thr Pro Phe Pro His
       290                 295                 300
   tac aag gct caa atg gcg acg gat gcg cta aag gag gcg tat ccc gac      960
   Tyr Lys Ala Gln Met Ala Thr Asp Ala Leu Lys Glu Ala Tyr Pro Asp
   305                 310                 315                 320
   ctc tac ctt tac gat cca act ccg atc gcg acc gct acg tgg cgc gtg     1008
   Leu Tyr Leu Tyr Asp Pro Thr Pro Ile Ala Thr Ala Thr Trp Arg Val
                   325                 330                 335
   ggg agc aag tgc atc gcc gtc gtg aag aag gga gac gaa tgg gtg ttc     1056
   Gly Ser Lys Cys Ile Ala Val Val Lys Lys Gly Asp Glu Trp Val Phe
               340                 345                 350
   acg gat aag caa ctc ccg gtc gcg gcg tga                             1086
   Thr Asp Lys Gln Leu Pro Val Ala Ala
           355                 360
<210> 32
   <211> 361
   <212> PRT
   <213> Ostreococcus tauri
<400> 32
   Met Gln Glu Gly Val Arg Asn Ile Pro Asn Glu Cys Phe Glu Thr Gly
   1               5                   10                  15
   His Leu Glu Arg Pro Trp Arg Ser Gly Arg Cys Gly Arg Asp Pro Gly
               20                  25                  30
   Ser Asn Trp Gly Ala Gly Phe Arg Phe Phe Ser Leu Lys Gly Phe Trp
           35                  40                  45
   Trp Pro Ala Trp Trp Ala Tyr Ala Phe Val Thr Gly Thr Ala Ala Thr
       50                  55                  60
   Gly Cys Trp Val Ala Ala His Glu Cys Gly His Gly Ala Phe Ser Asp
   65                  70                  75                  80
   Asn Lys Thr Leu Gln Asp Ala Val Gly Tyr Val Leu His Ser Leu Leu
                   85                  90                  95
   Leu Val Pro Tyr Phe Ser Trp Gln Arg Ser His Ala Val His His Ser
               100                 105                 110
   Arg Thr Asn His Val Leu Glu Gly Glu Thr His Val Pro Ala Arg Leu
           115                 120                 125
   Gly Thr Glu Asp Ala Asn Val Val Phe Lys Leu Arg Glu Leu Ile Gly
       130                 135                 140
   Glu Gly Pro Phe Thr Phe Phe Asn Leu Val Gly Val Phe Ala Leu Gly
   145                 150                 155                 160
   Trp Pro Ile Tyr Leu Leu Thr Gly Ala Ser Gly Gly Pro Val Arg Gly
                   165                 170                 175
   Asn Thr Asn His Phe Leu Pro Phe Met Gly Glu Lys Gly Lys His Ala
               180                 185                 190
   Leu Phe Pro Gly Lys Trp Ala Lys Lys Val Trp Gln Ser Asp Ile Gly
               195                     200                     205
   Val Val Ala Val Leu Gly Ala Leu Ala Ala Trp Ala Ala His Ser Gly
       210                     215                     220
   Ile Ala Thr Val Met Ala Leu Tyr Val Gly Pro Tyr Met Val Thr Asn
   225                 230                     235                 240
   Phe Trp Leu Val Leu Tyr Thr Trp Leu Gln His Thr Asp Val Asp Val
                   245                 250                     255
   Pro His Phe Glu Gly Asp Asp Trp Asn Leu Val Lys Gly Ala Phe Met
               260                 265                     270
   Thr Ile Asp Arg Pro Tyr Gly Pro Val Phe Asp Phe Leu His His Arg
           275                 280                     285
   Ile Gly Ser Thr His Val Ala His His Ile Asn Thr Pro Phe Pro His
       290                 295                     300
   Tyr Lys Ala Gln Met Ala Thr Asp Ala Leu Lys Glu Ala Tyr Pro Asp
   305                 310                     315                 320
   Leu Tyr Leu Tyr Asp Pro Thr Pro Ile Ala Thr Ala Thr Trp Arg Val
                   325                 330                     335
   Gly Ser Lys Cys Ile Ala Val Val Lys Lys Gly Asp Glu Trp Val Phe
               340                 345                     350
   Thr Asp Lys Gln Leu Pro Val Ala Ala
   355         360
<210> 33
   <211> 1077
   <212> DNA
   <213> Thalassiosira pseudonana
<220>
   <221> CDS
   <222> (1)..(1077)
   <223> Delta-5-Elongase
<400> 33
   atg tgc tca tca ccg ccg tca caa tcc aaa aca aca tcc ctc cta gca       48
   Met Cys Ser Ser Pro Pro Ser Gln Ser Lys Thr Thr Ser Leu Leu Ala
   1               5                   10                  15
   cgg tac acc acc gcc gcc ctc ctc ctc ctc acc ctc aca aca tgg tgc       96
   Arg Tyr Thr Thr Ala Ala Leu Leu Leu Leu Thr Leu Thr Thr Trp Cys
               20                  25                  30
   cac ttc gcc ttc cca gcc gcc acc gcc aca ccc ggc ctc acc gcc gaa      144
   His Phe Ala Phe Pro Ala Ala Thr Ala Thr Pro Gly Leu Thr Ala Glu
           35                  40                  45
   atg cac tcc tac aaa gtc cca ctc ggt ctc acc gta ttc tac ctg ctg      192
   Met His Ser Tyr Lys Val Pro Leu Gly Leu Thr Val Phe Tyr Leu Leu
       50                  55                  60
   agt cta ccg tca cta aag tac gtt acg gac aac tac ctt gcc aaa aag      240
   Ser Leu Pro Ser Leu Lys Tyr Val Thr Asp Asn Tyr Leu Ala Lys Lys
   65                  70                  75                  80
   tat gat atg aag tca ctc cta acg gaa tca atg gtg ttg tac aat gtg      288
   Tyr Asp Met Lys Ser Leu Leu Thr Glu Ser Met Val Leu Tyr Asn Val
                   85                  90                  95
   gcg caa gtg ctg ctc aat ggg tgg acg gtg tat gcg att gtg gat gcg      336
   Ala Gln Val Leu Leu Asn Gly Trp Thr Val Tyr Ala Ile Val Asp Ala
               100                 105                 110
   gtg atg aat aga gac cat ccg ttt att gga agt aga agt ttg gtt ggg      384
   Val Met Asn Arg Asp His Pro Phe Ile Gly Ser Arg Ser Leu Val Gly
           115                 120                 125
   gcg gcg ttg cat agt ggg agc tcg tat gcg gtg tgg gtt cat tat tgt      432
   Ala Ala Leu His Ser Gly Ser Ser Tyr Ala Val Trp Val His Tyr Cys
       130                 135                 140
   gat aag tat ttg gag ttc ttt gat acg tat ttt atg gtg ttg agg ggg      480
   Asp Lys Tyr Leu Glu Phe Phe Asp Thr Tyr Phe Met Val Leu Arg Gly
   145                 150                 155                 160
   aaa atg gac cag gtc tcc ttc ctc cac atc tac cac cac acg acc ata      528
   Lys Met Asp Gln Val Ser Phe Leu His Ile Tyr His His Thr Thr Ile
                   165                 170                 175
   gcg tgg gca tgg tgg atc gcc ctc cgc ttc tcc ccc ggt gga gac att      576
   Ala Trp Ala Trp Trp Ile Ala Leu Arg Phe Ser Pro Gly Gly Asp Ile
               180                 185                 190
   tac ttc ggg gca ctc ctc aac tcc atc atc cac gtc ctc atg tat tcc      624
   Tyr Phe Gly Ala Leu Leu Asn Ser Ile Ile His Val Leu Met Tyr Ser
           195                 200                 205
   tac tac gcc ctt gcc cta ctc aag gtc agt tgt cca tgg aaa cga tac      672
   Tyr Tyr Ala Leu Ala Leu Leu Lys Val Ser Cys Pro Trp Lys Arg Tyr
       210                 215                 220
   ctg act caa gct caa tta ttg caa ttc aca agt gtg gtg gtt tat acg      720
   Leu Thr Gln Ala Gln Leu Leu Gln Phe Thr Ser Val Val Val Tyr Thr
   225                 230                 235                 240
   ggg tgt acg ggt tat act cat tac tat cat acg aag cat gga gcg gat      768
   Gly Cys Thr Gly Tyr Thr His Tyr Tyr His Thr Lys His Gly Ala Asp
                   245                 250                 255
   gag aca cag cct agt tta gga acg tat tat ttc tgt tgt gga gtg cag      816
   Glu Thr Gln Pro Ser Leu Gly Thr Tyr Tyr Phe Cys Cys Gly Val Gln
               260                 265                 270
   gtg ttt gag atg gtt agt ttg ttt gta ctc ttt tcc atc ttt tat aaa      864
   Val Phe Glu Met Val Ser Leu Phe Val Leu Phe Ser Ile Phe Tyr Lys
           275                 280                 285
   cga tcc tat tcg aag aag aac aag tca gga gga aag gat agc aag aag      912
   Arg Ser Tyr Ser Lys Lys Asn Lys Ser Gly Gly Lys Asp Ser Lys Lys
       290                 295                 300
   aat gat gat ggg aat aat gag gat caa tgt cac aag gct atg aag gat      960
   Asn Asp Asp Gly Asn Asn Glu Asp Gln Cys His Lys Ala Met Lys Asp
   305                 310                 315                 320
   ata tcg gag ggt gcg aag gag gtt gtg ggg cat gca gcg aag gat gct     1008
   Ile Ser Glu Gly Ala Lys Glu Val Val Gly His Ala Ala Lys Asp Ala
                   325                 330                 335
   gga aag ttg gtg gct acg gcg agt aag gct gta aag agg aag gga act     1056
   Gly Lys Leu Val Ala Thr Ala Ser Lys Ala Val Lys Arg Lys Gly Thr
               340                 345                 350
   cgt gtt act ggt gcc atg tag                                         1077
   Arg Val Thr Gly Ala Met
           355
<210> 34
   <211> 358
   <212> PRT
   <213> Thalassiosira pseudonana
<400> 34
   Met Cys Ser Ser Pro Pro Ser Gln Ser Lys Thr Thr Ser Leu Leu Ala
   1               5                   10                  15
   Arg Tyr Thr Thr Ala Ala Leu Leu Leu Leu Thr Leu Thr Thr Trp Cys
               20                  25                  30
   His Phe Ala Phe Pro Ala Ala Thr Ala Thr Pro Gly Leu Thr Ala Glu
           35                  40                  45
   Met His Ser Tyr Lys Val Pro Leu Gly Leu Thr Val Phe Tyr Leu Leu
       50                  55                  60
   Ser Leu Pro Ser Leu Lys Tyr Val Thr Asp Asn Tyr Leu Ala Lys Lys
   65                  70                  75                  80
   Tyr Asp Met Lys Ser Leu Leu Thr Glu Ser Met Val Leu Tyr Asn Val
                   85                  90                  95
   Ala Gln Val Leu Leu Asn Gly Trp Thr Val Tyr Ala Ile Val Asp Ala
               100                 105                 110
   Val Met Asn Arg Asp His Pro Phe Ile Gly Ser Arg Ser Leu Val Gly
           115                 120                 125
   Ala Ala Leu His Ser Gly Ser Ser Tyr Ala Val Trp Val His Tyr Cys
       130                 135                 140
   Asp Lys Tyr Leu Glu Phe Phe Asp Thr Tyr Phe Met Val Leu Arg Gly
   145                 150                 155                 160
   Lys Met Asp Gln Val Ser Phe Leu His Ile Tyr His His Thr Thr Ile
                   165                 170                 175
   Ala Trp Ala Trp Trp Ile Ala Leu Arg Phe Ser Pro Gly Gly Asp Ile
               180                 185                 190
   Tyr Phe Gly Ala Leu Leu Asn Ser Ile Ile His Val Leu Met Tyr Ser
           195                 200                 205
   Tyr Tyr Ala Leu Ala Leu Leu Lys Val Ser Cys Pro Trp Lys Arg Tyr
       210                 215                 220
   Leu Thr Gln Ala Gln Leu Leu Gln Phe Thr Ser Val Val Val Tyr Thr
   225                 230                 235                 240
   Gly Cys Thr Gly Tyr Thr His Tyr Tyr His Thr Lys His Gly Ala Asp
                   245                 250                 255
   Glu Thr Gln Pro Ser Leu Gly Thr Tyr Tyr Phe Cys Cys Gly Val Gln
               260                 265                 270
   Val Phe Glu Met Val Ser Leu Phe Val Leu Phe Ser Ile Phe Tyr Lys
           275                 280                 285
   Arg Ser Tyr Ser Lys Lys Asn Lys Ser Gly Gly Lys Asp Ser Lys Lys
       290                 295                 300
   Asn Asp Asp Gly Asn Asn Glu Asp Gln Cys His Lys Ala Met Lys Asp
   305                 310                 315                 320
   Ile Ser Glu Gly Ala Lys Glu Val Val Gly His Ala Ala Lys Asp Ala
                   325                 330                 335
   Gly Lys Leu Val Ala Thr Ala Ser Lys Ala Val Lys Arg Lys Gly Thr
               340                 345                 350
   Arg Val Thr Gly Ala Met
           355
<210> 35
   <211> 819
   <212> DNA
   <213> Thalassiosira pseudonana
<220>
   <221> CDS
   <222> (1)..(819)
   <223> Delta-5-Elongase
<400> 35
   atg gac gcc tac aac gct gca atg gat aag atc ggt gcc gcc atc atc      48
   Met Asp Ala Tyr Asn Ala Ala Met Asp Lys Ile Gly Ala Ala Ile Ile
   1               5                   10                  15
   gat tgg tct gat ccc gat gga aag ttc cgt gcc gat aga gag gac tgg      96
   Asp Trp Ser Asp Pro Asp Gly Lys Phe Arg Ala Asp Arg Glu Asp Trp
               20                  25                  30
   tgg ctc tgc gac ttc cgt agc gcc atc acc atc gcc ctc atc tac atc     144
   Trp Leu Cys Asp Phe Arg Ser Ala Ile Thr Ile Ala Leu Ile Tyr Ile
           35                  40                  45
   gcc ttc gtc atc ctc ggt tcc gcc gtc atg caa tcc ctc ccc gca atg     192
   Ala Phe Val Ile Leu Gly Ser Ala Val Met Gln Ser Leu Pro Ala Met
       50                  55                  60
   gat ccc tac ccc atc aaa ttc ctc tac aac gtc tcc caa atc ttc ctt     240
   Asp Pro Tyr Pro Ile Lys Phe Leu Tyr Asn Val Ser Gln Ile Phe Leu
   65                  70                  75                  80
   tgt gcc tac atg act gtc gag gcg gga ttt ttg gcc tac cgc aat gga     288
   Cys Ala Tyr Met Thr Val Glu Ala Gly Phe Leu Ala Tyr Arg Asn Gly
                   85                  90                  95
   tat acc gtc atg cct tgc aat cat ttc aat gtg aat gat cct ccc gtg     336
   Tyr Thr Val Met Pro Cys Asn His Phe Asn Val Asn Asp Pro Pro Val
               100                 105                 110
   gcg aat ctt ctt tgg ttg ttt tat att tcc aag gtg tgg gac ttt tgg     384
   Ala Asn Leu Leu Trp Leu Phe Tyr Ile Ser Lys Val Trp Asp Phe Trp
           115                 120                 125
   gat acc att ttc att gtg ttg ggg aag aag tgg cgt caa tta tct ttc     432
   Asp Thr Ile Phe Ile Val Leu Gly Lys Lys Trp Arg Gln Leu Ser Phe
       130                 135                 140
   ttg cat gta tac cat cac acc acc atc ttt cta ttc tat tgg ctg aat     480
   Leu His Val Tyr His His Thr Thr Ile Phe Leu Phe Tyr Trp Leu Asn
   145                 150                 155                 160
   gcc aat gtc ttg tac gat ggt gac atc ttc ctt acc atc ttg ctc aat     528
   Ala Asn Val Leu Tyr Asp Gly Asp Ile Phe Leu Thr Ile Leu Leu Asn
                   165                 170                 175
   gga ttc atc cac acg gtg atg tac acg tat tac ttc atc tgt atg cat     576
   Gly Phe Ile His Thr Val Met Tyr Thr Tyr Tyr Phe Ile Cys Met His
               180                 185                 190
   acc aaa gat tcc aag acg ggc aag agt ctt cct ata tgg tgg aag tcg     624
   Thr Lys Asp Ser Lys Thr Gly Lys Ser Leu Pro Ile Trp Trp Lys Ser
           195                 200                 205
   agt ttg acg gcg ttt cag ttg ttg caa ttc act atc atg atg agt cag     672
   Ser Leu Thr Ala Phe Gln Leu Leu Gln Phe Thr Ile Met Met Ser Gln
       210                 215                 220
   gct acc tac ctt gtc ttc cac ggg tgt gat aag gtg tcg ctt cgt atc     720
   Ala Thr Tyr Leu Val Phe His Gly Cys Asp Lys Val Ser Leu Arg Ile
   225                 230                 235                 240
   acg att gtg tac ttt gtg tcc ctt ttg agt ttg ttc ttc ctt ttt gct     768
   Thr Ile Val Tyr Phe Val Ser Leu Leu Ser Leu Phe Phe Leu Phe Ala
                   245                 250                 255
   cag ttc ttt gtg caa tca tac atg gca ccc aaa aag aag aag agt gct     816
   Gln Phe Phe Val Gln Ser Tyr Met Ala Pro Lys Lys Lys Lys Ser Ala
               260                 265                 270
   tag                                                                 819
<210> 36
   <211> 272
   <212> PRT
   <213> Thalassiosira pseudonana
<400> 36
   Met Asp Ala Tyr Asn Ala Ala Met Asp Lys Ile Gly Ala Ala Ile Ile
   1               5                   10                  15
   Asp Trp Ser Asp Pro Asp Gly Lys Phe Arg Ala Asp Arg Glu Asp Trp
               20                  25                  30
   Trp Leu Cys Asp Phe Arg Ser Ala Ile Thr Ile Ala Leu Ile Tyr Ile
           35                  40                  45
   Ala Phe Val Ile Leu Gly Ser Ala Val Met Gln Ser Leu Pro Ala Met
       50                  55                  60
   Asp Pro Tyr Pro Ile Lys Phe Leu Tyr Asn Val Ser Gln Ile Phe Leu
   65                  70                  75                  80
   Cys Ala Tyr Met Thr Val Glu Ala Gly Phe Leu Ala Tyr Arg Asn Gly
                   85                  90                  95
   Tyr Thr Val Met Pro Cys Asn His Phe Asn Val Asn Asp Pro Pro Val
               100                 105                 110
   Ala Asn Leu Leu Trp Leu Phe Tyr Ile Ser Lys Val Trp Asp Phe Trp
           115                 120                 125
   Asp Thr Ile Phe Ile Val Leu Gly Lys Lys Trp Arg Gln Leu Ser Phe
       130                 135                 140
   Leu His Val Tyr His His Thr Thr Ile Phe Leu Phe Tyr Trp Leu Asn
   145                 150                 155                 160
   Ala Asn Val Leu Tyr Asp Gly Asp Ile Phe Leu Thr Ile Leu Leu Asn
                   165                 170                 175
   Gly Phe Ile His Thr Val Met Tyr Thr Tyr Tyr Phe Ile Cys Met His
               180                 185                 190
   Thr Lys Asp Ser Lys Thr Gly Lys Ser Leu Pro Ile Trp Trp Lys Ser
           195                 200                 205
   Ser Leu Thr Ala Phe Gln Leu Leu Gln Phe Thr Ile Met Met Ser Gln
       210                 215                 220
   Ala Thr Tyr Leu Val Phe His Gly Cys Asp Lys Val Ser Leu Arg Ile
   225                 230                 235                 240
   Thr Ile Val Tyr Phe Val Ser Leu Leu Ser Leu Phe Phe Leu Phe Ala
                   245                 250                 255
   Gln Phe Phe Val Gln Ser Tyr Met Ala Pro Lys Lys Lys Lys Ser Ala
               260                 265                 270
<210> 37
   <211> 1455
   <212> DNA
   <213> Thalassiosira pseudonana
<220>
   <221> CDS
   <222> (1)..(1455)
   <223> Delta-6-Desaturase
<400> 37
   atg gga aaa gga gga gac gca gcc gca gct acc aag cgt agt gga gca      48
   Met Gly Lys Gly Gly Asp Ala Ala Ala Ala Thr Lys Arg Ser Gly Ala
   1               5                   10                  15
   ttg aaa ttg gcg gag aag ccg cag aag tac act tgg cag gag gtg aag      96
   Leu Lys Leu Ala Glu Lys Pro Gln Lys Tyr Thr Trp Gln Glu Val Lys
               20                  25                  30
   aag cac atc acc ccc gac gat gcc tgg gta gtc cac caa aac aaa gtc     144
   Lys His Ile Thr Pro Asp Asp Ala Trp Val Val His Gln Asn Lys Val
           35                  40                  45
   tac gac gtc tcc aac tgg tac gac cac ccc ggt gga gcc gtg gtg ttc     192
   Tyr Asp Val Ser Asn Trp Tyr Asp His Pro Gly Gly Ala Val Val Phe
       50                  55                  60
   acc cac gcc gga gac gac atg acg gac atc ttc gcc gcc ttc cac gcc     240
   Thr His Ala Gly Asp Asp Met Thr Asp Ile Phe Ala Ala Phe His Ala
   65                  70                  75                  80
   caa ggc tct cag gcc atg atg aag aag ttt tac att gga gat ttg att     288
   Gln Gly Ser Gln Ala Met Met Lys Lys Phe Tyr Ile Gly Asp Leu Ile
                   85                  90                  95
   ccg gag agt gtg gag cat aag gat caa aga cag ttg gat ttc gag aag     336
   Pro Glu Ser Val Glu His Lys Asp Gln Arg Gln Leu Asp Phe Glu Lys
               100                 105                 110
   gga tat cgt gat tta cgg gcc aag ctt gtc atg atg ggg atg ttc aag     384
   Gly Tyr Arg Asp Leu Arg Ala Lys Leu Val Met Met Gly Met Phe Lys
           115                 120                 125
   tcg agt aag atg tat tat gca tac aag tgc tcg ttc aat atg tgc atg     432
   Ser Ser Lys Met Tyr Tyr Ala Tyr Lys Cys Ser Phe Asn Met Cys Met
       130                 135                 140
   tgg ttg gtg gcg gtg gcc atg gtg tac tac tcg gac agt ttg gca atg     480
   Trp Leu Val Ala Val Ala Met Val Tyr Tyr Ser Asp Ser Leu Ala Met
   145                 150                 155                 160
   cac att gga tcg gct ctc ttg ttg gga ttg ttc tgg cag cag tgt gga      528
   His Ile Gly Ser Ala Leu Leu Leu Gly Leu Phe Trp Gln Gln Cys Gly
                   165                 170                 175
   tgg ctt gcg cac gac ttt ctt cac cac caa gtc ttt aag caa cga aag      576
   Trp Leu Ala His Asp Phe Leu His His Gln Val Phe Lys Gln Arg Lys
               180                 185                 190
   tac gga gat ctc gtt ggc atc ttt tgg gga gat ctc atg cag ggg ttc      624
   Tyr Gly Asp Leu Val Gly Ile Phe Trp Gly Asp Leu Met Gln Gly Phe
           195                 200                 205
   tcg atg cag tgg tgg aag aac aag cac aat ggc cac cat gct gtt ccc      672
   Ser Met Gln Trp Trp Lys Asn Lys His Asn Gly His His Ala Val Pro
       210                 215                 220
   aac ttg cac aac tct tcc ttg gac agt cag gat ggt gat ccc gat att      720
   Asn Leu His Asn Ser Ser Leu Asp Ser Gln Asp Gly Asp Pro Asp Ile
   225                 230                 235                 240
   gat acc atg cca ctc ctt gct tgg agt ctc aag cag gct cag agt ttc      768
   Asp Thr Met Pro Leu Leu Ala Trp Ser Leu Lys Gln Ala Gln Ser Phe
                   245                 250                 255
   aga gag atc aat aag gga aag gac agt acc ttc gtc aag tac gct atc      816
   Arg Glu Ile Asn Lys Gly Lys Asp Ser Thr Phe Val Lys Tyr Ala Ile
               260                 265                 270
   aaa ttc cag gca ttc aca tac ttc ccc atc ctc ctc ttg gct cgc atc      864
   Lys Phe Gln Ala Phe Thr Tyr Phe Pro Ile Leu Leu Leu Ala Arg Ile
           275                 280                 285
   tct tgg ttg aat gaa tcc ttc aaa act gca ttc gga ctc gga gct gcc      912
   Ser Trp Leu Asn Glu Ser Phe Lys Thr Ala Phe Gly Leu Gly Ala Ala
       290                 295                 300
   tcg gag aat gcc aag ttg gag ttg gag aag cgt gga ctt cag tac cca      960
   Ser Glu Asn Ala Lys Leu Glu Leu Glu Lys Arg Gly Leu Gln Tyr Pro
   305                 310                 315                 320
   ctt ttg gag aag ctt gga atc acc ctt cat tac act tgg atg ttc gtc     1008
   Leu Leu Glu Lys Leu Gly Ile Thr Leu His Tyr Thr Trp Met Phe Val
                   325                 330                 335
   ctc tct tcc gga ttt gga agg tgg tct ctt cca tat tcc atc atg tat     1056
   Leu Ser Ser Gly Phe Gly Arg Trp Ser Leu Pro Tyr Ser Ile Met Tyr
               340                 345                 350
   ttc ttc act gcc aca tgc tcc tcg gga ctt ttc ctc gca ttg gtc ttt     1104
   Phe Phe Thr Ala Thr Cys Ser Ser Gly Leu Phe Leu Ala Leu Val Phe
           355                 360                 365
   gga ttg gga cac aac ggt atg tca gtg tac gat gcc acc acc cga cct     1152
   Gly Leu Gly His Asn Gly Met Ser Val Tyr Asp Ala Thr Thr Arg Pro
       370                 375                 380
   gac ttc tgg caa ctc caa gtc acc act aca cgt aac atc att ggt gga     1200
   Asp Phe Trp Gln Leu Gln Val Thr Thr Thr Arg Asn Ile Ile Gly Gly
   385                 390                 395                 400
   cac ggc att ccc caa ttc ttt gtg gat tgg ttc tgc ggt gga ttg caa     1248
   His Gly Ile Pro Gln Phe Phe Val Asp Trp Phe Cys Gly Gly Leu Gln
                   405                 410                 415
   tac caa gtg gat cac cac ctc ttc ccc atg atg cct aga aac aat atc     1296
   Tyr Gln Val Asp His His Leu Phe Pro Met Met Pro Arg Asn Asn Ile
               420                 425                 430
   gcg aaa tgc cac aag ctt gtg gag tca ttc tgt aag gag tgg ggt gtg      1344
   Ala Lys Cys His Lys Leu Val Glu Ser Phe Cys Lys Glu Trp Gly Val
           435                 440                     445
   aag tac cat gag gcc gat atg tgg gat ggt acc gtg gaa gtg ttg caa      1392
   Lys Tyr His Glu Ala Asp Met Trp Asp Gly Thr Val Glu Val Leu Gln
       450                 455                     460
   cat ctc tcc aag gtg tcg gat gat ttc ctt gtg gag atg gtg aag gat      1440
   His Leu Ser Lys Val Ser Asp Asp Phe Leu Val Glu Met Val Lys Asp
   465                 470                     475                 480
   ttc cct gcc atg taa                                                  1455
   Phe Pro Ala Met
<210> 38
   <211> 484
   <212> PRT
   <213> Thalassiosira pseudonana
<400> 38
   Met Gly Lys Gly Gly Asp Ala Ala Ala Ala Thr Lys Arg Ser Gly Ala
   1               5                   10                  15
   Leu Lys Leu Ala Glu Lys Pro Gln Lys Tyr Thr Trp Gln Glu Val Lys
               20                  25                  30
   Lys His Ile Thr Pro Asp Asp Ala Trp Val Val His Gln Asn Lys Val
           35                  40                  45
   Tyr Asp Val Ser Asn Trp Tyr Asp His Pro Gly Gly Ala Val Val Phe
       50                  55                  60
   Thr His Ala Gly Asp Asp Met Thr Asp Ile Phe Ala Ala Phe His Ala
   65                  70                  75                  80
   Gln Gly Ser Gln Ala Met Met Lys Lys Phe Tyr Ile Gly Asp Leu Ile
                   85                  90                  95
   Pro Glu Ser Val Glu His Lys Asp Gln Arg Gln Leu Asp Phe Glu Lys
               100                 105                 110
   Gly Tyr Arg Asp Leu Arg Ala Lys Leu Val Met Met Gly Met Phe Lys
           115                 120                 125
   Ser Ser Lys Met Tyr Tyr Ala Tyr Lys Cys Ser Phe Asn Met Cys Met
       130                 135                 140
   Trp Leu Val Ala Val Ala Met Val Tyr Tyr Ser Asp Ser Leu Ala Met
   145                 150                 155                 160
   His Ile Gly Ser Ala Leu Leu Leu Gly Leu Phe Trp Gln Gln Cys Gly
   165 170 175
   Trp Leu Ala His Asp Phe Leu His His Gln Val Phe Lys Gln Arg Lys
   180 185 190
   Tyr Gly Asp Leu Val Gly Ile Phe Trp Gly Asp Leu Met Gln Gly Phe
   195 200 205
   Ser Met Gln Trp Trp Lys Asn Lys His Asn Gly His His Ala Val Pro
   210 215 220
   Asn Leu His Asn Ser Ser Leu Asp Ser Gln Asp Gly Asp Pro Asp Ile
   225 230 235 240
   Asp Thr Met Pro Leu Leu Ala Trp Ser Leu Lys Gln Ala Gln Ser Phe
   245 250 255
   Arg Glu Ile Asn Lys Gly Lys Asp Ser Thr Phe Val Lys Tyr Ala Ile
   260 265 270
   Lys Phe Gln Ala Phe Thr Tyr Phe Pro Ile Leu Leu Leu Ala Arg Ile
   275 280 285
   Ser Trp Leu Asn Glu Ser Phe Lys Thr Ala Phe Gly Leu Gly Ala Ala
   290 295 300
   Ser Glu Asn Ala Lys Leu Glu Leu Glu Lys Arg Gly Leu Gln Tyr Pro
   305 310 315 320
   Leu Leu Glu Lys Leu Gly Ile Thr Leu His Tyr Thr Trp Met Phe Val
   325 330 335
   Leu Ser Ser Gly Phe Gly Arg Trp Ser Leu Pro Tyr Ser Ile Met Tyr
   340 345 350
   Phe Phe Thr Ala Thr Cys Ser Ser Gly Leu Phe Leu Ala Leu Val Phe
   355 360 365
   Gly Leu Gly His Asn Gly Met Ser Val Tyr Asp Ala Thr Thr Arg Pro
   370 375 380
   Asp Phe Trp Gln Leu Gln Val Thr Thr Thr Arg Asn Ile Ile Gly Gly
   385 390 395 400
   His Gly Ile Pro Gln Phe Phe Val Asp Trp Phe Cys Gly Gly Leu Gln
   405 410 415
   Tyr Gln Val Asp His His Leu Phe Pro Met Met Pro Arg Asn Asn Ile
   420 425 430
   Ala Lys Cys His Lys Leu Val Glu Ser Phe Cys Lys Glu Trp Gly Val
           435                 440                 445
   Lys Tyr His Glu Ala Asp Met Trp Asp Gly Thr Val Glu Val Leu Gln
       450                 455                 460
   His Leu Ser Lys Val Ser Asp Asp Phe Leu Val Glu Met Val Lys Asp
   465                 470                 475                 480
   Phe Pro Ala Met
<210> 39
   <211> 1449
   <212> DNA
   <213> Thalassiosira pseudonana
<220>
   <221> CDS
   <222> (1)..(1449)
   <223> Delta-5-Desaturase
<400> 39
   atg cca ccc aac gcc gag gtc aaa aac ctc cgt tca cgt tcc atc cca      48
   Met Pro Pro Asn Ala Glu Val Lys Asn Leu Arg Ser Arg Ser Ile Pro
   1               5                   10                  15
   acg aag aag tcc agt tca tcg tca tcc acc gcg aac gac gat ccg gct      96
   Thr Lys Lys Ser Ser Ser Ser Ser Ser Thr Ala Asn Asp Asp Pro Ala
               20                  25                  30
   acc caa tcc acc tca cct gtg aac cga acc ctc aag tct ttg aat gga     144
   Thr Gln Ser Thr Ser Pro Val Asn Arg Thr Leu Lys Ser Leu Asn Gly
           35                  40                  45
   aac gaa ata gct att gac ggt gtc atc tat gat att gat ggc ttt gtc     192
   Asn Glu Ile Ala Ile Asp Gly Val Ile Tyr Asp Ile Asp Gly Phe Val
       50                  55                  60
   cat cct gga gga gag gtt att agc ttc ttt gga ggc aac gat gtg act     240
   His Pro Gly Gly Glu Val Ile Ser Phe Phe Gly Gly Asn Asp Val Thr
   65                  70                  75                  80
   gta cag tac aaa atg att cat ccg tat cat aat agt aag cat ctc gag     288
   Val Gln Tyr Lys Met Ile His Pro Tyr His Asn Ser Lys His Leu Glu
                   85                  90                  95
   aag atg aga gcc gtt gga aag att gca gac tac tcc aca gag tac aag     336
   Lys Met Arg Ala Val Gly Lys Ile Ala Asp Tyr Ser Thr Glu Tyr Lys
               100                 105                 110
   ttc gac aca ccc ttt gaa cga gag atc aaa tcc gaa gtg ttc aaa atc     384
   Phe Asp Thr Pro Phe Glu Arg Glu Ile Lys Ser Glu Val Phe Lys Ile
           115                 120                 125
   gtc cgt cga gga cgt gaa ttc ggt aca aca gga tat ttc ctc cgt gcc     432
   Val Arg Arg Gly Arg Glu Phe Gly Thr Thr Gly Tyr Phe Leu Arg Ala
       130                 135                 140
   ttc ttc tac att gct ctc ttc ttc acc atg caa tac acc ttc gcc aca      480
   Phe Phe Tyr Ile Ala Leu Phe Phe Thr Met Gln Tyr Thr Phe Ala Thr
   145                 150                 155                 160
   tgc act acc ttc acc acc tac gat cat tgg tat caa agt ggt gta ttc      528
   Cys Thr Thr Phe Thr Thr Tyr Asp His Trp Tyr Gln Ser Gly Val Phe
                   165                 170                 175
   atc gcc att gtg ttt ggt atc tca caa gct ttc att ggg ttg aat gta      576
   Ile Ala Ile Val Phe Gly Ile Ser Gln Ala Phe Ile Gly Leu Asn Val
               180                 185                 190
   caa cat gat gcc aat cac gga gct gct agc aaa cga cct tgg gtg aat      624
   Gln His Asp Ala Asn His Gly Ala Ala Ser Lys Arg Pro Trp Val Asn
           195                 200                 205
   gat ctc ctt gga tct gga gct gat ctc atc ggt gga tgc aaa tgg aac      672
   Asp Leu Leu Gly Ser Gly Ala Asp Leu Ile Gly Gly Cys Lys Trp Asn
       210                 215                 220
   tgg ttg gct cag cat tgg act cat cat gcg tat acc aat cac gct gat      720
   Trp Leu Ala Gln His Trp Thr His His Ala Tyr Thr Asn His Ala Asp
   225                 230                 235                 240
   aaa gat cct gat agc ttt agt tcc gag ccg gtc ttc aac ttt aac gat      768
   Lys Asp Pro Asp Ser Phe Ser Ser Glu Pro Val Phe Asn Phe Asn Asp
                   245                 250                 255
   tat ccc att ggt cac ccc aaa aga aag tgg tgg cat agg ttc caa ggg      816
   Tyr Pro Ile Gly His Pro Lys Arg Lys Trp Trp His Arg Phe Gln Gly
               260                 265                 270
   ctc tac ttc cta atc atg ctg agt ttc tat tgg gta tcg atg gta ttc      864
   Leu Tyr Phe Leu Ile Met Leu Ser Phe Tyr Trp Val Ser Met Val Phe
           275                 280                 285
   aac cca caa gtt atc gac ctc cgt cat gct gga gct gcc tac gtt gga      912
   Asn Pro Gln Val Ile Asp Leu Arg His Ala Gly Ala Ala Tyr Val Gly
       290                 295                 300
   ttt cag atg gag aac gac ttt atc gtc aaa cgg aga aag tat gca atg      960
   Phe Gln Met Glu Asn Asp Phe Ile Val Lys Arg Arg Lys Tyr Ala Met
   305                 310                 315                 320
   gca ctt cgt gca atg tac ttc tat ttc aac atc tat tgt ccg att gtc     1008
   Ala Leu Arg Ala Met Tyr Phe Tyr Phe Asn Ile Tyr Cys Pro Ile Val
                   325                 330                 335
   aac aat gga ttg act tgg tcg aca gtt gga atc atc ctc tta atg gga     1056
   Asn Asn Gly Leu Thr Trp Ser Thr Val Gly Ile Ile Leu Leu Met Gly
               340                 345                 350
   gtt agc gaa agc ttc atg ctc tcc ggt cta ttc gta ctc tca cac aac     1104
   Val Ser Glu Ser Phe Met Leu Ser Gly Leu Phe Val Leu Ser His Asn
           355                 360                 365
   ttt gaa aat tcc gaa cgt gat cct acc tct gag tat cgc aag act ggt     1152
   Phe Glu Asn Ser Glu Arg Asp Pro Thr Ser Glu Tyr Arg Lys Thr Gly
       370                 375                 380
   gag caa gta tgt tgg ttc aag tct caa gtg gag act tct tct acc tac     1200
   Glu Gln Val Cys Trp Phe Lys Ser Gln Val Glu Thr Ser Ser Thr Tyr
   385                 390                 395                 400
   gga ggt atc gtt gct ggg tgt ctc act ggt gga ctc aac ttt caa gtg     1248
   Gly Gly Ile Val Ala Gly Cys Leu Thr Gly Gly Leu Asn Phe Gln Val
                   405                     410                     415
   gag cat cat ttg ttc ccg agg atg agc agt gct tgg tat cct ttc atc      1296
   Glu His His Leu Phe Pro Arg Met Ser Ser Ala Trp Tyr Pro Phe Ile
               420                     425                     430
   gcg ccg aag gtt aga gag att tgt aag aag cat gga gtt aga tac gct      1344
   Ala Pro Lys Val Arg Glu Ile Cys Lys Lys His Gly Val Arg Tyr Ala
           435                     440                     445
   tac tat ccg tac atc tgg cag aac ttg cat tct acc gtg agt tac atg      1392
   Tyr Tyr Pro Tyr Ile Trp Gln Asn Leu His Ser Thr Val Ser Tyr Met
       450                     455                     460
   cat ggg acg gga acg gga gct aga tgg gag ctt cag ccg ttg tct gga      1440
   His Gly Thr Gly Thr Gly Ala Arg Trp Glu Leu Gln Pro Leu Ser Gly
   465                     470                     475                     480
   agg gcg tag                                                          1449
   Arg Ala
<210> 40
   <211> 482
   <212> PRT
   <213> Thalassiosira pseudonana
<400> 40
   Met Pro Pro Asn Ala Glu Val Lys Asn Leu Arg Ser Arg Ser Ile Pro
   1               5                   10                  15
   Thr Lys Lys Ser Ser Ser Ser Ser Ser Thr Ala Asn Asp Asp Pro Ala
               20                  25                  30
   Thr Gln Ser Thr Ser Pro Val Asn Arg Thr Leu Lys Ser Leu Asn Gly
           35                  40                  45
   Asn Glu Ile Ala Ile Asp Gly Val Ile Tyr Asp Ile Asp Gly Phe Val
       50                  55                  60
   His Pro Gly Gly Glu Val Ile Ser Phe Phe Gly Gly Asn Asp Val Thr
   65                  70                  75                  80
   Val Gln Tyr Lys Met Ile His Pro Tyr His Asn Ser Lys His Leu Glu
                   85                  90                  95
   Lys Met Arg Ala Val Gly Lys Ile Ala Asp Tyr Ser Thr Glu Tyr Lys
               100                 105                 110
   Phe Asp Thr Pro Phe Glu Arg Glu Ile Lys Ser Glu Val Phe Lys Ile
           115                 120                 125
   Val Arg Arg Gly Arg Glu Phe Gly Thr Thr Gly Tyr Phe Leu Arg Ala
       130                 135                 140
   Phe Phe Tyr Ile Ala Leu Phe Phe Thr Met Gln Tyr Thr Phe Ala Thr
   145 150 155 160
   Cys Thr Thr Phe Thr Thr Tyr Asp His Trp Tyr Gln Ser Gly Val Phe
   165 170 175
   Ile Ala Ile Val Phe Gly Ile Ser Gln Ala Phe Ile Gly Leu Asn Val
   180 185 190
   Gln His Asp Ala Asn His Gly Ala Ala Ser Lys Arg Pro Trp Val Asn
   195 200 205
   Asp Leu Leu Gly Ser Gly Ala Asp Leu Ile Gly Gly Cys Lys Trp Asn
   210 215 220
   Trp Leu Ala Gln His Trp Thr His His Ala Tyr Thr Asn His Ala Asp
   225 230 235 240
   Lys Asp Pro Asp Ser Phe Ser Ser Glu Pro Val Phe Asn Phe Asn Asp
   245 250 255
   Tyr Pro Ile Gly His Pro Lys Arg Lys Trp Trp His Arg Phe Gln Gly
   260 265 270
   Leu Tyr Phe Leu Ile Met Leu Ser Phe Tyr Trp Val Ser Met Val Phe
   275 280 285
   Asn Pro Gln Val Ile Asp Leu Arg His Ala Gly Ala Ala Tyr Val Gly
   290 295 300
   Phe Gln Met Glu Asn Asp Phe Ile Val Lys Arg Arg Lys Tyr Ala Met
   305 310 315 320
   Ala Leu Arg Ala Met Tyr Phe Tyr Phe Asn Ile Tyr Cys Pro Ile Val
   325 330 335
   Asn Asn Gly Leu Thr Trp Ser Thr Val Gly Ile Ile Leu Leu Met Gly
   340 345 350
   Val Ser Glu Ser Phe Met Leu Ser Gly Leu Phe Val Leu Ser His Asn
   355 360 365
   Phe Glu Asn Ser Glu Arg Asp Pro Thr Ser Glu Tyr Arg Lys Thr Gly
   370 375 380
   Glu Gln Val Cys Trp Phe Lys Ser Gln Val Glu Thr Ser Ser Thr Tyr
   385 390 395 400
   Gly Gly Ile Val Ala Gly Cys Leu Thr Gly Gly Leu Asn Phe Gln Val
   405 410 415
   Glu His His Leu Phe Pro Arg Met Ser Ser Ala Trp Tyr Pro Phe Ile
               420                 425                 430
   Ala Pro Lys Val Arg Glu Ile Cys Lys Lys His Gly Val Arg Tyr Ala
           435                 440                 445
   Tyr Tyr Pro Tyr Ile Trp Gln Asn Leu His Ser Thr Val Ser Tyr Met
       450                 455                 460
   His Gly Thr Gly Thr Gly Ala Arg Trp Glu Leu Gln Pro Leu Ser Gly
   465                 470                 475                 480
   Arg Ala
<210> 41
   <211> 1512
   <212> DNA
   <213> Thalassiosira pseudonana
<220>
   <221> CDS
   <222> (1)..(1512)
   <223> Delta-4-Desaturase
<400> 41
   atg tgc aac ggc aac ctc cca gca tcc acc gca cag ctc aag tcc acc      48
   Met Cys Asn Gly Asn Leu Pro Ala Ser Thr Ala Gln Leu Lys Ser Thr
   1               5                   10                  15
   tcg aag ccc cag cag caa cat gag cat cgc acc atc tcc aag tcc gag      96
   Ser Lys Pro Gln Gln Gln His Glu His Arg Thr Ile Ser Lys Ser Glu
               20                  25                  30
   ctc gcc caa cac aac acg ccc aaa tca gca tgg tgt gcc gtc cac tcc     144
   Leu Ala Gln His Asn Thr Pro Lys Ser Ala Trp Cys Ala Val His Ser
           35                  40                  45
   act ccc gcc acc gac cca tcc cac tcc aac aac aaa caa cac gca cac     192
   Thr Pro Ala Thr Asp Pro Ser His Ser Asn Asn Lys Gln His Ala His
       50                  55                  60
   cta gtc ctc gac att acc gac ttt gcg tcc cgc cat cca ggg gga gac     240
   Leu Val Leu Asp Ile Thr Asp Phe Ala Ser Arg His Pro Gly Gly Asp
   65                  70                  75                  80
   ctc atc ctc ctc gct tcc ggc aaa gac gcc tcg gtg ctg ttt gaa aca     288
   Leu Ile Leu Leu Ala Ser Gly Lys Asp Ala Ser Val Leu Phe Glu Thr
                   85                  90                  95
   tac cat cca cgt gga gtt ccg acg tct ctc att caa aag ctg cag att     336
   Tyr His Pro Arg Gly Val Pro Thr Ser Leu Ile Gln Lys Leu Gln Ile
               100                 105                 110
   gga gtg atg gag gag gag gcg ttt cgg gat tcg ttt tac agt tgg act      384
   Gly Val Met Glu Glu Glu Ala Phe Arg Asp Ser Phe Tyr Ser Trp Thr
           115                 120                 125
   gat tct gac ttt tat act gtg ttg aag agg agg gtt gtg gag cgg ttg      432
   Asp Ser Asp Phe Tyr Thr Val Leu Lys Arg Arg Val Val Glu Arg Leu
       130                 135                 140
   gag gag agg ggg ttg gac agg agg gga tcg aaa gag att tgg atc aag      480
   Glu Glu Arg Gly Leu Asp Arg Arg Gly Ser Lys Glu Ile Trp Ile Lys
   145                 150                 155                 160
   gct ttg ttc ttg ttg gtt gga ttt tgg tac tgt ttg tac aag atg tat      528
   Ala Leu Phe Leu Leu Val Gly Phe Trp Tyr Cys Leu Tyr Lys Met Tyr
                   165                 170                 175
   act acg tcg gat atc gat cag tac ggt att gcc att gcc tat tct att      576
   Thr Thr Ser Asp Ile Asp Gln Tyr Gly Ile Ala Ile Ala Tyr Ser Ile
               180                 185                 190
   gga atg gga acc ttt gcg gca ttc atc ggc acg tgt att caa cac gat      624
   Gly Met Gly Thr Phe Ala Ala Phe Ile Gly Thr Cys Ile Gln His Asp
           195                 200                 205
   gga aat cac ggt gca ttc gct cag aac aag tta ctc aac aag ttg gct      672
   Gly Asn His Gly Ala Phe Ala Gln Asn Lys Leu Leu Asn Lys Leu Ala
       210                 215                 220
   ggg tgg acg ttg gat atg att ggt gcg agt gcg ttt acg tgg gag ctt      720
   Gly Trp Thr Leu Asp Met Ile Gly Ala Ser Ala Phe Thr Trp Glu Leu
   225                 230                 235                 240
   cag cac atg ctg ggg cat cat cca tat acg aat gtg ttg gat ggg gtg      768
   Gln His Met Leu Gly His His Pro Tyr Thr Asn Val Leu Asp Gly Val
                   245                 250                 255
   gag gag gag agg aag gag agg ggg gag gat gtt gct ttg gaa gaa aag      816
   Glu Glu Glu Arg Lys Glu Arg Gly Glu Asp Val Ala Leu Glu Glu Lys
               260                 265                 270
   gat cag gat ttt gaa gtt gcc aca tcc gga cga tta tat cat att gat      864
   Asp Gln Asp Phe Glu Val Ala Thr Ser Gly Arg Leu Tyr His Ile Asp
           275                 280                 285
   gcc aat gta cgt tat ggt tcg gta tgg aat gtc atg agg ttt tgg gct      912
   Ala Asn Val Arg Tyr Gly Ser Val Trp Asn Val Met Arg Phe Trp Ala
       290                 295                 300
   atg aag gtc att acg atg gga tat atg atg gga tta cca atc tac ttt      960
   Met Lys Val Ile Thr Met Gly Tyr Met Met Gly Leu Pro Ile Tyr Phe
   305                 310                 315                 320
   cat gga gta ctg agg gga gtt gga ttg ttt gtt att ggg cat ttg gcg     1008
   His Gly Val Leu Arg Gly Val Gly Leu Phe Val Ile Gly His Leu Ala
                   325                 330                 335
   tgt gga gag ttg ttg gcg acg atg ttt att gtg aat cac gtc att gag     1056
   Cys Gly Glu Leu Leu Ala Thr Met Phe Ile Val Asn His Val Ile Glu
               340                 345                 350
   ggt gtg agt tat gga acg aag gat ttg gtt ggt ggt gcg agt cat gta     1104
   Gly Val Ser Tyr Gly Thr Lys Asp Leu Val Gly Gly Ala Ser His Val
           355                 360                 365
   gat gag aag aag att gtc aag cca acg act gta ttg gga gat aca cca     1152
   Asp Glu Lys Lys Ile Val Lys Pro Thr Thr Val Leu Gly Asp Thr Pro
       370                 375                 380
   atg gta aag act cgc gag gag gca ttg aaa agc aac agc aat aac aac     1200
   Met Val Lys Thr Arg Glu Glu Ala Leu Lys Ser Asn Ser Asn Asn Asn
   385                 390                 395                 400
   aag aag aag gga gag aag aac tcg gta cca tcc gtt cca ttc aac gac      1248
   Lys Lys Lys Gly Glu Lys Asn Ser Val Pro Ser Val Pro Phe Asn Asp
                   405                 410                 415
   tgg gca gca gtc caa tgc cag acc tcc gtg aat tgg tct cca ggc tca      1296
   Trp Ala Ala Val Gln Cys Gln Thr Ser Val Asn Trp Ser Pro Gly Ser
               420                 425                 430
   tgg ttc tgg aat cac ttt tct ggg gga ctc tct cat cag att gag cat      1344
   Trp Phe Trp Asn His Phe Ser Gly Gly Leu Ser His Gln Ile Glu His
           435                 440                 445
   cac ttg ttc ccc agc att tgt cat aca aac tac tgt cat atc cag gat      1392
   His Leu Phe Pro Ser Ile Cys His Thr Asn Tyr Cys His Ile Gln Asp
       450                 455                 460
   gtt gtg gag agt acg tgt gct gag tac gga gtt ccg tat cag agt gag      1440
   Val Val Glu Ser Thr Cys Ala Glu Tyr Gly Val Pro Tyr Gln Ser Glu
   465                 470                 475                 480
   agt aat ttg ttt gtt gct tat gga aag atg att agt cat ttg aag ttt      1488
   Ser Asn Leu Phe Val Ala Tyr Gly Lys Met Ile Ser His Leu Lys Phe
                   485                 490                 495
   ttg ggt aaa gcc aag tgt gag tag                                      1512
   Leu Gly Lys Ala Lys Cys Glu
               500
<210> 42
   <211> 503
   <212> PRT
   <213> Thalassiosira pseudonana
<400> 42
   Met Cys Asn Gly Asn Leu Pro Ala Ser Thr Ala Gln Leu Lys Ser Thr
   1               5                   10                  15
   Ser Lys Pro Gln Gln Gln His Glu His Arg Thr Ile Ser Lys Ser Glu
               20                  25                  30
   Leu Ala Gln His Asn Thr Pro Lys Ser Ala Trp Cys Ala Val His Ser
           35                  40                  45
   Thr Pro Ala Thr Asp Pro Ser His Ser Asn Asn Lys Gln His Ala His
       50                  55                  60
   Leu Val Leu Asp Ile Thr Asp Phe Ala Ser Arg His Pro Gly Gly Asp
   65                  70                  75                  80
   Leu Ile Leu Leu Ala Ser Gly Lys Asp Ala Ser Val Leu Phe Glu Thr
                   85                  90                  95
   Tyr His Pro Arg Gly Val Pro Thr Ser Leu Ile Gln Lys Leu Gln Ile
   100 105 110
   Gly Val Met Glu Glu Glu Ala Phe Arg Asp Ser Phe Tyr Ser Trp Thr
   115 120 125
   Asp Ser Asp Phe Tyr Thr Val Leu Lys Arg Arg Val Val Glu Arg Leu
   130 135 140
   Glu Glu Arg Gly Leu Asp Arg Arg Gly Ser Lys Glu Ile Trp Ile Lys
   145 150 155 160
   Ala Leu Phe Leu Leu Val Gly Phe Trp Tyr Cys Leu Tyr Lys Met Tyr
   165 170 175
   Thr Thr Ser Asp Ile Asp Gln Tyr Gly Ile Ala Ile Ala Tyr Ser Ile
   180 185 190
   Gly Met Gly Thr Phe Ala Ala Phe Ile Gly Thr Cys Ile Gln His Asp
   195 200 205
   Gly Asn His Gly Ala Phe Ala Gln Asn Lys Leu Leu Asn Lys Leu Ala
   210 215 220
   Gly Trp Thr Leu Asp Met Ile Gly Ala Ser Ala Phe Thr Trp Glu Leu
   225 230 235 240
   Gln His Met Leu Gly His His Pro Tyr Thr Asn Val Leu Asp Gly Val
   245 250 255
   Glu Glu Glu Arg Lys Glu Arg Gly Glu Asp Val Ala Leu Glu Glu Lys
   260 265 270
   Asp Gln Asp Phe Glu Val Ala Thr Ser Gly Arg Leu Tyr His Ile Asp
   275 280 285
   Ala Asn Val Arg Tyr Gly Ser Val Trp Asn Val Met Arg Phe Trp Ala
   290 295 300
   Met Lys Val Ile Thr Met Gly Tyr Met Met Gly Leu Pro Ile Tyr Phe
   305 310 315 320
   His Gly Val Leu Arg Gly Val Gly Leu Phe Val Ile Gly His Leu Ala
   325 330 335
   Cys Gly Glu Leu Leu Ala Thr Met Phe Ile Val Asn His Val Ile Glu
   340 345 350
   Gly Val Ser Tyr Gly Thr Lys Asp Leu Val Gly Gly Ala Ser His Val
   355 360 365
   Asp Glu Lys Lys Ile Val Lys Pro Thr Thr Val Leu Gly Asp Thr Pro
   370 375 380
   Met Val Lys Thr Arg Glu Glu Ala Leu Lys Ser Asn Ser Asn Asn Asn
   385                 390                 395                 400
   Lys Lys Lys Gly Glu Lys Asn Ser Val Pro Ser Val Pro Phe Asn Asp
                   405                 410                 415
   Trp Ala Ala Val Gln Cys Gln Thr Ser Val Asn Trp Ser Pro Gly Ser
               420                 425                 430
   Trp Phe Trp Asn His Phe Ser Gly Gly Leu Ser His Gln Ile Glu His
           435                 440                 445
   His Leu Phe Pro Ser Ile Cys His Thr Asn Tyr Cys His Ile Gln Asp
       450                 455                 460
   Val Val Glu Ser Thr Cys Ala Glu Tyr Gly Val Pro Tyr Gln Ser Glu
   465                 470                 475                 480
   Ser Asn Leu Phe Val Ala Tyr Gly Lys Met Ile Ser His Leu Lys Phe
                   485                 490                 495
   Leu Gly Lys Ala Lys Cys Glu
               500
<210> 43
   <211> 1305
   <212> DNA
   <213> Thalassiosira pseudonana
<220>
   <221> CDS
   <222> (1)..(1305)
   <223> Delta-12-Desaturase
<400> 43
   atg gga aag gga gga aga tca gta acc cgc gct caa aca gca gaa aag      48
   Met Gly Lys Gly Gly Arg Ser Val Thr Arg Ala Gln Thr Ala Glu Lys
   1               5                   10                  15
   tca gca cac acc atc caa acc ttc acc gac ggc cga tgg gtc tcc ccc      96
   Ser Ala His Thr Ile Gln Thr Phe Thr Asp Gly Arg Trp Val Ser Pro
               20                  25                  30
   tac aac ccc ctc gca aaa gat gca cct gaa ctc ccc tcc aag ggt gaa     144
   Tyr Asn Pro Leu Ala Lys Asp Ala Pro Glu Leu Pro Ser Lys Gly Glu
           35                  40                  45
   atc aag gcg gtc atc ccc aaa gag tgc ttc gaa cga agc tac ctc cac     192
   Ile Lys Ala Val Ile Pro Lys Glu Cys Phe Glu Arg Ser Tyr Leu His
   50 55 60
   tcc atg tac ttc gtc ctc cgt gac acc gtc atg gcc gtg gcc tgc gcc 240
   Ser Met Tyr Phe Val Leu Arg Asp Thr Val Met Ala Val Ala Cys Ala
   65 70 75 80
   tac atc gcc cac tca acg ctc tcc acc gat att ccc tcc gag tta ctg 288
   Tyr Ile Ala His Ser Thr Leu Ser Thr Asp Ile Pro Ser Glu Leu Leu
   85 90 95
   agc gtg gac gca ctc aaa tgg ttc ctc gga tgg aac acc tac gcc ttt 336
   Ser Val Asp Ala Leu Lys Trp Phe Leu Gly Trp Asn Thr Tyr Ala Phe
   100 105 110
   tgg atg ggg tgc att ctc acc gga cac tgg gtc cta gcc cat gaa tgt 384
   Trp Met Gly Cys Ile Leu Thr Gly His Trp Val Leu Ala His Glu Cys
   115 120 125
   gga cat ggt gca ttc tct ccc tct cag acg ttt aat gac ttt tgg ggg 432
   Gly His Gly Ala Phe Ser Pro Ser Gln Thr Phe Asn Asp Phe Trp Gly
   130 135 140
   ttc att atg cat cag gcg gtg ttg gtt ccg tat ttc gcc tgg cag tac 480
   Phe Ile Met His Gln Ala Val Leu Val Pro Tyr Phe Ala Trp Gln Tyr
   145 150 155 160
   tct cat gcg aag cat cat cga cgt acc aac aac att atg gat ggg gag 528
   Ser His Ala Lys His His Arg Arg Thr Asn Asn Ile Met Asp Gly Glu
   165 170 175
   agc cat gtg ccc aat atc gcc aag gaa atg gga ttg aac gag aag aat 576
   Ser His Val Pro Asn Ile Ala Lys Glu Met Gly Leu Asn Glu Lys Asn
   180 185 190
   gag cgc agt gga gga tat gcc gcc att cat gag gct att gga gat gga 624
   Glu Arg Ser Gly Gly Tyr Ala Ala Ile His Glu Ala Ile Gly Asp Gly
   195 200 205
   ccc ttt gcg atg ttt caa atc ttt gct cac ttg gtg atc ggg tgg cct 672
   Pro Phe Ala Met Phe Gln Ile Phe Ala His Leu Val Ile Gly Trp Pro
   210 215 220
   att tac ttg atg gga ttt gct tcc act gga cgt ctc ggt cag gat ggg 720
   Ile Tyr Leu Met Gly Phe Ala Ser Thr Gly Arg Leu Gly Gln Asp Gly
   225 230 235 240
   aag gaa ctt cag gct gga gag atc atc gac cat tac cgt cct tgg agt 768
   Lys Glu Leu Gln Ala Gly Glu Ile Ile Asp His Tyr Arg Pro Trp Ser
   245 250 255
   aag atg ttc ccc acc aag ttg cga ttc aaa att gct ctt tcg aca ctt 816
   Lys Met Phe Pro Thr Lys Leu Arg Phe Lys Ile Ala Leu Ser Thr Leu
   260 265 270
   gga gtg att gcc gcc tgg gtt ggg ttg tac ttt gct gca caa gag tat 864
   Gly Val Ile Ala Ala Trp Val Gly Leu Tyr Phe Ala Ala Gln Glu Tyr
   275 280 285
   gga gtc ttg ccc gtg gtt ctt tgg tac att ggc cca ctc atg tgg aat 912
   Gly Val Leu Pro Val Val Leu Trp Tyr Ile Gly Pro Leu Met Trp Asn
   290 295 300
   cag gcg tgg ctt gtg ctc tac act tgg ctt cag cac aat gat ccc tcc 960
   Gln Ala Trp Leu Val Leu Tyr Thr Trp Leu Gln His Asn Asp Pro Ser
   305 310 315 320
   gtg cct caa tat gga agt gac gaa tgg aca tgg gtc aag gga gct ttg 1008
   Val Pro Gln Tyr Gly Ser Asp Glu Trp Thr Trp Val Lys Gly Ala Leu
   325 330 335
   tcg acg att gat cgc ccg tat ggt atc ttt gac ttc ttc cat cac aag      1056
   Ser Thr Ile Asp Arg Pro Tyr Gly Ile Phe Asp Phe Phe His His Lys
               340                 345                 350
   att gga agc act cac gta gct cat cat ttg ttc cac gag atg cca ttt      1104
   Ile Gly Ser Thr His Val Ala His His Leu Phe His Glu Met Pro Phe
           355                 360                 365
   tac aag gcg gat gtg gct act gcg tcg atc aag ggt ttc ttg gag ccg      1152
   Tyr Lys Ala Asp Val Ala Thr Ala Ser Ile Lys Gly Phe Leu Glu Pro
       370                 375                 380
   aag gga ctt tac aac tat gat cca acg cct tgg tat gtg gcc atg tgg      1200
   Lys Gly Leu Tyr Asn Tyr Asp Pro Thr Pro Trp Tyr Val Ala Met Trp
   385                 390                 395                 400
   agg gtg gcc aag act tgt cat tat att gag gat gtg gat gga gtt cag      1248
   Arg Val Ala Lys Thr Cys His Tyr Ile Glu Asp Val Asp Gly Val Gln
                   405                 410                 415
   tat tat aag agt ttg gag gat gtg cct ttg aag aag gat gcc aag aag      1296
   Tyr Tyr Lys Ser Leu Glu Asp Val Pro Leu Lys Lys Asp Ala Lys Lys
               420                 425                 430
   tct gat tag                                                          1305
   Ser Asp
<210> 44
   <211> 434
   <212> PRT
   <213> Thalassiosira pseudonana
<400> 44
   Met Gly Lys Gly Gly Arg Ser Val Thr Arg Ala Gln Thr Ala Glu Lys
   1               5                   10                  15
   Ser Ala His Thr Ile Gln Thr Phe Thr Asp Gly Arg Trp Val Ser Pro
               20                  25                  30
   Tyr Asn Pro Leu Ala Lys Asp Ala Pro Glu Leu Pro Ser Lys Gly Glu
           35                  40                  45
   Ile Lys Ala Val Ile Pro Lys Glu Cys Phe Glu Arg Ser Tyr Leu His
       50                  55                  60
   Ser Met Tyr Phe Val Leu Arg Asp Thr Val Met Ala Val Ala Cys Ala
   65                  70                  75                  80
   Tyr Ile Ala His Ser Thr Leu Ser Thr Asp Ile Pro Ser Glu Leu Leu
                   85                  90                  95
   Ser Val Asp Ala Leu Lys Trp Phe Leu Gly Trp Asn Thr Tyr Ala Phe
               100                 105                 110
   Trp Met Gly Cys Ile Leu Thr Gly His Trp Val Leu Ala His Glu Cys
           115                 120                 125
   Gly His Gly Ala Phe Ser Pro Ser Gln Thr Phe Asn Asp Phe Trp Gly
       130                 135                 140
   Phe Ile Met His Gln Ala Val Leu Val Pro Tyr Phe Ala Trp Gln Tyr
   145                 150                 155                 160
   Ser His Ala Lys His His Arg Arg Thr Asn Asn Ile Met Asp Gly Glu
                   165                 170                 175
   Ser His Val Pro Asn Ile Ala Lys Glu Met Gly Leu Asn Glu Lys Asn
               180                 185                 190
   Glu Arg Ser Gly Gly Tyr Ala Ala Ile His Glu Ala Ile Gly Asp Gly
           195                 200                 205
   Pro Phe Ala Met Phe Gln Ile Phe Ala His Leu Val Ile Gly Trp Pro
       210                 215                 220
   Ile Tyr Leu Met Gly Phe Ala Ser Thr Gly Arg Leu Gly Gln Asp Gly
   225                 230                 235                 240
   Lys Glu Leu Gln Ala Gly Glu Ile Ile Asp His Tyr Arg Pro Trp Ser
                   245                 250                 255
   Lys Met Phe Pro Thr Lys Leu Arg Phe Lys Ile Ala Leu Ser Thr Leu
               260                 265                 270
   Gly Val Ile Ala Ala Trp Val Gly Leu Tyr Phe Ala Ala Gln Glu Tyr
           275                 280                 285
   Gly Val Leu Pro Val Val Leu Trp Tyr Ile Gly Pro Leu Met Trp Asn
       290                 295                 300
   Gln Ala Trp Leu Val Leu Tyr Thr Trp Leu Gln His Asn Asp Pro Ser
   305                 310                 315                 320
   Val Pro Gln Tyr Gly Ser Asp Glu Trp Thr Trp Val Lys Gly Ala Leu
                   325                 330                 335
   Ser Thr Ile Asp Arg Pro Tyr Gly Ile Phe Asp Phe Phe His His Lys
               340                 345                 350
   Ile Gly Ser Thr His Val Ala His His Leu Phe His Glu Met Pro Phe
           355                 360                 365
   Tyr Lys Ala Asp Val Ala Thr Ala Ser Ile Lys Gly Phe Leu Glu Pro
       370                 375                 380
   Lys Gly Leu Tyr Asn Tyr Asp Pro Thr Pro Trp Tyr Val Ala Met Trp
   385 390 395 400
   Arg Val Ala Lys Thr Cys His Tyr Ile Glu Asp Val Asp Gly Val Gln
   405 410 415
   Tyr Tyr Lys Ser Leu Glu Asp Val Pro Leu Lys Lys Asp Ala Lys Lys
   420 425 430
   Ser Asp

## Beschreibung

Die vorliegende Beschreibung betrifft Polynucleotide aus Ostreococcus lucimarinus, die Desaturasen und Elongasen kodieren und zur rekombinanten Herstellung von mehrfach ungesättigten Fettsäuren eingesetzt werden können. Weiterhin betrifft die Erfindung Vektoren, Wirtszellen und transgene nicht-humane Organismen, die die Polynucleotide enthalten, sowie die von den Polynucleotiden kodierten Polypeptide. Schließlich betrifft die Erfindung noch Herstellungsverfahren für die mehrfach ungesättigten Fettsäuren und für Öl-, Lipid- und Fettsäurezusammensetzungen.

Fettsäuren und Triacylglyceride haben eine Vielzahl von Anwendungen in der Lebensmittelindustrie, der Tierernährung, der Kosmetik und im Pharmabereich. Je nachdem, ob es sich um freie gesättigte und ungesättigte Fettsäuren oder um Triacylglyceride mit einem erhöhten Gehalt an gesättigten oder ungesättigten Fettsäuren handelt, sind sie für die unterschiedlichsten Anwendungen geeignet. Mehrfachungesättigte Fettsäuren wie Linol- und Linolensäure sind für Säugetiere essentiell, da sie nicht von diesen selbst hergestellt werden können. Deshalb stellen mehrfach ungesättigte ω-3-Fettsäuren und ω-6-Fettsäuren einen wichtigen Bestandteil der tierischen und menschlichen Nahrung dar.

Mehrfach ungesättigte langkettige ω-3-Fettsäuren wie Eicosapentaensäure (= EPA, C20:5^{Δ5,8,11,14,17}) oder Docosahexaensäure (= DHA, c22:6^{Δ4,7,10,13,16,19}) sind wichtige Komponenten der menschlichen Ernährung aufgrund ihrer verschiedenen Rollen in der Gesundheit, die Aspekte wie die Entwicklung des kindlichen Gehirns, der Funktionalität des Auges, der Synthese von Hormonen und anderer Signalstoffe, sowie die Vorbeugung von Herz-Kreislauf-Beschwerden, Krebs und Diabetes umfassen (Poulos, A Lipids 30:1-14, 1995; Horrocks, LA und Yeo YK Pharmacol Res 40:211-225, 1999). Es besteht aus diesem Grund ein Bedarf an der Produktion mehrfach ungesättigter langkettiger Fettsäuren.

Aufgrund der Heute üblichen Zusammensetzung der menschlichen Nahrung ist ein Zusatz von mehrfach ungesättigten ω-3-Fettsäuren, die bevorzugt in Fischölen vorkommen, zur Nahrung besonders wichtig. So werden beispielsweise mehrfach ungesättigte Fettsäuren wie Docosahexaensäure (= DHA, c22:6^{Δ4,7,10,13,16,19}) oder Eisosapentaensäure (= EPA, C20:5^{Δ5,8,11,14,17}) Babynahrung zur Erhöhung des Nährwertes zugesetzt. Der ungesättigten Fettsäure DHA wird dabei ein positiver Effekt auf die Entwicklung und Aufrechterhaltung von Gehirnfunktionen zugeschrieben. Im folgenden werden mehrfach ungesättigte Fettsäuren als PUFA, PUFAs, LCPUFA oder LCPUFAs bezeichnet (poly unsaturated fatty acids, PUFA, mehrfach ungesättigte Fettsäuren; long chain poly unsaturated fatty acids, LCPUFA, langkettige mehrfach ungesättigte Fettsäuren).

Hauptsächlich werden die verschiedenen Fettsäuren und Triglyceride aus Mikroorganismen wie Mortierella oder Schizochytrium oder aus Öl-produzierenden Pflanzen wie Soja, Raps, Algen wie Crypthecodinium oder Phaeodactylum und weiteren gewonnen, wobei sie in der Regel in Form ihrer Triacylglyceride (= Triglyceride = Triglycerole) anfallen. Sie können aber auch aus Tieren wie z.B. Fischen gewonnen werden. Die freien Fettsäuren werden vorteilhaft durch Verseifung hergestellt. Sehr langkettige mehrfach ungesättigte Fettsäuren wie DHA, EPA, Arachidonsäure (= ARA, C20:4^{Δ5,8,11,4}), Dihomo-γ-linolensäure (C20:3^{Δ8,11,4}) oder Docosapentaensäure (DPA, C22:5^{Δ7,10,13,16,19}) werden in Ölfruchtpflanzen wie Raps, Soja, Sonnenblume, Färbersaflor nicht synthetisiert. Übliche natürliche Quellen für diese Fettsäuren sind Fische wie Hering, Lachs, Sardine, Goldbarsch, Aal, Karpfen, Forelle, Heilbutt, Makrele, Zander oder Thunfisch oder Algen.

Je nach Anwendungszweck werden Öle mit gesättigten oder ungesättigten Fettsäuren bevorzugt. So werden z.B. in der humanen Ernährung Lipide mit ungesättigten Fettsäuren speziell mehrfach ungesättigten Fettsäuren bevorzugt. Den mehrfach ungesättigten ω-3-Fettsäuren wird dabei ein positiver Effekt auf den Cholesterinspiegel im Blut und damit auf die Möglichkeit der Prävention einer Herzerkrankung zugeschrieben. Durch Zugabe dieser ω-3-Fettsäuren zur Nahrung kann das Risiko einer Herzerkrankung, eines Schlaganfalls oder von Bluthochdruck deutlich verringert werden. Auch entzündliche speziell chronisch entzündliche Prozesse im Rahmen immunologischer Erkrankungen wie rheumatroider Arthritis lassen sich durch w-3-Fettsäuren positiv beeinflussen. Sie werden deshalb Lebensmitteln speziell diätischen Lebensmitteln zugegeben oder finden in Medikamenten Anwendung. ω-6-Fettsäuren wie Arachidonsäure haben bei diesen rheumatischen Erkrankungen aufgrund unserer üblichen Nahrungsmittelzusammensetzung eher einen negativen Effekt auf diese Krankheiten.

w-3- und ω-6-Fettsäuren sind Vorläufer von Gewebshormonen, den sogenannten Eicosanoiden wie den Prostaglandinen, die sich von der Dihomo-γ-linolensäure, der Arachidonsäure und der Eicosapentaensäure ableiten, den Thromoxanen und Leukotrienen, die sich von der Arachidonsäure und der Eicosapentaensäure ableiten. Eicosanoide (sog. PG₂-Serie), die aus ω-6-Fettsäuren gebildet werden fördern in der Regel Entzündungsreaktionen, während Eicosanoide (sog. PG₃-Serie) aus ω-3-Fettsäuren geringe oder keine entzündungsfördernde Wirkung haben.

Aufgrund ihrer positiven Eigenschaften hat es in der Vergangenheit nicht an Ansätzen gefehlt, Gene, die an der Synthese von Fettsäuren bzw. Triglyceriden beteiligt sind, für die Herstellung von Ölen in verschiedenen Organismen mit geändertem Gehalt an ungesättigten Fettsäuren verfügbar zu machen. So wird in WO 91/13972 und seinem US-Äquivalent eine Δ-9-Desaturase beschrieben. In WO 93/11245 wird eine Δ-15-Desaturase in WO 94/11516 wird eine Δ-12-Desaturase beansprucht. Weitere Desaturasen werden beispielsweise in EP-A-0 550 162, WO 94/18337, WO 97/30582, WO 97/21340, WO 95/18222, EP-A-0 794 250, Stukey et al., J. Biol. Chem., 265, 1990: 20144-20149, Wada et al., Nature 347, 1990: 200-203 oder Huang et al., Lipids 34, 1999: 649-659 beschrieben. Die biochemische Charakterisierung der verschiedenen Desaturasen ist jedoch bisher nur unzureichend erfolgt, da die Enzyme als membrangebundene Proteine nur sehr schwer zu isolieren und zu charakterisieren sind (McKeon et al., Methods in Enzymol. 71, 1981: 12141-12147, Wang et al., Plant Physiol. Biochem., 26, 1988: 777-792). In der Regel erfolgt die Charakterisierung membrangebundener Desaturasen durch Einbringung in einen geeigneten Organismus, der anschließend auf Enzymaktivität mittels Edukt- und Produktanalyse untersucht wird. Δ-6-Desaturasen werden in WO 93/06712, US 5,614,393, US5614393, WO 96/21022, WO00/21557 und WO 99/27111 beschrieben und auch die Anwendung zur Produktion in transgenen Organismen beschrieben wie in WO98/46763 WO98/46764, WO9846765. Dabei wird auch die Expression verschiedener Desaturasen wie in WO99/64616 oder WO98/46776 und Bildung polyungesättigter Fettsäuren beschrieben und beansprucht. Bzgl. der Effektivität der Expression von Desaturasen und ihren Einfluss auf die Bildung polyungesättigter Fettsäuren ist anzumerken, dass durch Expression einer einzelnen Desaturase wie bisher beschrieben lediglich geringe Gehalte an ungesättigten Fettsäuren/Lipiden wie z.B. γ-Linolensäure und Stearidonsäure erreicht wurden. Weiterhin wurde in der Regel ein Gemisch aus w-3- und w-6-Fettsäuren erhalten.

Besonders geeignete Mikroorganismen zur Herstellung von PUFAs sind Mikroorganismen wie Mikroalgen wie Phaeodactylum tricornutum, Porphiridium-Arten, Thraustochytrien-Arten, Schizochytrien-Arten oder Crypthecodinium-Arten, Ciliaten, wie Stylonychia oder Colpidium, Pilze, wie Mortierella, Entomophthora oder Mucor und/oder Moosen wie Physcomitrella, Ceratodon und Marchantia (R. Vazhappilly & F. Chen (1998) Botanica Marina 41: 553-558; K. Totani & K. Oba (1987) Lipids 22: 1060-1062; M. Akimoto et al. (1998) Appl. Biochemistry and Biotechnology 73: 269-278). Durch Stammselektion ist eine Anzahl von Mutantenstämmen der entsprechenden Mikroorganismen entwickelt worden, die eine Reihe wünschenswerter Verbindungen, einschließlich PUFAs, produzieren. Die Mutation und Selektion von Stämmen mit verbesserter Produktion eines bestimmten Moleküls wie den mehrfach ungesättigten Fettsäuren ist jedoch ein zeitraubendes und schwieriges Verfahren. Deshalb werden, wann immer möglich wie oben beschrieben gentechnologische Verfahren bevorzugt. Mit Hilfe der vorgenannten Mikroorganismen lassen sich jedoch nur begrenzte Mengen der gewünschten mehrfach ungesättigten Fettsäuren wie DPA, EPA oder ARA herstellen. Wobei diese in der Regel je nach verwendeten Mikroorganismus als Fettsäuregemische aus beispielsweise EPA, DPA und ARA anfallen.

Für die Synthese von Arachidonsäure, Eicosapentaensäure (EPA) und Docosahexaensäure (DHA) werden verschiedene Synthesewege diskutiert. So erfolgt die Produktion von EPA bzw. DHA in marinen Bakterien wie Vibrio sp. oder Shewanella sp. nach dem Polyketid-Weg (Yu, R. et al. Lipids 35:1061-1064, 2000; Takeyama, H. et al. Microbiology 143:2725-2731, 1997).

Ein alternative Strategie verläuft über die wechselnde Aktivität von Desaturasen und Elongasen (Zank, T.K. et al. Plant Journal 31:255-268, 2002; Sakuradani, E. et al. Gene 238:445-453, 1999). Eine Modifikation des beschriebenen Weges über Δ6-Desaturase, Δ6-Elongase, Δ5-Desaturase, Δ5-Elongase, Δ4-Desaturase ist der Sprecher-Syntheseweg (Sprecher 2000, Biochim. Biophys. Acta 1486:219-231) in Säugetieren. Anstelle der Δ4-Desaturierung erfolgt hier ein weiterer Elongationsschritt auf C₂₄, eine weitere Δ6-Desaturierung und abschliessend eine β-Oxidation auf die C₂₂-Kettenlänge. Für die Herstellung in Pflanzen und Mikroorganismen ist der sogenannte Sprecher-Syntheseweg allerdings nicht geeignet, da die Regulationsmechanismen nicht bekannt sind.

Die polyungesättigten Fettsäuren können entsprechend ihrem Desaturierungsmuster in zwei große Klassen, in w-6- oder ω-3-Fettsäuren eingeteilt werden, die metabolisch und funktionell unterschiedlich Aktivitäten haben .

Als Ausgangsprodukt für den ω-6-Stoffwechselweg fungiert die Fettsäure Linolsäure (18:2^{Δ9,12}), während der ω-3-Weg über Linolensäure (18:3^{Δ9,12,15}) abläuft. Linolensäure wird dabei durch Aktivität einer ω-3-Desaturase gebildet (Tocher et al. 1998, Prog. Lipid Res. 37, 73-117 ; Domergue et al. 2002, Eur. J. Biochem. 269, 4105-4113). Säugetiere und damit auch der Mensch verfügen über keine entsprechende Desaturaseaktivität (Δ-12- und ω-3-Desaturase) und müssen diese Fettsäuren (essentielle Fettsäuren) über die Nahrung aufnehmen. Über die Abfolge von Desaturase- und Elongase-Reaktionen werden dann aus diesen Vorstufen die physiologisch wichtigen polyungesättigten Fettsäuren Arachidonsäure (= ARA, 20:4^{Δ5,8,11,14}), eine ω-6-Fettsäure und die beiden ω-3-Fettsäuren Eicosapentaen- (= EPA, 20:5^{Δ5,8,11,14,17}) und Docosahexaensäure (DHA, 22:6^{Δ4,7,10,13,17,19}) synthetisiert. Die Applikation von ω-3-Fettsäuren zeigt dabei die wie oben beschrieben therapeutische Wirkung bei der Behandlung von Herz-Kreislaufkrankheiten (Shimikawa 2001, World Rev. Nutr. Diet. 88, 100-108), Entzündungen (Calder 2002, Proc. Nutr. Soc. 61, 345-358) und Arthridis (Cleland und James 2000, J. Rheumatol. 27, 2305-2307).

Die Verlängerung von Fettsäuren durch Elongasen um 2 bzw. 4 C-Atome ist für die Produktion von C₂₀- bzw. C₂₂-PUFAs von entscheidender Bedeutung. Dieser Prozess verläuft über 4 Stufen. Der erste Schritt stellt die Kondensation von Malonyl-CoA an das Fettsäure-Acyl-CoA durch die Ketoacyl-CoA-Synthase (KCS, im weiteren Text als Elongase bezeichnet). Es folgt dann ein Reduktionschritt (Ketoacyl-CoA-Reduktase, KCR), ein Dehydratationsschritt (Dehydratase) und ein abschliessender Reduktionsschritt (enoyl-CoA-Reduktase). Es wurde postuliert, dass die Aktivität der Elongase die Spezifität und Geschwindigkeit des gesamten Prozesses beeinflussen (Millar and Kunst, 1997 Plant Journal 12:121-131).

In der Vergangenheit wurden zahlreiche Versuche unternommen, Elongase Gene zu erhalten. Millar and Kunst, 1997 (Plant Journal 12:121-131) und Millar et al. 1999, (Plant Cell 11:825-838) beschreiben die Charakterisierung von pflanzlichen Elongasen zur Synthese von einfachungesättigten langkettigen Fettsäuren (C22:1) bzw. zur Synthese von sehr langkettigen Fettsäuren für die Wachsbildung in Pflanzen (C₂₈-C₃₂). Beschreibungen zur Synthese von Arachidonsäure und EPA finden sich beispielsweise in WO0159128, WO0012720, WO02077213 und WO0208401. Die Synthese von mehrfachungesättigter C24 Fettsäuren ist beispielsweise in Tvrdik et al 2000, JCB 149:707-717 oder WO0244320 beschrieben.

Zur Herstellung von DHA (C22:6 n-3) in Organismen, die diese Fettsäure natürlicherweise nicht produzieren, wurde bisher keine spezifische Elongase beschrieben. Bisher wurden nur Elongasen beschrieben, die C₂₀- bzw. C₂₄-Fettsäuren bereitstellen. Eine Δ-5-Elongase-Aktivität wurde bisher noch nicht beschrieben.

Höhere Pflanzen enthalten mehrfach ungesättigte Fettsäuren wie Linolsäure (C18:2) und Linolensäure (C18:3). ARA, EPA und DHA kommen im Samenöl höherer Pflanzen gar nicht oder nur in Spuren vor (E. Ucciani: Nouveau Dictionnaire des Huiles Vegetales. Technique & Documentation - Lavoisier, 1995. ISBN: 2-7430-0009-0). Es wäre jedoch vorteilhaft, in höheren Pflanzen, bevorzugt in Ölsaaten wie Raps, Lein, Sonnenblume und Soja, LCPUFAs herzustellen, da auf diese Weise große Mengen qualitativ hochwertiger LCPUFAs für die Lebensmittelindustrie, die Tierernährung und für pharmazeutische Zwecke kostengünstig gewonnen werden können. Hierzu müssen vorteilhaft über gentechnische Methoden Gene kodierend für Enzyme der Biosynthese von LCPUFAs in Ölsaaten eingeführt und exprimiert werden. Dies sind Gene, die beispielsweise für Δ-6-Desaturasen, Δ-6-Elongasen, Δ-5-Desaturasen oder Δ-4-Desaturasen codieren. Diese Gene können vorteilhaft aus Mikroorganismen und niederen Pflanzen isoliert werden, die LCPUFAs herstellen und in den Membranen oder Triacylglyceriden einbauen. So konnten bereits Δ-6-Desaturase-Gene aus dem Moos Physcomitrella patens und Δ-6-Elongase-Gene aus P. patens und dem Nematoden C. elegans isoliert.

Erste transgene Pflanzen, die Gene kodierend für Enzyme der LCPUFA-Biosynthese enthalten und exprimieren und LCPUFAs produzieren wurden beispielsweise in DE 102 19 203 (Verfahren zur Herstellung mehrfach ungesättigter Fettsäuren in Pflanzen) erstmals beschrieben. Diese Pflanzen produzieren allerdings LCPUFAs in Mengen, die für eine Aufarbeitung der in den Pflanzen enthaltenen Öle noch weiter optimiert werden müssen.

Um eine Anreicherung der Nahrung und des Futters mit diesen mehrfach ungesättigten Fettsäuren zu ermöglichen, besteht daher ein großer Bedarf an Mitteln und Maßnahmen für eine einfache, kostengünstige Herstellung dieser mehrfach ungesättigten Fettsäuren speziell in eukaryontischen Systemen. Die der vorliegende Erfindung zugrunde liegende Aufgabe wäre also die ereitstellung von solchen Mitteln und Maßnahmen. Die Aufgabe wird durch die Ausführungsformen gelöst, die in den Patentansprüchen und im Folgenden beschrieben werden.

Die vorliegende Erfindung betrifft somit ein Polynucleotid umfassend eine Nukleinsäuresequenz, die ausgewählt ist aus der Gruppe bestehend aus:
(a) Nucleinsäuresequenz wie in SEQ ID No. 9, gezeigt;
(b) Nucleinsäuresequenz, die ein Polypeptid kodiert, das eine Aminosäuresequenz wie in SEQ ID No. 10, gezeigt aufweist;
(c) Nucleinsäuresequenz, die ein Polypeptid kodiert, das mindestens 85% identisch zu einem Polypeptid ist, das von der Nucleinsäuresequenzen aus (a) oder (b) kodiert wird, wobei das Polypeptid Desaturase oder Elongase Aktivität aufweist; und
(d) Nucleinsäuresequenz für ein Fragment mit mindestens 500 aufeinander folgenden Nucleotiden einer Nucleinsäure aus (a), (b) oder (c), woei das Fragment ein Polypeptid mit einer Desaturase oder Elongase Aktivität kodiert.

Der Begriff "Polynucleotid" betrifft Polynucleotide, die Nukleinsäuresequenzen umfassen, die Polypeptide mit Desaturase oder Elongase Aktivität kodieren. Die Desaturase oder Elongase Aktivitäten werden vorzugsweise für die Biosynthese von Lipiden oder Fettsäuren benötigt. Besonders bevorzugt handelt es sich um die folgenden Desaturase bzw. Elongase Aktivitäten: Δ-4-Desaturase, Δ-5-Desaturase, Δ-5-Elongase, Δ-6-Desaturase, Δ-6-Elongase oder Δ-12-Desaturase. Die Desaturasen und/oder Elongasen sind bevorzugt an der Synthese mehrfach ungesättigter Fettsäuren (PUFAs) und besonders bevorzugt an der Synthese lankettiger PUFAs (LCPUFAs) beteiligt. Geeignete Nachweissysteme für diese Desaturase bzw. Elongase Aktivitäten sind in den Beispielen oder in WO2005/083053 beschrieben. Besonders bevorzugt handelt es sich bei den zuvor genannten Aktivitäten hinsichtlich Substratspezifitäten und Umsetzungsraten um die der jeweiligen Enzyme aus Ostereococcus lucimarinus. Die spezifischen Polynucleotide, d.h. die Polynucleotide mit einer Nukleinsäuresequenz gemäß SEQ ID NO: 1, 3, 5, 7, 9, 11, 13 oder 15, wurden aus Ostereococcus lucimarinus gewonnen.

Insbesondere sind also Polynucleotide:
Polynucleotide, die ein Polypeptid mit Δ-12-Desaturase Aktivität kodieren und (i) eine Nucleinsäuresequenz umfassen, wie in SEQ ID NO: 1 oder 3 gezeigt, (ii) eine Nucleinsüresequenz umfassen, die für ein Polypeptid kodiert wie in SEQ ID NO: 2 oder 4 gezeigt, (iii) eine Nucleinsäuresequenz umfassen, die mindestens 83% identisch zu einer der Nucleinsäuresequenzen aus (i) oder (ii) ist, oder (iv) eine Nucleinsäuresequenz eines Fragments einer Nucleinsäure aus (i), (ii) oder (iii).

Polynucleotide, die ein Polypeptid mit Δ-4-Desaturase Aktivität kodieren und (i) eine Nucleinsäuresequenz umfassen, wie in SEQ ID NO: 5 gezeigt, (ii) eine Nucleinsüresequenz umfassen, die für ein Polypeptid kodiert wie in SEQ ID NO: 6 gezeigt, (iii) eine Nucleinsäuresequenz umfassen, die mindestens 72% identisch zu einer der Nucleinsäuresequenzen aus (i) oder (ii) ist, oder (iv) eine Nucleinsäuresequenz eines Fragments einer Nucleinsäure aus (i), (ii) oder (iii).

Polynucleotide, die ein Polypeptid mit Δ-5-Desaturase Aktivität kodieren und (i) eine Nucleinsäuresequenz umfassen, wie in SEQ ID NO: 7 oder 9 gezeigt, (ii) eine Nucleinsüresequenz umfassen, die für ein Polypeptid kodiert wie in SEQ ID NO: 8 oder 10 gezeigt, (iii) eine Nucleinsäuresequenz umfassen, die mindestens 72% identisch zu einer der Nucleinsäuresequenzen aus (i) oder (ii) ist, oder (iv) eine Nucleinsäuresequenz eines Fragments einer Nucleinsäure aus (i), (ii) oder (iii).

Polynucleotide, die ein Polypeptid mit Δ-5-Elongase Aktivität kodieren und (i) eine Nucleinsäuresequenz umfassen, wie in SEQ ID NO: 11 gezeigt, (ii) eine Nucleinsüresequenz umfassen, die für ein Polypeptid kodiert wie in SEQ ID NO: 12 gezeigt, (iii) eine Nucleinsäuresequenz umfassen, die mindestens 78% identisch zu einer der Nucleinsäuresequenzen aus (i) oder (ii) ist, oder (iv) eine Nucleinsäuresequenz eines Fragments einer Nucleinsäure aus (i), (ii) oder (iii).

Polynucleotide, die ein Polypeptid mit Δ-6-Desaturase Aktivität kodieren und (i) eine Nucleinsäuresequenz umfassen, wie in SEQ ID NO: 13 gezeigt, (ii) eine Nucleinsüresequenz umfassen, die für ein Polypeptid kodiert wie in SEQ ID NO: 14 gezeigt, (iii) eine Nucleinsäuresequenz umfassen, die mindestens 72% identisch zu einer der Nucleinsäuresequenzen aus (i) oder (ii) ist, oder (iv) eine Nucleinsäuresequenz eines Fragments einer Nucleinsäure aus (i), (ii) oder (iii).

Polynucleotide, die ein Polypeptid mit Δ-6-Elongase Aktivität kodieren und (i) eine Nucleinsäuresequenz umfassen, wie in SEQ ID NO: 15 gezeigt, (ii) eine Nucleinsüresequenz umfassen, die für ein Polypeptid kodiert wie in SEQ ID NO: 16 gezeigt, (iii) eine Nucleinsäuresequenz umfassen, die mindestens 71% identisch zu einer der Nucleinsäuresequenzen aus (i) oder (ii) ist, oder (iv) eine Nucleinsäuresequenz eines Fragments einer Nucleinsäure aus (i), (ii) oder (iii).

Es versteht sich, dass die zuvor genannten spezifischen Sequenzen angesichts des degenerierten genetischen Codes auch verändert werden können wobei von den veränderten Polynucleotiden immer noch Polypeptide mit einer Aminosäuresequenz gemäß einer der SEQ ID NO: 2, 4, 6, 8, 10, 12, 14 oder 16 kodiert werden, die die zuvor genannten Desaturase bzw. Elongase Aktivitäten aufweisen.

Der Begriff "Polynucleotid" umfasst auch Varianten der zuvor genannten spezifischen Polynucleotide. Bei diesen kann es sich um Homologe, Orthologe oder Paraloge Sequenzen handeln. Solche Varainaten umfassen Nukleinsäuresequenzen, die mindesten einen Basenaustausch, eine Basenaddition oder eine Basendeletion aufweisen, wobei die Varaianten immer noch ein Polypeptid mit der zuvor genannten biologischen Aktivität der jeweiligen Ausgangssequenz kodieren sollen. Varaianten umfassen Polynucleotide, die mit den zuvor genannten Polynucleotiden hybridisieren können, bevorzugt unter stringenten Bedingungen. Besonders bevorzugte stringente Bedingungen sind dem Fachmann bekannt und lassen sich in Current Protocols in Molecular Biology, John Wiley & Sons, N. Y. (1989), 6.3.1-6.3.6., finden. Ein bevorzugtes Beispiel für stringente Hybridisierungsbedingungen sind Hybridisierungen in 6 x Natriumchlorid/Natriumcitrat (sodium chloride/sodiumcitrate = SSC) bei etwa 45°C, gefolgt von einem oder mehreren Waschschritten in 0,2 x SSC, 0,1 % SDS bei 50 bis 65°C. Dem Fachmann ist bekannt, dass diese Hybridisierungsbedingungen sich je nach dem Typ der Nukleinsäure und, wenn beispielsweise organische Lösungsmittel vorliegen, hinsichtlich der Temperatur und der Konzentration des Puffers unterscheiden. Die Temperatur unterscheidet sich beispielsweise unter "Standard-Hybridisierungsbedingungen" je nach dem Typ der Nukleinsäure zwischen 42°C und 58°C in wässrigem Puffer mit einer Konzentration von 0,1 bis 5 x SSC (pH 7,2). Falls organisches Lösungsmittel im obengenannten Puffer vorliegt, zum Beispiel 50 % Formamid, ist die Temperatur unter Standardbedingungen etwa 42°C. Vorzugsweise sind die Hybridisierungsbedingungen für DNA:DNA-Hybride zum Beispiel 0,1 x SSC und 20°C bis 45°C, vorzugsweise zwischen 30°C und 45°C. Vorzugsweise sind die Hybridisierungsbedingungen für DNA:RNA-Hybride zum Beispiel 0,1 x SSC und 30°C bis 55°C, vorzugsweise zwischen 45°C und 55°C. Die vorstehend genannten Hybridisierungstemperaturen sind beispielsweise für eine Nukleinsäure mit etwa 100 bp (= Basenpaare) Länge und einem G + C-Gehalt von 50 % in Abwesenheit von Formamid bestimmt. Der Fachmann weiß, wie die erforderlichen Hybridisierungsbedingungen anhand von Lehrbüchern, wie dem vorstehend erwähnten oder aus den folgenden Lehrbüchern Sambrook et al., "Molecular Cloning", Cold Spring Harbor Laboratory, 1989; Hames und Higgins (Hrsgb.) 1985, "Nucleic Acids Hybridization: A Practical Approach", IRL Press at Oxford University Press, Oxford; Brown (Hrsgb.) 1991, "Essential Molecular Biology: A Practical Approach", IRL Press at Oxford University Press, Oxford, bestimmt werden können. Alternativ können Varaianten der spezifischen Polynucleotide auch durch Polymerasekettenreaktion (PCR) basierte Verfahren bereitgestellt werden. Hierzu können zunächst Primer von konservierten Sequenzen (z.B. Sequenzen, die funktionelle Domänen im Polypeptid kodieren) abgeleitet werden. Konserviere Sequenzen können durch Sequenzvergleiche mit Polynucleotiden, die Polypeptide ähnlicher Aktivität kodieren, ermittelt werden. Als Matritze kann DNA oder cDNA aus Bakterien, Pilzen, Pflanzen oder Tieren vedrwendet werden. DNA Fragmente, die durch die PCR erhalten wurde, können zum Screening von entsprechenden genomischen oder cDNA Bibliotheken verwendet werden um, - falls erforderlich - den kompletten offenen Leserahmen des Polynucleotids zu islieren und durch Sequenzierung zu ermitteln. Weitere Varianten umfassen Polynucleotide, die eine Nukleinsäuresequnz umfassen, die mindestens 70%, mindestens 75%, mindestens 80%, mindesten 81%, mindestens 82%, mindestens 83%, mindestens 84%, mindestens 85%, mindestens 86%, mindestens 87%, mindestens 88%, mindestens 89%, mindestens 90%, mindestens 91%, mindestens 92%, mindestens 93%, mindestens 94%, mindestens 95%, mindestens 96%, mindestens 97%, mindestens 98% oder mindestens 99% oder zu einem anderen hierin genannten Prozentsatz identisch ist mit einer der zuvor genannten spezifischen Nukleinsäuresequenzen und ein Polypeptid mit der jeweiligen biologischen Aktivität kodiert.. Ebenso umfasst sind Polynucleotide, die Nukleinsäuresequenzen umfassen, die ein Polypeptid mit einer Aminosäuresequenz kodieren, die mindestens 70%, mindestens 75%, mindestens 80%, mindesten 81%, mindestens 82%, mindestens 83%, mindestens 84%, mindestens 85%, mindestens 86%, mindestens 87%, mindestens 88%, mindestens 89%, mindestens 90%, mindestens 91%, mindestens 92%, mindestens 93%, mindestens 94%, mindestens 95%, mindestens 96%, mindestens 97%, mindestens 98% oder mindestens 99% oder zu einem anderen hierin genannten Prozentsatz identisch ist mit einer der zuvor genannten spezifischen Aminosäuresequenzen und wobei das Polypeptid die jeweilige biologische Aktivität der Ausgangssequenz aufweist. Der Prozentsatz identischer Nucleotide oder Aminosäuren bezieht sich vorzugsweise auf einen Sequenzabschnitt von mindestens 50% der zu vergleichenden Sequenzen und besonders bevorzugt überdie gesamte Länge der zu vergleichenden Sequenzen. Eine Vielzahl von Programmen, die Algorithmen für solche vergleiche implementieren ist im Stand der Technik eschrieben und kommerziell erhältlich. Insbesondere sei auf die Algorithmen von Needleman und Wunsch oder Smith und Waterman verwiesen, die besonders zuverlässige Ergebnisse liefern. Diese Algorithmen können vorzugsweise durch die folgenden Programme implementiert werden: PileUp (J. Mol. Evolution., 25, 351-360, 1987, Higgins et al., CABIOS, 5 1989: 151-153), Gap und BestFit (Needleman and Wunsch (J. Mol. Biol. 48; 443-453 (1970)) und Smith und Waterman (Adv. Appl. Math. 2; 482-489 (1981))), als Teil der GCG software [Genetics Computer Group, 575 Science Drive, Madison, Wisconsin, USA 53711 (1991)]. Besonders bevorzugt wird der Prozentsatz (%) der Sequenzidentität im Rahmen der vorliegenden Erfindung mit dem GAP Programm über die gesammte Sequenz mit folgenden festgelegten Größen bestimmt: Gap Weight: 50, Length Weight: 3, Average Match: 10.000 und Average Mismatch: 0.000.

Ein Polynucleotid, das lediglich ein Fragment der zuvor genannten Nukleinsäuresequenzen umfasst, ist auch ein Polynucleotid. Das Fragment soll hierbei ein Polypeptid kodieren, das die biologische Aktivität der Ausgangssequenz bzw. des davon kodierten Polypeptids aufweist. Polypeptide, die von solchen Polynucleotiden kodiert werden, umfassen oder bestehen daher aus Domänen der zuvor genannten spezifischen Polypeptide (Ausgangspolypeptide), die die biologische Aktivität vermitteln. Ein Fragment im Sinne der Erfindung umfasst vorzugsweise mindestens 500 aufeinanderfolgende Nucleotide der zuvor genannten spezifischen Sequenzen.

Die Polynucleotid- Varianten weisen vorzugsweise mindestens 10%, mindestens 20%, mindestens 30%, mindestens 40%, mindestens 50%, mindestens 60%, mindestens 70%, mindestens 80% oder mindestens 90% der jeweiligen biologischen Aktivität des Polypeptids, das von der Ausgangssequenz kodiert wird, auf. D.h. die Polypeptide, die von den Polynucleotiden kodiert werden, können am Stoffwechsel von zum Aufbau von Fettsäuren, Fettsäureester wie Diacylglyceride und/oder Triacylglyceride in einem Organismus, bevorzugt in einer Pflanze oder Pflanzenzelle, notwendigen Verbindungen oder am Transport von Molekülen über Membranen teilnehmen, wobei C₁₈-, C₂₀- oder C₂₂-Kohlenstoffketten im Fettsäuremolekül mit Doppelbindungen an mindestens zwei, vorteilhaft drei, vier, fünf oder sechs Stellen gemeint sind.

Die Polynucleotide umfassen entweder die zuvor genannten spezifischen Nukleinsäuresequenzen oder bestehen daraus. D.h. die erfindungsgemäßen Polynucleotide können grundsätzlich auch noch weitere Nukleotide umfassen. Dabei kann es sich vorzugsweise um 3' oder 5' untranslatierte Bereiche der genomischen Nukleinsäuresequenz handeln. Diese bestehen vorzugsweise aus mindestens 100, 200 oder 500 Nukleotiden am 5' Terminus und mindestens 20, 50 oder 100 Nukleotiden am 3' Terminus des kodierenden Bereichs. Weitere Polynucleotide, die zusätzliche Nukleinsäuresequenzen umfassen, sind solche, die für Fusionsproteine kodieren. Solche Fusionsproteine können zusätzlich zu den zuvor genannten Polypeptiden weiter Polypeptid bzw. Polypeptidanteile kodieren. Bei dem zusätzlichen Polypeptid bzw. Polypeptidanteil kann es sich um weitere Enzyme der Lipid- oder Fettsäure Biosynthese handeln. Auch denkbar sind Polypeptide, die als Marker für die Expression dienen können (Grün-, Gelb-, Rot-, Blau-fluoreszierende Proteine, alkalische Phosphatase u.a.) bzw. sogenannte "tags" als Marker oder Hilfe für die Aufreinigung (z.B. FLAG-tags, 6-Histidin-tags, MYC-tags u.a.).

Polynucleotid- Varianten können von verschiedenen natürlichen oder künstlichen Quellen isoliert werden. Beispielsweise können sie künstlich durch in vitro oder in vivo Mutagenese erzeugt werden. Homologe oder Orthologe der spezifischen Sequenzen können aus den verschiedensten Tieren, Pflanzen oder Mikroorganismen gewonnen werden. Vorzugsweise werden sie aus Algen gewonnen. Besonders bevorzugt sind Algen der Familie der Prasinophyceae wie aus den Gattungen Heteromastix, Mammella, Mantoniella, Micromonas, Nephroselmis, Ostreococcus, Prasinocladus, Prasinococcus, Pseudoscourfielda, Pycnococcus, Pyramimonas, Scherffelia oder Tetraselmis wie den Gattungen und Arten Heteromastix longifillis, Mamiella gilva, Mantoniella squamata, Micromonas pusilla, Nephroselmis olivacea, Nephroselmis pyriformis, Nephroselmis rotunda, Ostreococcus tauri, Ostreococcus sp. Prasinocladus ascus, Prasinocladus lubricus, Pycnococcus provasolii, Pyramimonas amylifera, Pyramimonas disomata, Pyramimonas obovata, Pyramimonas orientalis, Pyramimonas parkeae, Pyramimonas spinifera, Pyramimonas sp., Tetraselmis apiculata, Tetraselmis carteriaformis, Tetraselmis chui, Tetraselmis convolutae, Tetraselmis desikacharyi, Tetraselmis gracilis, Tetraselmis hazeni, Tetraselmis impellucida, Tetraselmis inconspicua, Tetraselmis levis, Tetraselmis maculata, Tetraselmis marina, Tetraselmis striata, Tetraselmis subcordiformis, Tetraselmis suecica, Tetraselmis tetrabrachia, Tetraselmis tetrathele, Tetraselmis verrucosa, Tetraselmis verrucosa fo. Rubens oder Tetraselmis sp. Vorteilhafterweise stammen die Polynucleotide aus Algen der Gattungen Mantonielle oder Ostreococcus. Ebenso bevorzugt sind Algen wie Isochrysis oder Crypthecodinium, Algen/Diatomeen wie Thalassiosira, Phaeodactylum oder Thraustochytrium, Moose wie Physcomitrella oder Ceratodon, ganz besonders bevorzugt sind die Algen der Gattungen Mantonielle oder Ostreococcus oder die Diatomeen der Gattungen Thalassiosira oder Crypthecodinium. Die Polynucleotide können ebenfalls bevorzugt aus höheren Pflanzen gewonnen werden, wie den Primulaceae wie Aleuritia, Calendula stellata, Osteospermum spinescens oder Osteospermum hyoseroides, Mikroorganismen wie Pilzen wie Aspergillus, Thraustochytrium, Phytophthora, Entomophthora, Mucor oder Mortierella, Bakterien wie Shewanella, Hefen oder Tieren wie Nematoden z.B. Caenorhabditis, Insekten oder Fischen. Die Polynucleotid- Varianten stammen ebenfalls bevorzugt aus einem Tier aus der Ordnung der Vertebraten. Besonders bevorzugt stammen die Polynucleotide aus der Klasse der Vertebrata; Euteleostomi, Actinopterygii; Neopterygii; Teleostei; Euteleostei, Protacanthopterygii, Salmoniformes; Salmonidae bzw. Oncorhynchus und, ganz besonders bevorzugt, aus der Ordnung der Salmoniformes wie der Familie der Salmonidae wie der Gattung Salmo beispielsweise aus den Gattungen und Arten Oncorhynchus mykiss, Trutta trutta oder Salmo trutta fario. Die Polynucleotide können hierbei mittels molekularbiologischer Standardtechniken und der hier bereitgestellten Sequenzinformation isoliert werden. Auch kann Mithilfe von Vergleichsalgorithmen beispielsweise eine homologe Sequenz oder homologe, konservierte Sequenzbereiche auf DNA oder Aminosäureebene identifiziert werden. Diese können als Hybridisierungssonde sowie Standard-Hybridisierungstechniken (wie z.B. beschrieben in Sambrook et al., Molecular Cloning: A Laboratory Manual. 2. Aufl., Cold Spring Harbor Laboratory, Cold Spring Harbor Laboratory Press, Cold Spring Harbor, NY, 1989) zur Isolierung weiterer im Verfahren nützlicher Nukleinsäuresequenzen verwendet werden. Überdies lassen sich Polynucleotide oder Fragmente davon, durch Polymerasekettenreaktion (PCR) isolieren, wobei Oligonukleotidprimer, die auf der Basis dieser Sequenz oder von Teilen davon, verwendet werden (z.B. kann ein Nukleinsäuremolekül, umfassend die vollständigen Sequenz oder einen Teil davon, durch Polymerasekettenreaktion unter Verwendung von Oligonukleotidprimern isoliert werden, die auf der Basis dieser gleichen Sequenz erstellt worden sind). Zum Beispiel lässt sich mRNA aus Zellen isolieren (z.B. durch das Guanidiniumthiocyanat-Extraktionsverfahren von Chirgwin et al. (1979) Biochemistry 18:5294-5299) und cDNA mittels Reverser Transkriptase (z.B. Moloney-MLV-Reverse-Transkriptase, erhältlich von Gibco/BRL, Bethesda, MD, oder AMV-Reverse-Transkriptase, erhältlich von Seikagaku America, Inc., St.Petersburg, FL) herstellen. Synthetische Oligonukleotidprimer zur Amplifizierung mittels Polymerasekettenreaktion lassen sich auf der Basis der in den SEQ ID Nummern dargestellten Aminosäuresequenzen erstellen. Eine Nukleinsäure kann unter Verwendung von cDNA oder alternativ von genomischer DNA als Matrize und geeigneten Oligonukleotidprimern gemäß Standard-PCR-Amplifikationstechniken amplifiziert werden. Die so amplifizierte Nukleinsäure kann in einen geeigneten Vektor kloniert werden und mittels DNA-Sequenzanalyse charakterisiert werden. Oligonukleotide, die einer Desaturase-Nukleotidsequenz entsprechen, können durch Standard-Syntheseverfahren, beispielsweise mit einem automatischen DNA-Synthesegerät, hergestellt werden.

Die Polynucleotide können entweder als isolierte Polynucleotide (d.h. isoliert aus ihrem natürlichen Ursprung, z.B. dem genomischen Locus) oder in genetisch veränderter Form bereitgestellt werden (d.h. die Polynucleotide können auch an ihrem natürlichen genetischen Locus vorliegen, müssen dann aber genetisch verändert werden). Ein isoliertes Polynucleotid umfasst vorzugsweise weniger als 5 kb, 4 kb, 3 kb, 2 kb, 1 kb, 0,5 kb oder 0,1 kb an Nukleinsäuresequenz, die natürlicherweise in seiner Nachbarschaft vorkommt. Das erfindugsgemäße Polynucleotid kann als einzelsträngiges oder doppelsträngiges Nukleinsäuremolekül vorliegen und genomische DNA, cDNA oder RNA sein. Die Polynucleotide umfassen alle Orientierungen der in den SEQ ID Nummern aufgezeigten Sequenzen, d.h. auch komplementäre Stränge sowie reverse oder revers-komplementäre Orientierungen. Ferner umfasst der Begriff auch chemisch modifizierte Nucleinsäuren, wie die natürlich vorkommenden methylierten DNA Moleküle, oder artifizielle Nucleinsäuren z.B. biotinylierte Nukleinsären.

Auch Oligonucleotide von mindestens 15 bp, bevorzugt mindestens 20 bp, mindestens 25 bp, mindestens 30 bp, mindestens 35 bp, oder mindestens 50 bp, die spezifisch mit einem der zuvor genannten Polynucleotide unter stringenten Bedingungen hybridisieren können sind beschrieben. Die Oliguncleotide können aus DNA oder RNA oder beidem bestehen. Solche Oligonucleotide können als Primer für die PCR, als die Expression hemmende, antisense Oligonucleotide, für RNA Interferenz (RNAi) oder für Chimero- oder Genoplastie Ansätze verwendet werden. RNAi Verfahren sind beispielsweise in Fire et al., Nature (1998) 391:806-811; Fire, Trends Genet. 15, 358-363 (1999); Sharp, RNA interference 2001. Genes Dev. 15,485-490 (2001); Hammond et al. Nature Rev. Genet. 2, 1110-1119 (2001); Tuschl, Chem. Biochem. 2, 239-245 (2001); Hamilton et al., Science 286, 950-952 (1999); Hammond et al., Nature 404, 293-296 (2000); Zamore et al., Cell 101, 25-33 (2000); Bernstein et al., Nature 409, 363-366 (2001); Elbashir et al., Genes Dev. 15, 188-200 (2001); WO 01/29058; WO 99/32619; oder Elbashir et al., 2001 Nature 411: 494-498 beschrieben und dienen der Inhibierung der Genexpression durch Abbau der mRNA. Chimero- oder Genoplastie Ansätze dienen der in vivo Modifizierung (z.B. dem Einfügen von Punktmutationen) in Genen an deren endogenen Loci. Entsprechende Verfahren sind offenbart in US5,565,350, US5,756,325, US5,871,984, US5,731,181, US5,795,972, US6,573,046, US6,211,351, US6,586,184, US6,271,360 und US6,479,292.

Vorteilhafterweise hat sich gezeigt, dass die Polynucleotide zur rekombinanten Herstellung mehrfach ungesättigter Fettsäuren in Wirtszellen und transgenen Organismen besonders effektiv eingesetzt weden können. Insbesondere können die von den Polynucleotiden kodierten Polypeptide mit Δ-12-Desaturase, Δ-4-Desaturase, Δ-5-Desaturase, Δ-5-Elongase, Δ-6-Desaturase und Δ-6-Elongase Aktivität C₁₈-, C₂₀- und C₂₂-Fettsäuren mit ein, zwei, drei, vier oder fünf Doppelbindungen und bevorzugt mehrfach ungesättigte C₁₈-Fettsäuren mit ein, zwei oder drei Doppelbindungen wie C18:1^{Δ9}, C18:2^{Δ9,12} oder C18:3 ^{Δ9,12,15}, mehrfach ungesättigte C₂₀-Fettsäuren mit drei oder vier Doppelbindungen wie C20:3^{Δ8,11,14} oder C20:4^{Δ8,11,14,17} oder mehrfach ungesättigte C₂₂-Fettsäuren mit vier oder fünf Doppelbindungen wie C22:4^{Δ7,10,13,16} oder C22:5^{Δ7,10,13,16,19} umsetzen. Bevorzugt werden die Fettsäuren in Phospholipiden oder CoA-Fettsäureestern desaturiert, vorteilhaft in den CoA-Fettsäureester. Somit ist eine einfache, kostengünstige Herstellung dieser mehrfach ungesättigten Fettsäuren speziell in eukaryontischen Systemen möglich. Die mittels der erfindungsgemäßen Polynucleotide hergestellten ungesättigten Fettsäuren können dann als Öl-, Lipid- und Fettsäurezusammensetzungen formuliert und entsprechend eingesetzt werden.

Die vorliegende Erfindung betrifft weiterhin einen Vektor, der das erfindungsgemäße Polynucleotid umfasst.

Der Begriff "Vektor" bezeichnet ein Nukleinsäuremolekül, das ein anderes Nukleinsäuremolekül, wie die erfindungsgemäßen Polynucleotide, transportieren kann, an welche es gebunden ist. Ein Vektortyp ist ein Plasmid, das für eine zirkuläre doppelsträngige DNA-Schleife steht, in die zusätzlichen DNA-Segmente ligiert werden können. Ein weiterer Vektortyp ist ein viraler Vektor, wobei zusätzliche DNA-Segmente in das virale Genom ligiert werden können. Bestimmte Vektoren können in einer Wirtszelle, in die sie eingebracht worden sind, autonom replizieren (z.B. Bakterienvektoren mit bakteriellem Replikationsursprung). Andere Vektoren werden vorteilhaft beim Einbringen in die Wirtszelle in das Genom einer Wirtszelle integriert und dadurch zusammen mit dem Wirtsgenom repliziert. Zudem können bestimmte Vektoren die Expression von Genen, mit denen sie funktionsfähig verbunden sind, steuern. Diese Vektoren werden hier auch als Expressionsvektoren bezeichnet. Gewöhnlich haben Expressionsvektoren, die für DNA-Rekombinationstechniken geeignet sind, die Form von Plasmiden. In der vorliegenden Beschreibung können die Begriffe Plasmid und Vektor austauschbar verwendet werden, da das Plasmid die am häufigsten verwendete Vektorform ist. Die Erfindung soll jedoch diese anderen Expressionsvektorformen, wie virale Vektoren, die ähnliche Funktionen ausüben, umfassen. Ferner soll der Begriff Vektor auch andere Vektoren, die dem Fachmann bekannt sind, wie Phagen, Viren, wie SV40, CMV, TMV, Transposons, IS-Elemente, Phasmide, Phagemide, Cosmide, lineare oder zirkuläre DNA, künstliche Chromosomen, umfassen. Der Begriff umfasst schließlich auch Konstrukte für zielgerichtete, d.h. homologe Rekombination, oder heterologe Insertion von Polynucleotiden.

Vektoren lassen sich in prokaryotische oder eukaryotische Zellen über herkömmliche Transformations- oder Transfektionstechniken einbringen. Die Begriffe "Transformation" und "Transfektion", Konjugation und Transduktion, wie hier verwendet, sollen eine Vielzahl von im Stand der Technik bekannten Verfahren zum Einbringen fremder Nukleinsäure (z.B. DNA) in eine Wirtszelle, einschließlich Calciumphosphat- oder Calciumchlorid-Copräzipitation, DEAE-Dextran-vermittelte Transfektion, Lipofektion, natürliche Kompetenz, chemisch vermittelter Transfer, Elektroporation oder Teilchenbeschuss, umfassen. Geeignete Verfahren zur Transformation oder Transfektion von Wirtszellen, einschließlich Pflanzenzellen, lassen sich finden in Sambrook et al. (Molecular Cloning: A Laboratory Manual., 2. Aufl., Cold Spring Harbor Laboratory, Cold Spring Harbor Laboratory Press, Cold Spring Harbor, NY, 1989) und anderen Labor-Handbüchern, wie Methods in Molecular Biology, 1995, Bd. 44, Agrobacterium protocols, Hrsgb: Gartland und Davey, Humana Press, Totowa, New Jersey.

Geeignete Klonierungsvektoren sind dem Fachmann allgemein bekannt. Hierzu gehören insbesondere Vektoren, die in mikrobiellen Systemen replizierbar sind, also vor allem Vektoren, die eine effiziente Klonierung in Hefen oder Pilze gewährleisten, und die stabile Transformation von Pflanzen ermöglichen. Zu nennen sind insbesondere verschiedene für die T-DNA-vermittelte Transformation geeignete, binäre und cointegrierte Vektorsysteme. Derartige Vektorsysteme sind in der Regel dadurch gekennzeichnet, dass sie zumindest die für die Agrobakterium-vermittelte Transformation benötigten vir-Gene sowie die T-DNA begrenzenden Sequenzen (T-DNA-Border) beinhalten. Vorzugsweise umfassen diese Vektorsysteme auch weitere cis-regulatorische Regionen wie Promotoren und Terminatoren und/oder Selektionsmarker, mit denen entsprechend transformierte Organismen identifiziert werden können. Während bei co-integrierten Vektorsystemen vir-Gene und T-DNA-Sequenzen auf demselben Vektor angeordnet sind, basieren binäre Systeme auf wenigstens zwei Vektoren, von denen einer vir-Gene, aber keine T-DNA und ein zweiter T-DNA, jedoch kein vir-Gen trägt. Dadurch sind letztere Vektoren relativ klein, leicht zu manipulieren und sowohl in E.-coli als auch in Agrobacterium zu replizieren. Zu diesen binären Vektoren gehören Vektoren der Serien pBIB-HYG, pPZP, pBecks, pGreen. Erfindungsgemäß bevorzugt verwendet werden Bin19, pBl101, pBinAR, pGPTV und pCAMBIA. Eine Übersicht über binäre Vektoren und ihre Verwendung gibt Hellens et al, Trends in Plant Science (2000) 5, 446-451. Die Vektoren mit den inseriernten erfindungsgemäßen Polynucleotiden lassen sich in Mikroorganismen, insbesondere Escherichia coli und Agrobacterium tumefaciens, unter selektiven Bedingungen stabil propagieren und ermöglichen einen Transfer von heterologer DNA in Pflanzen oder Mikroorganismen. Mittels der Klonierungsvektoren können die erfindungsgemäßen Polynucleotide in Organismen wie Mikroorganismen oder Pflanzen eingebracht werden und damit zur Pflanzentransformation verwendet werden. Geeignet Vektoren dafür sind veröffentlicht in: Plant Molecular Biology and Biotechnology (CRC Press, Boca Raton, Florida), Kapitel 6/7, S. 71-119 (1993); F.F. White, Vectors for Gene Transfer in Higher Plants; in: Transgenic Plants, Bd. 1, Engineering and Utilization, Hrsgb.: Kung und R. Wu, Academic Press, 1993, 15-38; B. Jenes et al., Techniques for Gene Transfer, in: Transgenic Plants, Bd. 1, Engineering and Utilization, Hrsgb.: Kung und R. Wu, Academic Press (1993), 128-143; Potrykus, Annu. Rev. Plant Physiol. Plant Molec. Biol. 42 (1991), 205-225)).

Bevorzugt handelt es sich bei dem Vektor um einen Expressionsvektor. Bei dem erfindungsgemäßen Expressionsvektor liegt das Polynucleotid in operativer (d.h. funktioneller) Verbindung zu einer Expressionskontrollsequenz vor. Die Expressionskontrollsequenz zusammen mit dem Polynucleotid und optional weiteren Sequenzelementen des Vektors wird auch als Expressionskassette bezeichnet. Durch die Expressionskotrollsequenz wird sichergestellt, dass das Polynucleotid nach Transformation oder Transfektion in eine Wirtszelle exprimiert werden kann. Die zu verwendende Expressionskontrollsequenz enthält vorzugsweise cis-regulatorische Elemente wie Promotor und/oder Enhancer Nukleinsäuresequenzen, die von der Transkriptionsmaschinerie der Wirtszellen erkannt werden. Der Begriff umfasst zudem andere Expressionskontrollelemente z.B. Polyadenylierungssignale und RNA stabilisierende Sequenzen. Diese Regulationssequenzen sind z.B. beschrieben in Goeddel: Gene Expression Technology: Methods in Enzymology 185, Academic Press, San Diego, CA (1990), oder siehe: Gruber und Crosby, in: Methods in Plant Molecular Biology and Biotechnolgy, CRC Press, Boca Raton, Florida, Hrsgb.: Glick und Thompson, Kapitel 7, 89-108, einschließlich der Literaturstellen darin. Expressionskontrollsequenzen umfassen solche, welche die konstitutive Expression einer Nukleotidsequenz in vielen Wirtszelltypen steuern, und solche, welche die direkte Expression der Nukleotidsequenz nur in bestimmten Wirtszellen unter bestimmten Bedingungen steuern. Der Fachmann weiß, dass die Gestaltung des Expressionsvektors von Faktoren, wie der Auswahl der zu transformierenden Wirtszelle, dem Ausmaß der Expression des gewünschten Proteins usw., abhängen kann. Die erfindungsgemäßen Polynucleotide können in einer oder mehreren Kopien in der Expressionskassette oder dem erfindungsgemäßen Expressionsvektor vorliegen (z.B. in Form mehrerer Expressionskassetten). Die regulatorischen Sequenzen bzw. Faktoren können dabei wie oben beschrieben vorzugsweise die Genexpression der eingeführten Gene positiv beeinflussen und dadurch erhöhen. So kann eine Verstärkung der regulatorischen Elemente vorteilhafterweise auf der Transkriptionsebene erfolgen, indem starke Transkriptionssignale wie Promotoren und/oder "Enhancer" verwendet werden. Daneben ist aber auch eine Verstärkung der Translation möglich, indem beispielsweise die Stabilität der mRNA verbessert wird. Weitere Expressionskontrollsequenzen im Sinne der vorliegenden Erfindung sind Translationsterminatoren am 3'-Ende der zu translatierenen Polynucleotide. Verwendet werden kann hier z.B. der OCS1 Terminator. Wie auch für die Promotoren, so sollte hier für jedes zu exprimierende Polynucleotid eine unterschiedliche Terminatorsequenz verwendet werden.

Bevorzugte Expressionskontrollsequenzen bzw. Regulationssequenzen liegen in Promotoren vor, wie dem cos-, tac-, trp-, tet-, trp-tet-, lpp-, lac-, lpp-lac-, laclq-, T7-, T5-, T3-, gal-, trc-, ara-, SP6-, λ-PR- oder λ-PL-Promotor und werden vorteilhafterweise in Gram-negativen Bakterien angewendet. Weitere vorteilhafte Regulationssequenzen liegen beispielsweise in den Gram-positiven Promotoren amy und SPO2, in den Hefe- oder Pilzpromotoren ADC1, MFα, AC, P-60, CYC1, GAPDH, TEF, rp28, ADH oder in den Pflanzenpromotoren CaMV/35S [Franck et al., Cell 21 (1980) 285-294], PRP1 [Ward et al., Plant. Mol. Biol. 22 (1993)], SSU, OCS, lib4, usp, STLS1, B33, nos oder im Ubiquitin- oder Phaseolin-Promotor vor. In diesem Zusammenhang vorteilhaft sind ebenfalls induzierbare Promotoren, wie die in EP-A-0 388 186 (Benzylsulfonamid-induzierbar), Plant J. 2, 1992:397-404 (Gatz et al., Tetracyclininduzierbar), EP-A-0 335 528 (Abzisinsäure-induzierbar) oder WO 93/21334 (Ethanol- oder Cyclohexenol-induzierbar) beschriebenen Promotoren. Weitere geeignete Pflanzenpromotoren sind der Promotor von cytosolischer FBPase oder der ST-LSI-Promotor der Kartoffel (Stockhaus et al., EMBO J. 8, 1989, 2445), der Phosphoribosylprophosphatamidotransferase-Promotor aus Glycine max (Genbank-Zugangsnr. U87999) oder der in EP-A-0 249 676 beschriebene nodienspezifische Promotor. Besonders vorteilhafte Promotoren sind Promotoren, welche die Expression in Geweben ermöglichen, die an der Fettsäurebiosynthese beteiligt sind. Ganz besonders vorteilhaft sind samenspezifische Promotoren, wie der ausführungsgemäße USP Promotor aber auch andere Promotoren wie der LeB4-, DC3, Phaseolin- oder Napin-Promotor. Weitere besonders vorteilhafte Promotoren sind samenspezifische Promotoren, die für monokotyle oder dikotyle Pflanzen verwendet werden können und in US 5,608,152 (Napin-Promotor aus Raps), WO 98/45461 (Oleosin-Promotor aus Arobidopsis), US 5,504,200 (Phaseolin-Promotor aus Phaseolus vulgaris), WO 91/13980 (Bce4-Promotor aus Brassica), von Baeumlein et al., Plant J., 2, 2, 1992:233-239 (LeB4-Promotor aus einer Leguminose) beschrieben sind, wobei sich diese Promotoren für Dikotyledonen eignen. Die folgenden Promotoren eignen sich beispielsweise für Monokotyledonen Ipt-2- oder Ipt-1-Promotor aus Gerste (WO 95/15389 und WO 95/23230), Hordein-Promotor aus Gerste und andere, in WO 99/16890 beschriebene geeignete Promotoren. Es ist im Prinzip möglich, alle natürlichen Promotoren mit ihren Regulationssequenzen, wie die oben genannten, als Expressionskontrollsequenzen zu verwenden. Es ist ebenfalls möglich, zusätzlich oder alleine synthetische Promotoren zu verwenden, besonders wenn sie eine Samenspezifische Expression vermitteln, wie z.B. beschrieben in WO 99/16890.

Um einen besonders hohen Gehalt an PUFAs vor allem in transgenen Pflanzen zu erzielen, sollten die Polynucleotide der vorliegenden Erfindung bevorzugt samenspezifisch in Ölsaaten exprimiert werden. Hierzu können Samen-spezifische Promotoren verwendet werden, bzw. solche Promotoren die im Embryo und/oder im Endosperm aktiv sind. Samen-spezifische Promotoren können prinzipiell sowohl aus dikotolydonen als auch aus monokotolydonen Pflanzen isoliert werden. Im Folgenden sind vorteilhafte bevorzugte Promotoren aufgeführt: USP (= unknown seed protein) und Vicilin (Vicia faba) [Bäumlein et al., Mol. Gen Genet., 1991, 225(3)], Napin (Raps) [US 5,608,152], Acyl-Carrier Protein (Raps) [US 5,315,001 und WO 92/18634], Oleosin (Arabidopsis thaliana) [WO 98/45461 und WO 93/20216], Phaseolin (Phaseolus vulgaris) [US 5,504,200], Bce4 [WO 91/13980], Leguminosen B4 (LegB4-Promotor) [Bäumlein et al., Plant J., 2,2, 1992], Lpt2 und Ipt1 (Gerste) [WO 95/15389 u. WO95/23230], Samenspezifische Promotoren aus Reis, Mais u. Weizen [WO 99/16890], Amy32b, Amy 6-6 und Aleurain [US 5,677,474], Bce4 (Raps) [US 5,530,149], Glycinin (Soja) [EP 571 741], Phosphoenol-Pyruvatcarboxylase (Soja) [JP 06/62870], ADR12-2 (Soja) [WO 98/08962], Isocitratlyase (Raps) [US 5,689,040] oder α-Amylase (Gerste) [EP 781 849].

Die Pflanzengenexpression lässt sich auch über einen chemisch induzierbaren Promotor erleichtern (siehe eine Übersicht in Gatz 1997, Annu. Rev. Plant Physiol. Plant Mol. Biol., 48:89-108). Chemisch induzierbare Promotoren eignen sich besonders, wenn gewünscht wird, dass die Genexpression auf zeitspezifische Weise erfolgt. Beispiele für solche Promotoren sind ein Salicylsäure-induzierbarer Promotor (WO 95/19443), ein Tetracyclin-induzierbarer Promotor (Gatz et al. (1992) Plant J. 2, 397-404) und ein Ethanol-induzierbarer Promotor.

Um eine stabile Integration der verschiedenen Biosynthesegene in die transgene Pflanze über mehrere Generation sicherzustellen, sollte jedes der erfindungsgemäßen Polynucleotide unter der Kontrolle eines eigenen bevorzugt eines unterschiedlichen Promotors exprimiert werden, da sich wiederholende Sequenzmotive zu Instabilität der T-DNA bzw. zu Rekombinationsereignissen führen können. Die Expressionskassette ist dabei vorteilhaft so aufgebaut, dass einem Promotor eine geeignete Schnittstelle zur Insertion der zu exprimierenden Nukleinsäure folgt vorteilhaft in einem Polylinker anschließend gegebenenfalls ein Terminator hinter dem Polylinker liegt. Diese Abfolge wiederholt sich mehrfach bevorzugt drei-, vier- oder fünfmal, so dass bis zu fünf Gene in einem Konstrukt zusammengeführt werden und so zur Expression in die transgene Pflanze eingebracht werden können. Vorteilhaft wiederholt sich die Abfolge bis zu dreimal. Die Nukleinsäuresequenzen werden zur Expression über die geeignete Schnittstelle, beispielsweise im Polylinker, hinter den Promotor inseriert. Vorteilhaft hat jede Nukleinsäuresequenz ihren eigenen Promotor und gegebenenfalls ihren eigenen Terminator. Derartige vorteilhafte Konstrukte werden beispielsweise in DE 10102337 oder DE 10102338 offenbart. Es ist aber auch möglich mehrere Nukleinsäuresequenzen hinter einem Promotor und ggf. vor einem Terminator zu inserieren. Dabei ist die Insertionsstelle bzw. die Abfolge der inserierten Nukleinsäuren in der Expressionskassette nicht von entscheidender Bedeutung, das heißt eine Nukleinsäuresequenz kann an erster oder letzter Stelle in der Kassette inseriert sein, ohne dass dadurch die Expression wesentlich beeinflusst wird. Es können in der Expressionskassette vorteilhaft unterschiedliche Promotoren wie beispielsweise der USP-, LegB4 oder DC3-Promotor und unterschiedliche Terminatoren verwendet werden. Es ist aber auch möglich nur einen Promotortyp in der Kassette zu verwenden. Dies kann jedoch zu unerwünschten Rekombinationsereignissen führen.

Die verwendeten rekombinanten Expressionsvektoren können zur Expression in prokaryotischen oder eukaryotischen Zellen gestaltet sein. Dies ist vorteilhaft, da häufig Zwischenschritte der Vektorkonstruktion der Einfachheit halber in Mikroorganismen durchgeführt werden. Beispielsweise können die Δ-12-Desaturase-, Δ-6-Desaturase-, Δ-6-Elongase-, Δ-5-Desaturase-, Δ-5-Elongase- und/oder Δ-4-Desaturase-Gene in bakteriellen Zellen, Insektenzellen (unter Verwendung von Baculovirus-Expressionsvektoren), Hefe- und anderen Pilzzellen (siehe Romanos, M.A., et al. (1992) "Foreign gene expression in yeast: a review", Yeast 8:423-488; van den Hondel, C.A.M.J.J., et al. (1991) "Heterologous gene expression in filamentous fungi", in: More Gene Manipulations in Fungi, J.W. Bennet & L.L. Lasure, Hrsgb., S. 396-428: Academic Press: San Diego; und van den Hondel, C.A.M.J.J., & Punt, P.J. (1991) "Gene transfer systems and vector development for filamentous fungi, in: Applied Molecular Genetics of Fungi, Peberdy, J.F., et al., Hrsgb., S. 1-28, Cambridge University Press: Cambridge), Algen (Falciatore et al., 1999, Marine Biotechnology.1, 3:239-251), Ciliaten der Typen: Holotrichia, Peritrichia, Spirotrichia, Suctoria, Tetrahymena, Paramecium, Colpidium, Glaucoma, Platyophrya, Potomacus, Desaturaseudocohnilembus, Euplotes, Engelmaniella und Stylonychia, insbesondere der Gattung Stylonychia lemnae, mit Vektoren nach einem Transformationsverfahren, wie beschrieben in WO 98/01572, sowie bevorzugt in Zellen vielzelliger Pflanzen (siehe Schmidt, R. und Willmitzer, L. (1988) "High efficiency Agrobacterium tumefaciens-mediated transformation of Arabidopsis thaliana leaf and cotyledon explants" Plant Cell Rep.:583-586; Plant Molecular Biology and Biotechnology, C Press, Boca Raton, Florida, Kapitel 6/7, S.71-119 (1993); F.F. White, B. Jenes et al., Techniques for Gene Transfer, in: Transgenic Plants, Bd. 1, Engineering and Utilization, Hrsgb.: Kung und R. Wu, Academic Press (1993), 128-43; Potrykus, Annu. Rev. Plant Physiol. Plant Molec. Biol. 42 (1991), 205-225 (und darin zitierte Literaturstellen)) exprimiert werden. Geeignete Wirtszellen werden ferner erörtert in Goeddel, Gene Expression Technology: Methods in Enzymology 185, Academic Press, San Diego, CA (1990). Der rekombinante Expressionsvektor kann alternativ, zum Beispiel unter Verwendung von T7-Promotor-Regulationssequenzen und T7-Polymerase, in vitro transkribiert und translatiert werden.

Die Expression von Proteinen in Prokaryoten erfolgt meist mit Vektoren, die konstitutive oder induzierbare Promotoren enthalten, welche die Expression von Fusions- oder nicht-Fusionsproteinen steuern. Typische Fusions-Expressionsvektoren sind u.a. pGEX (Pharmacia Biotech Inc; Smith, D.B., und Johnson, K.S. (1988) Gene 67:31-40), pMAL (New England Biolabs, Beverly, MA) und pRIT5 (Pharmacia, Piscataway, NJ), bei denen Glutathion-S-Transferase (GST), Maltose E-bindendes Protein bzw. Protein A an das rekombinante Zielprotein fusioniert wird. Beispiele für geeignete induzierbare nicht-Fusions-E. coli-Expressionsvektoren sind u.a. pTrc (Amann et al. (1988) Gene 69:301-315) und pET 11d (Studier et al., Gene Expression Technology: Methods in Enzymology 185, Academic Press, San Diego, Kalifornien (1990) 60-89). Die Zielgenexpression vom pTrc-Vektor beruht auf der Transkription durch Wirts-RNA-Polymerase von einem Hybrid-trp-lac-Fusionspromotor. Die Zielgenexpression aus dem pET 11d-Vektor beruht auf der Transkription von einem T7-gn10-lac-FusionsPromotor, die von einer coexprimierten viralen RNA-Polymerase (T7 gn1) vermittelt wird. Diese virale Polymerase wird von den Wirtsstämmen BL21 (DE3) oder HMS174 (DE3) von einem residenten λ-Prophagen bereitgestellt, der ein T7 gn1-Gen unter der Transkriptionskontrolle des lacUV 5-Promotors birgt. Andere in prokaryotischen Organismen geeignete Vektoren sind dem Fachmann bekannt, diese Vektoren sind beispielsweise in E. coli pLG338, pACYC184, die pBR-Reihe, wie pBR322, die pUC-Reihe, wie pUC18 oder pUC19, die M113mp-Reihe, pKC30, pRep4, pHS1, pHS2, pPLc236, pMBL24, pLG200, pUR290, pIN-III113-B1, λgt11 or pBdCl, in Streptomyces pIJ101, pIJ364, pIJ702 oder pIJ361, in Bacillus pUB110, pC194 oder pBD214, in Corynebacterium pSA77 oder pAJ667.

Bei einer weiteren Ausführungsform ist der Expressionsvektor ein Hefe-Expressionsvektor. Beispiele für Vektoren zur Expression in der Hefe S. cerevisiae umfassen pYeDesaturasec1 (Baldari et al. (1987) Embo J. 6:229-234), pMFa (Kurjan und Herskowitz (1982) Cell 30:933-943), pJRY88 (Schultz et al. (1987) Gene 54:113-123) sowie pYES2 (Invitrogen Corporation, San Diego, CA). Vektoren und Verfahren zur Konstruktion von Vektoren, die sich zur Verwendung in anderen Pilzen, wie den filamentösen Pilzen, eignen, umfassen diejenigen, die eingehend beschrieben sind in: van den Hondel, C.A.M.J.J., & Punt, P.J. (1991) "Gene transfer systems and vector development for filamentous fungi, in: Applied Molecular Genetics of fungi, J.F. Peberdy et al., Hrsgb., S. 1-28, Cambridge University Press: Cambridge, oder in: More Gene Manipulations in Fungi [J.W. Bennet & L.L. Lasure, Hrsgb., S. 396-428: Academic Press: San Diego]. Weitere geeignete Hefevektoren sind beispielsweise pAG-1, YEp6, YEp13 oder pEMBLYe23.

Alternativ können die Polynucleotide der vorliegenden Erfindung auch in Insektenzellen unter Verwendung von Baculovirus-Expressionsvektoren exprimiert werden. Baculovirus-Vektoren, die zur Expression von Proteinen in gezüchteten Insektenzellen (z.B. Sf9-Zellen) verfügbar sind, umfassen die pAc-Reihe (Smith et al. (1983) Mol. Cell Biol.. 3:2156-2165) und die pVL-Reihe (Lucklow und Summers (1989) Virology 170:31-39).

Bevorzugte Pflanzen-Expressionsvektoren umfassen solche, die eingehend beschrieben sind in: Becker, D., Kemper, E., Schell, J., und Masterson, R. (1992) "New plant binary vectors with selectable markers located proximal to the left border", Plant Mol. Biol. 20:1195-1197; und Bevan, M.W. (1984) "Binary Agrobacterium vectors for plant transformation", Nucl. Acids Res. 12:8711-8721; Vectors for Gene Transfer in Higher Plants; in: Transgenic Plants, Bd. 1, Engineering and Utilization, Hrsgb.: Kung und R. Wu, Academic Press, 1993, S. 15-38. Eine Pflanzen-Expressionskassette enthält vorzugsweise Expressionskontrollsequenzen, welche die Genexpression in Pflanzenzellen steuern können und funktionsfähig verbunden sind, so dass jede Sequenz ihre Funktion, wie Termination der Transkription, erfüllen kann, beispielsweise Polyadenylierungssignale. Bevorzugte Polyadenylierungssignale sind diejenigen, die aus Agrobacterium tumefaciens-T-DNA stammen, wie das als Octopinsynthase bekannte Gen 3 des Ti-Plasmids pTiACH5 (Gielen et al., EMBO J. 3 (1984) 835ff.) oder funktionelle Äquivalente davon, aber auch alle anderen in Pflanzen funktionell aktiven Terminatoren sind geeignet. Da die Pflanzengenexpression sehr oft nicht auf Transkriptionsebenen beschränkt ist, enthält eine Pflanzen-Expressionskassette vorzugsweise andere funktionsfähig verbunden Sequenzen, wie Translationsenhancer, beispielsweise die Overdrive-Sequenz, welche die 5'-untranslatierte Leader-Sequenz aus Tabakmosaikvirus, die das Protein/RNA-Verhältnis erhöht, enthält (Gallie et al., 1987, Nucl. Acids Research 15:8693-8711). Die Pflanzengenexpression muss wie oben beschrieben funktionsfähig mit einem geeigneten Promotor verbunden sein, der die Genexpression auf rechtzeitige, zell- oder gewebespezifische Weise durchführt. Nutzbare Promotoren sind konstitutive Promotoren (Benfey et al., EMBO J. 8 (1989) 2195-2202), wie diejenigen, die von Pflanzenviren stammen, wie 35S CAMV (Franck et al., Cell 21 (1980) 285-294), 19S CaMV (siehe auch US 5352605 und WO 84/02913) oder Pflanzenpromotoren, wie der in US 4,962,028 beschriebene der kleinen Untereinheit der Rubisco. Andere bevorzugte Sequenzen für die Verwendung zur funktionsfähigen Verbindung in Pflanzengenexpressions-Kassetten sind Targeting-Sequenzen, die zur Steuerung des Genproduktes in sein entsprechendes Zellkompartiment notwendig sind (siehe eine Übersicht in Kermode, Crit. Rev. Plant Sci. 15, 4 (1996) 285-423 und darin zitierte Literaturstellen), beispielsweise in die Vakuole, den Zellkern, alle Arten von Plastiden, wie Amyloplasten, Chloroplasten, Chromoplasten, den extrazellulären Raum, die Mitochondrien, das Endoplasmatische Retikulum, Ölkörper, Peroxisomen und andere Kompartimente von Pflanzenzellen.

Die Pflanzengenexpression lässt sich auch wie oben beschrieben über einen chemisch induzierbaren Promotor erleichtern (siehe eine Übersicht in Gatz 1997, Annu. Rev. Plant Physiol. Plant Mol. Biol., 48:89-108). Chemisch induzierbare Promotoren eignen sich besonders, wenn gewünscht wird, dass die Genexpression auf zeitspezifische Weise erfolgt. Beispiele für solche Promotoren sind ein Salicylsäure-induzierbarer Promotor (WO 95/19443), ein Tetracyclin-induzierbarer Promotor (Gatz et al. (1992) Plant J. 2, 397-404) und ein Ethanol-induzierbarer Promotor. Auch Promotoren, die auf biotische oder abiotische Stressbedingungen reagieren, sind geeignete Promotoren, beispielsweise der pathogeninduzierte PRP1-Gen-Promotor (Ward et al., Plant. Mol. Biol. 22 (1993) 361-366), der hitzeinduzierbare hsp80-Promotor aus Tomate (US 5,187,267), der kälteinduzierbare Alpha-Amylase-Promotor aus Kartoffel (WO 96/12814) oder der durch Wunden induzierbare pinll-Promotor (EP-A-0 375 091).

Es sind insbesondere solche Promotoren bevorzugt, welche die Genexpression in Geweben und Organen herbeiführen, in denen die Fettsäure-, Lipid- und Ölbiosynthese stattfindet, in Samenzellen, wie den Zellen des Endosperms und des sich entwickelnden Embryos. Geeignete Promotoren sind der Napingen-Promotor aus Raps (US 5,608,152), der USP-Promotor aus Vicia faba (Baeumlein et al., Mol Gen Genet, 1991, 225 (3):459-67), der Oleosin-Promotor aus Arabidopsis (WO 98/45461), der Phaseolin-Promotor aus Phaseolus vulgaris (US 5,504,200), der Bce4-Promotor aus Brassica (WO 91/13980) oder der Legumin-B4-Promotor (LeB4; Baeumlein et al., 1992, Plant Journal, 2 (2):233-9) sowie Promotoren, welche die samenspezifische Expression in Monokotyledonen-Pflanzen, wie Mais, Gerste, Weizen, Roggen, Reis usw. herbeiführen. Geeignete beachtenswerte Promotoren sind der Ipt2- oder Ipt1-Gen-Promotor aus Gerste (WO 95/15389 und WO 95/23230) oder die in WO 99/16890 beschriebenen (Promotoren aus dem Gersten-Hordein-Gen, dem Reis-Glutelin-Gen, dem Reis-Oryzin-Gen, dem Reis-Prolamin-Gen, dem Weizen-Gliadin-Gen, Weizen-Glutelin-Gen, dem Mais-Zein-Gen, dem Hafer-Glutelin-Gen, dem Sorghum-Kasirin-Gen, dem Roggen-Secalin-Gen). Ebenfalls besonders geeignet sind Promotoren, welche die plastidenspezifische Expression herbeiführen, da Plastiden dasKompartiment sind, in dem die Vorläufer sowie einige Endprodukte der Lipidbiosynthese synthetisiert werden. Geeignete Promotoren, wie der virale RNA-Polymerase-Promotor, sind beschrieben in WO 95/16783 und WO 97/06250, und der cIpP-Promotor aus Arabidopsis, beschrieben in WO 99/46394.

Die oben genannten Vektoren bieten nur einen kleinen Überblick über mögliche geeignete Vektoren. Weitere Plasmide sind dem Fachmann bekannt und sind zum Beispiel beschrieben in: Cloning Vectors (Hrsgb. Pouwels, P.H., et al., Elsevier, Amsterdam-New York-Oxford, 1985, ISBN 0 444 904018). Weitere geeignete Expressionssysteme für prokaryotische und eukaryotische Zellen siehe in den Kapiteln 16 und 17 von Sambrook, J., Fritsch, E.F., und Maniatis, T., Molecular Cloning: A Laboratory Manual, 2. Auflage, Cold Spring Harbor Laboratory, Cold Spring Harbor Laboratory Press, Cold Spring Harbor, NY, 1989.

Der Expressionsvektor kann, wie oben beschrieben, auch weitere Gene umfassen, die in die Organismen eingebracht werden sollen. Es ist möglich und bevorzugt, in die Wirtsorganismen Regulationsgene, wie Gene für Induktoren, Repressoren oder Enzyme, welche durch ihre Enzymaktivität in die Regulation eines oder mehrerer Gene eines Biosynthesewegs eingreifen, einzubringen und darin zu exprimieren. Diese Gene können heterologen oder homologen Ursprungs sein. Heterologe Gene oder Polynucleotide stammen aus einem Ursprungsorganismus, der sich von dem Zielorganismus unterscheidet, in den die Gene oder Polynucleotide eingebracht werden sollen. Bei homologen Gene oder Polynucleotide sind Zielorganismus und Ursprungsorganismus gleich. Vorzugsweise umfasst der Vektor daher mindestens ein weiteres Polynucleotid, das ein weiteres Enzym kodiert, das in die Biosynthese von Lipiden oder Fettsäuren eingebunden ist. Das Enzym ist vorzugsweise ausgewählt aus der Gruppe bestehend aus: Acyl-CoA-Dehydrogenase(n), Acyl-ACP[= acyl carrier protein]-Desaturase(n), Acyl-ACP-Thioesterase(n), Fettsäure-Acyl-Transferase(n), Acyl-CoA:Lysophospholipid-Acyltransferase(n), Fettsäure-Synthase(n), Fettsäure-Hydroxylase(n), Acetyl-Coenzym A-Carboxylase(n), Acyl-Coenzym A-Oxidase(n), Fettsäure-Desaturase(n), Fettsäure-Acetylenase(n), Lipoxygenase(n), Triacylglycerol-Lipase(n), Allenoxid-Synthase(n), Hydroperoxid-Lyase(n), Fettsäure-Elongase(n), Δ4-Desaturase(n), Δ5-Desaturase(n), Δ6-Desaturase(n), Δ8-Desaturase(n), Δ9-Desaturase(n), Δ12-Desaturase(n), Δ5-Elongase(n), Δ6-Elongase(n) und Δ9-Elongase(n).

Die Erfindung betrifft auch eine Wirtszelle, die das erfindungsgemäße Polynucleotid oder den erfindungsgemäßen Vektor umfasst.

Wirtszellen im Sinne der vorliegenden Erfindung können prinzipiell alle eukaryotischen oder prokaryotischn Zellen sein. Es können primäre Zellen aus Tieren, Pflanzen oder mehrzelligen Mikroorganismen sein, z.B. aus denen, die in der Beschreibung an anderer Stelle genannt sind. Ferner umfasst der Begriff auch Zelllinien, die aus diesen Organismen gewonnen werden können.

Wirtszellen im Sinne der Erfindung können aber auch einzellige Mikroorganismen sein, z.B. Bakterien oder Pilze. Besonders bevorzugte Mikroorganismen sind Pilze ausgewählt aus der Gruppe der Familien Chaetomiaceae, Choanephoraceae, Cryptococcaceae, Cunninghamellaceae, Demetiaceae, Moniliaceae, Mortierellaceae, Mucoraceae, Pythiaceae, Sacharomycetaceae, Saprolegniaceae, Schizosacharomycetaceae, Sodariaceae oder Tuberculariaceae. Weitere evorzugte Mikroorganismen sind ausgewählt aus der Gruppe: Choanephoraceae wie den Gattungen Blakeslea, Choanephora z.B. die Gattungen und Arten *Blakeslea trispora, Choanephora cucurbitarum, Choanephora infundibulifera* var. *cucurbitarum,* Mortierellaceae wie der Gattung Mortierella z.B. die Gattungen und Arten *Mortierella isabellina, Mortierella polycephala, Mortierella ramanniana, Mortierella vinacea, Mortierella zonata,* Pythiaceae wie den Gattungen Phytium, Phytophthora z.B. die Gattungen und Arten *Pythium debaryanum, Pythium intermedium, Pythium irregulare, Pythium megalacanthum, Pythium paroecandrum, Pythium sylvaticum, Pythium ultimum, Phytophthora cactorum, Phytophthora cinnamomi, Phytophthora citricola, Phytophthora citrophthora, Phytophthora cryptogea, Phytophthora drechsleri, Phytophthora erythroseptica, Phytophthora lateralis, Phytophthora megasperma, Phytophthora nicotianae, Phytophthora nicotianae var. parasitica, Phytophthora palmivora, Phytophthora parasitica, Phytophthora syringae,* Saccharomycetaceae wie den Gattungen Hansenula, Pichia, Saccharomyces, Saccharomycodes, Yarrowia z.B. die Gattungen und Arten *Hansenula anomala, Hansenula californica, Hansenula canadensis, Hansenula capsulata, Hansenula ciferrii, Hansenula glucozyma, Hansenula henricii, Hansenula holstii, Hansenula minuta, Hansenula nonfermentans, Hansenula philodendri, Hansenula polymorpha, Hansenula saturnus, Hansenula subpelliculosa, Hansenula wickerhamii, Hansenula wingei, Pichia alcoholophila, Pichia angusta, Pichia anomala, Pichia bispora, Pichia burtonii, Pichia canadensis, Pichia capsulata, Pichia carsonii, Pichia cellobiosa, Pichia ciferrii, Pichia farinosa, Pichia fermentans, Pichia finlandica, Pichia glucozyma, Pichia guilliermondii, Pichia haplophila, Pichia henricii, Pichia holstii, Pichia jadinii, Pichia lindnerii, Pichia membranaefaciens, Pichia methanolica, Pichia minuta var. minuta, Pichia minuta var. nonfermentans, Pichia norvegensis, Pichia ohmeri, Pichia pastoris, Pichia philodendri, Pichia pini, Pichia polymorpha, Pichia quercuum, Pichia rhodanensis, Pichia sargentensis, Pichia stipitis, Pichia strasburgensis, Pichia subpelliculosa, Pichia toletana, Pichia trehalophila, Pichia vini, Pichia xylosa, Saccharomyces aceti, Saccharomyces bailii, Saccharomyces bayanus, Saccharomyces bisporus, Saccharomyces capensis, Saccharomyces carlsbergensis, Saccharomyces cerevisiae, Saccharomyces cerevisiae var. ellipsoideus, Saccharomyces chevalieri, Saccharomyces delbrueckii, Saccharomyces diastaticus, Saccharomyces drosophilarum, Saccharomyces elegans, Saccharomyces ellipsoideus, Saccharomyces fermentati, Saccharomyces florentinus, Saccharomyces fragilis, Saccharomyces heterogenicus, Saccharomyces hienipiensis, Saccharomyces inusitatus, Saccharomyces italicus, Saccharomyces kluyveri, Saccharomyces krusei, Saccharomyces lactis, Saccharomyces marxianus, Saccharomyces microellipsoides, Saccharomyces montanus, Saccharomyces norbensis, Saccharomyces oleaceus, Saccharomyces paradoxus, Saccharomyces pastorianus, Saccharomyces pretoriensis, Saccharomyces rosei, Saccharomyces rouxii, Saccharomyces uvarum, Saccharomycodes ludwigii, Yarrowia lipolytica,* Schizosacharomycetaceae such as the genera Schizosaccharomyces e.g. the species *Schizosaccharomyces japonicus var. japonicus, Schizosaccharomyces japonicus var. versatilis, Schizosaccharomyces malidevorans, Schizosaccharomyces octosporus, Schizosaccharomyces pombe var. malidevorans, Schizosaccharomyces pombe var. pombe,* Thraustochytriaceae such as the genera Althornia, Aplanochytrium, Japonochytrium, Schizochytrium, Thraustochytrium e.g. the species *Schizochytrium aggregatum, Schizochytrium limacinum, Schizochytrium mangrovei, Schizochytrium minutum, Schizochytrium octosporum, Thraustochytrium aggregatum, Thraustochytrium amoeboideum, Thraustochytrium antacticum, Thraustochytrium arudimentale, Thraustochytrium aureum, Thraustochytrium benthicola, Thraustochytrium globosum, Thraustochytrium indicum, Thraustochytrium kerguelense, Thraustochytrium kinnei, Thraustochytrium motivum, Thraustochytrium multirudimentale, Thraustochytrium pachydermum, Thraustochytrium proliferum, Thraustochytrium roseum, Thraustochytrium rossii, Thraustochytrium striatum oder Thraustochytrium visurgense.*

Ebenfalls bevorzugt als Mikroorganismen sind Bakterien ausgewählt aus der Gruppe der Familien Bacillaceae, Enterobacteriacae oder Rhizobiaceae. Besonders bevorzugt seien die folgenden Bakterinen genannt ausgewählt aus der Gruppe: Bacillaceae wie die Gattung Bacillus z.B die Gattungen und Arten *Bacillus acidocaldarius, Bacillus acidoterrestris, Bacillus alcalophilus, Bacillus amyloliquefaciens, Bacillus amylolyticus, Bacillus brevis, Bacillus cereus, Bacillus circulans, Bacillus coagulans, Bacillus sphaericus subsp. fusiformis, Bacillus galactophilus, Bacillus globisporus, Bacillus globisporus subsp. marinus, Bacillus halophilus, Bacillus lentimorbus, Bacillus lentus, Bacillus licheniformis, Bacillus megaterium, Bacillus polymyxa, Bacillus psychrosaccharolyticus, Bacillus pumilus, Bacillus sphaericus, Bacillus subtilis subsp. spizizenii, Bacillus subtilis subsp. subtilis* oder *Bacillus thuringiensis;* Enterobacteriacae wie die Gattungen Citrobacter, Edwardsiella, Enterobacter, Erwinia, Escherichia, Klebsiella, Salmonella oder Serratia z.B die Gattungen und Arten *Citrobacter amalonaticus, Citrobacter diversus, Citrobacter freundii, Citrobacter genomospecies, Citrobacter gillenii, Citrobacter intermedium, Citrobacter koseri, Citrobacter murliniae, Citrobacter sp., Edwardsiella hoshinae, Edwardsiella ictaluri, Edwardsiella tarda, Erwinia alni, Erwinia amylovora, Erwinia ananatis, Erwinia aphidicola, Erwinia billingiae, Erwinia cacticida, Erwinia cancerogena, Erwinia carnegieana, Erwinia carotovora* subsp. *atroseptica, Erwinia carotovora* subsp. *betavasculorum, Erwinia carotovora* subsp. *odorifera, Erwinia carotovora* subsp. *wasabiae, Erwinia chrysanthemi, Erwinia cypripedii, Erwinia dissolvens, Erwinia herbicola, Erwinia mallotivora, Erwinia milletiae, Erwinia nigrifluens, Erwinia nimipressuralis, Erwinia persicina, Erwinia psidii, Erwinia pyrifoliae, Erwinia quercina, Erwinia rhapontici, Erwinia rubrifaciens, Erwinia salicis, Erwinia stewartii, Erwinia tracheiphila, Erwinia uredovora, Escherichia adecarboxylata, Escherichia anindolica, Escherichia aurescens, Escherichia blattae, Escherichia coli, Escherichia coli var. communior, Escherichia coli-mutabile, Escherichia fergusonii, Escherichia hermannii, Escherichia sp., Escherichia vulneris, Klebsiella aerogenes, Klebsiella edwardsii subsp. atlantae, Klebsiella ornithinolytica, Klebsiella oxytoca, Klebsiella planticola, Klebsiella pneumoniae, Klebsiella pneumoniae subsp. pneumoni ae, Klebsiella sp., Klebsiella terrigena, Klebsiella trevisanii, Salmonella abony, Salmonella arizonae, Salmonella bongori, Salmonella choleraesuis subsp. arizonae, Salmonella choleraesuis subsp. bongori, Salmonella choleraesuis subsp. cholereasuis, Salmonella choleraesuis subsp. diarizonae, Salmonella choleraesuis subsp. houtenae, Salmonella choleraesuis subsp. indica, Salmonella choleraesuis subsp. salamae, Salmonella daressalaam, Salmonella enterica subsp. houtenae, Salmonella enterica subsp. salamae, Salmonella enteritidis, Salmonella gallinarum, Salmonella heidelberg, Salmonella panama, Salmonella senftenberg, Salmonella typhimurium, Serratia entomophila, Serratia ficaria, Serratia fonticola, Serratia grimesii, Serratia liquefaciens, Serratia marcescens, Serratia marcescens subsp. marcescens, Serratia marinorubra, Serratia odorifera, Serratia plymouthensis, Serratia plymuthica, Serratia proteamaculans, Serratia proteamaculans subsp. quinovora, Serratia quinivorans* oder *Serratia rubidaea;* Rhizobiaceae wie die Gattungen Agrobacterium, Carbophilus, Chelatobacter, Ensifer, Rhizobium, Sinorhizobium z.B. die Gattungen und Arten *Agrobacterium atlanticum, Agrobacterium ferrugineum, Agrobacterium gelatinovorum, Agrobacterium larrymoorei, Agrobacterium meteori, Agrobacterium radiobacter, Agrobacterium rhizogenes, Agrobacterium rubi, Agrobacterium stellulatum, Agrobacterium tumefaciens, Agrobacterium vitis, Carbophilus carboxidus, Chelatobacter heintzii, Ensifer adhaerens, Ensifer arboris, Ensifer fredii, Ensifer kostiensis, Ensifer kummerowiae, Ensifer medicae, Ensifer meliloti, Ensifer saheli, Ensifer terangae, Ensifer xinjiangensis, Rhizobium ciceri Rhizobium etli, Rhizobium fredii, Rhizobium galegae, Rhizobium gallicum, Rhizobium giardinii, Rhizobium hainanense, Rhizobium huakuii, Rhizobium huautlense, Rhizobium indigoferae, Rhizobium japonicum, Rhizobium leguminosarum, Rhizobium loessense, Rhizobium loti, Rhizobium lupini, Rhizobium mediterraneum, Rhizobium meliloti, Rhizobium mongolense, Rhizobium phaseoli, Rhizobium radiobacter, Rhizobium rhizogenes, Rhizobium rubi, Rhizobium sullae, Rhizobium tianshanense, Rhizobium trifolii, Rhizobium tropici, Rhizobium undicola, Rhizobium vitis, Sinorhizobium adhaerens, Sinorhizobium arboris, Sinorhizobium fredii, Sinorhizobium kostiense, Sinorhizobium kummerowiae, Sinorhizobium medicae, Sinorhizobium meliloti, Sinorhizobium morelense, Sinorhizobium saheli* oder *Sinorhizobium xinjiangense.*

Nutzbare Wirtszellen sind weiterhin genannt in: Goeddel, Gene Expression Technology: Methods in Enzymology 185, Academic Press, San Diego, CA (1990).Verwendbare Expressionsstämme z.B. solche, die eine geringere Proteaseaktivität aufweisen sind beschrieben in: Gottesman, S., Gene Expression Technology: Methods in Enzymology 185, Academic Press, San Diego, California (1990) 119-128. Hierzu gehören Pflanzenzellen und bestimmte Gewebe, Organe und Teile von Pflanzen in all ihren Erscheinungsformen, wie Antheren, Fasern, Wurzelhaare, Stängel, Embryos, Kalli, Kotelydonen, Petiolen, Erntematerial, pflanzliches Gewebe, reproduktives Gewebe und Zellkulturen, das von der eigentlichen transgenen Pflanze abgeleitet ist und/oder dazu verwendet werden kann, die transgene Pflanze hervorzubringen.

Polynucleotide oder Vektoren können mit im Stand der Technik bekannten Transformations- bzw. Transfektionsverfahren in die Wirtszelle eingebracht werden wie zuvor beschrieben. Bedingungen und Medien für die Kultivierung der Wirtszellen sind dem Fachmann ebenfalls bekannt.

Bevorzugt umfasst die erfindungsgemäße Wirtszelle zusätzlich mindestens ein weiteres Enzym, das in die Biosynthese von Lipiden oder Fettsäuren eingebunden ist. Bevorzugte Enzyme sind bereits an anderer Stelle in der Beschreibung genannt. Das Enzym kann endogen in der Wirtszelle vorliegen, d.h. die Wirtszelle exprimiert bereits natürlicherweise ein Gen, das für ein entsprechendes Enzym kodiert. Alternativ kann auch ein heterologes Polynucleotid in die Wirtszelle eingebracht werden, das für das Enzym kodiert. Geeignete Verfahren und Maßnahmen für die Expression eines heterologen Polynucleotids sind im Stand der Technik bekannt und in Zusammenhang mit den efindungsgemäßen Polynucleotiden, Vektoren und Wirtszellen hier beschrieben.

Die Erfindung betrifft auch ein Verfahren zur Herstellung eines Polypeptids mit Desaturase oder Elongase Aktivität umfassend die Schritte:
(a) Exprimieren eines erfindungsgemäßen Polynucleotids in einer Wirtszelle; und
(b) Gewinnen des Polypeptids, das von dem Polynucleotid kodiert wird, aus der Wirtszelle.

Das Polypeptid kann hierbei durch alle gängigen Verfahren zur Proteinreinigung gewonnen werden. Die Verfahren umfassen beispielseweise Affinitätschromatographie, Molsiebchromatographie, Hochdruck Flüssigkeitschromatographie oder auch Proteinpräzipitation ggf. mit spezifischen Antikörpern. Obwohl dies bevorzugt ist, muss das Verfahren nicht notwendigerweise ein reines Präparat des Polypeptids bereitstellen.

Die Erfindung betrifft somit auch ein Polypeptid, das von dem erfindungsgemäßen Polynucleotid kodiert wird oder das erhätlich ist durch das zuvor genannte erfindungsgemäße Verfahren.

Der Begriff "Polypeptid" bezeichnet sowohl ein im Wesentlichen reines Polypeptid als auch ein Polypeptid Präparat, das noch weitere Komponenten oder Verunreinigungen aufweist. Der Begriff wird auch für Fusionsproteine oder Proteinaggregate verwendet, die das erfindungsgemäße Polypeptid und zusätzlich noch weitere Komponenten umfassen. Der Begriff bezeichnet auch chemisch modifizierte Polypeptide. Chemische Modifikationen beinhalten in diesem Zusammenhang künstliche Modifikationen oder natürlich auftretende Modifikationen, z.B posttranslationale Modifikationen wie Phosphorylierung, Myristylierung, Glykosylierung usw.. Die Begriffe Polypeptid, Peptid oder Protein sind austauschbar und werden entsprechend in der Beschreibung und im Stand der Technik verwendet. Die erfindungsgemäßen Polypeptide haben die zuvor genannten biologischen Aktivitäten, also Desaturase oder Elongase Aktivitäten, und können die Biosynthese von mehrfach ungesättigten Fettsäuren (PUFAs), vorzugsweise den langkettigen PUFAs (LCPUFAs), wie hier beschrieben beeinflussen.

Die Erfindung betrifft ferner einen transgenen, nicht-humanen Organismus, der das Polynucleotid, den Vektor oder die Wirtszelle der vorliegenden Erfindung umfasst. Bevorzugt handelt es sich bei dem transgenen, nicht humanen Organismus um ein Tier, eine Pflanze oder ein multizellulärere Mikroorganismus.

Unter dem Begriff "transgen" ist zu verstehen, dass ein heterologes Polynucleotid, also ein in dem jeweiligen Organismus nicht natürlicherweise vorkommendes Polynucleotid, in den Organismus eingebracht wird. Dies kann entweder durch zufällige Insertion des Polynucleotids oder durch homologe Rekombination erreicht werden. Selbstverständlich kann statt des Polynucleotids auch der erfindungsgemäße Vektor eingebracht werden. Verfahren zum Einbringen von Polynucleotiden oder Vektoren zwecks zufälliger Insertion oder homologer Rekombination sind im Stand der Technik bekannt und auch nachfolgend genauer beschrieben. Wirtszellen, die das Polynucleotid oder den Vektor enthalten, können ebenfalls in einen Organismus eingebracht weden und so einen transgenen Organismus erzeugen. Bei einem solchen Organsimus handelt es sich dann aber um einen chimären Organismus bei dem lediglich die Zellen, die sich von den eingebrachten Zellen ableiten, transgen sind, d.h. das heterologe Polynucleotid umfassen.

Bevorzugt sind die transgenen, nicht-humanen Organismen Öl-produzierenden Organismen, das heißt die für die Herstellung von Ölen verwendet werden, wie Pilze wie Mortierella oder Thraustochytrium, Algen wie Nephroselmis, Pseudoscourfielda, Prasinococcus, Scherffelia, Tetraselmis, Mantoniella, Ostreococcus, Crypthecodinium, Phaeodactylum oder Pflanzen.

Als transgene Pflanzen können grundsätzlich alle Pflanzen verwendet werden, d.h. sowohl zweikeimblättrige als auch einkeimblättrige Pflanzen. Vorzugsweise handelt es sich um Ölfruchtpflanzen, die große Mengen an Lipidverbindungen enthalten, wie Erdnuss, Raps, Canola, Sonnenblume, Saflor (Carthamus tinctoria), Mohn, Senf, Hanf, Rizinus, Olive, Sesam, Calendula, Punica, Nachtkerze, Königskerze, Distel, Wildrosen, Haselnuss, Mandel, Macadamia, Avocado, Lorbeer, Kürbis, Lein, Soja, Pistazien, Borretsch, Bäume (Ölpalme, Kokosnuss oder Walnuss) oder Feldfrüchte, wie Mais, Weizen, Roggen, Hafer, Triticale, Reis, Gerste, Baumwolle, Maniok, Pfeffer, Tagetes, Solanaceen-Pflanzen, wie Kartoffel, Tabak, Aubergine und Tomate, Vicia-Arten, Erbse, Alfalfa oder Buschpflanzen (Kaffee, Kakao, Tee), Salix-Arten sowie ausdauernde Gräser und Futterfeldfrüchte. Bevorzugte erfindungsgemäße Pflanzen sind Ölfruchtpflanzen, wie Erdnuss, Raps, Canola, Sonnenblume, Saflor , Mohn, Senf, Hanf, Rhizinus, Olive, Calendula, Punica, Nachtkerze, Kürbis, Lein, Soja, Borretsch, Bäume (Ölpalme, Kokosnuss). Besonders bevorzugt sind C18:2- und/oder C18:3-Fettsäure reiche Pflanzen wie Sonnenblume, Färberdistel, Tabak, Königskerze, Sesam, Baumwolle, Kürbis, Mohn, Nachtkerze, Walnuss, Lein, Hanf, Distel oder Färberdistel. Ganz besonders bevorzugt sind Pflanzen wie Färberdistel, Sonnenblume, Mohn, Nachtkerze, Walnuss, Lein oder Hanf. Prinzipiell kommen aber alle Pflanzen in Frage, die in der Lage sind Fettsäuren zu synthetisieren wie alle dicotylen oder monokotylen Pflanzen, Algen oder Moose. Vorteilhaft Pflanzen sind ausgewählt aus der Gruppe der Pflanzenfamilien Adelotheciaceae, Anacardiaceae, Asteraceae, Apiaceae, Betulaceae, Boraginaceae, Brassicaceae, Bromeliaceae, Caricaceae, Cannabaceae, Convolvulaceae, Chenopodiaceae, Crypthecodiniaceae, Cucurbitaceae, Ditrichaceae, Elaeagnaceae, Ericaceae, Euphorbiaceae, Fabaceae, Geraniaceae, Gramineae, Juglandaceae, Lauraceae, Leguminosae, Linaceae, Prasinophyceae oder Gemüsepflanzen oder Zierpflanzen wie Tagetes in Betracht.

Besonders bevorzugt seien die folgenden Pflanzen genannt ausgewählt aus der Gruppe: Adelotheciaceae wie die Gattungen Physcomitrella z.B. die Gattung und Arten *Physcomitrella patens,* Anacardiaceae wie die Gattungen Pistacia, Mangifera, Anacardium z.B. die Gattung und Arten *Pistacia vera* [Pistazie], *Mangifer indica* [Mango] oder *Anacardium occidentale* [Cashew], Asteraceae wie die Gattungen Calendula, Carthamus, Centaurea, Cichorium, Cynara, Helianthus, Lactuca, Locusta, Tagetes, Valeriana z.B. die Gattung und Arten Calendula officinalis [Garten-Ringelblume], Carthamus tinctorius [Färberdistel, safflower], *Centaurea cyanus* [Kornblume], *Cichorium intybus* [Wegwarte], Cynara scolymus [Artichoke], *Helianthus annus* [Sonnenblume], *Lactuca sativa, Lactuca crispa, Lactuca esculenta, Lactuca scariola L. ssp. sativa, Lactuca scariola L. var. integrata, Lactuca scariola L. var. integrifolia, Lactuca sativa subsp. romana, Locusta communis, Valeriana locusta* [Salat], *Tagetes lucida, Tagetes erecta* oder *Tagetes tenuifolia* [Studentenblume], Apiaceae wie die Gattung Daucus z.B. die Gattung und Art *Daucus carota* [Karotte], Betulaceae wie die Gattung Corylus z.B. die Gattungen und Arten *Corylus avellana* oder *Corylus colurna* [Haselnuss], Boraginaceae wie die Gattung Borago z.B. die Gattung und Art *Borago officinalis* [Borretsch], Brassicaceae wie die Gattungen Brassica, Camelina, Melanosinapis, Sinapis, Arabadopsis z.B. die Gattungen und Arten *Brassica napus, Brassica rapa* ssp. [Raps], *Sinapis arvensis Brassica juncea, Brassica juncea var. juncea, Brassica juncea var. crispifolia, Brassica juncea var. foliosa, Brassica nigra, Brassica sinapioides,* Camelina sativa, *Melanosinapis communis* [Senf], *Brassica oleracea* [Futterrübe] oder Arabidopsis thaliana, Bromeliaceae wie die Gattungen Anana, Bromelia (Ananas) z.B. die Gattungen und Arten *Anana comosus, Ananas ananas* oder *Bromelia comosa* [Ananas], Caricaceae wie die Gattung Carica wie die Gattung und Art *Carica papaya* [Papaya], Cannabaceae wie die Gattung Cannabis wie die Gattung und Art *Cannabis sative* [Hanf], Convolvulaceae wie die Gattungen Ipomea, Convolvulus z.B. die Gattungen und Arten *Ipomoea batatus, Ipomoea pandurata, Convolvulus batatas, Convolvulus tiliaceus, Ipomoea fastigiata, Ipomoea tiliacea, Ipomoea triloba* oder *Convolvulus panduratus* [Süßkartoffel, Batate], Chenopodiaceae wie die Gattung Beta wie die Gattungen und Arten *Beta vulgaris, Beta vulgaris var. altissima, Beta vulgaris var. Vulgaris, Beta maritima, Beta vulgaris var. perennis, Beta vulgaris var. conditiva* oder *Beta vulgaris var. esculenta* [Zuckerrübe], Crypthecodiniaceae wie die Gattung Crypthecodinium z.B. die Gattung und Art *Cryptecodinium cohnii,* Cucurbitaceae wie die Gattung Cucubita z.B. die Gattungen und Arten *Cucurbita maxima, Cucurbita mixta, Cucurbita pepo* oder *Cucurbita moschata* [Kürbis], Cymbellaceae wie die Gattungen Amphora, Cymbella, Okedenia, Phaeodactylum, Reimeria z.B. die Gattung und Art *Phaeodactylum tricornutum,* Ditrichaceae wie die Gattungen Ditrichaceae, Astomiopsis, Ceratodon, Chrysoblastella, Ditrichum, Distichium, Eccremidium, Lophidion, Philibertiella, Pleuridium, Saelania, Trichodon, Skottsbergia z.B. die Gattungen und Arten *Ceratodon antarcticus, Ceratodon columbiae, Ceratodon heterophyllus, Ceratodon purpurascens, Ceratodon purpureus, Ceratodon purpureus ssp. convolutus, Ceratodon purpureus ssp. stenocarpus, Ceratodon purpureus var. rotundifolius, Ceratodon ratodon, Ceratodon stenocarpus, Chysoblastella chilensis, Ditrichum ambiguum, Ditrichum brevisetum, Ditrichum crispatissimum, Ditrichum difficile, Ditrichum falcifolium, Ditrichum flexicaule, Ditrichum giganteum, Ditrichum heteromaltum, Ditrichum lineare, Ditrichum lineare, Ditrichum montanum, Ditrichum montanum, Ditrichum pallidum, Ditrichum punctulatum, Ditrichum pusillum, Ditrichum pusillum var. tortile, Ditrichum rhynchostegium, Ditrichum schimperi, Ditrichum tortile, Distichium capillaceum, Distichium hagenii, Distichium inclinatum, Distichium macounii, Eccremidium floridanum, Eccremidium whiteleggei, Lophidion strictus, Pleuridium acuminatum, Pleuridium alternifolium, Pleuridium holdridgei, Pleuridium mexicanum, Pleuridium ravenelii, Pleuridium subulatum, Saelania glaucescens, Trichodon borealis, Trichodon cylindricus oder Trichodon cylindricus var. oblongus,* Elaeagnaceae wie die Gattung Elaeagnus z.B. die Gattung und Art *Olea europaea* [Olive], Ericaceae wie die Gattung Kalmia z.B. die Gattungen und Arten *Kalmia latifolia, Kalmia angustifolia, Kalmia microphylla, Kalmia polifolia, Kalmia occidentalis, Cistus chamaerhodendros* oder *Kalmia lucida* [Berglorbeer], Euphorbiaceae wie die Gattungen Manihot, Janipha, Jatropha, Ricinus z.B. die Gattungen und Arten *Manihot utilissima, Janipha manihot" Jatropha manihot., Manihot aipil, Manihot dulcis, Manihot manihot, Manihot melanobasis, Manihot esculenta* [Manihot] oder *Ricinus communis* [Rizinus], Fabaceae wie die Gattungen Pisum, Albizia, Cathormion, Feuillea, Inga, Pithecolobium, Acacia, Mimosa, Medicajo, Glycine, Dolichos, Phaseolus, Soja z.B. die Gattungen und Arten *Pisum sativum, Pisum arvense, Pisum humile* [Erbse], *Albizia berteriana, Albizia julibrissin, Albizia lebbeck, Acacia berteriana, Acacia littoralis, Albizia berteriana, Albizzia berteriana, Cathormion berteriana, Feuillea berteriana, Inga fragrans, Pithecellobium berterianum, Pithecellobium fragrans, Pithecolobium berterianum, Pseudalbizzia berteriana, Acacia julibrissin, Acacia nemu, Albizia nemu, Feuilleea julibrissin, Mimosa julibrissin, Mimosa speciosa, Sericanrda julibrissin, Acacia lebbeck, Acacia macrophylla, Albizia lebbek, Feuilleea lebbeck, Mimosa lebbeck, Mimosa speciosa* [Seidenbaum], *Medicago sativa, Medicago falcata, Medicago varia* [Alfalfa] *Glycine max Dolichos soja, Glycine gracilis, Glycine hispida, Phaseolus max, Soja hispida* oder *Soja max* [Sojabohne], Funariaceae wie die Gattungen Aphanorrhegma, Entosthodon, Funaria, Physcomitrella, Physcomitrium z.B. die Gattungen und Arten *Aphanorrhegma serratum, Entosthodon attenuatus, Entosthodon bolanderi, Entosthodon bonplandii, Entosthodon californicus, Entosthodon drummondii, Entosthodon jamesonii, Entosthodon leibergii, Entosthodon neoscoticus, Entosthodon rubrisetus, Entosthodon spathulifolius, Entosthodon tucsoni, Funaria americana, Funaria bolanderi, Funaria calcarea, Funaria californica, Funaria calvescens, Funaria convoluta, Funaria flavicans, Funaria groutiana, Funaria hygrometrica, Funaria hygrometrica var. arctica, Funaria hygrometrica var. calvescens, Funaria hygrometrica var. convoluta, Funaria hygrometrica var. muralis, Funaria hygrometrica var. utahensis, Funaria microstoma, Funaria microstoma var. obtusifolia, Funaria muhlenbergii, Funaria orcuttii, Funaria planoconvexa, Funaria polaris, Funaria ravenelii, Funaria rubriseta, Funaria serrata, Funaria sonorae, Funaria sublimbatus, Funaria tucsoni, Physcomitrella californica, Physcomitrella patens, Physcomitrella readeri, Physcomitrium australe, Physcomitrium californicum, Physcomitrium collenchymatum, Physcomitrium coloradense, Physcomitrium cupuliferum, Physcomitrium drummondii, Physcomitrium eurystomum, Physcomitrium flexifolium, Physcomitrium hookeri, Physcomitrium hookeri var. serratum, Physcomitrium immersum, Physcomitrium kellermanii, Physcomitrium megalocarpum, Physcomitrium pyriforme, Physcomitrium pyriforme var. serratum, Physcomitrium rufipes, Physcomitrium sandbergii, Physcomitrium subsphaericum, Physcomitrium washingtoniense,* Geraniaceae wie die Gattungen Pelargonium, Cocos, Oleum z.B. die Gattungen und Arten *Cocos nucifera, Pelargonium grossularioides* oder *Oleum cocois* [Kokusnuss], Gramineae wie die Gattung Saccharum z.B. die Gattung und Art Saccharum officinarum, Juglandaceae wie die Gattungen Juglans, Wallia z.B. die Gattungen und Arten *Juglans regia, Juglans ailanthifolia, Juglans sieboldiana, Juglans cinerea, Wallia cinerea, Juglans bixbyi, Juglans californica, Juglans hindsii, Juglans intermedia, Juglans jamaicensis, Juglans major, Juglans microcarpa, Juglans nigra* oder *Wallia nigra* [Walnuss], Lauraceae Wie die Gattungen Persea, Laurus z.B. die Gattungen und Arten *Laurus nobilis* [Lorbeer], *Persea americana, Persea gratissi ma* oder *Persea persea* [Avocado], Leguminosae wie die Gattung Arachis z.B. die Gattung und Art *Arachis hypogaea* [Erdnuss], Linaceae wie die Gattungen Linum, Adenolinum z.B. die Gattungen und Arten *Linum usitatissimum, Linum humile, Linum austriacum, Linum bienne, Linum angustifolium, Linum catharticum, Linum flavum, Linum grandiflorum, Adenolinum grandiflorum, Linum lewisii, Linum narbonense, Linum perenne, Linum perenne var. lewisii, Linum pratense* oder *Linum trigynum* [Lein], Lythrarieae wie die Gattung Punica z.B. die Gattung und Art *Punica granatum* [Granatapfel], Malvaceae wie die Gattung Gossypium z.B. die Gattungen und Arten *Gossypium hirsutum, Gossypium arboreum, Gossypium barbadense, Gossypium herbaceum* oder *Gossypium thurberi* [Baumwolle], Marchantiaceae wie die Gattung Marchantia z.B. die Gattungen und Arten *Marchantia berteroana, Marchantia foliacea, Marchantia macropora,* Musaceae wie die Gattung Musa z.B. die Gattungen und Arten *Musa nana, Musa acuminata, Musa paradisiaca, Musa* spp. [Banane], Onagraceae wie die Gattungen Camissonia, Oenothera z.B. die Gattungen und Arten *Oenothera biennis* oder *Camissonia brevipes* [Nachtkerze], Palmae wie die Gattung Elacis z.B. die Gattung und Art *Elaeis guineensis* [Ölpalme], Papaveraceae wie die Gattung Papaver z.B. die Gattungen und Arten *Papaver orientale, Papaver rhoeas, Papaver dubium* [Mohn], Pedaliaceae wie die Gattung Sesamum z.B. die Gattung und Art *Sesamum indicum* [Sesam], Piperaceae wie die Gattungen Piper, Artanthe, Peperomia, Steffensia z.B. die Gattungen und Arten *Piper aduncum, Piper amalago, Piper angustifolium, Piper auritum, Piper betel, Piper cubeba, Piper longum, Piper nigrum, Piper retrofractum, Artanthe adunca, Artanthe elongata, Peperomia elongata, Piper elongatum, Steffensia elongata.* [Cayennepfeffer], Poaceae wie die Gattungen Hordeum, Secale, Avena, Sorghum, Andropogon, Holcus, Panicum, Oryza, Zea (Mais), Triticum z.B. die Gattungen und Arten *Hordeum vulgare, Hordeum jubatum, Hordeum murinum, Hordeum secalinum, Hordeum distichon Hordeum aegiceras, Hordeum hexastichon., Hordeum hexastichum, Hordeum irregulare, Hordeum sativum, Hordeum secalinum* [Gerste], *Secale cereale* [Roggen], Avena sativa, *Avena fatua, Avena byzantina, Avena fatua var. sativa, Avena hybrida* [Hafer], *Sorghum bicolor, Sorghum halepense, Sorghum saccharatum, Sorghum vulgare, Andropogon drummondii, Holcus bicolor, Holcus sorghum, Sorghum aethiopicum, Sorghum arundinaceum, Sorghum caffrorum, Sorghum cernuum, Sorghum dochna, Sorghum drummondii, Sorghum durra, Sorghum guineense, Sorghum lanceolatum, Sorghum nervosum, Sorghum saccharatum, Sorghum subglabrescens, Sorghum verticilliflorum, Sorghum vulgare, Holcus halepensis, Sorghum miliaceum, Panicum militaceum [Hirse], Oryza sativa, Oryza latifolia* [Reis], *Zea mays* [Mais] *Triticum aestivum, Triticum durum, Triticum turgidum, Triticum hybernum, Triticum macha, Triticum sativum* oder *Triticum vulgare* [Weizen], Porphyridiaceae wie die Gattungen Chroothece, Flintiella, Petrovanella, Porphyridium, Rhodella, Rhodosorus, Vanhoeffenia z.B. die Gattung und Art *Porphyridium cruentum,* Proteaceae wie die Gattung Macadamia z.B. die Gattung und Art *Macadamia intergrifolia* [Macadamia], Prasinophyceae wie die Gattungen Nephroselmis, Prasinococcus, Scherffelia, Tetraselmis, Mantoniella, Ostreococcus z.B. die Gattungen und Arten *Nephroselmis olivacea, Prasinococcus capsulatus, Scherffelia dubia, Tetraselmis chui, Tetraselmis suecica,* Mantoniella squamata, Ostreococcus tauri, Rubiaceae wie die Gattung Coffea z.B. die Gattungen und Arten Cofea spp., *Coffea arabica, Coffea canephora* oder *Coffea liberica* [Kaffee], Scrophulariaceae wie die Gattung Verbascum z.B. die Gattungen und Arten *Verbascum blattaria, Verbascum chaixii, Verbascum densiflorum, Verbascum lagurus, Verbascum longifolium, Verbascum lychnitis, Verbascum nigrum, Verbascum olympicum, Verbascum phlomoides, Verbascum phoenicum, Verbascum pulverulentum* oder *Verbascum thapsus* [*Königskerze*], Solanaceae wie die Gattungen Capsicum, Nicotiana, Solanum, Lycopersicon z.B. die Gattungen und Arten *Capsicum annuum, Capsicum annuum var. glabriusculum, Capsicum frutescens* [Pfeffer], *Capsicum annuum* [Paprika], *Nicotiana tabacum, Nicotiana alata, Nicotiana attenuata, Nicotiana glauca, Nicotiana langsdorffii, Nicotiana obtusifolia, Nicotiana quadrivalvis, Nicotiana repanda, Nicotiana rustica, Nicotiana sylvestris* [Tabak], *Solanum tuberosum* [Kartoffel], *Solanum melongena* [Aubergine] *Lycopersicon esculentum, Lycopersicon lycopersicum., Lycopersicon pyriforme, Solanum integrifolium* oder *Solanum lycopersicum* [Tomate], Sterculiaceae wie die Gattung Theobroma z.B. die Gattung und Art *Theobroma* cacao [Kakao] oder Theaceae wie die Gattung Camellia z.B. die Gattung und Art *Camellia sinensis* [Tee].

Multizelluläre Mikroorganismen, die als transgene nicht-humane Organismen eingesetzt werden können, sind vorzugsweise Protisten oder Diatomeen ausgewählt aus der Gruppe der Familien Dinophyceae, Turaniellidae oder Oxytrichidae wie die Gattungen und Arten: *Crypthecodinium cohnii, Phaeodactylum tricornutum, Stylonychia mytilus, Stylonychia pustulata, Stylonychia putrina, Stylonychia notophora, Stylonychia sp.,* Colpidium campylum oder Colpidium sp.

Die Erfindung betrifft ein Verfahren zur Herstellung eines Stoffes, der die Struktur aufweist, die in der folgenden allgemeinen Formel I gezeigt wird 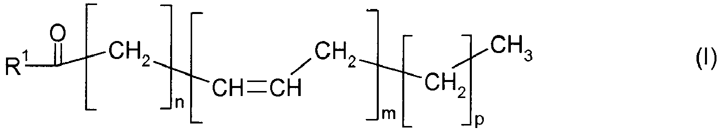 wobei die Variablen und Substiuenten die folgenden sind
- R¹ =: Hydroxyl, Coenzym A (Thioester), Lysophosphatidylcholin, Lysophosphatidylethanolamin, Lysophosphatidylglycerol, Lysodiphosphatidylglycerol, Lysophosphatidylserin, Lysophosphatidylinositol, Sphingo- Base oder ein Radikal der Formel II 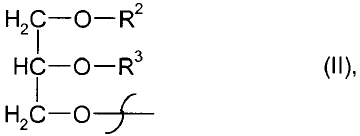
R² = Wasserstoff, Llysophosphatidylcholin, Lysophosphatidylethanolamin, Lysophosphatidylglycerol, Llysodiphosphatidylglycerol, Lysophosphatidylserin, Lysophosphatidylinositol oder gesättigtes oder ungesättigtes C₂-C₂₄-Alkylcarbonyl,
R³ = Wasserstoff, gesättigtes oder ungesättigtes C₂-C₂₄-Alkylcarbonyl, oder R² und R³ sind unabhängig voneinander ein Radikal der Formel la: 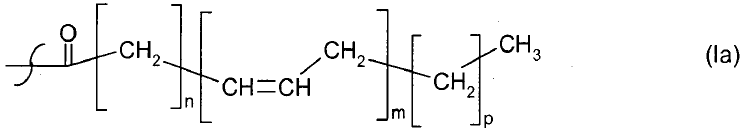
n = 2, 3, 4, 5, 6, 7 or 9, m = 2, 3, 4, 5 oder 6 und p = 0 oder 3;
und wobei das Verfahren das Kultivieren von (i) einer erfindungsgemäßen Wirtszelle oder (ii) eines erfindungsgemäßen transgenen, nicht-humanen unter Bedingungen umfasst, die die Biosynthese des Stoffes erlauben. Vorzugsweise wird dabei der zuvor genannte Stoff in einer Menge von mindestens 1 Gew.-% bezogen auf den Gesamtgehalt der Lipide in der Wirtszelle oder dem transgenen Organismus bereitgestellt.

R¹ bedeutet in der allgemeinen Formel I Hydroxyl-, CoenzymA-(Thioester), Lyso-Phosphatidylcholin-, Lyso-Phosphatidylethanolamin-, Lyso-Phosphatidylglycerol-, Lyso-Diphosphatidylglycerol-, Lyso-Phosphatidylserin-, Lyso-Phosphatidylinositol-, Sphingobase-, oder einen Rest der allgemeinen Formel II

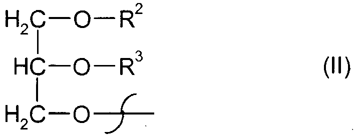

Die oben genannten Reste von R¹ sind immer in Form ihrer Thioester an die Verbindungen der allgemeinen Formel I gebunden.

R² bedeutet in der allgemeinen Formel Wasserstoff-, Lyso-Phosphatidylcholin-, Lyso-Phosphatidylethanolamin-, Lyso-Phosphatidylglycerol-, Lyso-Diphosphatidylglycerol-, Lyso-Phosphatidylserin-, Lyso-Phosphatidylinositol- oder gesättigtes oder ungesättigtes C₂-C₂₄-Alkylcarbonyl.

Als Alkylreste seien substituiert oder unsubstituiert, gesättigt oder ungesättigte C₂-C₂₄-Alkylcarbonyl-Ketten wie Ethylcarbonyl-, n-Propylcarbonyl-, n-Butylcarbonyl-, n-Pentylcarbonyl-, n-Hexylcarbonyl-,n-Heptylcarbonyl-, n-Octylcarbonyl-, n-Nonylcarbonyl-, n-Decylcarbonyl-, n-Undecylcarbonyl-, n-Dodecylcarbonyl-, n-Tridecylcarbonyl-, n-Tetradecylcarbonyl-, n-Pentadecylcarbonyl-, n-Hexadecylcarbonyl-, n-Heptadecylcarbonyl-, n-Octadecylcarbonyl-, n-Nonadecylcarbonyl-, n-Eicosylcarbonyl-, n-Docosanylcarbonyl- oder n-Tetracosanylcarbonyl- genannt, die ein oder mehrere Doppelbindungen enthalten. Gesättigte oder ungesättigte C₁₀-C₂₂-Alkylcarbonylreste wie n-Decylcarbonyl-, n-Undecylcarbonyl-, n-Dodecylcarbonyl-, n-Tridecylcarbonyl-, n-Tetradecylcarbonyl-, n-Pentadecylcarbonyl-, n-Hexadecylcarbonyl-, n-Heptadecylcarbonyl-, n-Octadecylcarbonyl-, n-Nonadecylcarbonyl-, n-Eicosylcarbonyl-, n-Docosanylcarbonyl- oder n-Tetracosanylcarbonyl-., die ein oder mehrere Doppelbindungen enthalten, sind bevorzugt. Besonders bevorzugt sind gesättigte und/oder ungesättigte C₁₀-C₂₂-Alkylcarbonylreste wie C₁₀-Alkylcarbonyl-, C₁₁-Alkylcarbonyl-, C₁₂-Alkylcarbonyl-, C₁₃-Alkylcarbonyl-, C₁₄-Alkylcarbonyl-, C₁₆-Alkylcarbonyl-, C₁₈-Alkylcarbonyl-, C₂₀-Alkylcarbonyl- oder C₂₂-Alkylcarbonylreste, die ein oder mehrere Doppelbindungen enthalten. Ganz besonders bevorzugt sind gesättigte oder ungesättigte C₁₆-C₂₂-Alkylcarbonylreste wie C₁₆-Alkylcarbonyl-, C₁₈-Alkylcarbonyl-, C₂₀-Alkylcarbonyl- oder C₂₂-Alkylcarbonylreste, die ein oder mehrere Doppelbindungen enthalten. Diese vorteilhaften Reste können zwei, drei, vier, fünf oder sechs Doppelbindungen enthalten. Die besonders vorteilhaften Reste mit 20 oder 22 Kohlenstoffatomen in der Fettsäurekette enthalten bis zu sechs Doppelbindungen, vorteilhaft drei, vier, fünf oder sechs Doppelbindungen, besonders bevorzugt fünf oder sechs Doppelbindungen. Alle genannten Reste leiten sich von den entsprechenden Fettsäuren ab.

R³ bedeutet in der allgemeinen Formel II Wasserstoff-, gesättigtes oder ungesättigtes C₂-C₂₄-Alkylcarbonyl.

Als Alkylreste seien substituiert oder unsubstituiert, gesättigt oder ungesättigte C₂-C₂₄-Alkylcarbonyl-Ketten wie Ethylcarbonyl-, n-Propylcarbonyl-, n-Butylcarbonyl-, n-Pentylcarbonyl-, n-Hexylcarbonyl-, n-Heptylcarbonyl-, n-Octylcarbonyl-, n-Nonylcarbonyl-, n-Decylcarbonyl-, n-Undecylcarbonyl-, n-Dodecylcarbonyl-, n-Tridecylcarbonyl-, n-Tetradecylcarbonyl-, n-Pentadecylcarbonyl-, n-Hexadecylcarbonyl-, n-Heptadecylcarbonyl-, n-Octadecylcarbonyl-, n-Nonadecylcarbonyl-, n-Eicosylcarbonyl-, n-Docosanylcarbonyl- oder n-Tetracosanylcarbonyl- genannt, die ein oder mehrere Doppelbindungen enthalten. Gesättigte oder ungesättigte C₁₀-C₂₂-Alkylcarbonylreste wie n-Decylcarbonyl-, n-Undecylcarbonyl-, n-Dodecylcarbonyl-, n-Tridecylcarbonyl-, n-Tetradecylcarbonyl-, n-Pentadecylcarbonyl-, n-Hexadecylcarbonyl-, n-Heptadecylcarbonyl-, n-Octadecylcarbonyl-, n-Nonadecylcarbonyl-, n-Eicosylcarbonyl-, n-Docosanylcarbonyl- oder n-Tetracosanylcarbonyl-, die ein oder mehrere Doppelbindungen enthalten, sind bevorzugt. Besonders bevorzugt sind gesättigte und/oder ungesättigte C₁₀-C₂₂-Alkylcarbonylreste wie C₁₀-Alkylcarbonyl-, C₁₁-Alkylcarbonyl-, C₁₂-Alkylcarbonyl-, C₁₃-Alkylcarbonyl-, C₁₄-Alkylcarbonyl-, C₁₆-Alkylcarbonyl-, C₁₈-Alkylcarbonyl-, C₂₀-Alkylcarbonyl- oder C₂₂-Alkylcarbonylreste, die ein oder mehrere Doppelbindungen enthalten. Ganz besonders bevorzugt sind gesättigte oder ungesättigte C₁₆-C₂₂-Alkylcarbonylreste wie C₁₆-Alkylcarbonyl-, C₁₈-Alkylcarbonyl-, C₂₀-Alkylcarbonyl- oder C₂₂-Alkylcarbonylreste, die ein oder mehrere Doppelbindungen enthalten. Diese vorteilhaften Reste können zwei, drei, vier, fünf oder sechs Doppelbindungen enthalten. Die besonders vorteilhaften Reste mit 20 oder 22 Kohlenstoffatomen in der Fettsäurekette enthalten bis zu sechs Doppelbindungen, vorteilhaft drei, vier, fünf oder sechs Doppelbindungen, besonders bevorzugt fünf oder sechs Doppelbindungen. Alle genannten Reste leiten sich von den entsprechenden Fettsäuren ab.

Die oben genannten Reste von R¹, R² and R³ können mit Hydroxyl- und/oder Epoxygruppen substituierte sein und/oder können Dreifachbindungen enthalten.

Vorteilhaft enthalten die im erfindungsgemäßen Verfahren hergestellten mehrfach ungesättigten Fettsäuren mindestens zwei vorteilhaft drei, vier, fünf oder sechs Doppelbindungen. Besonders vorteilhaft enthalten die Fettsäuren vier fünf oder sechs Doppelbindungen. Im Verfahren hergestellte Fettsäuren haben vorteilhaft 18-, 20- oder 22-C-Atome in der Fettsäurekette, bevorzugt enthalten die Fettsäuren 20 oder 22 Kohlenstoffatome in der Fettsäurekette. Vorteilhaft werden gesättigte Fettsäuren mit den im Verfahren verwendeten Nukleinsäuren wenig oder gar nicht umgesetzt. Unter wenig ist zu verstehen, das im Vergleich zu mehrfach ungesättigten Fettsäuren die gesättigten Fettsäuren mit weniger als 5 % der Aktivität, vorteilhaft weniger als 3 %, besonders vorteilhaft mit weniger als 2 %, ganz besonders bevorzugt mit weniger als 1; 0,5; 0,25 oder 0,125 % umgesetzt werden. Diese hergestellten Fettsäuren können als einziges Produkt im Verfahren hergestellt werden oder in einem Fettsäuregemisch vorliegen.

Vorteilhaft bedeuten die Substituenten R² oder R³ in den allgemeinen Formeln I und II unabhängig voneinander gesättigtes oder ungesättigtes C₁₈-C₂₂-Alkylcarbonyl-, besonders vorteilhaft bedeuten sie unabhängig voneinander ungesättigtes C₁₈-, C₂₀- oder C₂₂-Alkylcarbonyl- mit mindestens zwei Doppelbindungen.

Die im Verfahren hergestellten mehrfach ungesättigten Fettsäuren sind vorteilhaft in Membranlipiden und/oder Triacylglyceriden gebunden, können aber auch als freie Fettsäuren oder aber gebunden in Form anderer Fettsäureester in den Organismen vorkommen. Dabei können sie als "Reinprodukte" oder aber vorteilhaft in Form von Mischungen verschiedener Fettsäuren oder Mischungen unterschiedlicher Glyceride vorliegen. Die in den Triacylglyceriden gebundenen verschieden Fettsäuren lassen sich dabei von kurzkettigen Fettsäuren mit 4 bis 6 C-Atomen, mittelkettigen Fettsäuren mit 8 bis 12 C-Atomen oder langkettigen Fettsäuren mit 14 bis 24 C-Atomen ableiten, bevorzugt sind die langkettigen Fettsäuren besonders bevorzugt sind die langkettigen Fettsäuren LCPUFAs von C₁₈-, C₂₀- und/oder C₂₂-Fettsäuren.

Im erfindungsgemäßen Verfahren werden vorteilhaft Fettsäureester mit mehrfach ungesättigten C₁₈-, C₂₀- und/oder C₂₂-Fettsäuremolekülen mit mindestens zwei Doppelbindungen im Fettsäureester, vorteilhaft mit mindestens drei, vier, fünf oder sechs Doppelbindungen im Fettsäureester, besonders vorteilhaft von mindestens fünf oder sechs Doppelbindungen im Fettsäureester hergestellt und führen vorteilhaft zur Synthese von Linolsäure (=LA, C18:2^{Δ9,12}), γ-Linolensäure (=GLA, C18:3^{Δ6,9,12}), Stearidonsäure (= SDA, C18:4 ^{Δ6,9,12,15}), Dihomo-γ-Linolensäure (=DGLA, 20:3 ^{Δ8,11,14}), ω-3-Eicosatetraensäure (= ETA, C20:4 ^{Δ5,8,11,14}), Arachidonsäure (ARA, C20:4 ^{Δ5,8,11,14}), Eicosapentaensäure (EPA, C20:5^{Δ5,8,11,14,17}), ω-6-Docosapentaensäure (C22:5^{Δ4,7,10,13,16}), ω-6-Docosatetraensäure (C22:4^{Δ,7,10,13,16}), ω-3-Docosapentaensäure (= DPA, C22:5^{Δ7,10,13,16,19}), Docosahexaensäure (= DHA, C22:6^{Δ4,7,10,13,16,19}) oder deren Mischungen, bevorzugt ARA, EPA und/oder DHA. Ganz besonders bevorzugt werden, ω-3-Fettsäuren wie EPA und/oder DHA hergestellt.

Die Fettsäureester mit mehrfach ungesättigten C₁₈-, C₂₀- und/oder C₂₂-Fettsäuremolekülen können aus den Organismen, die für die Herstellung der Fettsäureester verwendet wurden, in Form eines Öls oder Lipids beispielsweise in Form von Verbindungen wie Sphingolipide, Phosphoglyceride, Lipide, Glycolipide wie Glycosphingolipide, Phospholipide wie Phosphatidylethanolamin, Phosphatidylcholin, Phosphatidylserin, Phosphatidylglycerol, Phosphatidylinositol oder Diphosphatidylglycerol, Monoacylglyceride, Diacylglyceride, Triacylglyceride oder sonstige Fettsäureester wie die AcetylCoenzymA-Ester, die die mehrfach ungesättigten Fettsäuren mit mindestens zwei, drei, vier, fünf oder sechs bevorzugt fünf oder sechs Doppelbindungen enthalten, isoliert werden, vorteilhaft werden sie in der Form ihrer Diacylglyceride, Triacylglyceride und/oder in Form des Phosphatidylcholin isoliert, besonders bevorzugt in der Form der Triacylglyceride. Neben diesen Estern sind die mehrfach ungesättigten Fettsäuren auch als freie Fettsäuren oder gebunden in anderen Verbindungen in den Organismen vorteilhaft den Pflanzen enthalten. In der Regel liegen die verschiedenen vorgenannten Verbindungen (Fettsäureester und frei Fettsäuren) in den Organismen in einer ungefähren Verteilung von 80 bis 90 Gew.-% Triglyceride, 2 bis 5 Gew.-% Diglyceride, 5 bis 10 Gew.-% Monoglyceride, 1 bis 5 Gew.-% freie Fettsäuren, 2 bis 8 Gew.-% Phospholipide vor, wobei sich die Summe der verschiedenen Verbindungen zu 100 Gew.-% ergänzt.

Im erfindungsgemäßen Verfahren werden die hergestellten LCPUFAs mit einem Gehalt von mindestens 3 Gew.-%, vorteilhaft von mindestens 5 Gew.-%, bevorzugt von mindestens 8 Gew.-%, besonders bevorzugt von mindestens 10 Gew.-%, ganz besonders bevorzugt von mindestens 15 Gew.-% bezogen auf die gesamten Fettsäuren in den transgenen Organismen vorteilhaft in einer transgenen Pflanze hergestellt. Dabei werden vorteilhaft C₁₈- und/oder C₂₀-Fettsäuren, die in den Wirtsorganismen vorhanden sind, zu mindestens 10 %, vorteilhaft zu mindestens 20 %, besonders vorteilhaft zu mindestens 30 %, ganz besonders vorteilhaft zu mindestens 40 % in die entsprechenden Produkte wie DPA oder DHA, um nur zwei beispielhaft zu nennen, umgesetzt. Vorteilhaft werden die Fettsäuren in gebundener Form hergestellt. Mit Hilfe der im erfindungsgemäßen Verfahren verwendeten Nukleinsäuren lassen sich diese ungesättigten Fettsäuren an sn1-, sn2- und/oder sn3-Position der vorteilhaft hergestellten Triglyceride bringen. Da im erfindungsgemäßen Verfahren von den Ausgangsverbindungen Linolsäure (C18:2) bzw. Linolensäure (C18:3) mehrere Reaktionsschritte durchlaufen werden, fallen die Endprodukte des Verfahrens wie beispielsweise Arachidonsäure (ARA), Eicosapentaensäure (EPA), ω-6-Docosapentaensäure oder DHA nicht als absolute Reinprodukte an, es sind immer auch geringe Spuren der Vorstufen im Endprodukt enthalten. Sind in dem Ausgangsorganismus bzw. in der Ausgangspflanze beispielsweise sowohl Linolsäure als auch Linolensäure vorhanden, so liegen die Endprodukte wie ARA, EPA oder DHA als Mischungen vor. Die Vorstufen sollten vorteilhaft nicht mehr als 20 Gew.-%, bevorzugt nicht mehr als 15Gew.-%, besonders bevorzugt nicht als 10 Gew.-%, ganz besonders bevorzugt nicht mehr als 5 Gew.-% bezogen auf die Menge des jeweilige Endproduktes betragen. Vorteilhaft werden in einer transgenen Pflanze als Endprodukte nur ARA, EPA oder nur DHA im erfindungsgemäßen Verfahren gebunden oder als freie Säuren hergestellt. Werden die Verbindungen ARA, EPA und DHA gleichzeitig hergestellt, werden sie vorteilhaft in einem Verhältnis von mindesten 1:1:2 (EPA:ARA:DHA), vorteilhaft von mindestens 1:1:3, bevorzugt von 1:1:4, besonders bevorzugt von 1:1:5 hergestellt.

Fettsäureester bzw. Fettsäuregemische, die nach dem erfindungsgemäßen Verfahren hergestellt wurden, enthalten vorteilhaft 6 bis 15 % Palmitinsäure, 1 bis 6 % Stearinsäure; 7 - 85 % Ölsäure; 0,5 bis 8 % Vaccensäure, 0,1 bis 1 % Arachinsäure, 7 bis 25 % gesättigte Fettsäuren, 8 bis 85 % einfach ungesättigte Fettsäuren und 60 bis 85% mehrfach ungesättigte Fettsäuren jeweils bezogen auf 100 % und auf den Gesamtfettsäuregehalt der Organismen. Als vorteilhafte mehrfach ungesättigte Fettsäure sind in den Fettsäureester bzw. Fettsäuregemische bevorzugt mindestens 0,1; 0,2; 0,3; 0,4; 0,5; 0,6; 0,7; 0,8; 0,9 oder 1 % bezogen auf den Gesamtfettsäuregehalt an Arachidonsäure enthalten. Weiterhin enthalten die Fettsäureester bzw. Fettsäuregemische, die nach dem erfindungsgemäßen Verfahren hergestellt wurden, vorteilhaft Fettsäuren ausgewählt aus der Gruppe der Fettsäuren Erucasäure (13-Docosaensäure), Sterculinsäure (9,10-Methylene octadec-9-enonsäure), Malvalinsäure (8,9-Methylen Heptadec-8-enonsäure), Chaulmoogrinsäure (Cyclopenten-dodecansäure), Furan-Fettsäure (9,12-Epoxy-octadeca-9,11-dienonsäure), Vernonsäure (9,10-Epoxyoctadec-12-enonsäure), Tarinsäure (6-Octadecynonsäure),6-Nonadecynonsäure, Santalbinsäure (t11-Octadecen-9-ynoic acid), 6,9-Octadecenynonsäure, Pyrulinsäure (t10-Heptadecen-8-ynonsäure), Crepenyninsäure (9-Octadecen-12-ynonsäure), 13,14-Dihydrooropheinsäure, Octadecen-13-ene-9,11-diynonsäure, Petroselensäure (cis-6-Octadecenonsäure), 9c,12t-Octadecadiensäure, Calendulasäure (8t10t12c-Octadecatriensäure), Catalpinsäure (9t11t13c-Octadecatriensäure), Eleosterinsäure (9c11t13t-Octadecatriensäure), Jacarinsäure (8c10t12c-Octadecatriensäure), Punicinsäure (9c11t13c-Octadecatriensäure), Parinarinsäure (9c11t13t15c-Octadecatetraensäure), Pinolensäure (all-cis-5,9,12-Octadecatriensäure), Laballensäure (5,6-Octadecadienallensäure), Ricinolsäure (12-Hydroxyölsäure) und/oder Coriolinsäure (13-Hydroxy-9c,11t-Octadecadienonsäure). Die vorgenannten Fettsäuren kommen in den nach dem erfindungsgemäßen Verfahren hergestellten Fettsäureester bzw. Fettsäuregemischen in der Regel vorteilhaft nur in Spuren vor, das heißt sie kommen bezogen auf die Gesamtfettsäuren zu weniger als 30 %, bevorzugt zu weniger als 25 %, 24 %, 23 %, 22 % oder 21 %, besonders bevorzugt zu weniger als 20 %, 15 %, 10 %, 9 %, 8 %, 7%, 6 % oder 5%, ganz besonders bevorzugt zu weniger als 4 %, 3 %, 2 % oder 1 % vor. Vorteilhaft enthalten die nach dem erfindungsgemäßen Verfahren hergestellten Fettsäureester bzw. Fettsäuregemische weniger als 0,1 % bezogen auf die Gesamtfettsäuren oder keine Buttersäure, kein Cholesterin, keine Clupanodonsäure (= Docosapentaensäure, C22:5^{Δ4,8,12,15,21}) sowie keine Nisinsäure (Tetracosahexaensäure, C23:6^{Δ3,8,12,15,18,21}). Durch die erfindungsgemäßen Nukleinsäuresequenzen bzw. im erfindungsgemäßen Verfahren verwendeten Nukleinsäuresequenzen kann eine Steigerung der Ausbeute an mehrfach ungesättigten Fettsäuren von mindestens 50 %, vorteilhaft von mindestens 80 %, besonders vorteilhaft von mindestens 100 %, ganz besonders vorteilhaft von mindestens 150 % gegenüber den nicht transgenen Ausgangsorganismus beispielsweise einer Hefe, einer Alge, einem Pilz oder einer Pflanze wie Arabidopsis oder Lein beim Vergleich in der GC-Analyse siehe Beispiele erreicht werden.

Auch chemisch reine mehrfach ungesättigte Fettsäuren oder Fettsäurezusammensetzungen sind nach den vorbeschriebenen Verfahren darstellbar. Dazu werden die Fettsäuren oder die Fettsäurezusammensetzungen aus dem Organismus wie den Mikroorganismen oder den Pflanzen oder dem Kulturmedium, in dem oder auf dem die Organismen angezogen wurden, oder aus dem Organismus und dem Kulturmedium in bekannter Weise beispielsweise über Extraktion, Destillation, Kristallisation, Chromatographie oder Kombinationen dieser Methoden isoliert. Diese chemisch reinen Fettsäuren oder Fettsäurezusammensetzungen sind für Anwendungen im Bereich der Lebensmittelindustrie, der Kosmetikindustrie und besonders der Pharmaindustrie vorteilhaft.

Im Prinzip können alle Gene des Fettsäure- oder Lipidstoffwechsels vorteilhaft in Kombination mit der(den) erfinderischen Polynucleotiden (im Sinne dieser Anmeldung soll der Plural den Singular und umgekehrt beinhalten) im Verfahren zur Herstellung mehrfach ungesättigter Fettsäuren verwendet werden vorteilhaft werden Gene des Fettsäure- oder Lipidstoffwechsels ausgewählt aus der Gruppe Acyl-CoA-Dehydrogenase(n), Acyl-ACP[= acyl carrier protein]-Desaturase(n), Acyl-ACP-Thioesterase(n), Fettsäure-Acyl-Transferase(n), Acyl-CoA:Lysophospholipid-Acyltransferasen, Fettsäure-Synthase(n), Fettsäure-Hydroxylase(n), Acetyl-Coenzym A-Carboxylase(n), Acyl-Coenzym A-Oxidase(n), Fettsäure-Desaturase(n), Fettsäure-Acetylenasen, Lipoxygenasen, Triacylglycerol-Lipasen, Allenoxid-Synthasen, Hydroperoxid-Lyasen oder Fettsäure-Elongase(n) verwendet. Besonders bevorzugt werden Gene ausgewählt aus der Gruppe der Δ-4-Desaturasen, Δ-5-Desaturasen, Δ-6-Desaturasen, Δ-9-Desaturasen, Δ-12-Desaturasen, Δ-6-Elongasen oder Δ-5-Elongasen in Kombination mit den erfindungsgemäßen Polynucleotiden verwendet, wobei einzelne Gene oder mehrere Gene in Kombination verwendet werden können.

Vorteilhaft setzen die im erfindungsgemäßen Verfahren verwendeten Desaturasen ihre jeweiligen Substrate in Form der CoA-Fettsäureester um. Dies führt, wenn vorher ein Elongationsschritt stattgefunden hat, vorteilhaft zu einer erhöhten Produktausbeute. Die jeweiligen Desaturierungsprodukte werden dadurch in höheren Mengen synthetisiert, da der Elongationsschritt in der Regel an den CoA-Fettsäureestern erfolgt, während der Desaturierungsschritt überwiegend an den Phospholipiden oder an den Triglyceriden erfolgt. Eine Ausstauschreaktion, die eine weitere möglicherweise limitierende Enzymreaktion erforderlich machen würde, zwischen den CoA-Fettsäureestern und den Phospholipiden oder Triglyceriden ist somit nicht erforderlich.

Durch die enzymatische Aktivität der im erfindungsgemäßen Verfahren verwendeten Polypeptide können unterschiedlichste mehrfach ungesättigte Fettsäuren im erfindungsgemäßen Verfahren hergestellt werden. Je nach Auswahl der für das erfindungsgemäße Verfahren verwendeten Organismen wie den bevorzugte Pflanze lassen sich Mischungen der verschiedenen mehrfach ungesättigten Fettsäuren oder einzelne mehrfach ungesättigte Fettsäuren wie EPA oder ARA in freier oder gebundener Form herstellen. Je nachdem welche Fettsäurezusammensetzung in der Ausgangspflanze vorherrscht (C18:2- oder C18:3-Fettsäuren) entstehen so Fettsäuren, die sich von C18:2-Fettsäuren ableiten, wie GLA, DGLA oder ARA oder, die sich von C18:3-Fettsäuren ableiten, wie SDA, ETA oder EPA. Liegt in der für das Verfahren verwendeten Pflanze als ungesättigte Fettsäure nur Linolsäure (= LA, C18:2^{Δ9,12}) vor, so können als Produkte des Verfahrens nur GLA, DGLA und ARA entstehen, die als freie Fettsäuren oder gebunden vorliegen können. Ist in der im Verfahren verwendeten Pflanze als ungesättigte Fettsäure nur α-Linolensäure (=ALA, C18:3^{Δ9,12,15}) beispielsweise wie in Lein, so können als Produkte des Verfahrens nur SDA, ETA, EPA und/oder DHA entstehen, die wie oben beschrieben als freie Fettsäuren oder gebunden vorliegen können. Durch Modifikation der Aktivität der an der Synthese beteiligten Enzyme Δ-5-Desaturase, Δ-6-Desaturase, Δ-4-Desaturase, Δ-12-Desaturase, Δ-5-Elongase und/oder Δ-6-Elongase lassen sich gezielt in den vorgenannten Organismen vorteilhaft in den vorgenannten Pflanzen nur einzelne Produkte herstellten. Durch die Aktivität der Δ-6-Desaturase und Δ-6-Elongase entstehen beispielsweise GLA und DGLA bzw. SDA und ETA, je nach Ausgangspflanze und ungesättigter Fettsäure. Bevorzugt entstehen DGLA bzw. ETA oder deren Mischungen. Werden die Δ-5-Desaturase, die Δ-5-Elongase und die Δ-4-Desaturase zusätzlich in die Organismen vorteilhaft in die Pflanze eingebracht, so entstehen zusätzlich ARA, EPA und/oder DHA. Vorteilhaft werden nur ARA, EPA oder DHA oder deren Mischungen synthetisiert, abhängig von den im Organismus bzw. in der Pflanze vorliegenden Fettsäuren, die als Ausgangssubstanz für die Synthese dient. Da es sich um Biosyntheseketten handelt, liegen die jeweiligen Endprodukte nicht als Reinsubstanzen in den Organismen vor. Es sind immer auch geringe Mengen der Vorläuferverbindungen im Endprodukt enthalten. Diese geringen Mengen betragen weniger als 20 Gew.-%, vorteilhaft weniger als 15 Gew.-%, besonders vorteilhaft weniger als 10 Gew.-%, ganz besonders vorteilhaft weniger als 5, 4, 3, 2 oder 1 Gew.-% bezogen auf das Endprodukt DGLA, ETA oder deren Mischungen bzw. ARA, EPA, DHA oder deren Mischungen vorteilhaft EPA oder DHA oder deren Mischungen.

Neben der Produktion der Ausgangsfettsäuren für die im erfindungsgemäßen Verfahren verwendeten Polypeptide direkt im Organismus können die Fettsäuren auch von außen gefüttert werden. Aus kostengründen ist die Produktion im Organismus bevorzugt. Bevorzugte Substrate sind die Linolsäure (C18:2^{Δ9,12}), die γ-Linolensäure (C18:3^{Δ6,9,12}), die Eicosadiensäure (C20:2^{Δ11,4}), die Dihomo-γ-linolensäure (C20:3^{Δ8,11,14}), die Arachidonsäure (C20:4^{Δ5,8,11,14}), die Docosatetraensäure (C22:4^{Δ7,10,13,16}) und die Docosapentaensäure (C22:5^{Δ4,7,10,13,15}).

Zur Steigerung der Ausbeute im beschriebenen Verfahren zur Herstellung von Ölen und/oder Triglyceriden mit einem vorteilhaft erhöhten Gehalt an mehrfach ungesättigten Fettsäuren ist es vorteilhaft die Menge an Ausgangsprodukt für die Fettsäuresynthese zu steigern, dies kann beispielsweise durch das Einbringen einer Nukleinsäure in den Organismus, die für ein Polypeptid mit Δ-12-Desaturase codiert, erreicht werden. Dies ist besonders vorteilhaft in Öl-produzierenden Organismen wie der Familie der Brassicaceae wie der Gattung Brassica z.B. Raps; der Familie der Elaeagnaceae wie die Gattung Elaeagnus z.B. die Gattung und Art *Olea europaea* oder der Familie Fabaceae wie der Gattung Glycine z.B. die Gattung und Art *Glycine max,* die einen hohen Ölsäuregehalt aufweisen. Da diese Organismen nur einen geringen Gehalt an Linolsäure aufweisen (Mikoklajczak et al., Journal of the American Oil Chemical Society, 38, 1961, 678 - 681) ist die Verwendung der genannten Δ-12-Desaturasen zur Herstellung des Ausgangsprodukts Linolsäure vorteilhaft.

Vorteilhaft werden im erfindungsgemäßen Verfahren die vorgenannten Nukleinsäuresequenzen oder deren Derivat oder Homologe, die für Polypeptide codieren, die noch die enzymatische Aktivität der durch Nukleinsäuresequenzen codierten Proteine besitzen. Diese Sequenzen werden einzeln oder in Kombination mit den erfindungsgemäßen Polynucleotiden in Expressionskonstrukte cloniert und zum Einbringen und zur Expression in Organismen verwendet. Diese Expressionskonstrukte ermöglichen durch ihre Konstruktion eine vorteilhafte optimale Synthese der im erfindungsgemäßen Verfahren produzierten mehrfach ungesättigten Fettsäuren.

Bei einer bevorzugten Ausführungsform umfasst das Verfahren ferner den Schritt des Gewinnens einer Zelle oder eines ganzen Organismus, der die im Verfahren verwendeten Nukleinsäuresequenzen enthält, wobei die Zelle und/oder der Organismus mit einem erfindungsgemäßen Polynucleotiden einem Genkonstrukt oder einem Vektor wie nachfolgend beschrieben, allein oder in Kombination mit weiteren Nukleinsäuresequenzen, die für Proteine des Fettsäure- oder Lipidsstoffwechsels codieren, transformiert wird. Bei einer weiteren bevorzugten Ausführungsform umfasst dieses Verfahren ferner den Schritt des Gewinnens der Öle, Lipide oder freien Fettsäuren aus dem Organismus oder aus der Kultur. Bei der Kultur kann es sich beispielsweise um eine Fermentationskultur beispielsweise im Falle der Kultivierung von Mikroorganismen wie z.B. Mortierella, Thalassiosira, Mantoniella, Ostreococcus, Saccharomyces oder Thraustochytrium oder um eine Treibhaus oder Feldkultur einer Pflanze handeln. Die so hergestellte Zelle oder der so hergestellte Organismus ist vorteilhaft eine Zelle eines Öl-produzierenden Organismus wie einer Ölfruchtpflanze wie beispielsweise Erdnuss, Raps, Canola, Lein, Hanf, Erdnuss, Soja, Safflower, Hanf, Sonnenblumen oder Borretsch.

Unter Anzucht ist beispielsweise die Kultivierung im Falle von Pflanzenzellen, -gewebe oder -organe auf oder in einem Nährmedium oder der ganzen Pflanze auf bzw. in einem Substrat beispielsweise in Hydrokultur, Blumentopferde oder auf einem Ackerboden zu verstehen.

Als Organismen bzw. Wirtszellen für das erfindungsgemäße Verfahren sind solche geeignet, die in der Lage sind Fettsäuren speziell ungesättigte Fettsäuren zu synthetisieren bzw. für die Expression rekombinanter Gene geeignet sind. Beispielhaft seien Pflanzen wie Arabidopsis, Asteraceae wie Calendula oder Kulturpflanzen wie Soja, Erdnuss, Rizinus, Sonnenblume, Mais, Baumwolle, Flachs, Raps, Kokosnuss, Ölpalme, FärberSaflor (Carthamus tinctorius) oder Kakaobohne, Mikroorganismen wie Pilze beispielsweise die Gattung Mortierella, Thraustochytrium, Saprolegnia, Phytophtora oder Pythium, Bakterien wie die Gattung Escherichia oder Shewanella, Hefen wie die Gattung Saccharomyces, Cyanobakterien, Ciliaten, Algen wie Mantoniella oder Ostreococcus oder Protozoen wie Dinoflagellaten wie Thalassiosira oder Crypthecodinium genannt. Bevorzugt werden Organismen, die natürlicherweise Öle in größeren Mengen synthetisieren können wie Pilze wie Mortierella alpina, Pythium insidiosum, Phytophtora infestans oder Pflanzen wie Soja, Raps, Kokosnuss, Ölpalme, FärberSaflor, Flachs, Hanf, Rizinus, Calendula, Erdnuss, Kakaobohne oder Sonnenblume oder Hefen wie Saccharomyces cerevisiae, besonders bevorzugt werden Soja, Flachs, Raps, FärberSaflor, Sonnenblume, Calendula, Mortierella oder Saccharomyces cerevisiae. Prinzipiell sind als Wirtsorganismen neben den vorgenannten transgenen Organismen auch transgene Tiere vorteilhaft nicht-humane Tiere geeignet beispielsweise C. elegans. Weitere geeignete Wirtszellen und Organismen wurden bereits zuvor ausführlich beschrieben.

Transgene Pflanzen, die die im erfindungsgemäßen Verfahren synthetisierten mehrfach ungesättigten Fettsäuren enthalten, können vorteilhaft direkt vermarktet werden ohne dass die synthetisierten Öle, Lipide oder Fettsäuren isoliert werden müssen. Unter Pflanzen im erfindungsgemäßen Verfahren sind ganze Pflanzen sowie alle Pflanzenteile, Pflanzenorgane oder Pflanzenteile wie Blatt, Stiel, Samen, Wurzel, Knollen, Antheren, Fasern, Wurzelhaare, Stängel, Embryos, Kalli, Kotelydonen, Petiolen, Erntematerial, pflanzliches Gewebe, reproduktives Gewebe, Zellkulturen, die sich von der transgenen Pflanze abgeleiten und/oder dazu verwendet werden können, die transgene Pflanze hervorzubringen. Der Samen umfasst dabei alle Samenteile wie die Samenhüllen, Epidermis- und Samenzellen, Endosperm oder Embyrogewebe. Die im erfindungsgemäßen Verfahren hergestellten Verbindungen können aber auch aus den Organismen vorteilhaft Pflanzen in Form ihrer Öle, Fett, Lipide und/oder freien Fettsäuren isoliert werden. Durch dieses Verfahren hergestellte mehrfach ungesättigte Fettsäuren lassen sich durch Ernten der Organismen entweder aus der Kultur, in der sie wachsen, odervom Feld ernten. Dies kann über Pressen oder Extraktion der Pflanzenteile bevorzugt der Pflanzensamen erfolgen. Dabei können die Öle, Fette, Lipide und/oder freien Fettsäuren durch so genanntes kalt schlagen oder kalt pressen ohne Zuführung von Wärme durch Pressen gewonnen werden. Damit sich die Pflanzenteile speziell die Samen leichter aufschließen lassen, werden sie vorher zerkleinert, gedämpft oder geröstet. Die so vorbehandelten Samen können anschließend gepresst werden oder mit Lösungsmittel wie warmes Hexan extrahiert werden. Anschließend wird das Lösungsmittel wieder entfernt. Im Falle von Mikroorganismen werden diese nach Ernte beispielsweise direkt ohne weitere Arbeitsschritte extrahiert oder aber nach Aufschluss über verschiedene dem Fachmann bekannte Methoden extrahiert. Auf diese Weise können mehr als 96 % der im Verfahren hergestellten Verbindungen isoliert werden. Anschließend werden die so erhaltenen Produkte weiter bearbeitet, das heißt raffiniert. Dabei werden zunächst beispielsweise die Pflanzenschleime und Trübstoffe entfernt. Die sogenannte Entschleimung kann enzymatisch oder beispielsweise chemisch/physikalisch durch Zugabe von Säure wie Phosphorsäure erfolgen. Anschließend werden die freien Fettsäuren durch Behandlung mit einer Base beispielsweise Natronlauge entfernt. Das erhaltene Produkt wird zur Entfernung der im Produkt verbliebenen Lauge mit Wasser gründlich gewaschen und getrocknet. Um die noch im Produkt enthaltenen Farbstoffe zu entfernen werden die Produkte einer Bleichung mit beispielsweise Bleicherde oder Aktivkohle unterzogen. Zum Schluss wird das Produkt noch beispielsweise mit Wasserdampf noch desodoriert.

Vorzugsweise sind die durch dieses Verfahren produzierten PUFAs bzw. LCPUFAs C₁₈-, C₂₀- oder C₂₂-Fettsäuremoleküle vorteilhaft C₂₀- oder C₂₂-Fettsäuremoleküle mit mindestens zwei Doppelbindungen im Fettsäuremolekül, vorzugsweise drei, vier, fünf oder sechs Doppelbindungen. Diese C₁₈-, C₂₀- oder C₂₂-Fettsäuremoleküle lassen sich aus dem Organismus in Form eines Öls, Lipids oder einer freien Fettsäure isolieren. Geeignete Organismen sind beispielsweise die vorstehend erwähnten. Bevorzugte Organismen sind transgene Pflanzen.

Eine Ausführungsform der Erfindung sind deshalb Öle, Lipide oder Fettsäuren oder Fraktionen davon, die durch das oben beschriebene Verfahren hergestellt worden sind, besonders bevorzugt Öl, Lipid oder eine Fettsäurezusammensetzung, die PUFAs umfassen und von transgenen Pflanzen herrühren.

Diese Öle, Lipide oder Fettsäuren enthalten wie oben beschrieben vorteilhaft 6 bis 15 % Palmitinsäure, 1 bis 6 % Stearinsäure; 7 - 85 % Ölsäure; 0,5 bis 8 % Vaccensäure, 0,1 bis 1 % Arachinsäure, 7 bis 25 % gesättigte Fettsäuren, 8 bis 85 % einfach ungesättigte Fettsäuren und 60 bis 85 % mehrfach ungesättigte Fettsäuren jeweils bezogen auf 100 % und auf den Gesamtfettsäuregehalt der Organismen. Als vorteilhafte mehrfach ungesättigte Fettsäure sind in den Fettsäureester bzw. Fettsäuregemische bevorzugt mindestens 0,1; 0,2; 0,3; 0,4; 0,5; 0,6; 0,7; 0,8; 0,9 oder 1 % bezogen auf den Gesamtfettsäuregehalt an Arachidonsäure enthalten. Weiterhin enthalten die Fettsäureester bzw. Fettsäuregemische, die nach dem erfindungsgemäßen Verfahren hergestellt wurden, vorteilhaft Fettsäuren ausgewählt aus der Gruppe der Fettsäuren Erucasäure (13-Docosaensäure), Sterculinsäure (9,10-Methylene octadec-9-enonsäure), Malvalinsäure (8,9-Methylen Heptadec-8-enonsäure), Chaulmoogrinsäure (Cyclopenten-dodecansäure), Furan-Fettsäure (9,12-Epoxy-octadeca-9,11-dienonsäure), Vernonsäure (9,10-Epoxyoctadec-12-enonsäure), Tarinsäure (6-Octadecynonsäure),6-Nonadecynonsäure, Santalbinsäure (t11-Octadecen-9-ynoic acid), 6,9-Octadecenynonsäure, Pyrulinsäure (t10-Heptadecen-8-ynonsäure), Crepenyninsäure (9-Octadecen-12-ynonsäure), 13,14-Dihydrooropheinsäure, Octadecen-13-ene-9,11-diynonsäure, Petroselensäure (cis-6-Octadecenonsäure), 9c,12t-Octadecadiensäure, Calendulasäure (8t10t12c-Octadecatriensäure), Catalpinsäure (9t11t13c-Octadecatriensäure), Eleosterinsäure (9c11t13t-Octadecatriensäure), Jacarinsäure (8c10t12c-Octadecatriensäure), Punicinsäure (9c11t13c-Octadecatriensäure),Parinarinsäure (9c11t13t15c-Octadecatetraensäure), Pinolensäure (all-cis-5,9,12-Octadecatriensäure), Laballensäure (5,6-Octadecadienallensäure), Ricinolsäure (12-Hydroxyölsäure) und/oder Coriolinsäure (13-Hydroxy-9c,11t-Octadecadienonsäure). Die vorgenannten Fettsäuren kommen in den nach dem erfindungsgemäßen Verfahren hergestellten Fettsäureester bzw. Fettsäuregemischen in der Regel vorteilhaft nur in Spuren vor, das heißt sie kommen bezogen auf die Gesamtfettsäuren zu weniger als 30 %, bevorzugt zu weniger als 25 %, 24 %, 23 %, 22 % oder 21 %, besonders bevorzugt zu weniger als 20 %, 15 %, 10 %, 9 %, 8 %, 7%, 6 % oder 5%, ganz besonders bevorzugt zu weniger als 4 %, 3 %, 2 % oder 1 % vor. Vorteilhaft enthalten die nach dem erfindungsgemäßen Verfahren hergestellten Fettsäureester bzw. Fettsäuregemische weniger als 0,1 % bezogen auf die Gesamtfettsäuren oder keine Butterbuttersäure, kein Cholesterin, keine Clupanodonsäure (= Docosapentaensäure, C22:5^{Δ4,8,12,15,21}) sowie keine Nisinsäure (Tetracosahexaensäure, C23:6^{Δ3,8,121518,21}).

Bevorzugt enthalten die erfindungsgemäßen Öle, Lipide oder Fettsäuren mindestens 0,5%, 1%, 2%, 3%, 4% oder 5%, vorteilhaft mindestens 6%, 7%, 8%, 9% oder 10%, besonders vorteilhaft mindestens 11 %, 12%, 13%, 14% oder 15% ARA oder mindestens 0,5%, 1%, 2%, 3%, 4% oder 5%, vorteilhaft mindestens 6%, oder 7%, besonders vorteilhaft mindestens 8%, 9% oder 10% EPA und/oder DHA bezogen auf den Gesamtfettsäuregehalt des Produktionsorganismus vorteilhaft einer Pflanze, besonders vorteilhaft einer Ölfruchtpflanze wie Soja, Raps, Kokosnuss, Ölpalme, Färbersafflor, Flachs, Hanf, Rizinus, Calendula, Erdnuss, Kakaobohne, Sonnenblume oder den oben genannten weiteren ein- oder zweikeimblättrigen Ölfruchtpflanzen.

Eine weitere erfindungsgemäße Ausführungsform ist die Verwendung des Öls, Lipids, der Fettsäuren und/oder der Fettsäurezusammensetzung in Futtermitteln, Nahrungsmitteln, Kosmetika oder Pharmazeutika. Die erfindungsgemäßen Öle, Lipide, Fettsäuren oder Fettsäuregemische können in der dem Fachmann bekannten Weise zur Abmischung mit anderen Ölen, Lipiden, Fettsäuren oder Fettsäuregemischen tierischen Ursprungs wie z.B. Fischölen verwendet werden. Auch diese Öle, Lipide, Fettsäuren oder Fettsäuregemische, die aus pflanzlichen und tierischen Bestandteilen bestehen, können zur Herstellung von Futtermitteln, Nahrungsmitteln, Kosmetika oder Pharmazeutika verwendet werden.

Unter dem Begriff "Öl", "Lipid" oder "Fett" wird ein Fettsäuregemisch verstanden, das ungesättigte, gesättigte, vorzugsweise veresterte Fettsäure(n) enthält. Bevorzugt ist, dass das Öl, Lipid oder Fett einen hohen Anteil an mehrfach ungesättigten freien oder vorteilhaft veresterten Fettsäure(n), insbesondere Linolsäure, γ-Linolensäure, Dihomo-γ-Linolensäure, Arachidonsäure, α-Linolensäure, Stearidonsäure, Eicosatetraensäure, Eicosapentaensäure, Docosapentaensäure oder Docosahexaensäure hat. Vorzugsweise ist der Anteil an ungesättigten veresterten Fettsäuren ungefähr 30 %, mehr bevorzugt ist ein Anteil von 50 %, noch mehr bevorzugt ist ein Anteil von 60 %, 70 %, 80 % oder mehr. Zur Bestimmung kann z.B. der Anteil an Fettsäure nach Überführung der Fettsäuren in die Methylestern durch Umesterung gaschromatographisch bestimmt werden. Das Öl, Lipid oder Fett kann verschiedene andere gesättigte oder ungesättigte Fettsäuren, z.B. Calendulasäure, Palmitin-, Palmitolein-, Stearin-, Ölsäure etc., enthalten. Insbesondere kann je nach Ausgangsorganismus der Anteil der verschiedenen Fettsäuren in dem Öl oder Fett schwanken.

Bei den im Verfahren hergestellten mehrfach ungesättigte Fettsäuren mit vorteilhaft mindestens zwei Doppelbindungen enthalten, handelt es sich wie oben beschrieben beispielsweise um Sphingolipide, Phosphoglyceride, Lipide, Glycolipide, Phospholipide, Monoacylglycerin, Diacylglycerin, Triacylglycerin oder sonstige Fettsäureester.

Aus den so im erfindungsgemäßen Verfahren hergestellten mehrfach ungesättigte Fettsäuren mit vorteilhaft mindestens fünf oder sechs Doppelbindungen lassen sich die enthaltenden mehrfach ungesättigten Fettsäuren beispielsweise über eine Alkalibehandlung beispielsweise wässrige KOH oder NaOH oder saure Hydrolyse vorteilhaft in Gegenwart eines Alkohols wie Methanol oder Ethanol oder über eine enzymatische Abspaltung freisetzen und isolieren über beispielsweise Phasentrennung und anschließender Ansäuerung über z.B. H₂SO₄. Die Freisetzung der Fettsäuren kann auch direkt ohne die vorhergehend beschriebene Aufarbeitung erfolgen.

Die im Verfahren verwendeten Nukleinsäuren können nach Einbringung in einem Organismus vorteilhaft einer Pflanzenzelle bzw. Pflanze entweder auf einem separaten Plasmid liegen oder vorteilhaft in das Genom der Wirtszelle integriert sein. Bei Integration in das Genom kann die Integration zufallsgemäß sein oder durch derartige Rekombination erfolgen, dass das native Gen durch die eingebrachte Kopie ersetzt wird, wodurch die Produktion der gewünschten Verbindung durch die Zelle moduliert wird, oder durch Verwendung eines Gens in "trans", so dass das Gen mit einer funktionellen Expressionseinheit, welche mindestens eine die Expression eines Gens gewährleistende Sequenz und mindestens eine die Polyadenylierung eines funktionell transkribierten Gens gewährleistende Sequenz enthält, funktionell verbunden ist. Vorteilhaft werden die Nukleinsäuren über Multiexpressionskassetten oder Konstrukte zur multiparallelen Expression in die Organismen vorteilhaft zur multiparallelen samenspezifischen Expression von Genen in die Pflanzen gebracht.

Moose und Algen sind die einzigen bekannten Pflanzensysteme, die erhebliche Mengen an mehrfach ungesättigten Fettsäuren, wie Arachidonsäure (ARA) und/oder Eicosapentaensäure (EPA) und/oder Docosahexaensäure (DHA) herstellen. Moose enthalten PUFAs in Membranlipiden während Algen, algenverwandte Organismen und einige Pilze auch nennenswerte Mengen an PUFAs in der Triacylglycerolfraktion akkumulieren. Daher eignen sich Nukleinsäuremoleküle, die aus solchen Stämmen isoliert werden, die PUFAs auch in der Triacylglycerolfraktion akkumulieren, besonders vorteilhaft für das erfindungsgemäße Verfahren und damit zur Modifikation des Lipid- und PUFA-Produktionssystems in einem Wirt, insbesondere Pflanzen, wie Ölfruchtpflanzen, beispielsweise Raps, Canola, Lein, Hanf, Soja, Sonnenblumen, Borretsch. Sie sind deshalb vorteilhaft im erfindungsgemäßen Verfahren verwendbar.

Als Substrate der im erfindungsgemäßen Polypeptide oder des Polypeptids des Fettsäure- oder Lipidstoffwechsels ausgewählt aus der Gruppe Acyl-CoA-Dehydrogenase(n), Acyl-ACP[= acyl carrier protein]-Desaturase(n), Acyl-ACP-Thioesterase(n), Fettsäure-Acyl-Transferase(n), Acyl-CoA:Lysophospholipid-Acyltransferase(n), Fettsäure-Synthase(n), Fettsäure-Hydroxylase(n), Acetyl-Coenzym A-Carboxylase(n), Acyl-Coenzym A-Oxidase(n), Fettsäure-Desaturase(n), Fettsäure-Acetylenase(n), Lipoxygenase(n), Triacylglycerol-Lipase(n), Allenoxid-Synthase(n), Hydroperoxid-Lyase(n) oder Fettsäure-Elongase(n) eignen sich bevorzugt C₁₆-, C₁₈- oder C₂₀-Fettsäuren. Bevorzugt werden die im Verfahren als Substrate umgesetzten Fettsäuren in Form ihrer Acyl-CoA-Ester und/oder ihrer Phospholipid-Ester umgesetzt.

Zur Herstellung der erfindungsgemäßen langkettigen PUFAs müssen die mehrfach ungesättigten C₁₈-Fettsäuren zunächst durch die enzymatische Aktivität einer Desaturase zunächst desaturiert und anschließend über eine Elongase um mindestens zwei Kohlenstoffatome verlängert werden. Nach einer Elongationsrunde führt diese Enzymaktivität zu C₂₀-Fettsäuren, und nach zwei Elongationsrunden zu C₂₂-Fettsäuren. Die Aktivität der erfindungsgemäßen Verfahren verwendeten Desaturasen und Elongasen führt vorzugsweise zu C₁₈-, C₂₀- und/oder C₂₂-Fettsäuren vorteilhaft mit mindestens zwei Doppelbindungen im Fettsäuremolekül, vorzugsweise mit drei, vier, fünf oder sechs Doppelbindungen, besonders bevorzugt zu C₂₀- und/oder C₂₂-Fettsäuren mit mindestens zwei Doppelbindungen im Fettsäuremolekül, vorzugsweise mit drei, vier, fünf oder sechs Doppelbindungen, ganz besonders bevorzugt mit fünf oder sechs Doppelbindungen im Molekül. Nachdem eine erste Desaturierung und die Verlängerung stattfand, können weitere Desaturierungs- und Elongierungsschritte wie z.B. eine solche Desaturierung in Δ-5- und Δ-4-Position erfolgen. Besonders bevorzugt als Produkte des erfindungsgemäßen Verfahrens sind Dihomo-γ-linolensäure, Arachidonsäure, Eicosapentaensäure, Docosapetaensäure und/oder Docosahesaensäure. Die C₂₀-Fettsäuren mit mindestens zwei Doppelbindungen in der Fettsäure können durch die erfindungsgemäße enzymatische Aktivität in Form der freien Fettsäure oder in Form der Ester, wie Phospholipide, Glycolipide, Sphingolipide, Phosphoglyceride, Monoacylglycerin, Diacylglycerin oder Triacylglycerin, verlängert werden.

Der bevorzugte Biosyntheseort von Fettsäuren, Ölen, Lipiden oder Fette in den vorteilhaft verwendeten Pflanzen ist beispielsweise im allgemeinen der Samen oder Zellschichten des Samens, so dass eine samenspezifische Expression der im Verfahren verwendeten Nukleinsäuren sinnvoll ist. Es ist jedoch naheliegend, dass die Biosynthese von Fettsäuren, Ölen oder Lipiden nicht auf das Samengewebe beschränkt sein muss, sondern auch in allen übrigen Teilen der Pflanze - beispielsweise in Epidermiszellen oder in den Knollen - gewebespezifisch erfolgen kann.

Werden im erfindungsgemäßen Verfahren als Organismen Mikroorganismus wie Hefen wie Saccharomyces oder Schizosaccharomyces, Pilze wie Mortierella, Aspergillus, Phytophtora, Entomophthora, Mucor oder Thraustochytrium Algen wie Isochrysis, Mantoniella, Ostreococcus, Phaeodactylum oder Crypthecodiniu verwendet, so werden diese Organismen vorteilhaft fermentativ angezogen.

Durch die Verwendung der erfindungsgemäßen Nukleinsäuren, die für eine Δ-5-Elongase codieren, können im Verfahren die hergestellten mehrfach ungesättigten Fettsäuren mindestens um 5 %, bevorzugt mindestens um 10 %, besonders bevorzugt mindestens um 20 %, ganz besonders bevorzugt um mindestens 50 % gegenüber dem Wildtyp der Organismen, die die Nukleinsäuren nicht rekombinant enthalten, erhöht werden.

Durch das erfindungsgemäße Verfahren können die hergestellten mehrfach ungesättigten Fettsäuren in den im Verfahren verwendeten Organismen prinzipiell auf zwei Arten erhöht werden. Es kann vorteilhaft der Pool an freien mehrfach ungesättigten Fettsäuren und/oder der Anteil der über das Verfahren hergestellten veresterten mehrfach ungesättigten Fettsäuren erhöht werden. Vorteilhaft wird durch das erfindungsgemäße Verfahren der Pool an veresterten mehrfach ungesättigten Fettsäuren in den transgenen Organismen erhöht.

Werden im erfindungsgemäßen Verfahren als Organismen Mikroorganismen verwendet, so werden sie je nach Wirtsorganismus in dem Fachmann bekannter Weise angezogen bzw. gezüchtet. Mikroorganismen werden in der Regel in einem flüssigen Medium, das eine Kohlenstoffquelle meist in Form von Zuckern, eine Stickstoffquelle meist in Form von organischen Stickstoffquellen wie Hefeextrakt oder Salzen wie Ammoniumsulfat, Spurenelemente wie Eisen-, Mangan-, Magnesiumsalze und gegebenenfalls Vitamine enthält, bei Temperaturen zwischen 0°C und 100°C, bevorzugt zwischen 10°C bis 60°C unter Sauerstoffbegasung angezogen. Dabei kann der pH der Nährflüssigkeit auf einen festen Wert gehalten werden, das heißt während der Anzucht reguliert werden oder nicht. Die Anzucht kann batch weise, semi batch weise oder kontinuierlich erfolgen. Nährstoffe können zu Beginn der Fermentation vorgelegt oder semikontinuierlich oder kontinuierlich nachgefüttert werden. Die hergestellten mehrfach ungesättigten Fettsäuren können nach dem Fachmann bekannten Verfahren wie oben beschrieben aus den Organismen isoliert werden. Beispielsweise über Extraktion, Destillation, Kristallisation, ggf. Salzfällung und/oder Chromatographie. Die Organismen können dazu vorher noch vorteilhaft aufgeschlossen werden.

Das erfindungsgemäße Verfahren wird, wenn es sich bei den Wirtsorganismen um Mikroorganismen handelt, vorteilhaft bei einer Temperatur zwischen 0°C bis 95° , bevorzugt zwischen 10°C bis 85°C, besonders bevorzugt zwischen 15°C bis 75°C, ganz besonders bevorzugt zwischen 15°C bis 45°C durchgeführt.

Der pH Wert wird dabei vorteilhaft zwischen pH 4 und 12, bevorzugt zwischen pH 6 und 9, besonders bevorzugt zwischen pH 7 und 8 gehalten.

Das erfindungsgemäße Verfahren kann batchweise, semi-batchweise oder kontinuierlich betrieben werden. Eine Zusammenfassung über bekannte Kultivierungsmethoden ist im Lehrbuch von Chmiel (Bioprozeßtechnik 1. Einführung in die Bioverfahrenstechnik (Gustav Fischer Verlag, Stuttgart, 1991)) oder im Lehrbuch von Storhas (Bioreaktoren und periphere Einrichtungen (Vieweg Verlag, Braunschweig/Wiesbaden, 1994)) zu finden.

Das zu verwendende Kulturmedium hat in geeigneter Weise den Ansprüchen der jeweiligen Stämme zu genügen. Beschreibungen von Kulturmedien verschiedener Mikroorganismen sind im Handbuch "Manual of Methods für General Bacteriology" der American Society für Bacteriology (Washington D. C., USA, 1981) enthalten.

Diese erfindungsgemäß einsetzbaren Medien umfassen wie oben beschrieben gewöhnlich eine oder mehrere Kohlenstoffquellen, Stickstoffquellen, anorganische Salze, Vitamine und/oder Spurenelemente.

Bevorzugte Kohlenstoffquellen sind Zucker, wie Mono-, Di- oder Polysaccharide. Sehr gute Kohlenstoffquellen sind beispielsweise Glucose, Fructose, Mannose, Galactose, Ribose, Sorbose, Ribulose, Lactose, Maltose, Saccharose, Raffinose, Stärke oder Cellulose. Man kann Zucker auch über komplexe Verbindungen, wie Melassen, oder andere Nebenprodukte der Zucker-Raffinierung zu den Medien geben. Es kann auch vorteilhaft sein, Gemische verschiedener Kohlenstoffquellen zuzugeben. Andere mögliche Kohlenstoffquellen sind Öle und Fette wie z.B. Sojaöl, Sonnenblumenöl, Erdnussöl und/oder Kokosfett, Fettsäuren wie z.B. Palmitinsäure, Stearinsäure und/oder Linolsäure, Alkohole und/oder Polyalkohole wie z. B. Glycerin, Methanol und/oder Ethanol und/oder organische Säuren wie z.B. Essigsäure und/oder Milchsäure.

Stickstoffquellen sind gewöhnlich organische oder anorganische Stickstoffverbindungen oder Materialien, die diese Verbindungen enthalten. Beispielhafte Stickstoffquellen umfassen Ammoniak in flüssiger- oder gas- Form oder Ammoniumsalze, wie Ammoniumsulfat, Ammoniumchlorid, Ammoniumphosphat, Ammoniumcarbonat oder Ammoniumnitrat, Nitrate, Harnstoff, Aminosäuren oder komplexe Stickstoffquellen, wie Maisquellwasser, Sojamehl, Sojaprotein, Hefeextrakt, Fleischextrakt und andere. Die Stickstoffquellen können einzeln oder als Mischung verwendet werden.

Anorganische Salzverbindungen, die in den Medien enthalten sein können, umfassen die Chlorid-, Phosphor- oder Sulfatsalze von Calcium, Magnesium, Natrium, Kobalt, Molybdän, Kalium, Mangan, Zink, Kupfer und Eisen.

Als Schwefelquelle für die Herstellung von schwefelhaltigen Feinchemikalien, insbesondere von Methionin, können anorganische schwefelhaltige Verbindungen wie beispielsweise Sulfate, Sulfite, Dithionite, Tetrathionate, Thiosulfate, Sulfide aber auch organische Schwefelverbindungen, wie Mercaptane und Thiole, verwendet werden.

Als Phosphorquelle können Phosphorsäure, Kaliumdihydrogenphosphat oder Dikaliumhydrogenphosphat oder die entsprechenden Natriumhaltigen Salze verwendet werden.

Chelatbildner können zum Medium gegeben werden, um die Metallionen in Lösung zu halten. Besonders geeignete Chelatbildner umfassen Dihydroxyphenole, wie Catechol oder Protocatechuat, oder organische Säuren, wie Zitronensäure.

Die erfindungsgemäß zur Kultivierung von Mikroorganismen eingesetzten Fermentationsmedien enthalten üblicherweise auch andere Wachstumsfaktoren, wie Vitamine oder Wachstumsförderer, zu denen beispielsweise Biotin, Riboflavin, Thiamin, Folsäure, Nikotinsäure, Panthothenat und Pyridoxin gehören. Wachstumsfaktoren und Salze stammen häufig von komplexen Medienkomponenten, wie Hefeextrakt, Melassen, Maisquellwasser und dergleichen. Dem Kulturmedium können überdies geeignete Vorstufen zugesetzt werden. Die genaue Zusammensetzung der Medienverbindungen hängt stark vom jeweiligen Experiment ab und wird für jeden spezifischen Fall individuell entschieden. Information über die Medienoptimierung ist erhältlich aus dem Lehrbuch "Applied Microbiol. Physiology, A Practical Approach" (Hrsg. P.M. Rhodes, P.F. Stanbury, IRL Press (1997) S. 53-73, ISBN 0 19 963577 3). Wachstumsmedien lassen sich auch von kommerziellen Anbietern beziehen, wie Standard 1 (Merck) oder BHI (Brain heart infusion, DIFCO) und dergleichen.

Sämtliche Medienkomponenten werden, entweder durch Hitze (20 min bei 1,5 bar und 121 °C) oder durch Sterilfiltration, sterilisiert. Die Komponenten können entweder zusammen oder nötigenfalls getrennt sterilisiert werden. Sämtliche Medienkomponenten können zu Beginn der Anzucht zugegen sein oder wahlfrei kontinuierlich oder Chargenweise hinzu gegeben werden.

Die Temperatur der Kultur liegt normalerweise zwischen 15°C und 45°C, vorzugsweise bei 25°C bis 40°C und kann während des Experimentes konstant gehalten oder verändert werden. Der pH-Wert des Mediums sollte im Bereich von 5 bis 8,5, vorzugsweise um 7,0 liegen. Der pH-Wert für die Anzucht lässt sich während der Anzucht durch Zugabe von basischen Verbindungen wie Natriumhydroxid, Kaliumhydroxid, Ammoniak bzw. Ammoniakwasser oder sauren Verbindungen wie Phosphorsäure oder Schwefelsäure kontrollieren. Zur Kontrolle der Schaumentwicklung können Antischaummittel wie z. B. Fettsäurepolyglykolester, eingesetzt werden. Zur Aufrechterhaltung der Stabilität von Plasmiden können dem Medium geeignete selektiv wirkende Stoffe, wie z. B. Antibiotika, hinzugefügt werden. Um aerobe Bedingungen aufrechtzuerhalten, werden Sauerstoff oder Sauerstoffhaltige Gasmischungen, wie z.B. Umgebungsluft, in die Kultur eingetragen. Die Temperatur der Kultur liegt normalerweise bei 20°C bis 45°C und vorzugsweise bei 25°C bis 40°C. Die Kultur wird solange fortgesetzt, bis sich ein Maximum des gewünschten Produktes gebildet hat. Dieses Ziel wird normalerweise innerhalb von 10 Stunden bis 160 Stunden erreicht.

Die so erhaltenen, insbesondere mehrfach ungesättigte Fettsäuren enthaltenden, Fermentationsbrühen haben üblicherweise eine Trockenmasse von 7,5 bis 25 Gew.-%.

Die Fermentationsbrühe kann anschließend weiterverarbeitet werden. Je nach Anforderung kann die Biomasse ganz oder teilweise durch Separationsmethoden, wie z. B. Zentrifugation, Filtration, Dekantieren oder einer Kombination dieser Methoden aus der Fermentationsbrühe entfernt oder vollständig in ihr belassen werden. Vorteilhaft wird die Biomasse nach Abtrennung aufgearbeitet.

Die Fermentationsbrühe kann aber auch ohne Zellabtrennung mit bekannten Methoden, wie z. B. mit Hilfe eines Rotationsverdampfers, Dünnschichtverdampfers, Fallfilmverdampfers, durch Umkehrosmose, oder durch Nanofiltration, eingedickt beziehungsweise aufkonzentriert werden. Diese aufkonzentrierte Fermentationsbrühe kann schließlich zur Gewinnung der darin enthaltenen Fettsäuren aufgearbeitet werden.

Die Polynucleotide bzw. Polypeptide der vorliegenden Erfindung, die am Stoffwechsel von Lipiden und Fettsäuren, PUFA-Cofaktoren und Enzymen oder am Transport lipophiler Verbindungen über Membranen beteiligt sind, werden im erfindungsgemäßen Verfahren zur Modulation der Produktion von PUFAs in transgenen Organismen vorteilhaft in Pflanzen, wie Mais, Weizen, Roggen, Hafer, Triticale, Reis, Gerste, Sojabohne, Erdnuss, Baumwolle, Linum Arten wie Öl- oder Faserlein, Brassica-Arten, wie Raps, Canola und Rübsen, Pfeffer, Sonnenblume, Borretsch, Nachtkerze und Tagetes, Solanacaen-Pflanzen, wie Kartoffel, Tabak, Aubergine und Tomate, Vicia-Arten, Erbse, Maniok, Alfalfa, Buschpflanzen (Kaffee, Kakao, Tee), Salix-Arten, Bäume (Ölpalme, Kokosnuss) und ausdauernden Gräsern und Futterfeldfrüchten, entweder direkt (z.B. wenn die Überexpression oder Optimierung eines Fettsäurebiosynthese-Proteins einen direkten Einfluss auf die Ausbeute, Produktion und/oder Effizienz der Produktion der Fettsäure aus modifizierten Organismen hat) verwendet und/oder können eine indirekt Auswirkung haben, die dennoch zu einer Steigerung der Ausbeute, Produktion und/oder Effizienz der Produktion der PUFAs oder einer Abnahme unerwünschter Verbindungen führt (z.B. wenn die Modulation des Stoffwechsels von Lipiden und Fettsäuren, Cofaktoren und Enzymen zu Veränderungen der Ausbeute, Produktion und/oder Effizienz der Produktion oder der Zusammensetzung der gewünschten Verbindungen innerhalb der Zellen führt, was wiederum die Produktion einer oder mehrerer Fettsäuren beeinflussen kann).

Die Kombination verschiedener Vorläufermoleküle und Biosyntheseenzyme führt zur Herstellung verschiedener Fettsäuremoleküle, was eine entscheidende Auswirkung auf die Zusammensetzung der Lipide hat. Da mehrfach ungesättigte Fettsäuren (= PUFAs) nicht nur einfach in Triacylglycerin sondern auch in Membranlipide eingebaut werden.

Besonders zur Herstellung von PUFAs, beispielsweise Stearidonsäure, Eicosapentaensäure und Docosahexaensäure eignen sich Brasicaceae, Boraginaceen, Primulaceen, oder Linaceen. Besonders vorteilhaft eignet sich Lein (Linum usitatissimum) zur Herstellung von PUFAS mit dem erfindungsgemäßen Nukleinsäuresequenzen vorteilhaft, wie beschrieben, in Kombination mit weiteren Desaturasen und Elongasen.

Die Lipidsynthese lässt sich in zwei Abschnitte unterteilen: die Synthese von Fettsäuren und ihre Bindung an sn-Glycerin-3-Phosphat sowie die Addition oder Modifikation einer polaren Kopfgruppe. Übliche Lipide, die in Membranen verwendet werden, umfassen Phospholipide, Glycolipide, Sphingolipide und Phosphoglyceride. Die Fettsäuresynthese beginnt mit der Umwandlung von Acetyl-CoA in Malonyl-CoA durch die Acetyl-CoA-Carboxylase oder in Acetyl-ACP durch die Acetyltransacylase. Nach einer Kondensationsreaktion bilden diese beiden Produktmoleküle zusammen Acetoacetyl-ACP, das über eine Reihe von Kondensations-, Reduktions- und Dehydrätisierungsreaktionen umgewandelt wird, so dass ein gesättigtes Fettsäuremolekül mit der gewünschten Kettenlänge erhalten wird. Die Produktion der ungesättigten Fettsäuren aus diesen Molekülen wird durch spezifische Desaturasen katalysiert, und zwar entweder aerob mittels molekularem Sauerstoff oder anaerob (bezüglich der Fettsäuresynthese in Mikroorganismen siehe F.C. Neidhardt et al. (1996) E. coli und Salmonella. ASM Press: Washington, D.C., S. 612-636 und darin enthaltene Literaturstellen; Lengeler et al. (Hrsgb.) (1999) Biology of Procaryotes. Thieme: Stuttgart, New York, und die enthaltene Literaturstellen, sowie Magnuson, K., et al. (1993) Microbiological Reviews 57:522-542 und die enthaltenen Literaturstellen). Die so hergestellten an Phospholipide gebundenen Fettsäuren müssen anschließend wieder für die weitere Elongationen aus den Phospholipiden in den FettsäureCoA-Ester-Pool überführt werden. Dies ermöglichen Acyl-CoA:Lysophospholipid-Acyltransferasen. Weiterhin können diese Enzyme die elongierten Fettsäuren wieder von den CoA-Estern auf die Phospholipide übertragen. Diese Reaktionsabfolge kann gegebenenfalls mehrfach durchlaufen werden.

Vorläufer für die PUFA-Biosynthese sind beispielsweise Ölsäure, Linol- und Linolensäure. Diese C₁₈-Kohlenstoff-Fettsäuren müssen auf C₂₀ und C₂₂ verlängert werden, damit Fettsäuren vom Eicosa- und Docosa-Kettentyp erhalten werden. Mithilfe der im Verfahren verwendeten Desaturasen wie der Δ-12-, Δ-4-, Δ-5- und Δ-6-Desaturasen und/oder der Δ-5-, Δ-6-Elongasen können Arachidonsäure, Eicosapentaensäure, Docosapentaensäure oder Docosahexaensäure vorteilhaft Eicosapentaensäure und/oder Docosahexaensäure hergestellt werden und anschließend für verschiedene Zwecke bei Nahrungsmittel-, Futter-, Kosmetik- oder pharmazeutischen Anwendungen verwendet werden. Mit den genannten Enzymen können C₂₀- und/oder C₂₂-Fettsäuren mit mindestens zwei vorteilhaft mindestens drei, vier, fünf oder sechs Doppelbindungen im Fettsäuremolekül, vorzugsweise C₂₀- oder C₂₂-Fettsäuren mit vorteilhaft vier, fünf oder sechs Doppelbindungen im Fettsäuremolekül hergestellt werden. Die Desaturierung kann vor oder nach Elongation der entsprechenden Fettsäure erfolgen. Daher führen die Produkte der Desaturaseaktivitäten und der möglichen weiteren Desaturierung und Elongation zu bevorzugten PUFAs mit höherem Desaturierungsgrad, einschließlich einer weiteren Elongation von C₂₀ zu C₂₂-Fettsäuren,zu Fettsäuren wie γ-Linolensäure, Dihomo-γ-linolensäure, Arachidonsäure, Stearidonsäure, Eicosatetraensäure oder Eicosapentaensäure. Substrate der verwendeten Desaturasen und Elongasen im erfindungsgemäßen Verfahren sind C₁₆-, C₁₈- oder C₂₀-Fettsäuren wie zum Beispiel Linolsäure, γ-Linolensäure, α-Linolensäure, Dihomo-γ-linolensäure, Eicosatetraensäure oder Stearidonsäure. Bevorzugte Substrate sind Linolsäure, γ-Linolensäure und/oder α-Linolensäure, Dihomo-γ-linolensäure bzw. Arachidonsäure, Eicosatetraensäure oder Eicosapentaensäure. Die synthetisierten C₂₀- oder C₂₂-Fettsäuren mit mindestens zwei, drei, vier, fünf oder sechs Doppelbindungen in der Fettsäure fallen im erfindungsgemäßen Verfahren in Form der freien Fettsäure oder in Form ihrer Ester beispielsweise in Form ihrer Glyceride an.

Unter dem Begriff "Glycerid" wird ein mit ein, zwei oder drei Carbonsäureresten verestertes Glycerin verstanden (Mono-, Di- oder Triglycerid). Unter "Glycerid" wird auch ein Gemisch an verschiedenen Glyceriden verstanden. Das Glycerid oder das Glyceridgemisch kann weitere Zusätze, z.B. freie Fettsäuren, Antioxidantien, Proteine, Kohlenhydrate, Vitamine und/oder andere Substanzen enthalten.

Unter einem "Glycerid" im Sinne des erfindungsgemäßen Verfahrens werden ferner vom Glycerin abgeleitete Derivate verstanden. Dazu zählen neben den oben beschriebenen Fettsäureglyceriden auch Glycerophospholipide und Glyceroglycolipide. Bevorzugt seien hier die Glycerophospholipide wie Lecithin (Phosphatidylcholin), Cardiolipin, Phosphatidylglycerin, Phosphatidylserin und Alkylacylglycerophospholipide beispielhaft genannt.

Ferner müssen Fettsäuren anschließend an verschiedene Modifikationsorte transportiert und in das Triacylglycerin-Speicherlipid eingebaut werden. Ein weiterer wichtiger Schritt bei der Lipidsynthese ist der Transfer von Fettsäuren auf die polaren Kopfgruppen, beispielsweise durch Glycerin-Fettsäure-Acyltransferase (siehe Frentzen, 1998, Lipid, 100(4-5):161-166).

Veröffentlichungen über die Pflanzen-Fettsäurebiosynthese, Desaturierung, den Lipidstoffwechsel und Membrantransport von fetthaltigen Verbindungen, die Betaoxidation, Fettsäuremodifikation und Cofaktoren, Triacylglycerin-Speicherung und - Assemblierung einschließlich der Literaturstellen darin siehe in den folgenden Artikeln: Kinney, 1997, Genetic Engeneering, Hrsgb.: JK Setlow, 19:149-166; Ohlrogge und Browse, 1995, Plant Cell 7:957-970; Shanklin und Cahoon, 1998, Annu. Rev. Plant Physiol. Plant Mol. Biol. 49:611-641; Voelker, 1996, Genetic Engeneering, Hrsgb.: JK Setlow, 18:111-13; Gerhardt, 1992, Prog. Lipid R. 31:397-417; Gühnemann-Schäfer & Kindl, 1995, Biochim. Biophys Acta 1256:181-186; Kunau et al., 1995, Prog. Lipid Res. 34:267-342; Stymne et al., 1993, in: Biochemistry and Molecular Biology of Membrane and Storage Lipids of Plants, Hrsgb.: Murata und Somerville, Rockville, American Society of Plant Physiologists, 150-158, Murphy & Ross 1998, Plant Journal. 13(1):1-16.

Die im Verfahren hergestellten PUFAs, umfassen eine Gruppe von Molekülen, die höhere Tiere nicht mehr synthetisieren können und somit aufnehmen müssen oder die höhere Tiere nicht mehr ausreichend selbst herstellen können und somit zusätzlich aufnehmen müssen, obwohl sie leicht von anderen Organismen, wie Bakterien, synthetisiert werden, beispielsweise können Katzen Arachidonsäure nicht mehr synthetisieren.

Unter Phospholipiden im Sinne der Erfindung sind zu verstehen Phosphatidylcholin, Phosphatidylethanolamin, Phosphatidylserin, Phosphatidylglycerin und/oder Phosphatidylinositol vorteilhafterweise Phosphatidylcholin. Die Begriffe Produktion oder Produktivität sind im Fachgebiet bekannt und beinhalten die Konzentration des Fermentationsproduktes (Verbindungen der Formel I), das in einer bestimmten Zeitspanne und einem bestimmten Fermentationsvolumen gebildet wird (z.B. kg Produkt pro Stunde pro Liter). Es umfasst auch die Produktivität innerhalb einer Pflanzenzelle oder einer Pflanze, das heißt den Gehalt an den gewünschten im Verfahren hergestellten Fettsäuren bezogen auf den Gehalt an allen Fettsäuren in dieser Zelle oder Pflanze. Der Begriff Effizienz der Produktion umfasst die Zeit, die zur Erzielung einer bestimmten Produktionsmenge nötig ist (z.B. wie lange die Zelle zur Aufrichtung einer bestimmten Durchsatzrate einer Feinchemikalie benötigt). Der Begriff Ausbeute oder Produkt/Kohlenstoff-Ausbeute ist im Fachgebiet bekannt und umfasst die Effizienz der Umwandlung der Kohlenstoffquelle in das Produkt (d.h. die Feinchemikalie). Dies wird gewöhnlich beispielsweise ausgedrückt als kg Produkt pro kg Kohlenstoffquelle. Durch Erhöhen der Ausbeute oder Produktion der Verbindung wird die Menge der gewonnenen Moleküle oder der geeigneten gewonnenen Moleküle dieser Verbindung in einer bestimmten Kulturmenge über einen festgelegten Zeitraum erhöht. Die Begriffe Biosynthese oder Biosyntheseweg sind im Fachgebiet bekannt und umfassen die Synthese einer Verbindung, vorzugsweise einer organischen Verbindung, durch eine Zelle aus Zwischenverbindungen, beispielsweise in einem Mehrschritt- und stark regulierten Prozess. Die Begriffe Abbau oder Abbauweg sind im Fachgebiet bekannt und umfassen die Spaltung einer Verbindung, vorzugsweise einer organischen Verbindung, durch eine Zelle in Abbauprodukte (allgemeiner gesagt, kleinere oder weniger komplexe Moleküle) beispielsweise in einem Mehrschritt- und stark regulierten Prozess. Der Begriff Stoffwechsel ist im Fachgebiet bekannt und umfasst die Gesamtheit der biochemischen Reaktionen, die in einem Organismus stattfinden. Der Stoffwechsel einer bestimmten Verbindung (z.B. der Stoffwechsel einer Fettsäure) umfasst dann die Gesamtheit der Biosynthese-, Modifikations- und Abbauwege dieser Verbindung in der Zelle, die diese Verbindung betreffen.

Durch den Einsatz der erfindungsgemäßen Polynucleotide und optional weiterer Polynucleotide, die für Enzyme des Lipid oder Fettsäurestoffwechsels kodieren, in dem erfindungsgemäßen Verfahren können verschiedene vorteilhafte Effekte erzielt werden. So kann die Ausbeute, Produktion und/oder Effizienz der Produktion der mehrfach ungesättigten Fettsäuren in einer Pflanze, bevorzugt in einer Ölfruchtpflanze, oder einem Mikroorganismus beeinflusst werden kann. Die Anzahl oder Aktivität der erfindungsgemäßen Polypeptide bzw. Polynucleotide kann erhöht werden, so dass größere Mengen der Genprodukte und damit letztlich größere Mengen der Verbindungen der allgemeinen Formel I hergestellt werden. Auch eine de novo Synthese in einem Organismus, dem die Aktivität und Fähigkeit zur Biosynthese der Verbindungen vor dem Einbringen des/der entsprechenden Gens/Gene fehlte, ist möglich. Entsprechendes gilt für die Kombination mit weiteren Desaturasen oder Elongasen oder weiteren Enzymen aus dem Fettsäure- und Lipidstoffwechsel. Auch die Verwendung verschiedener divergenter, d.h. auf DNA-Sequenzebene unterschiedlicher Sequenzen kann dabei vorteilhaft sein bzw. die Verwendung von Promotoren zur Genexpression, die eine andere zeitliche Genexpression z.B. abhängig vom Reifegrad eines Samens oder Öl-speichernden Gewebes ermöglicht.

Durch das Einbringen eines erfindungsgemäßen Polynucleotids in einen Organismus allein oder in Kombination mit anderen Genen in eine Zelle kann nicht nur den Biosynthesefluss zum Endprodukt erhöht, sondern auch die entsprechende Triacylglycerin- Zusammensetzung erhöht oder de novo geschaffen werden. Ebenso kann die Anzahl oder Aktivität anderer Gene, die am Import von Nährstoffen, die zur Biosynthese einer oder mehrerer Fettsäuren, Ölen, polaren und/oder neutralen Lipiden nötig sind, erhöht sein, so dass die Konzentration dieser Vorläufer, Cofaktoren oder Zwischenverbindungen innerhalb der Zellen oder innerhalb des Speicherkompartiments erhöht ist, wodurch die Fähigkeit der Zellen zur Produktion von PUFAs weiter gesteigert wird. Durch Optimierung der Aktivität oder Erhöhung der Anzahl einer oder mehrerer erfindungsgemäßer Polynucleotide bzw. Polypeptide, die an der Biosynthese dieser Verbindungen beteiligt sind, oder durch Zerstören der Aktivität einer oder mehrerer Gene, die am Abbau dieser Verbindungen beteiligt sind, kann es möglich sein, die Ausbeute, Produktion und/oder Effizienz der Produktion von Fettsäure- und Lipidmolekülen aus Organismen insbesondere aus Pflanzen zu steigern. Die im Verfahren gewonnenen Fettsäuren eignen sich als Ausgangsmaterial für die chemische Synthese von weiteren Wertprodukten. Sie können beispielsweise in Kombination miteinander oder allein zur Herstellung von Pharmaka, Nahrungsmittel, Tierfutter oder Kosmetika verwendet werden.

Aus den zuvor gemachten Ausführungen versteht sich, dass die Erfindung auch ein Verfahren zur Herstellung einer Öl-, Lipid- oder Fettsäurezusammensetzung umfassend die Schritte des erfindungsgemäßen Verfahrens und den weiteren Schritt des Formulierens des Stoffes als Öl-, Lipid- oder Fettsäurezusammensetzung betrifft.

In einer bevorzugten Ausführungsform dieses Verfahren wird die Öl-, Lipid- oder Fettsäurezusammensetzung weiter formuliert zu einem Arzneimittel, zu Kosmetik, zu einem Nahrungsmittel, zu einem Futtermittel, vorzugsweise Fischfutter, oder zu einem Nahrungsergänzungsmittel.

Schließlich betrifft die Erfindung grundsätzlich die Verwendung des Polynucleotids, des Vektors, der Wirtszelle, des Polypeptids oder des transgenen, nicht-humanen Organsismus der vorliegenden Erfindung für die Herstellung einer Öl-, Lipid- oder Fettsäurezusammensetzung. Diese ist dann bevorzugt als Arzneimittel, Kosmetik, Nahrungsmittel, Futtermittel, vorzugsweise Fischfutter, oder Nahrungsergänzungsmittel einzusetzen.

Der Inhalt sämtlicher in dieser Patentanmeldung zitierten Literaturstellen, Patentanmeldungen, Patente und veröffentlichten Patentanmeldungen ist hiermit durch Bezugnahme auf den jeweiligen speziellen Offenbarungsgehalt aufgenommen.

### Figuren

Figur 1 zeigt einen Sequenzvergleich der Δ5- und Δ6-Elongase Aminosäuresequenzen aus O. lucimarinus, O. tauri und T. pseudonana im ClustalW-Vergleich.
Figur 2 zeigt einen Sequenzvergleich der Δ4-Desaturase Aminosäuresequenzen aus O. lucimarinus, O. tauri und T. pseudonana im ClustalW-Vergleich.
Figur 3 zeigt einen Sequenzvergleich der Δ5-Desaturase Aminosäuresequenzen aus O. lucimarinus, O. tauri und T. pseudonana im ClustalW-Vergleich.
Figur 4 zeigt einen Sequenzvergleich der Δ6-Desaturase Aminosäuresequenzen aus O. lucimarinus, O. tauri und T. pseudonana im ClustalW-Vergleich.
Figur 5 zeigt einen Sequenzvergleich der Δ12-Desaturase Aminosäuresequenzen aus O. lucimarinus, O. tauri und T. pseudonana im ClustalW-Vergleich.
Figur 6 zeigt die gaschromatographische Bestimmung der Fettsäuren aus Hefen, die mit dem Plasmid pYES (A, B) bzw. pYES-D5Elo(Ol) (C) transformiert wurden. Die Fettsäure 20:4Δ5,8,11,14 wurde zugefüttert (B, C).
Figur 7 zeigt die gaschromatographische Bestimmung der Fettsäuren aus Hefen, die mit dem Plasmid pYES (A, B, C) bzw. pYES-D6Elo(Ol) (D,E) transformiert wurden. Die Fettsäuren 18:3Δ6,9,12 bzw. 18:4Δ6,9,12,15 wurde zugefüttert (B, D) bzw. (C, E).
Figur 8 zeigt die gaschromatographische Bestimmung der Fettsäuren aus Hefen, die mit dem Plasmid pYES (A, B) bzw. pYES-D5Des(Ol_2) (C) transformiert wurden. Die Fettsäuren 20:3Δ5,8,11,14 wurde zugefüttert in (B) und (C).
Figur 9 zeigt die gaschromatographische Bestimmung der Fettsäuren aus Hefen, die mit dem Plasmid pYES (A) bzw. pYES-D12Des(Ol) (B) transformiert wurden.
Figur 10 zeigt die gaschromatographische Bestimmung der Fettsäuren aus Hefe. pYes-d5Des(Ol_1) in Hefe Stamm InvSc ohne Zugabe von Fettsäuren (A); pYes-d5Des(Ol_1) in Hefe Stamm InvSc nach Zugabe der Fettsäure 20:3n-6 (B), pYes-d5Des(Ol_1) in Hefe Stamm InvSc nach Zugabe der Fettsäure 20:4n-3 (C).

Die Erfindung wird durch die nachstehenden Beispiele weiter veranschaulicht, die nicht als beschränkend aufgefasst werden sollten.

### Beispiele

### Beispiel 1: Allgemeine Klonierungsverfahren

Die Klonierungsverfahren wie z.B. Restriktionsspaltungen, Agarose-Gelelektrophorese, Reinigung von DNA-Fragmenten, Transfer von Nukleinsäuren auf Nitrozellulose und Nylon Membranen, Verknüpfen von DNA-Fragmenten, Transformation von Escherichia coli Zellen, Anzucht von Bakterien und die Sequenzanalyse rekombinanter DNA wurden wie bei Sambrook et al. (1989) (Cold Spring Harbor Laboratory Press: ISBN 0-87969-309-6) beschrieben durchgeführt.

### Beispiel 2: Sequenzanalyse rekombinanter DNA

Die Sequenzierung rekombinanter DNA-Moleküle erfolgte mit einem Laserfluoreszenz-DNA-Sequenzierer der Firma ABI nach der Methode von Sanger (Sanger et al. (1977) Proc. Natl. Acad. Sci. USA74, 5463-5467). Fragmente resultierend aus einer Polymerase Kettenreaktion wurden zur Vermeidung von Polymerasefehlern in zu exprimierenden Konstrukten sequenziert und überprüft.

### Beispiel 3: Lipidextraktion aus Hefen

Die Auswirkung der genetischen Modifikation in Pflanzen, Pilzen, Algen, Ciliaten oder auf die Produktion einer gewünschten Verbindung (wie einer Fettsäure) kann bestimmt werden, indem die modifizierten Mikroorganismen oder die modifizierte Pflanze unter geeigneten Bedingungen (wie den vorstehend beschriebenen) gezüchtet werden und das Medium und/oder die zellulären Komponenten auf die erhöhte Produktion des gewünschten Produktes (d.h. von Lipiden oder einer Fettsäure) untersucht wird. Diese Analysetechniken sind dem Fachmann bekannt und umfassen Spektroskopie, Dünnschichtchromatographie, Färbeverfahren verschiedener Art, enzymatische und mikrobiologische Verfahren sowie analytische Chromatographie, wie Hochleistungs-Flüssigkeitschromatographie (siehe beispielsweise Ullman, Encyclopedia of Industrial Chemistry, Bd. A2, S. 89-90 und S. 443-613, VCH: Weinheim (1985); Fallon, A., et al., (1987) "Applications of HPLC in Biochemistry" in: Laboratory Techniques in Biochemistry and Molecular Biology, Bd. 17; Rehm et al. (1993) Biotechnology, Bd. 3, Kapitel III: "Product recovery and purification", S. 469-714, VCH: Weinheim; Belter, P.A., et al. (1988) Bioseparations: downstream processing for Biotechnology, John Wiley and Sons; Kennedy, J.F., und Cabral, J.M.S. (1992) Recovery processes for biological Materials, John Wiley and Sons; Shaeiwitz, J.A., und Henry, J.D. (1988) Biochemical Separations, in: Ullmann's Encyclopedia of Industrial Chemistry, Bd. B3; Kapitel 11, S. 1-27, VCH: Weinheim; und Dechow, F.J. (1989) Separation and purification techniques in biotechnology, Noyes Publications).

Neben den oben erwähnten Verfahren werden Pflanzenlipide aus Pflanzenmaterial wie von Cahoon et al. (1999) Proc. Natl. Acad. Sci. USA 96 (22):12935-12940, und Browse et al. (1986) Analytic Biochemistry 152:141-145, beschrieben extrahiert. Die qualitative und quantitative Lipid- oder Fettsäureanalyse ist beschrieben bei Christie, William W., Advances in Lipid Methodology, Ayr/Scotland: Oily Press (Oily Press Lipid Library; 2); Christie, William W., Gas Chromatography and Lipids. A Practical Guide - Ayr, Scotland: Oily Press, 1989, Repr. 1992, IX, 307 S. (Oily Press Lipid Library; 1); "Progress in Lipid Research, Oxford: Pergamon Press, 1 (1952) - 16 (1977) u.d.T.: Progress in the Chemistry of Fats and Other Lipids CODEN.

Zusätzlich zur Messung des Endproduktes der Fermentation ist es auch möglich, andere Komponenten der Stoffwechselwege zu analysieren, die zur Produktion der gewünschten Verbindung verwendet werden, wie Zwischen- und Nebenprodukte, um die Gesamteffizienz der Produktion der Verbindung zu bestimmen. Die Analyseverfahren umfassen Messungen der Nährstoffmengen im Medium (z.B. Zucker, Kohlenwasserstoffe, Stickstoffquellen, Phosphat und andere Ionen), Messungen der Biomassezusammensetzung und des Wachstums, Analyse der Produktion üblicher Metabolite von Biosynthesewegen und Messungen von Gasen, die während der Fermentation erzeugt werden. Standardverfahren für diese Messungen sind in Applied Microbial Physiology; A Practical Approach, P.M. Rhodes und P.F. Stanbury, Hrsgb., IRL Press, S. 103-129; 131-163 und 165-192 (ISBN: 0199635773) und darin angegebenen Literaturstellen beschrieben.

Ein Beispiel ist die Analyse von Fettsäuren (Abkürzungen: FAME, Fettsäuremethylester; GC-MS, Gas-Flüssigkeitschromatographie-Massenspektrometrie; TAG, Triacylglycerin; TLC, Dünnschichtchromatographie).

Der unzweideutige Nachweis für das Vorliegen von Fettsäureprodukten kann mittels Analyse rekombinanter Organismen nach Standard-Analyseverfahren erhalten werden: GC, GC-MS oder TLC, wie verschiedentlich beschrieben von Christie und den Literaturstellen darin (1997, in: Advances on Lipid Methodology, Vierte Aufl.: Christie, Oily Press, Dundee, 119-169; 1998, Gaschromatographie-Massenspektrometrie-Verfahren, Lipide 33:343-353).

Das zu analysierende Material kann durch Ultraschallbehandlung, Mahlen in der Glasmühle, flüssigen Stickstoff und Mahlen oder über andere anwendbare Verfahren aufgebrochen werden. Das Material muss nach dem Aufbrechen zentrifugiert werden. Das Sediment wird in Aqua dest. resuspendiert, 10 min bei 100°C erhitzt, auf Eis abgekühlt und erneut zentrifugiert, gefolgt von Extraktion in 0,5 M Schwefelsäure in Methanol mit 2 % Dimethoxypropan für 1 Std. bei 90°C, was zu hydrolysierten Öl- und Lipidverbindungen führt, die transmethylierte Lipide ergeben. Diese Fettsäuremethylester werden in Petrolether extrahiert und schließlich einer GC-Analyse unter Verwendung einer Kapillarsäule (Chrompack, WCOT Fused Silica, CP-Wax-52 CB, 25 mikrom, 0,32 mm) bei einem Temperaturgradienten zwischen 170°C und 240°C für 20 min und 5 min bei 240°C unterworfen. Die Identität der erhaltenen Fettsäuremethylester muss unter Verwendung von Standards, die aus kommerziellen Quellen erhältlich sind (d.h. Sigma), definiert werden.

### Beispiel 4: Klonierung und Charakterisierung von Elongase-Genen aus Ostreococcus lucimarinus

Durch Suche nach konservierten Bereichen in den Proteinsequenzen in Elongase-Genen konnten zwei Sequenzen mit entsprechenden Motiven in einer *Ostreococcus lucimarinus* Sequenzdatenbank identifiziert werden. In einem weiteren Schritt wurden die Gene mittels Sequenzvergleich, Genvorhersage und der Suche nach kodierenden Bereichen charakterisiert. Folgende kodierende Bereiche wurden gefunden:

**Tabelle 1: Kodierende Bereiche**

| Gen-Name | SEQ ID | Aminosäuren |
|---|---|---|
| D5Elo(Ol) | 12 | 298 |
| D6Elo(Ol) | 16 | 287 |

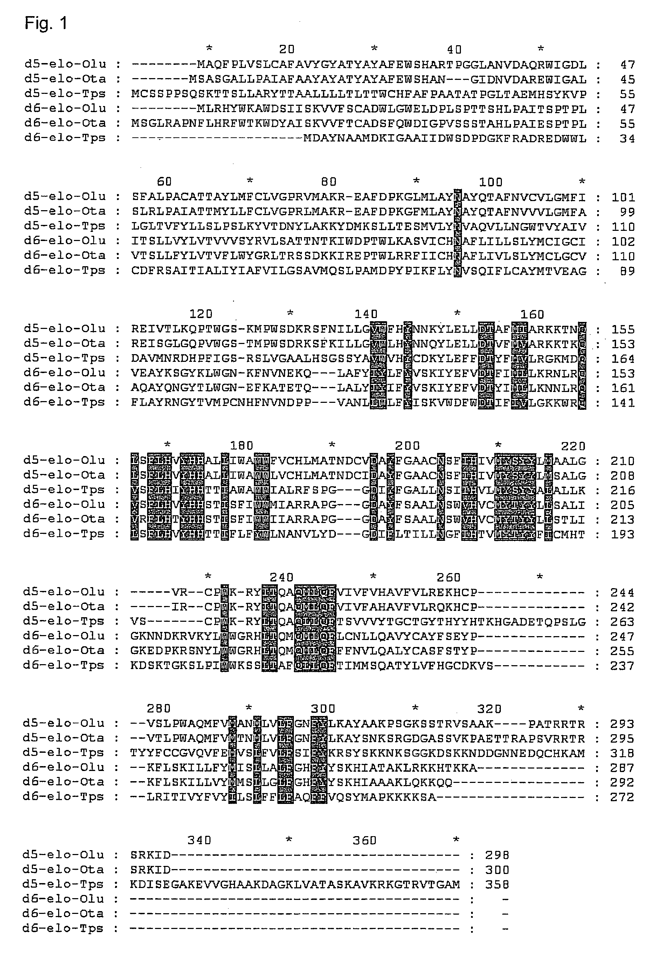

Figur 1 zeigt die Sequenzähnlichkeiten zu anderen Algen (*Ostreococcus tauri, Thalassiosira pseudonana*) für die verschiedenen Elongase-Aminosäure Sequenzen im ClustalW Sequenzvergleich. Überraschenderweise unterscheiden sich die Sequenzen von *O*. *lucimarinus* in ihrer Aminosäuresequenz deutlich von den anderen Algen.

**Tabelle 2: Sequenzidentitäten einzelner Elongasen**

| Gen-Name | SEQ ID | Organismus | Identität in % |
|---|---|---|---|
| D5Elo(Ol) | 28 | O. lucimarinus | 100 |
| | | O. tauri | 77 |
| | | T. pseudonana | 21 |
| D6Elo(Ol) | 32 | O. lucimarinus | 100 |
| | | O. tauri | 71 |
| | | T. pseudonana | 25 |

Die Klonierung wurde wie folgt durchgeführt:
40 ml einer *Ostreococcus lucimarinus* Kultur in der stationären Phase wurden abzentrifugiert und in 100 µl Aqua bidest resuspendiert und bei -20°C gelagert. Auf der Basis des PCR-Verfahren wurden die zugehörigen genomischen DNAs amplifiziert. Die entsprechenden Primerpaare wurden so ausgewählt, dass sie die Hefe-Konsensus-Sequenz für hocheffiziente Translation (Kozak, Cell 1986, 44:283-292) neben dem Startcodon trugen. Die Amplifizierung der DNAs wurde jeweils mit 1 µl aufgetauten Zellen, 200 µM dNTPs, 2,5 U *Taq*-Polymerase und 100 pmol eines jeden Primers in einem Gesamtvolumen von 50 µl durchgeführt. Die Bedingungen für die PCR waren wie folgt: Erste Denaturierung bei 95°C für 5 Minuten, gefolgt von 30 Zyklen bei 94°C für 30 Sekunden, 55°C für 1 Minute und 72°C für 2 Minuten sowie ein letzter Verlängerungsschritt bei 72°C für 10 Minuten.

Zur Charakterisierung der Funktion der Elongasen aus *Ostreococcus lucimarinus* werden die offenen Leserahmen der jeweiligen DNAs stromabwärts des Galactose-induzierbaren GAL1-Promotors von pYES2.1/V5-His-TOPO (Invitrogen) kloniert, wobei pOLE1 und pOLE2 erhalten werden.

Der *Saccharomyces cerevisiae-*Stamm 334 wird durch Elektroporation (1500 V) mit dem Vektor pOLE1 bzw. pOLE2 transformiert. Als Kontrolle wird eine Hefe verwendet, die mit dem leeren Vektor pYES2 transformiert wird. Die Selektion der transformierten Hefen erfolgt auf Komplett-Minimalmedium (CMdum)-Agarplatten mit 2% Glucose, aber ohne Uracil. Nach der Selektion werden je drei Transformanten zur weiteren funktionellen Expression ausgewählt.

Für die Expresssion der Ol-Elongasen werden zunächst Vorkulturen aus jeweils 5 ml CMdum-Flüssigmedium mit 2% (w/v) Raffinose aber ohne Uracil mit den ausgewählten Transformanten angeimpft und 2 Tage bei 30°C, 200 rpm inkubiert. 5 ml CMdum-Flüssigmedium (ohne Uracil) mit 2% Raffinose und 300 µM verschiedener Fettsäuren werden dann mit den Vorkulturen auf eine OD₆₀₀ von 0,05 angeimpft. Die Expression wird durch die Zugabe von 2% (w/v) Galactose induziert. Die Kulturen wurden für weitere 96 h bei 20°C inkubiert.

Hefen, die mit den Plasmiden pYES2, pOLE1 und pOLE2 transformiert sind, werden folgendermaßen analysiert:
Die Hefezellen aus den Hauptkulturen werden durch Zentrifugation (100 x g, 5 min, 20°C) geerntet und mit 100 mM NaHCO₃, pH 8,0 gewaschen, um restliches Medium und Fettsäuren zu entfernen. Aus den Hefe-Zellsedimenten werden Fettsäuremethylester (FAMEs) durch saure Methanolyse hergestellt. Hierzu werden die Zellsedimente mit 2 ml 1 N methanolischer Schwefelsäure und 2% (v/v) Dimethoxypropan für 1 h bei 80°C inkubiert. Die Extraktion der FAMES erfolgt durch zweimalige Extraktion mit Petrolether (PE). Zur Entfernung nicht derivatisierter Fettsäuren werden die organischen Phasen je einmal mit 2 ml 100 mM NaHCO₃, pH 8,0 und 2 ml Aqua dest. gewaschen. Anschließend wurden die PE-Phasen mit Na₂SO₄ getrocknet, unter Argon eingedampft und in 100 µl PE aufgenommen. Die Proben werden auf einer DB-23-Kapillarsäule (30 m, 0,25 mm, 0,25 µm, Agilent) in einem Hewlett-Packard 6850-Gaschromatographen mit Flammenionisationsdetektor getrennt. Die Bedingungen für die GLC-Analyse sind wie folgt: Die Ofentemperatur wurde von 50°C bis 250°C mit einer Rate von 5°C/min und schließlich 10 min bei 250°C(halten) programmiert.

Die Identifikation der Signale erfolgt durch Vergleiche der Retentionszeiten mit entsprechenden Fettsäurestandards (Sigma). Die Methodik ist beschrieben zum Beispiel in Napier and Michaelson, 2001,Lipids. 36(8):761-766; Sayanova et al., 2001, Journal of Experimental Botany. 52(360):1581-1585, Sperling et al., 2001, Arch. Biochem. Biophys. 388(2):293-298 und Michaelson et al., 1998, FEBS Letters. 439(3):215-218.

### Aktivitäts- und Substratbestimmung von D5Elo(OI):

Zur Ermittlung der Aktivität und Substratspezifität der d5Elo(OI) wurden verschiedene Fettsäuren gefüttert (Tab. 3). Die gefütterten Substrate sind dabei in großen Mengen in allen transgenen Hefen nachzuweisen. Die transgenen Hefen zeigen die Synthese neuer Fettsäuren, den Produkten der d5Elo(OI)-Reaktion. Dies bedeutet, dass das Gen d5Elo(Ol) funktional exprimiert wurde.

**Tabelle 3: Fütterung von Hefen mit den Plasmiden pYES und pYES-DSEIo(OI)**

| Probenname/gefütterte Fettsäure | Erwartete Umsetzung | Substrat | Produkt |
|---|---|---|---|
| pYES | Kontrolle | - | - |
| pYES-DSElo(Ol_GA) 20:4 | 20:4 -> 22:4 | 98,0 | 48,5 |
| pYES-DSElo(Ol_GA) 20:4 | 20:4 -> 22:4 | 62,6 | 32,2 |

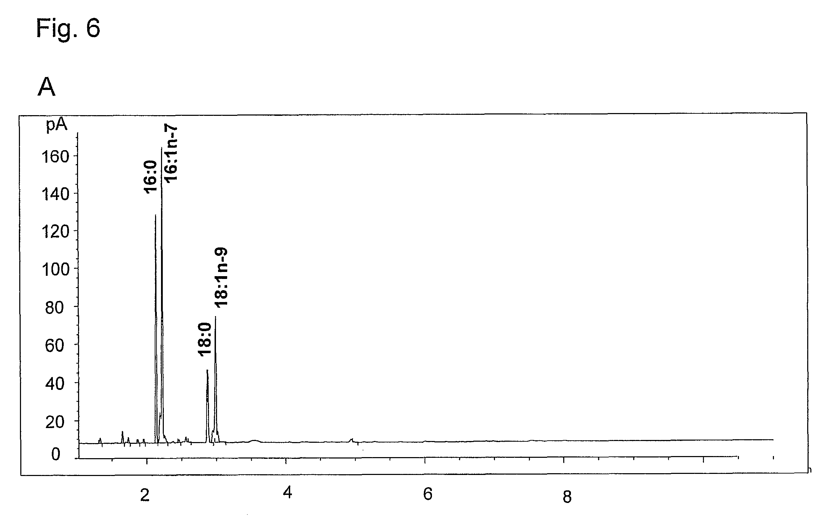

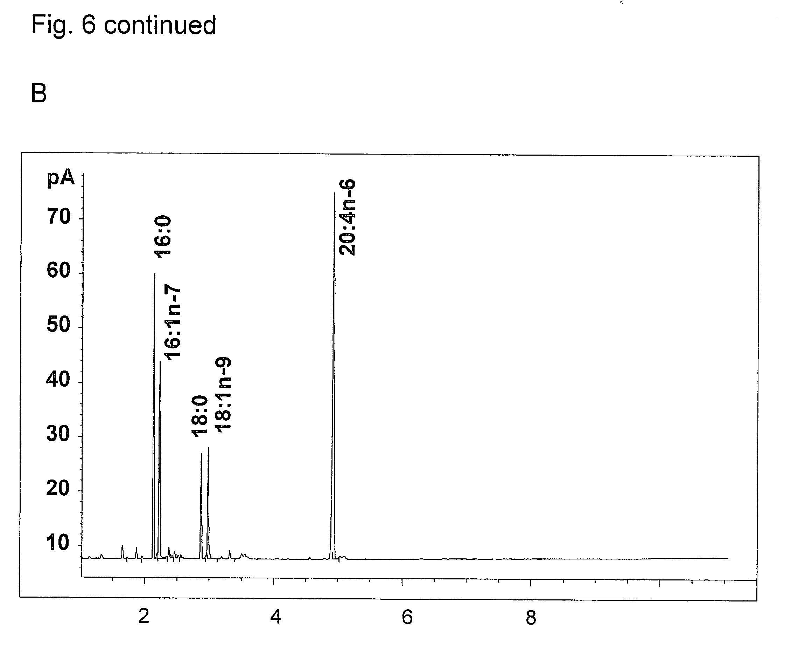

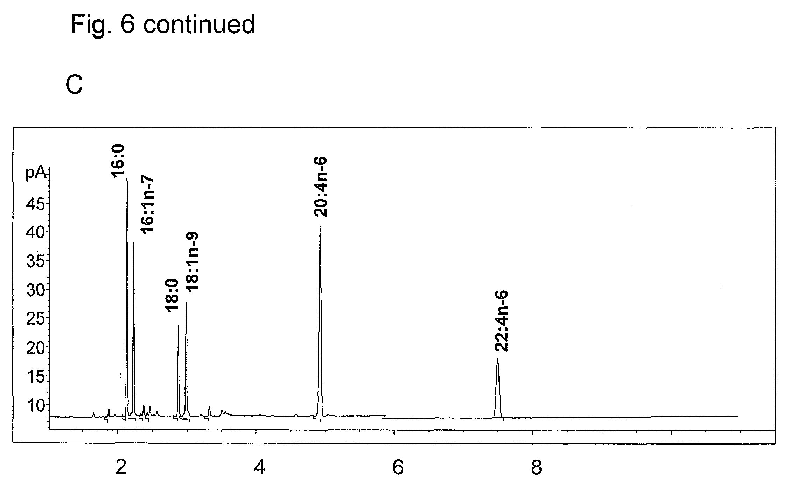

Figur 6 zeigt die Chromatogramme der einzelnen Experimente. Als Kontrolle wurde in Fig. 6A Hefen transformiert mit pYES ohne Zugabe von Fettsäuren analysiert. In Fig. 1B wurde zu den Hefen transformiert mit pYES die Fettsäure 20:4Δ5,8,11,14 gefüttert. Dabei kann die gefütterte Fettsäure in großen Mengen detektiert werden. Dasselbe Experiment ist in Fig. 6C für Hefen transformiert mit dem Plasmid pYES-DSElo(Ol) durchgeführt. Im Unterschied zu Fig. 6B kann in den Hefen mit pYES-D5Elo(Ol) eine zusätzliche Fettsäure detektiert werden, die auf die Aktivität der DSEIo(OI) zurückgehen muss. Anhand der Aktivität kann DSElo(Ol) als Δ5-Elongase charakterisiert werden.

### Zusammenfassung der Ergebnisse zu D5Elo(Ol):

In den Hefefütterungsexperimenten konnte gezeigt werden, dass das klonierte Gen D5Elo(OI) SEQ ID12 funktional exprimiert wurde und Elongase-Aktivität besitzt. Anhand der gefütterten Fettsäure kann D5Elo(Ol) als Δ5-Elongase charakterisiert werden, d.h. C20-Fettsäuren mit einer Δ5-Doppelbindung werden spezifisch verlängert.

### Aktivitäts- und Substratbestimmung von D6Elo(OI):

Zur Ermittlung der Aktivität und Substratspezifität der D6Elo(OI) wurden verschiedene Fettsäuren gefüttert (Tab. 4). Die gefütterten Substrate sind dabei in großen Mengen in allen transgenen Hefen nachzuweisen. Die transgenen Hefen zeigen die Synthese neuer Fettsäuren, den Produkten der D6Elo(OI)-Reaktion. Dies bedeutet, dass das Gen D6Elo(OI) funktional exprimiert wurde.

**Tabelle 4: Fütterung/ Umsetzung verschiedener Fettsäuren durch D6Elo(Ol)**

| Probenname/gefütterte Fettsäure | Erwartete Umsetzung | Substrat | Produkt |
|---|---|---|---|
| pYES | Kontrolle | - | - |
| pYES-D6Elo(Ol_GA) γ18:3 | γ18:3 -> 20:3 | 236,5 | 232,1 |
| pYES-D6Elo(Ol_GA) γ18:3 | γ18:3 -> 20:3 | 111,2 | 126,5 |
| pYES-D6Elo(Ol_GA) 18:4 | 18:4 -> 20:4 | 94,3 | 82,9 |
| pYES-D6Elo(Ol_GA) 18:4 | 18:4 -> 20:4 | 73,2 | 68,3 |

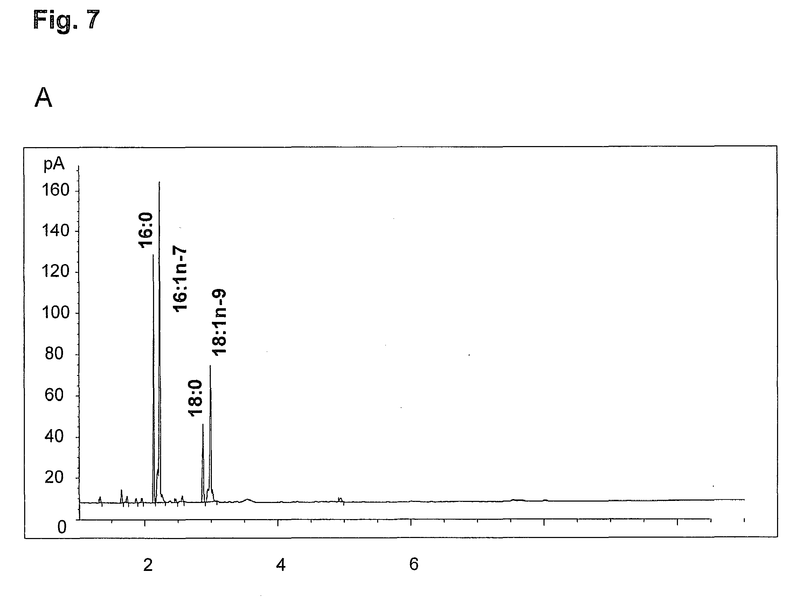

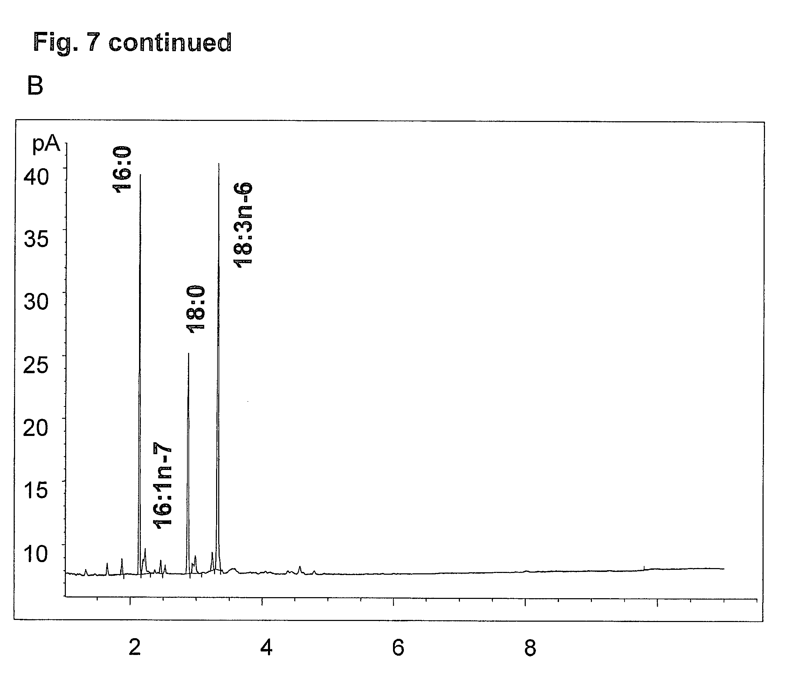

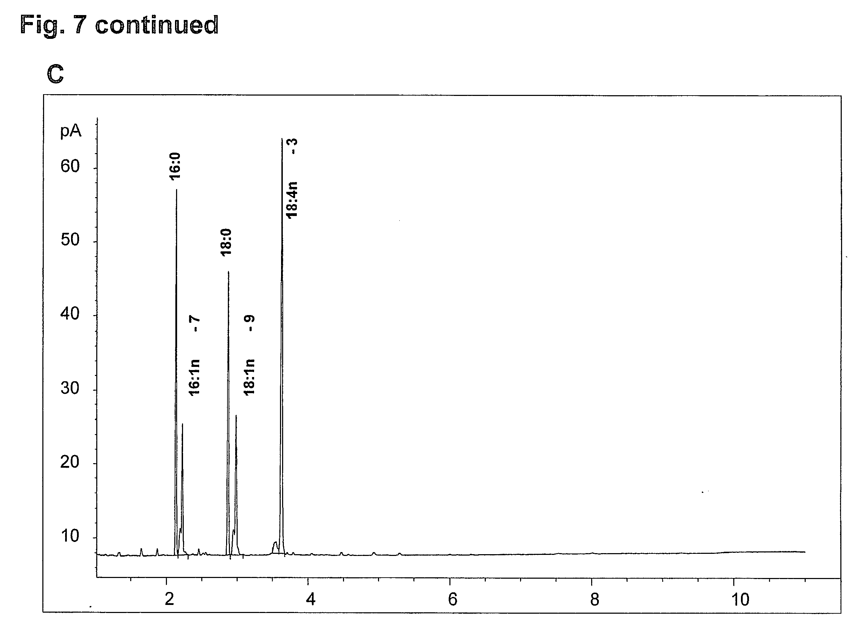

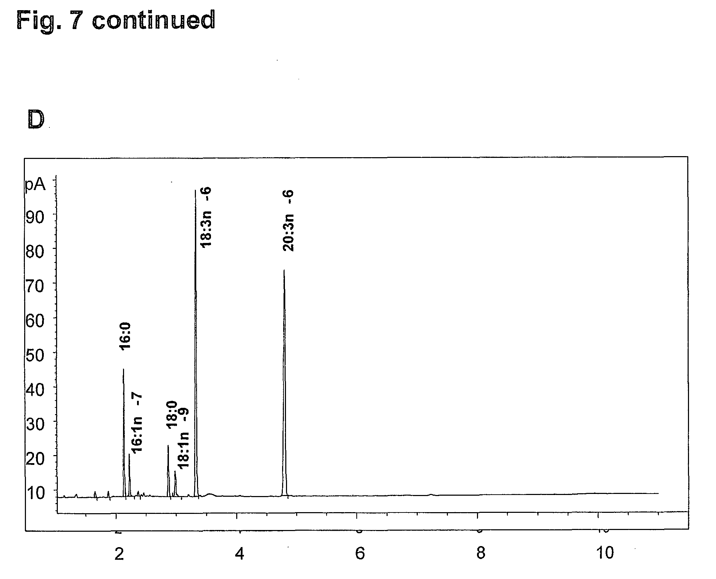

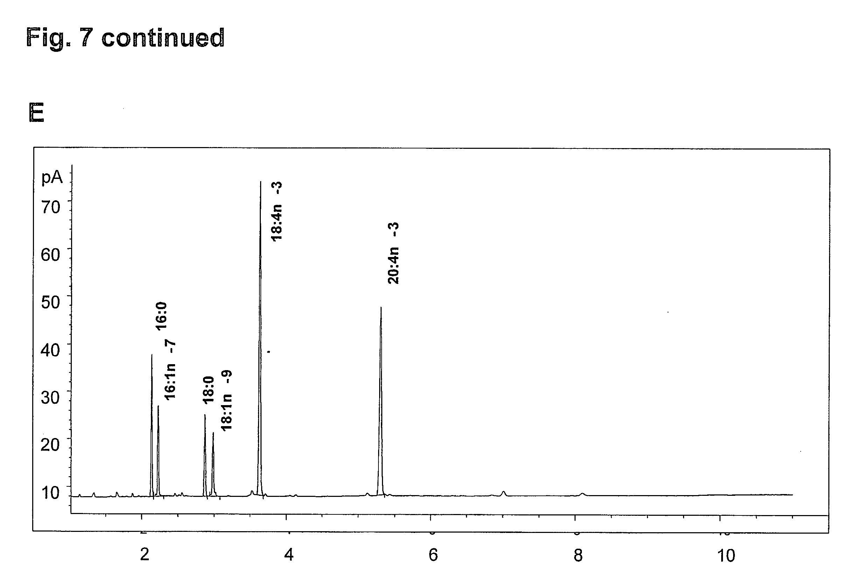

Figur 7 zeigt die Chromatogramme der einzelnen Experimente. Als Kontrolle wurde in Fig. 7A Hefen transformiert mit pYES ohne Zugabe von Fettsäuren analysiert. In Fig. 7B und 7C wurden den Hefen transformiert mit pYES die Fettsäure 18:3Δ6,9,12 (B) bzw. 18:4Δ6,9,12,15 (C) zugegeben. Dabei können die gefütterte Fettsäure in großen Mengen detektiert werden. Dasselbe Experiment ist in Fig. 7C und 7D für Hefen transformiert mit dem Plasmid pYES-D6Elo(OI) durchgeführt. Im Unterschied zu Fig. 7B und 7C kann in den Hefen mit pYES-D6Elo(OI) eine zusätzliche Fettsäure detektiert werden, die auf die Aktivität der D6Elo(OI) zurückgehen muss. Anhand der Aktivität kann D6Elo(OI) als Δ6-Elongase charakterisiert werden.

### Zusammenfassung der Ergebnisse zu D6Elo(OI):

In den Hefefütterungsexperimenten konnte gezeigt werden, dass das klonierte Gen D6Elo(OI) SEQ ID16 funktional exprimiert wurde und Elongase-Aktivität besitzt. Anhand der gefütterten Fettsäure kann D6Elo(OI) als Δ6-Elongase charakterisiert werden, d.h. C18-Fettsäuren mit einer Δ6-Doppelbindung werden spezifisch verlängert.

### Beispiel 5: Klonierung und Charakterisierung von Desaturase-Genen aus Ostreococcus lucimarinus

Durch Suche nach konservierten Bereichen in den Proteinsequenzen mit Hilfe von konservierten Motiven (His-Boxen, Domergue et al. 2002, Eur. J. Biochem. 269, 4105-4113) konnten fünf Sequenzen mit entsprechenden Motiven in einer Ostreococcus lucimarinus Sequenzdatenbank (genomische Sequenzen) identifiziert werden. In einem weiteren Schritt wurden die Gene mittels Sequenzvergleich, Genvorhersage und der Suche nach kodierenden Bereichen charakterisiert. Folgende kodierende Bereiche wurden gefunden::

**Tabelle 5: Kodierende Bereiche**

| Gen-Name | SEQ ID | Aminosäuren |
|---|---|---|
| D4Des(Ol) | 22 | 466 |
| D5Des(Ol) | 26 | 491 |
| D5Des_2(Ol) | 24 | 459 |
| D6Des(Ol) | 30 | 482 |
| D12Des(Ol) | 18 | 362 |
| D12Des_2(Ol) | 20 | 442 |

Zur Charakterisierung der Funktion der Desaturase d6Des(OI) (= Δ-6-Desaturase) aus *Ostreococcus lucimarinus* wird der offenen Leserahmen der DNA stromabwärts des Galactose-induzierbaren GAL1-Promotors von pYES2.1/V5-His-TOPO (Invitrogen) kloniert, wobei der entsprechenden pYES2.1-d6Elo(OI) Klon erhalten wird. In entsprechender Art und Weise können weitere Desaturase-Gene aus Ostreococcus kloniert werden.

Der *Saccharomyces cerevisiae-*Stamm 334 wird durch Elektroporation (1500 V) mit dem Vektor pYES2.1-d6Elo(Ol) transformiert. Als Kontrolle wurde eine Hefe verwendet, die mit dem leeren Vektor pYES2 transformiert wird. Die Selektion der transformierten Hefen erfolgt auf Komplett-Minimalmedium (CMdum)-Agarplatten mit 2% Glucose, aber ohne Uracil. Nach der Selektion werden je drei Transformanten zur weiteren funktionellen Expression ausgewählt.

Für die Expresssion der d6Elo(OI) Desaturase werden zunächst Vorkulturen aus jeweils 5 ml CMdum-Flüssigmedium mit 2% (w/v) Raffinose aber ohne Uracil mit den ausgewählten Transformanten angeimpft und 2 Tage bei 30°C, 200 rpm inkubiert. 5 ml CMdum-Flüssigmedium (ohne Uracil) mit 2% Raffinose und 300 µM verschiedener Fettsäuren werden dann mit den Vorkulturen auf eine OD₆₀₀ von 0,05 angeimpft. Die Expression wird durch die Zugabe von 2% (w/v) Galactose induziert. Die Kulturen werden für weitere 96 h bei 20°C inkubiert.

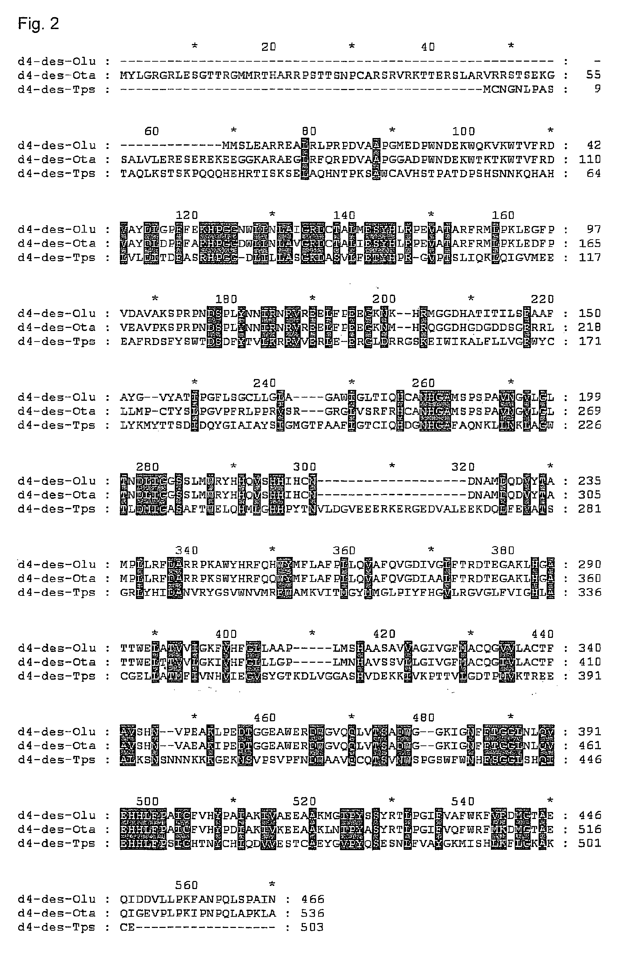

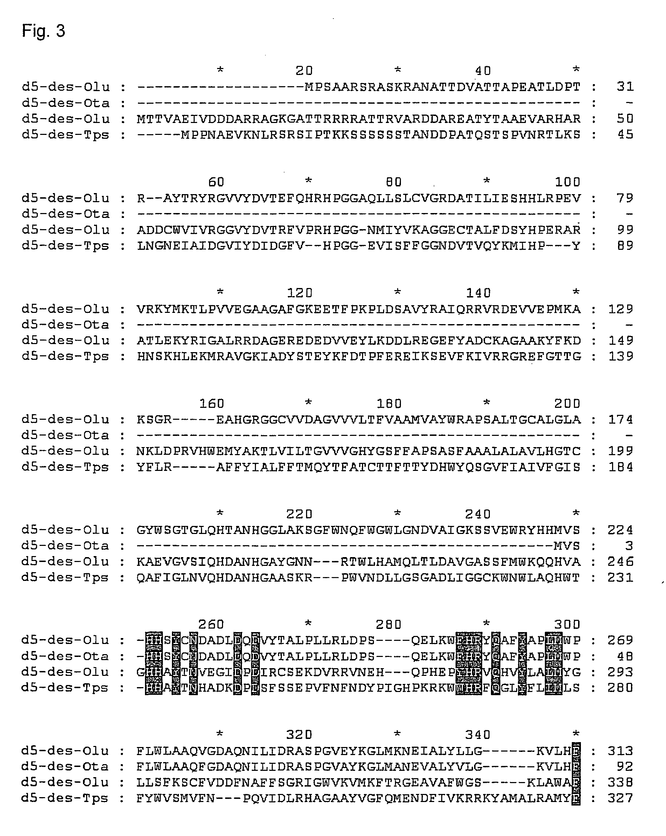

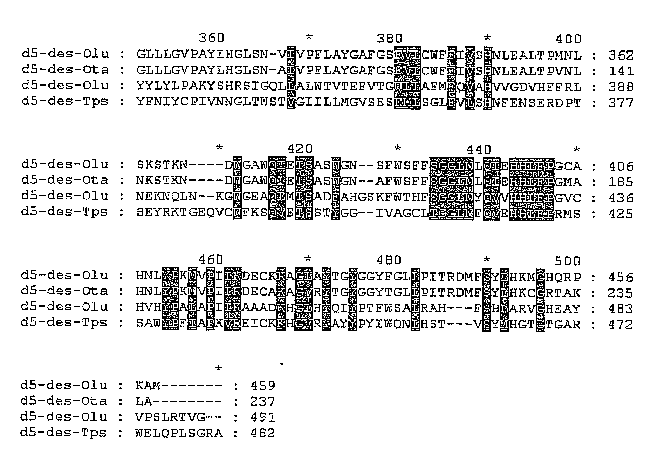

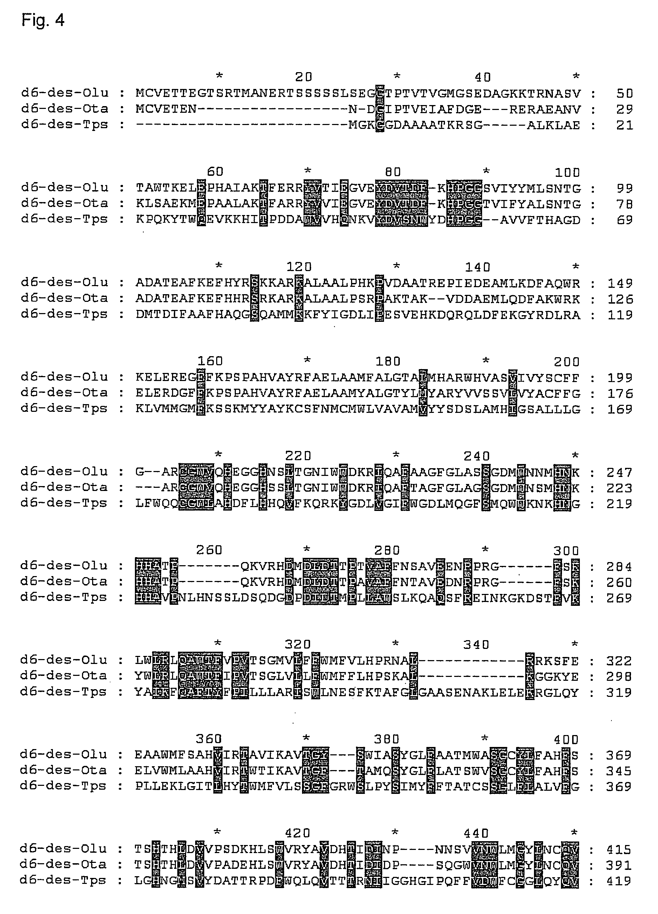

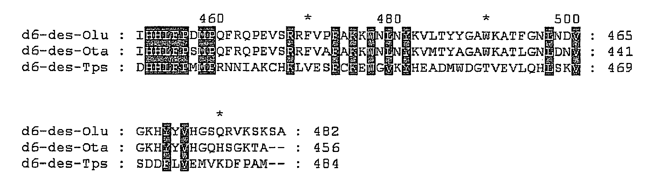

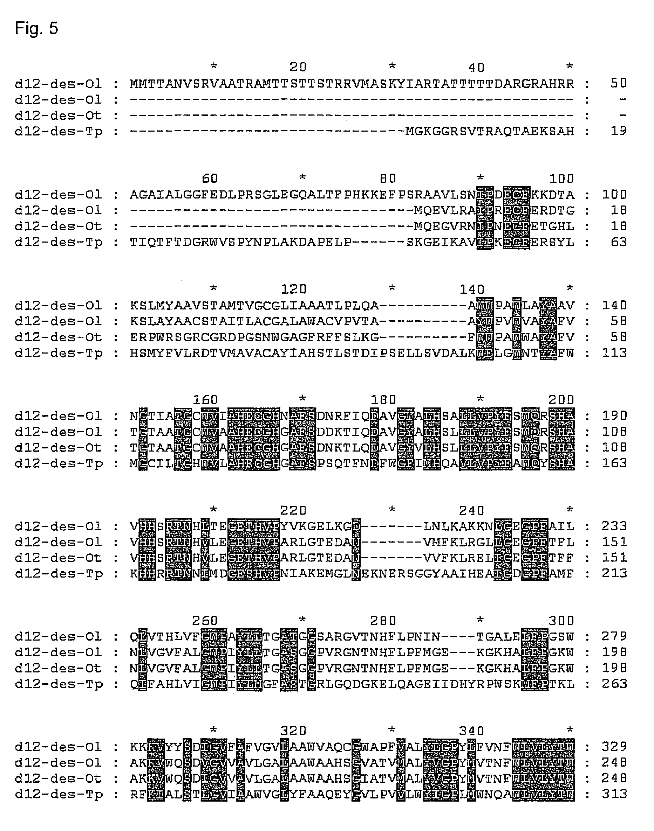

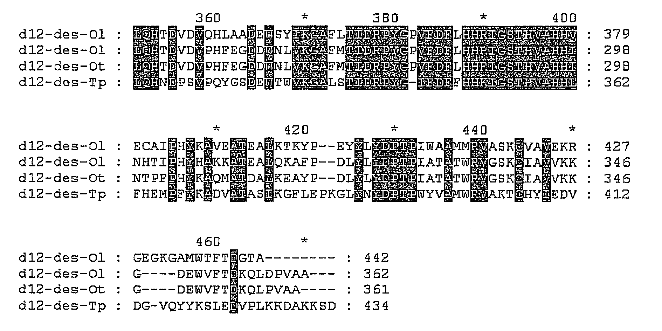

Die Figuren 2 bis 5 zeigen die Sequenzähnlichkeiten zu anderen Algen (*Ostreococcus tauri, Thalassiosira pseudonana*) für die verschiedenen Desaturase-Aminosäure Sequenzen im ClustalW Sequenzvergleich. Überraschenderweise unterscheiden sich die Sequenzen von *O. lucimarinus* in ihrer Aminosäuresequenz deutlich von den anderen Algen.

**Tabelle 6: Sequenzidentitäten einzelner Desaturasen**

| Gen-Name | SEQ ID | Organismus | Identität in % |
|---|---|---|---|
| D4Des(Ol) | 22 | O. lucimarinus | 100 |
| | | O. tauri | 69 |
| | | T. pseudonana | 20 |
| D5Des(Ol) | 26 | D5Des_2(Ol) | 23 |
| | | O. tauri_2 | 47 |
| | | T. pseudonana | 22 |
| D5Des_2(Ol) | 24 | D5Des(Ol) | 23 |
| | | O. tauri_2 | 14 |
| | | T. pseudonana | 19 |
| D6Des(Ol) | 30 | O. lucimarinus | 100 |
| | | O. tauri | 62 |
| | | T. pseudonana | 15 |
| D12Des(Ol) | 18 | D12Des_2(Ol) | 51 |
| | | O. tauri | 82 |
| | | T. pseudonana | 34 |
| D12Des_2(Ol) | 20 | D12Des(Ol) | 51 |
| | | O. tauri | 47 |
| | | T. pseudonana | 32 |

Die Gene werden wie folgt characterisiert:
Zur Expression der Desaturasen in Hefezellen werden aus den Hauptkulturen durch Zentrifugation (100 x g, 5 min, 20°C) geerntet und mit 100 mM NaHCO₃, pH 8,0 gewaschen, um restliches Medium und Fettsäuren zu entfernen. Die Hefe-Zellsedimente werden 4 h mit Chloroform/Methanol (1:1) extrahiert. Die resultierende organische Phase wird mit 0,45 % NaCl extrahiert, mit Na₂SO₄ getrocknet und unter Vakuum evaporiert. Der Lipidextrakt wird durch Dünnschicht-Chromatographie (Horizontal-Tank, Chloroform:Methanol:Essigsäure 65:35:8) weiter in die Lipidklassen Phosphatidylcholin (PC), Phosphatidiylinositol (PI), Phosphatidyserin (PS), Phosphatidylethanolamine (PE) und Neutral-Lipide (NL) aufgetrennt. Die verschiedenen aufgetrennten Spots auf der Dünnschichtplatte weden abgekratzt. Für die gaschromatografische Analyse wurde Fettsäuremethylester (FAMEs) durch saure Methanolyse hergestellt. Hierzu werden die Zellsedimente mit 2 ml 1 N methanolischer Schwefelsäure und 2% (v/v) Dimethoxypropan für 1 h bei 80°C inkubiert. Die Extraktion der FAMES erfolgte durch zweimalige Extraktion mit Petrolether (PE). Zur Entfernung nicht derivatisierter Fettsäuren wird die organische Phase je einmal mit 2 ml 100 mM NaHCO₃, pH 8,0 und 2 ml Aqua dest. gewaschen. Anschließend werden die PE-Phasen mit Na₂SO₄ getrocknet, unter Argon eingedampft und in 100 µl PE aufgenommen. Die Proben werden auf einer DB-23-Kapillarsäule (30 m, 0,25 mm, 0,25 µm, Agilent) in einem Hewlett-Packard 6850-Gaschromatographen mit Flammenionisationsdetektor getrennt. Die Bedingungen für die GLC-Analyse sind wie folgt: Die Ofentemperatur wird von 50°C bis 250°C mit einer Rate von 5°C/min und schließlich 10 min bei 250°C(halten) programmiert.

Die Identifikation der Signale erfolgt durch Vergleiche der Retentionszeiten mit entsprechenden Fettsäurestandards (Sigma). Die Methodik ist beschrieben zum Beispiel in Napier and Michaelson, 2001,Lipids. 36(8):761-766; Sayanova et al., 2001, Journal of Experimental Botany. 52(360):1581-1585, Sperling et al., 2001, Arch. Biochem. Biophys. 388(2):293-298 und Michaelson et al., 1998, FEBS Letters. 439(3):215-218.

### Aktivitäts- und Substratbestimmung von D5Des_2 (OI):

Zur Ermittlung der Aktivität und Substratspezifität der D5Des_2 (OI) SEQ ID 24 wurden verschiedene Fettsäuren gefüttert (Tab. 7). Die gefütterten Substrate sind dabei in großen Mengen in allen transgenen Hefen nachzuweisen. Die transgenen Hefen zeigen die Synthese neuer Fettsäuren, den Produkten der D5Des_2 (OI)-Reaktion. Dies bedeutet, dass das Gen D5Des_2 (OI) funktional exprimiert wurde.

**Tabelle 7: Fütterung/ Umsetzung verschiedener Fettsäuren durch D5Des(Ol_2).**

| Probenname/ gefütterte Fettsäure | Erwartete Umsetzung | Substrat | Produkt |
|---|---|---|---|
| pYES | Kontrolle | - | - |
| pYES-d5Des(Ol_GA) w/o FS | - | | |
| pYES-d5Des(Ol_GA) 20:3 | 20:3 -> 20:4ara | 11,1 | 0,9 |

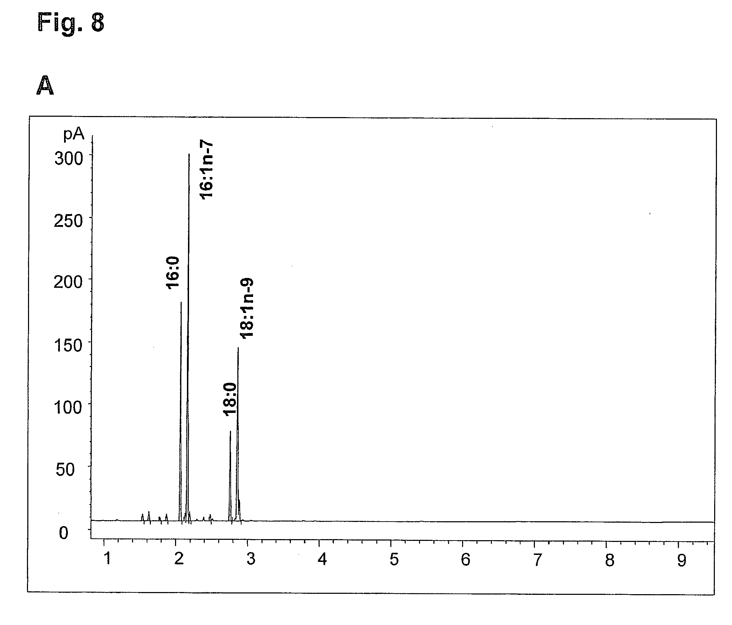

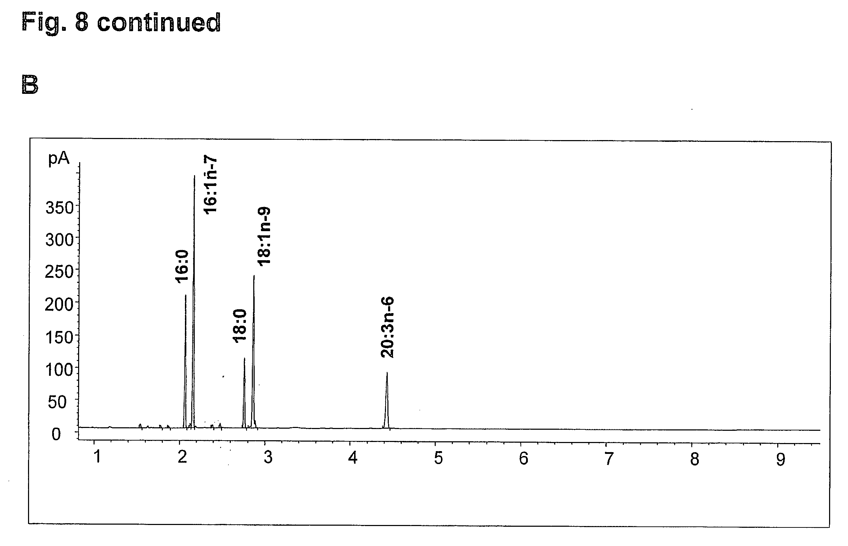

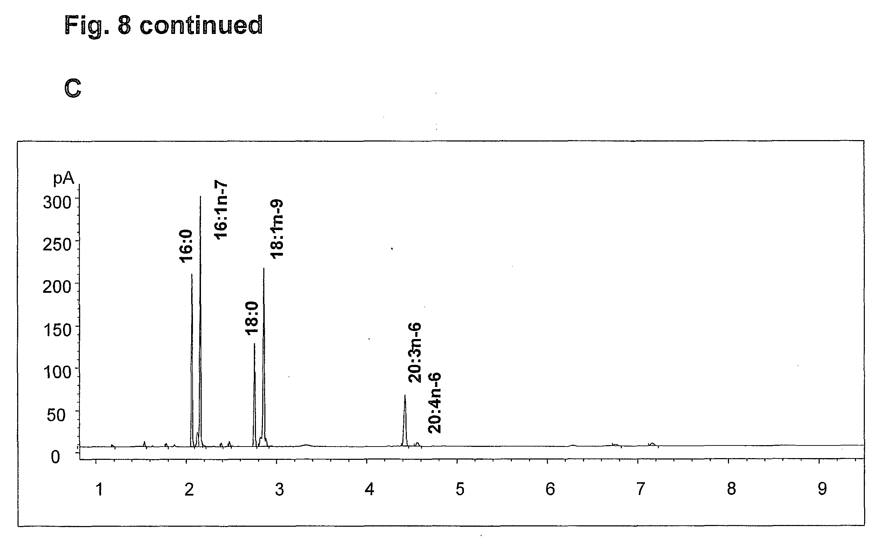

Figur 8 zeigt die Chromatogramme der einzelnen Experimente. Als Kontrolle wurde in Fig. 8A Hefen, transformiert mit pYES, ohne Zugabe von Fettsäuren analysiert. In Fig. 8B wurden den Hefen transformiert mit pYES die Fettsäure 3Δ8,11,14 zugegeben. Dabei können die gefütterte Fettsäure in großen Mengen detektiert werden. Dasselbe Experiment ist in Fig. 8C für Hefen transformiert mit dem Plasmid pYES-D5Des(Ol_2) durchgeführt worden. Im Unterschied zu Fig. 8B kann in den Hefen mit pYES-D5Des(Ol_2) eine zusätzliche Fettsäure detektiert werden, die auf die Aktivität der D5Des(Ol_2) zurückgehen muss. Anhand der Aktivität kann D5Des(Ol_2) als Δ5-Desaturase charakterisiert werden.

### Zusammenfassung der Ergebnisse zu D5Des_2 (OI):

In den Hefefütterungsexperimenten konnte gezeigt werden, dass das klonierte Gen DSDes_2 (OI) SEQ ID 24 funktional exprimiert wurde und Desaturase-Aktivität besitzt. Anhand der gefütterten Fettsäure kann DSDes_2 (OI) als Δ5-Desaturase charakterisiert werden, d.h. C20-Fettsäuren mit einer Δ8-Doppelbindung werden spezifisch an der Δ5-Position dehydriert.

### Aktivitäts- und Substratbestimmung von D12Des(OI):

Zur Ermittlung der Aktivität und Substratspezifität der D12Des(OI) SEQ ID 18 wurden verschiedene Fettsäuren gefüttert (Tab. 8). Die gefütterten Substrate sind dabei in großen Mengen in allen transgenen Hefen nachzuweisen. Die transgenen Hefen zeigen die Synthese neuer Fettsäuren, den Produkten der D12Des(OI)-Reaktion. Dies bedeutet, dass das Gen D12Des(OI) funktional exprimiert wurde.

**Tabelle 8: Fütterung/ Umsetzung verschiedener Fettsäuren durch D12Des(Ol).**

| Probenname/ gefütterte Fettsäure | Erwartete Umsetzung | Substrat | Produkt |
|---|---|---|---|
| pYES | Kontrolle | - | - |
| pYES-D12Des(Ol) | 18:1 -> 18:2 | 24,9 | 1,1 |
| pYES-D12Des(Ol) | 18:1 -> 18:2 | 24,1 | 1,0 |

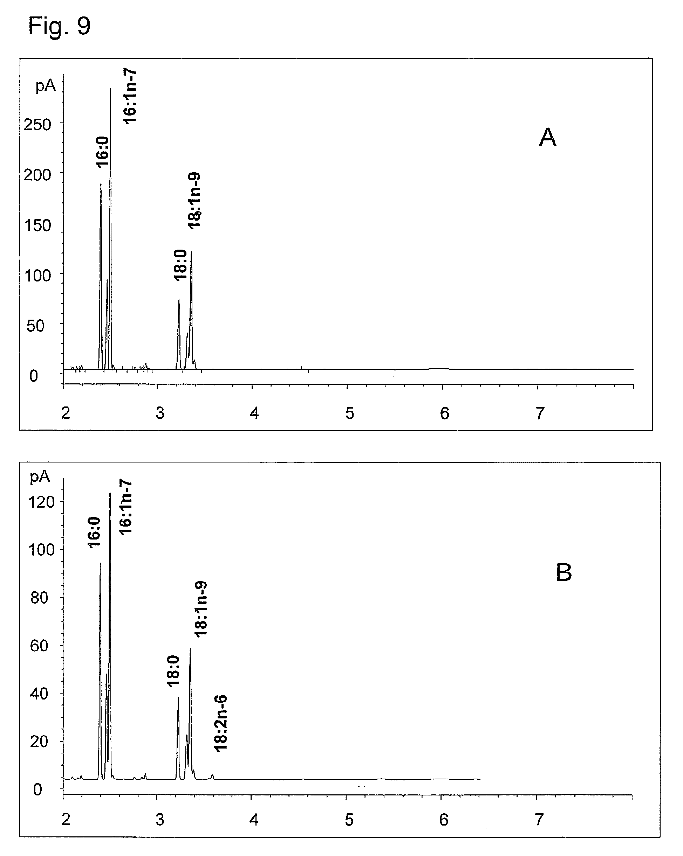

Figur 9 zeigt die Chromatogramme der einzelnen Experimente. Als Kontrolle wurde in Fig. 9A Hefen, transformiert mit pYES, ohne Zugabe von Fettsäuren analysiert. In Fig. 9B wurden den Hefen transformiert mit pYES-D12Des(OI) analysiert. Im Unterschied zu Fig. 9A kann in den Hefen mit pYES-D12Des(OI) eine zusätzliche Fettsäure detektiert werden, die auf die Aktivität der D12Des(OI) zurückgehen muss. Anhand der Aktivität kann D12Des(OI) als Δ12-Desaturase charakterisiert werden.

### Zusammenfassung der Ergebnisse zu D12Des(OI):

In den Hefefütterungsexperimenten konnte gezeigt werden, dass das klonierte Gen D12Des(OI) SEQ ID 18 funktional exprimiert wurde und Desaturase-Aktivität besitzt. Anhand des Fettsäurespektrums kann D12Des(OI) als Δ12-Desaturase charakterisiert werden, d.h. C18-Fettsäuren mit einer Δ9-Doppelbindung werden spezifisch an der Δ12-Position dehydriert.

### Aktivitäts- und Substratbestimmung von D5Des(OI):

Zur Ermittlung der Aktivität und Substratspezifität der D5Des(Oi) SEQ ID 26 wurden verschiedene Fettsäuren gefüttert (Tab. 9). Die gefütterten Substrate sind dabei in großen Mengen in allen transgenen Hefen nachzuweisen. Die transgenen Hefen zeigen die Synthese neuer Fettsäuren, den Produkten der D5Des(OI)-Reaktion. Dies bedeutet, dass das Gen D5Des(OI) funktional exprimiert wurde.

**Tabelle 9: Umsetzung verschiedener Fettsäuren durch D5Des(Ol)**

| Sample | Erwartete Umsetzung | Substrat | Produkt | Konversionsrate [%] |
|---|---|---|---|---|
| d5Des(Ol_febit) | 20:3n-6 -> 20:4ara | 29,4 | 12,4 | 29,6 |
| d5Des(Ol_febit) | 20:3n-6 -> 20:4ara | 19,8 | 10,3 | 34,3 |
| d5Des(Ol_febit) | 20:4n-3 -> 20:5 | nd | 1,2 | >50% |

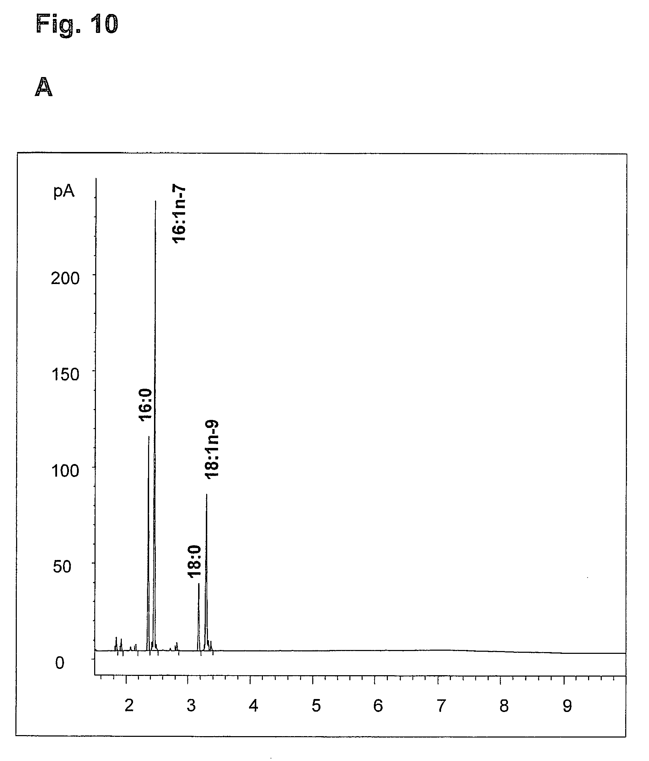

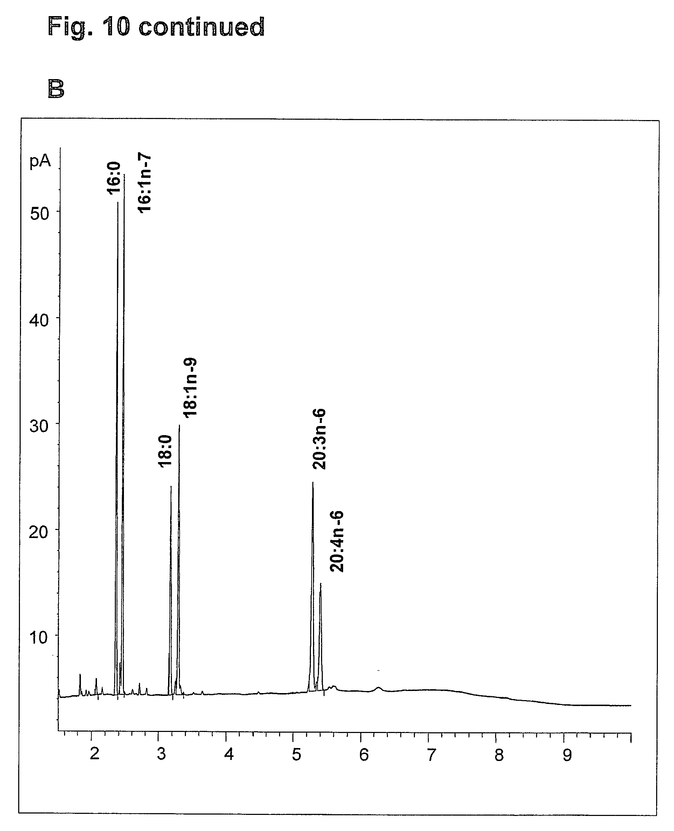

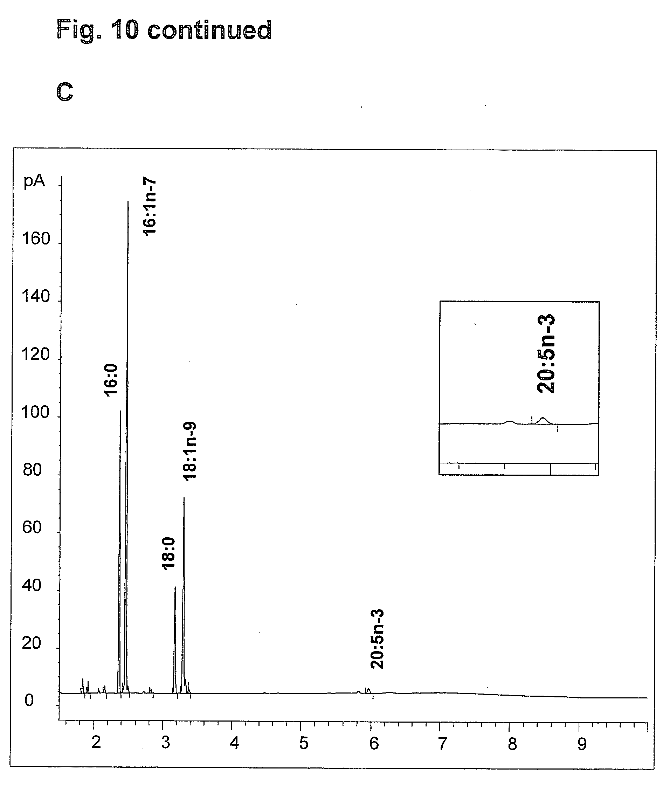

Figur 10 zeigt die gaschromatographische Analyse von Hefefütterungsexperimenten. Nach Expression von pYes-d5Des(OI_1) in Hefe Stamm InvSc ohne Zugabe von Fettsäuren (Fig. 10A) wurde keine Umsetzung der vorhandenen Fettsäuren festgestellt. pYes-d5Des(OI_1) Expression in Hefe Stamm InvSc nach Zugabe der Fettsäure 20:3n-6 (B) führt zur spezifischen Umsetzung von 20:3n-6 zu 20:4n-6 (Arachidonsäure) und Expression von pYes-d5Des(OI_1) in Hefe Stamm InvSc nach Zugabe der Fettsäure 20:4n-3 (C) zur spezifische Umsetzung von 20:4n-3 zu 20:5n-3 (Eicosapentaensäure). Die spezifische Inkorporation von d5-Doppelbindungen in die gefütterten Fettsäuren zeigt die d5-Desaturase Aktivität von d5Des(Ol).

### Zusammenfassung der Ergebnisse zu D5Des(OI):

In den Hefefütterungsexperimenten konnte gezeigt werden, dass das klonierte Gen D5Des(OI) SEQ ID 26 funktional exprimiert wurde und Desaturase-Aktivität besitzt. Anhand des Fettsäurespektrums kann D5Des(OI) als Δ5-Desaturase charakterisiert werden, d.h. C20-Fettsäuren mit einer Δ8-Doppelbindung werden spezifisch an der Δ5-Position dehydriert.

## Patentansprüche

1. Polynucleotid umfassend eine Nukleinsäuresequenz, die ausgewählt ist aus der Gruppe bestehend aus:
(a) Nukleinsäuresequenz wie in SEQ ID No. 9 gezeigt;
(b) Nukleinsäuresequenz, die ein Polypeptid kodiert, das eine Aminosäuresequenz wie in SEQ ID No. 10 gezeigt aufweist;
(c) Nukleinsäuresequenz, die ein Polypeptid kodiert, das über die gesamte Länge mindestens 85% identisch zu einem Polypeptid ist, das von der Nucleinsäuresequenzen aus (a) oder (b) kodiert wird, wobei das Polypeptid Desaturase Aktivität aufweist; und
(d) Nukleinsäuresequenz für ein Fragment mit mindestens 500 aufeinanderfolgenden Nucleotiden einer Nukleinsäure aus (a), (b) oder (c), wobei das Fragment ein Polypeptid mit einer Desaturase Aktivität kodiert.

2. Polynucleotid nach Anspruch 1, wobei das Polynucleotid aus RNA oder DNA besteht.

3. Vektor umfassend das Polynucleotid nach Anspruch 1 oder 2.

4. Vektor nach Anspruch 3, wobei der Vektor ein Expressionsvektor ist.

5. Vektor nach Anspruch 3 oder 4, wobei der Vektor mindestens ein weiteres Polynucleotid umfasst, das ein weiteres Enzym kodiert, das in die Biosynthese von Lipiden oder Fettsäuren eingebunden ist.

6. Wirtszelle umfassend das heterologe Polynucleotid nach Anspruch 1 oder 2 oder den Vektor nach einem der Ansprüche 3 bis 5.

7. Wirtszelle nach Anspruch 6, wobei die Wirtszelle zusätzlich mindestens ein weiteres Enzym umfasst, das in die Biosynthese von Lipiden oder Fettsäuren eingebunden ist.

8. Vektor nach Anspruch 5 oder Wirtszelle nach Anspruch 7 wobei das Enzym ausgewählt ist aus der Gruppe bestehend aus: Acyl-CoA-Dehydrogenase(n), Acyl-ACP[= acyl carrier protein]-Desaturase(n), Acyl-ACP-Thioesterase(n), Fettsäure-Acyl-Transferase(n), Acyl-CoA:Lysophospholipid-Acyltransferase(n), Fettsäure-Synthase(n), Fettsäure-Hydroxylase(n), Acetyl-Coenzym A-Carboxylase(n), Acyl-Coenzym A-Oxidase(n), Fettsäure-Desaturase(n), Fettsäure-Acetylenase(n), Lipoxygenase(n), Triacylglycerol-Lipase(n), Allenoxid-Synthase(n), Hydroperoxid-Lyase(n), Fettsäure-Elongase(n), Δ4-Desaturase(n), Δ5-Desaturase(n), Δ6-Desaturase(n), Δ8-Desaturase(n), Δ9-Desaturase(n), Δ12-Desaturase(n), Δ5-Elongase(n), Δ6-Elongase(n) und Δ9-Elongase(n).

9. Verfahren zur Herstellung eines Polypeptids mit Desaturase Aktivität umfassend die Schritte:
(a) Exprimieren eines Polynucleotids nach Anspruch 1 oder 2 in einer Wirtszelle; und
(b) Gewinnen des Polypeptids, das von dem Polynucleotid kodiert wird, aus der Wirtszelle.

10. Polypeptid, das von dem Polynucleotid gemäß Anspruch 1 oder 2 kodiert wird oder das erhätlich durch das Verfahren nach Anspruch 9 ist.

11. Antikörper, der das Polypeptid nach Anspruch 10 spezifisch erkennt.

12. Transgener, nicht-humaner Organismus umfassend das Polynucleotid nach Anspruch 1 oder 2, den Vektor nach einem der Ansprüche 3 bis 5 oder die Wirtszelle nach Anspruch 8.

13. Transgener, nicht-humaner Organismus nach Anspruch 12, wobei der Organismus ein Tier, eine Pflanze oder ein multizellulärere Mikroorganismus ist.

14. Verfahren zur Herstellung eines Stoffes, der die Struktur aufweist, die in der folgenden allgemeinen Formel I gezeigt wird 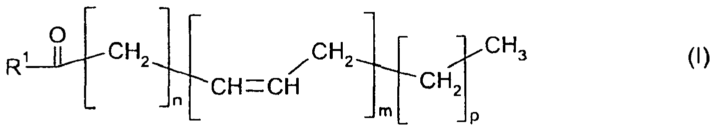 wobei die Variablen und Substiuenten die folgenden sind
R¹ = Hydroxyl, Coenzym A (Thioester), Lysophosphatidylcholin, Lysophosphatidylethanolamin, Lysophosphatidylglycerol, Lysodiphosphatidylglycerol, Lysophosphatidylserin, Lysophosphatidylinositol, Sphingo- Base oder ein Radikal der Formel II 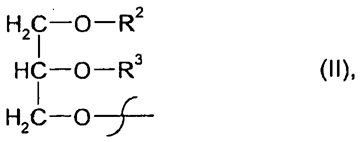
R² = Wasserstoff, Llysophosphatidylcholin, Lysophosphatidylethanolamin, Lysophosphatidylglycerol, Llysodiphosphatidylglycerol, Lysophosphatidylserin, Lysophosphatidylinositol oder gesättigtes oder ungesättigtes C₂-C₂₄-Alkylcarbonyl,
R³ = Wasserstoff, gesättigtes oder ungesättigtes C₂-C₂₄-Alkylcarbonyl, oder R² und R³ sind unabhängig voneinander ein Radikal der Formel la: 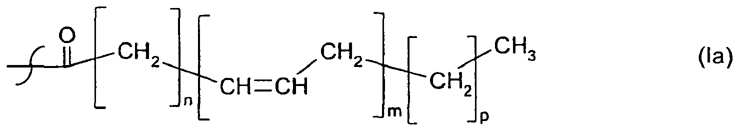
n = 2, 3, 4, 5, 6, 7 or 9, m = 2, 3, 4, 5 oder 6 und p = 0 oder 3;
und
wobei das Verfahren das Kultivieren von (i) einer Wirtszelle nach einem der Ansprüche 3 bis 5 oder (ii) eines transgenen, nicht-humanen Organismus nach Anspruch 12 oder 13 unter Bedingungen umfasst, die die Biosynthese des Stoffes erlauben.

15. Verfahren zur Herstellung einer Öl-, Lipid- oder Fettsäurezusammensetzung umfassend die Schritte des Verfahrens nach Anspruch 14 und den weiteren Schritt des Formulierens des Stoffes als Öl-, Lipid- oder Fettsäurezusammensetzung.

16. Verfahren nach Anspruch 15, wobei die Öl-, Lipid- oder Fettsäurezusammensetzung weiter formuliert wird zu einem Arzneimittel, zu Kosmetik, zu einem Nahrungsmittel, zu einem Futtermittel, vorzugsweise Fischfutter, oder zu einem Nahrungsergänzungsmittel.

17. Verwendung des Polynucleotids nach Anspruch 1 oder 2, des Vektors nach einem der Ansprüche 3 bis 5, der Wirtszelle nach Anspruch 6 oder 7, des Vektors oder der Wirtszelle nach Anspruch 8, des Polypeptids nach Anspruch 10 oder des transgenen, nicht-humanen Organismus nach Anspruch 12 oder 13 für die Herstellung einer Öl-, Lipid- oder Fettsäurezusammensetzung.

18. Verwendung nach Anspruch 17, wobei die Öl-, Lipid- oder Fettsäurezusammensetzung einzusetzen ist als Arzneimittel, Kosmetik, Nahrungsmittel, Futtermittel, vorzugsweise Fischfutter, oder Nahrungsergänzungsmittel.

## Claims

1. A polynucleotide, comprising a nucleic acid sequence which is selected from the group consisting of:
(a) nucleic acid sequence as shown in SEQ ID No. 9;
(b) nucleic acid sequence which codes for a polypeptide which has an amino acid sequence as shown in SEQ ID No. 10;
(c) nucleic acid sequence which codes for a polypeptide which is identical to at least 85% over the entire length to a polypeptide which is encoded by the nucleic acid sequences of (a) or (b), where the polypeptide has desaturase activity; and
(d) nucleic acid sequence for a fragment with at least 500 contiguous nucleotides of a nucleic acid from (a), (b) or (c), where the fragment codes for a polypeptide with a desaturase activity.

2. The polynucleotide according to claim 1, wherein the polynucleotide is composed of RNA or DNA.

3. A vector comprising the polynucleotide according to claim 1 or 2.

4. The vector according to claim 3, wherein the vector is an expression vector.

5. The vector according to claim 3 or 4, wherein the vector comprises at least one further polynucleotide which codes for a further enzyme which is involved in the biosynthesis of lipids or fatty acids.

6. A host cell, comprising the heterologous polynucleotide according to claim 1 or 2 or the vector according to any of claims 3 to 5.

7. The host cell according to claim 6, wherein the host cell comprises additionally at least one further enzyme which is involved in the biosynthesis of lipids or fatty acids.

8. The vector according to claim 5 or the host cell according to claim 7, wherein the enzyme is selected from the group consisting of: acyl-CoA dehydrogenase(s), acyl-ACP [= acyl carrier protein] desaturase(s), acyl-ACP thioesterase(s), fatty acid acyl transferase(s), acyl-CoA:lysophospholipid acyltransferase(s), fatty acid synthase(s), fatty acid hydroxylase(s), acetyl-coenzyme A carboxylase(s), acyl-coenzyme A oxidase(s), fatty acid desaturase(s), fatty acid acetylenase(s), lipoxygenase(s), triacylglycerol lipase(s), allene oxide synthase(s), hydroperoxide lyase(s), fatty acid elongase(s), Δ4 desaturase(s), Δ5 desaturase(s), Δ6 desaturase(s), Δ8 desaturase(s), Δ9 desaturase(s), Δ12 desaturase(s), Δ5 elongase(s), Δ6 elongase (s) and Δ9 elongase (s).

9. A process for the production of a polypeptide with desaturase activity, comprising the steps:
(a) expressing a polynucleotide according to claim 1 or 2 in a host cell; and
(b) obtaining, from the host cell, the polypeptide which is encoded by the polynucleotide.

10. A polypeptide which is encoded by the polynucleotide according to claim 1 or 2 or which is obtainable by the process according to claim 9.

11. An antibody which specifically recognizes the polypeptide according to claim 10.

12. A transgenic nonhuman organism, comprising the polynucleotide according to claim 1 or 2, the vector according to any of claims 3 to 5 or the host cell according to claim 8.

13. The transgenic nonhuman organism according to claim 12, wherein the organism is an animal, a plant or a multicellular microorganism.

14. A process for the production of a substance which has the structure shown in the following general formula I 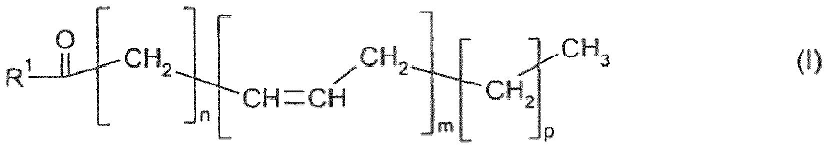 where the variables and substituents are the following:
R¹ = hydroxyl, coenzyme A (thioester), lysophosphatidylcholine, lysophosphatidylethanolamine, lysophosphatidylglycerol, lysodiphosphatidylglycerol, lysophosphatidylserine, lysophosphatidylinositol, sphingo base or a radical of the formula II 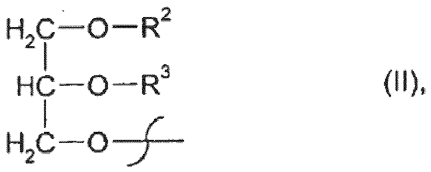
R² = hydrogen, lysophosphatidylcholine, lysophosphatidylethanolamine, lysophosphatidylglycerol, lysodiphosphatidylglycerol, lysophosphatidylserine, lysophosphatidylinositol or saturated or unsaturated C₂-C₂₄-alkylcarbonyl,
R³ = hydrogen, saturated or unsaturated C₂-C₂₄-alkyl-carbonyl, or R² and R³ independently of one another are a radical of the formula Ia: 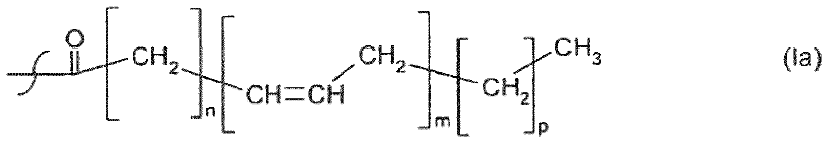
n = 2, 3, 4, 5, 6, 7 or 9, m = 2, 3, 4, 5 or 6 and p = 0 or 3;
and
wherein the process comprises the culturing of (i) a host cell according to any of claims 3 to 5 or (ii) a transgenic nonhuman organism according to claim 12 or 13 under conditions which permit the biosynthesis of the substance.

15. A process for producing an oil, lipid or fatty acid composition, comprising the steps of the process according to claim 14 and the further step of formulating the substance as an oil, lipid or fatty acid composition.

16. The process according to claim 15, wherein the oil, lipid or fatty acid composition is formulated further to give a pharmaceutical, cosmetic, foodstuff, feedstuff, preferably fish feed, or a food supplement.

17. The use of the polynucleotide according to claim 1 or 2, of the vector according to any of claims 3 to 5, of the host cell according to claim 6 or 7, of the vector or the host cell according to claim 8, of the polypeptide according to claim 10 or of the transgenic nonhuman organism according to claim 12 or 13 for the preparation of an oil, lipid or fatty acid composition.

18. The use according to claim 17, wherein the oil, lipid or fatty acid composition is to be employed as a pharmaceutical, cosmetic, foodstuff, feedstuff, preferably fish feed, or food supplement.

## Revendications

1. Polynucléotide comprenant une séquence d'acide nucléique qui est choisie dans le groupe consistant en :
(a) la séquence d'acide nucléique telle que présentée dans SEQ ID NO:9 ;
(b) la séquence d'acide nucléique qui code pour un polypeptide qui comprend une séquence d'acides aminés telle que présentée dans SEQ ID NO:10;
(c) la séquence d'acide nucléique qui code pour un polypeptide qui sur la totalité de sa longueur a une identité d'au moins 85 % avec un polypeptide qui est codé par les séquences d'acides nucléiques de (a) ou (b), le polypeptide présentant une activité de désaturase ; et
(d) la séquence d'acide nucléique d'un fragment ayant au moins 500 nucléotides successifs d'un acide nucléique de (a), (b) ou (c), le fragment codant pour un polypeptide ayant une activité de désaturase.

2. Polynucléotide selon la revendication 1, le polynucléotide étant constitué d'ARN ou d'ADN.

3. Vecteur comprenant le polynucléotide selon la revendication 1 ou 2.

4. Vecteur selon la revendication 3, le vecteur étant un vecteur d'expression.

5. Vecteur selon la revendication 3 ou 4, le vecteur comprenant au moins un polynucléotide supplémentaire, qui code pour une enzyme supplémentaire, qui participe à la biosynthèse de lipides ou d'acides gras.

6. Cellule hôte comprenant le polynucléotide hétérologue selon la revendication 1 ou 2 ou le vecteur selon l'une des revendications 3 à 5.

7. Cellule hôte selon la revendication 6, la cellule hôte comprenant en outre au moins une enzyme supplémentaire, qui participe à la biosynthèse de lipides ou d'acides gras.

8. Vecteur selon la revendication 5 ou cellule hôte selon la revendication 7, l'enzyme étant choisie dans le groupe consistant en la ou les acyl-CoA-déshydrogénases, la ou les acyl-ACP[= protéine porteuse d'acyle]-désaturases, la ou les acyl-ACP-thioestérases, la ou les acides gras-acyl-transférases, la ou les acyl-CoA:lysophospholipide-acyltransférases, la ou les acide gras-synthases, la ou les acide gras-hydroxylases, la ou les acétyl-coenzyme A-carboxylases, la ou les acyl-coenzyme A-oxydases, la ou les acide gras-désaturases, la ou les acide gras-acétylénases, la ou les lipoxygénases, la ou les triacylglycérol-lipases, la ou les oxyde d'allène-synthases, la ou les hydroperoxyde-lyases, la ou les acide gras-élongases, la ou les Δ4-désaturases, la ou les Δ5-désaturases, la ou les Δ6-désaturases, la ou les Δ8-désaturases, la ou les Δ9-désaturases, la ou les Δ12-désaturases, la ou les Δ5-élongases, la ou les Δ6-élongases et la ou les Δ9-élongases.

9. Procédé de fabrication d'un polypeptide ayant une activité de désaturase, comprenant les étapes :
(a) expression d'un polynucléotide selon la revendication 1 ou 2 dans une cellule hôte ; et
(b) obtention du polypeptide qui est codé par le polynucléotide, à partir de la cellule hôte.

10. Polypeptide qui est codé par le polynucléotide selon la revendication 1 ou 2 ou qui peut être obtenu par le procédé selon la revendication 9.

11. Anticorps qui reconnaît spécifiquement le polypeptide selon la revendication 10.

12. Organisme transgénique non humain comprenant le polynucléotide selon la revendication 1 ou 2, le vecteur selon l'une des revendications 3 à 5 ou la cellule hôte selon la revendication 8.

13. Organisme transgénique non humain selon la revendication 12, l'organisme étant un animal, une plante ou un microorganisme multicellulaire.

14. Procédé de préparation d'une substance ayant la structure qui est présentée dans la formule générale I ci-après : 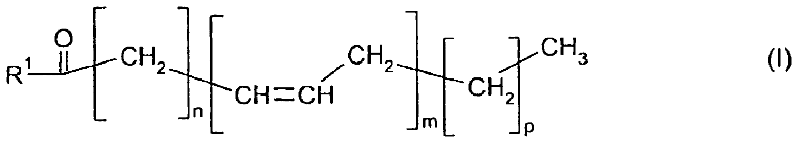 les variables et substituants étant les suivants :
R¹ = hydroxyle, coenzyme A (thioester), lysophosphatidylcholines, lysophosphatidyléthanolamine, lysophosphatidylglycérol, lysodiphosphatidylglycérol, lysophosphatidylsérine, lysophosphatidylinositol, sphingo-base ou un radical de formule II 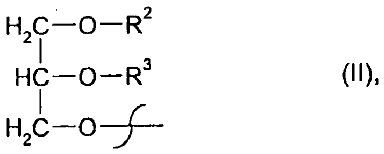
R² = hydrogène, lysophosphatidylcholines, lysophosphatidyléthanolamine, lysophosphatidylglycérol, lysodiphosphatidylglycérol, lysophosphatidylsérine, lysophosphatidylinositol, ou alkylcarbonyle en C₂-C₂₄ saturé ou insaturé,
R³ = hydrogène, alkylcarbonyle en C₂-C₂₄ saturé ou insaturé, ou R² et R³ représentent chacun indépendamment de l'autre un radical de formule Ia : 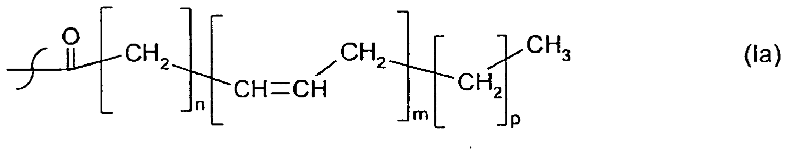
n = 2, 3, 4, 5, 6, 7, ou 9, m = 2, 3, 4, 5 ou 6, et p = 0 ou 3 ;
et
le procédé comprenant la culture (i) d'une cellule hôte selon l'une des revendications 3 à 5 ou (ii) d'un organisme transgénique non humain selon la revendication 12 ou 13, dans des conditions permettant la biosynthèse de la substance.

15. Procédé de préparation d'une composition d'huiles, de lipides ou d'acides gras, comprenant les étapes du procédé selon la revendication 14 et l'étape supplémentaire de formulation de la substance en tant que composition d'huiles, de lipides ou d'acides gras.

16. Procédé selon la revendication 15, dans lequel la composition d'huiles, de lipides ou d'acides gras est en outre formulé pour former un médicament, un produit cosmétique, un produit pour l'alimentation humaine, un produit pour l'alimentation animale, de préférence un aliment pour poissons, ou un complément alimentaire.

17. Utilisation du polynucléotide selon la revendication 1 ou 2, du vecteur selon l'une des revendications 3 à 5, de la cellule hôte selon la revendication 6 ou 7, du vecteur ou de la cellule hôte selon la revendication 8, du polypeptide selon la revendication 10 ou de l'organisme transgénique non humain selon la revendication 12 ou 13 pour préparer une composition d'huiles, de lipides ou d'acides gras.

18. Utilisation selon la revendication 17, pour laquelle la composition d'huiles, de lipides ou d'acides gras doit être utilisée en tant que médicament, produit cosmétique, produit pour l'alimentation humaine, produit pour l'alimentation animale, de préférence aliment pour poissons, ou complément alimentaire.
